(19) Europäisches Patentamt
European Patent Office
Office européen des brevets

(11) EP 4 190 791 A1

(12) **EUROPEAN PATENT APPLICATION**
published in accordance with Art. 153(4) EPC

(43) Date of publication:
**07.06.2023 Bulletin 2023/23**

(21) Application number: **21850149.2**

(22) Date of filing: **30.07.2021**

(51) International Patent Classification (IPC):
***C07D 519/00*** *(2006.01)* ***C09K 11/06*** *(2006.01)*
***H01L 51/50*** *(2006.01)*

(52) Cooperative Patent Classification (CPC):
**C07D 209/94; C07D 519/00; C09K 11/06; H10K 50/00**

(86) International application number:
**PCT/JP2021/028342**

(87) International publication number:
**WO 2022/025248 (03.02.2022 Gazette 2022/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.07.2020 JP 2020130053**
**26.04.2021 JP 2021074499**

(71) Applicant: **Kyulux, Inc.**
**Fukuoka-shi, Fukuoka 819-0388 (JP)**

(72) Inventors:
- **YAMASHITA Masataka**
**Fukuoka-shi, Fukuoka 819-0388 (JP)**
- **CHENG Shuo-Hsien**
**Fukuoka-shi, Fukuoka 819-0388 (JP)**
- **YANG YuSeok**
**Fukuoka-shi, Fukuoka 819-0388 (JP)**

(74) Representative: **Zimmermann & Partner Patentanwälte mbB**
**Postfach 330 920**
**80069 München (DE)**

# (54) COMPOUND, LIGHT-EMITTING MATERIAL, AND LIGHT-EMITTING ELEMENT

(57) A compound represented by the following general formula is used as a light emitting material. Any one of $R^1$, $R^2$ and $R^4$ is a hydrogen atom or a deuterium atom, the remaining ones are donor groups but at least one is a carbazol-9-yl group condensed with a benzofuran ring, a benzothiophene ring, an indole ring, an indene ring or a silaindene ring.

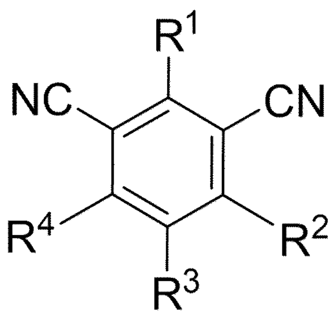

EP 4 190 791 A1

## Description

### Technical Field

The present invention relates to a compound useful as a light emitting material, and a light emitting device using the compound.

### Background Art

Studies for enhancing the light emission efficiency of light emitting devices such as organic electroluminescent devices (organic EL devices) are being made actively. In particular, various kinds of efforts have been made for increasing light emission efficiency by newly developing and combining an electron transporting material and a hole transporting material to constitute an organic electroluminescent device. Among them, there are seen some reports relating to an organic electroluminescent device that utilizes a delayed fluorescent material.

A delayed fluorescent material is a material which, in an excited state, after having undergone reverse intersystem crossing from an excited triplet state to an excited singlet state, emits fluorescence when returning back from the excited singlet state to a ground state thereof. Fluorescence through the route is observed later than fluorescence from the excited singlet state directly occurring from the ground state (ordinary fluorescence), and is therefore referred to as delayed fluorescence. Here, for example, in the case where a light emitting compound is excited through carrier injection thereinto, the occurring probability of the excited singlet state to the excited triplet state is statistically 25%/75%, and therefore improvement of light emission efficiency by the fluorescence alone from the directly occurring excited singlet state is limited. On the other hand, in a delayed fluorescent material, not only the excited singlet state thereof but also the excited triplet state can be utilized for fluorescent emission through the route via the above-mentioned reverse intersystem crossing, and therefore as compared with an ordinary fluorescent material, a delayed fluorescent material can realize a higher emission efficiency.

Since such principles have been clarified, various delayed fluorescent materials have become discovered by various studies. However, every material capable of emitting delayed fluorescence is not always immediately useful as a light emitting material. Some delayed fluorescent materials are relatively less likely to undergo reverse intersystem crossing, and some delayed fluorescent materials have a long lifetime. In a high current density region, excitons may accumulate to lower emission efficiency, or in continuous long-time driving, some materials may rapidly worsen. Consequently, in fact, there are very many delayed fluorescent materials with room for improvement in point of practicability. Therefore, it is pointed out that benzonitrile compounds that are known as delayed fluorescent materials also have various problems. For example, 2CzPN having the following structure is a material that emits delayed fluorescence, but has a problem in that the emission efficiency is not high and, in addition, the emission efficiency greatly reduces in a high-current density region (see NPL 1).

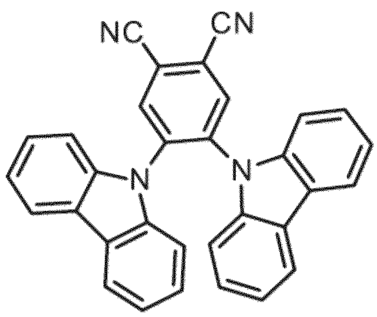

### Citation List

### Non-Patent Literature

NPL 1: Organic Electronics 14 (2013) 2721-2726

### Summary of Invention

### Technical Problem

Although it has been pointed out that such a problem has been encountered, it can be said that the relationship

between the chemical structure and the characteristics of the delayed fluorescent material has been sufficiently elucidated. Consequently, at present, it is difficult to generalize the chemical structure of a compound useful as a light emitting material, and there are many unclear points.

Given the situation, the present inventors have made repeated studies for the purpose of providing a compound more useful as a light emitting material for light emitting devices. With that, the inventors have further made assiduous studies in order to derive and generalize a general formula of a compound more useful as a light emitting material.

Solution to Problem

As a result of further promoting assiduous studies for attaining the above-mentioned object, the present inventors have found that, among isophthalonitrile derivatives, compounds having a structure that satisfies a specific requirement are useful as a light emitting material. The present invention has been proposed on the basis of these findings, and specifically has the following constitution.

[1] A compound represented by the following general formula (1):

General Formula (1)

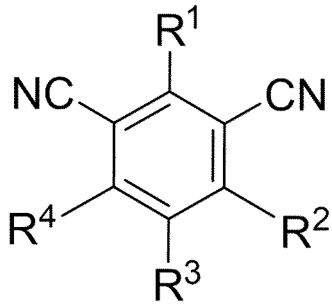

In the general formula (1):

any one of $R^1$, $R^2$ and $R^4$ is a hydrogen atom or a deuterium atom,
the remaining two and $R^3$ each independently represent a donor group, of which at least one is a benzofuran-condensed carbazol-9-yl group having a skeleton of a carbazole ring condensed with a benzofuran ring at the 2,3-positions, a benzothiophene-condensed carbazol-9-yl group having a skeleton of a carbazole ring condensed with a benzothiophene ring at the 2,3-positions, an indole-condensed carbazol-9-yl group having a skeleton of a carbazole ring condensed with an indole ring at the 2,3-positions, an indene-condensed carbazol-9-yl group having a skeleton of a carbazole ring condensed with an indene ring at the 2,3-positions, or a silaindene-condensed carbazol-9-yl group having a skeleton of a carbazole ring condensed with a silaindene ring at the 2,3-positions.

[2] The compound according to [1], wherein the remaining two and $R^3$ all contain a carbazole ring.
[3] The compound according to [1] or [2], wherein $R^3$ is the benzofuran-condensed carbazol-9-yl group, the benzothiophene-condensed carbazol-9-yl group, the indole-condensed carbazol-9-yl group, the indene-condensed carbazol-9-yl group, or the silaindene-condensed carbazol-9-yl group.
[4] The compound according to any one of [1] to [3], wherein $R^2$ and $R^4$ each are independently the benzofuran-condensed carbazol-9-yl group, the benzothiophene-condensed carbazol-9-yl group, the indole-condensed carbazol-9-yl group, the indene-condensed carbazol-9-yl group, or the silaindene-condensed carbazol-9-yl group.
[5] The compound according to any one of [1] to [4], wherein $R^2$ and $R^4$ are the same.
[6] The compound according to any one of [1] to [5], wherein at least one of the remaining two and $R^3$ is the benzofuran-condensed carbazol-9-yl group or the benzothiophene-condensed carbazol-9-yl group.
[7] The compound according to any one of [1] to [5], wherein the remaining two and $R^3$ each are independently the benzofuran-condensed carbazol-9-yl group or the benzothiophene-condensed carbazol-9-yl group.
[8] The compound according to any one of [1] to [7], wherein $R^1$ is a hydrogen atom or a deuterium atom.
[9] The compound according to any one of [1] to [8], wherein the remaining two and $R^3$ are the same.
[10] The compound according to any one of [1] to [9], wherein at least one of the remaining two and $R^3$ is a benzofuran-condensed carbazol-9-yl group having a skeleton of a carbazole ring condensed with two benzofuran rings at the 2,3-positions, a benzothiophene-condensed carbazol-9-yl group having a skeleton of a carbazole ring condensed

with two benzothiophene rings at the 2,3-positions, an indole-condensed carbazol-9-yl group having a skeleton of a carbazole ring condensed with two indole rings at the 2,3-positions, an indene-condensed carbazol-9-yl group having a skeleton of a carbazole ring condensed with two indene rings at the 2,3-positions, or a silaindene-condensed carbazol-9-yl group having a skeleton of a carbazole ring condensed with two silaindene rings at the 2,3-positions.
[11] The compound according to any one of [1] to [10], having a symmetric structure.
[12] The compound according to any one of [1] to [11], composed of atoms selected from the group consisting of a carbon atom, a hydrogen atom, a deuterium atom, a nitrogen atom, an oxygen atom and a sulfur atom.
[13] The compound according to any one of [1] to [12], wherein the benzofuran-condensed carbazol-9-yl group has a structure of any of the following:

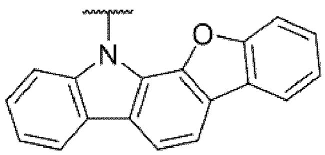
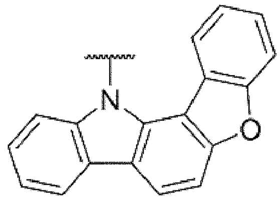
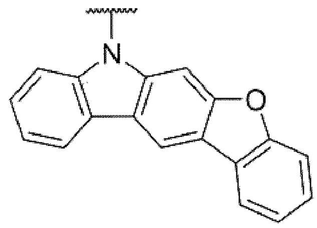
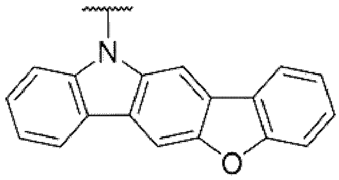
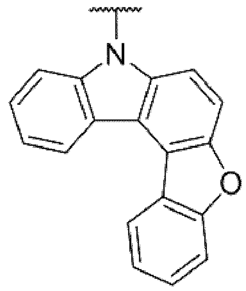
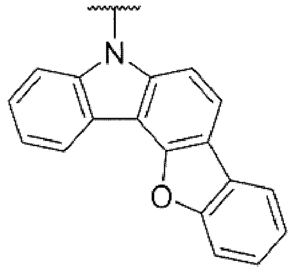

In the above structures, the hydrogen atom can be substituted, but is not further condensed.
[14] The compound according to any one of [1] to [13], wherein the benzothiophene-condensed carbazol-9-yl group has a structure of any of the following:

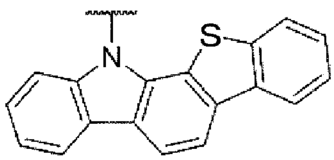
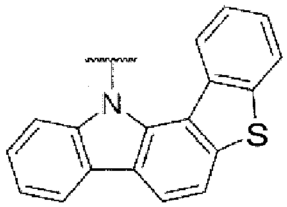
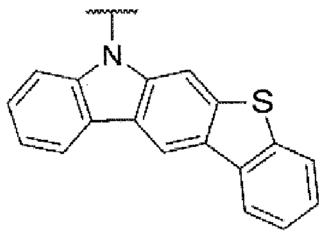
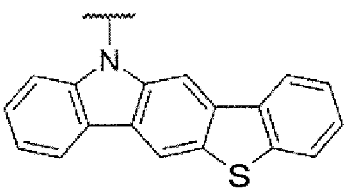
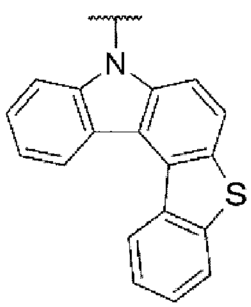
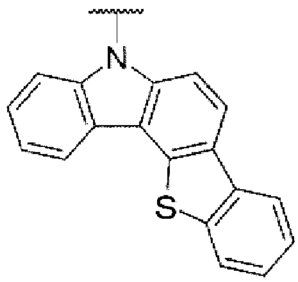

In the above structures, the hydrogen atom can be substituted, but is not further condensed.
[15] A light emitting material of a compound of any one of [1] to [14].

[16] A light emitting device containing a compound of any one of [1] to [14].
[17] The light emitting device according to [16], wherein the light emitting device has a light emitting layer and the light emitting layer contains the above compound and a host material.
[18] The light emitting device according to [16], wherein the light emitting device has a light emitting layer, the light emitting layer contains the above compound and a light emitting material, and the light emitting material mainly emits light.

Advantageous Effects of Invention

The compound of the present invention is useful as a light emitting material. Also, the compound of the present invention includes a compound that emits delayed fluorescence. Also, the compound of the present invention is useful as a material for organic light emitting devices.

Brief Description of Drawing

[Fig. 1] This is a schematic cross-sectional view showing an example of a layer configuration of an organic electroluminescent device.

Description of Embodiments

The contents of the invention will be described in detail below. The constitutional elements may be described below with reference to representative embodiments and specific examples of the invention, but the invention is not limited to the embodiments and the examples. In the description herein, a numerical range expressed as "to" means a range that includes the numerical values described before and after "to" as the upper limit and the lower limit. A part or all of hydrogen atoms existing in the molecule of the compound for use in the present invention can be substituted with deuterium atoms ($^2H$, deuterium D). In the chemical structural formulae in the description herein, the hydrogen atom is expressed as H, or the expression thereof is omitted. For example, when expression of the atoms bonding to the ring skeleton-constituting carbon atoms of a benzene ring is omitted, H is considered to bond to the ring skeleton-constituting carbon atom at the site having the omitted expression. In the chemical structural formulae in the present description, a deuterium atom is expressed as D.

[Compound Represented by General Formula (1)]

General Formula (1)

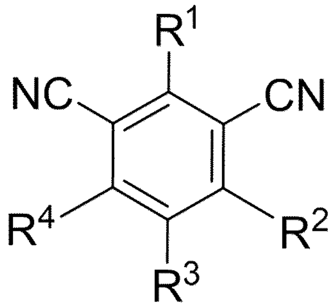

In the general formula (1), any one of $R^1$, $R^2$ and $R^4$ is a hydrogen atom or a deuterium atom. In one preferred embodiment of the present invention, $R^1$ is a hydrogen atom or a deuterium atom. However, an embodiment where $R^2$ is a hydrogen atom or a deuterium atom, and an embodiment where $R^4$ is a hydrogen atom or a deuterium atom are also employable.
In the general formula (1), two of $R^1$, $R^2$ and $R^4$ not a hydrogen atom or a deuterium atom, and $R^3$ each independently represent a donor group. For example, an embodiment where $R^1$ is a hydrogen atom or a deuterium atom, and $R^2$, $R^3$ and $R^4$ each are independently a donor group is preferred. However, an embodiment where $R^2$ is a hydrogen atom or a deuterium atom, and $R^1$, $R^3$ and $R^4$ each are independently a donor group, and an embodiment where $R^4$ is a hydrogen atom or a deuterium atom, and $R^1$, $R^2$ and $R^3$ each are independently a donor group are also employable. In the following description, "two of $R^1$, $R^2$ and $R^4$ not a hydrogen atom or a deuterium atom, and $R^3$" are

collectively called "three R's of donor groups".

Of the three R's of donor groups, at least one is a benzofuran-condensed carbazol-9-yl group having a skeleton of a carbazole ring condensed with a benzofuran ring at the 2,3-positions (hereinafter abbreviated as "benzofuran-condensed carbazol-9-yl group"), a benzothiophene-condensed carbazol-9-yl group having a skeleton of a carbazole ring condensed with a benzothiophene ring at the 2,3-positions (hereinafter abbreviated as "benzothiophene-condensed carbazol-9-yl group"), an indole-condensed carbazol-9-yl group having a skeleton of a carbazole ring condensed with an indole ring at the 2,3-positions (hereinafter abbreviated as "indole-condensed carbazol-9-yl group"), an indene-condensed carbazol-9-yl group having a skeleton of a carbazole ring condensed with an indene ring at the 2,3-positions (hereinafter abbreviated as "indene-condensed carbazol-9-yl group"), or a silaindene-condensed carbazol-9-yl group having a skeleton of a carbazole ring condensed with a silaindene ring at the 2,3-positions (hereinafter abbreviated as "silaindene-condensed carbazol-9-yl group").

In one embodiment of the present invention, at least one of the three R's of donor groups is selected from a benzofuran-condensed carbazol-9-yl group, a benzothiophene-condensed carbazol-9-yl group, and an indole-condensed carbazol-9-yl group. In one embodiment of the present invention, at least one of the three R's of donor groups is selected from a benzofuran-condensed carbazol-9-yl group, a benzothiophene-condensed carbazol-9-yl group, and an indene-condensed carbazol-9-yl group. In one embodiment of the present invention, at least one of the three R's of donor groups is selected from a benzofuran-condensed carbazol-9-yl group, a benzothiophene-condensed carbazol-9-yl group, and a silaindene-condensed carbazol-9-yl group. At least one of the three R's of donor groups is preferably a benzofuran-condensed carbazol-9-yl group or a benzothiophene-condensed carbazol-9-yl group, more preferably a benzofuran-condensed carbazol-9-yl group. At least one of the three R's of donor groups can be a benzothiophene-condensed carbazol-9-yl group. At least one of the three R's of donor groups can be an indole-condensed carbazol-9-yl group. At least one of the three R's of donor groups can be an indene-condensed carbazol-9-yl group. At least one of the three R's of donor groups can be a silaindene-condensed carbazol-9-yl group.

In one embodiment of the present invention, at least two of the three R's of donor groups are selected from a benzofuran-condensed carbazol-9-yl group, a benzothiophene-condensed carbazol-9-yl group, and an indole-condensed carbazol-9-yl group. In one embodiment of the present invention, at least two of the three R's of donor groups are selected from a benzofuran-condensed carbazol-9-yl group, a benzothiophene-condensed carbazol-9-yl group, and an indene-condensed carbazol-9-yl group. In one embodiment of the present invention, at least two of the three R's of donor groups are selected from a benzofuran-condensed carbazol-9-yl group, a benzothiophene-condensed carbazol-9-yl group, and a silaindene-condensed carbazol-9-yl group. At least two of the three R's of donor groups are preferably a benzofuran-condensed carbazol-9-yl group or a benzothiophene-condensed carbazol-9-yl group, more preferably a benzofuran-condensed carbazol-9-yl group. At least two of the three R's of donor groups can be a benzothiophene-condensed carbazol-9-yl group. At least two of the three R's of donor groups can be an indole-condensed carbazol-9-yl group. At least two of the three R's of donor groups can be an indene-condensed carbazol-9-yl group. At least two of the three R's of donor groups can be a silaindene-condensed carbazol-9-yl group.

In one embodiment of the present invention, all the three R's of donor groups are selected from a benzofuran-condensed carbazol-9-yl group, a benzothiophene-condensed carbazol-9-yl group, and an indole-condensed carbazol-9-yl group. In one embodiment of the present invention, all the three R's of donor groups are selected from a benzofuran-condensed carbazol-9-yl group, a benzothiophene-condensed carbazol-9-yl group, and an indene-condensed carbazol-9-yl group. In one embodiment of the present invention, all the three R's of donor groups are selected from a benzofuran-condensed carbazol-9-yl group, a benzothiophene-condensed carbazol-9-yl group, and a silaindene-condensed carbazol-9-yl group. All the three R's of donor groups are preferably a benzofuran-condensed carbazol-9-yl group or a benzothiophene-condensed carbazol-9-yl group, more preferably a benzofuran-condensed carbazol-9-yl group. All the three R's of donor groups can be a benzothiophene-condensed carbazol-9-yl group. All the three R's of donor groups can be an indole-condensed carbazol-9-yl group. All the three R's of donor groups can be an indene-condensed carbazol-9-yl group. All the three R's of donor groups can be a silaindene-condensed carbazol-9-yl group.

In the benzofuran-condensed carbazol-9-yl group, one benzofuran ring only is condensed at the 2,3-positions, or two or more benzofuran rings can be condensed. In addition, a benzofuran ring can be condensed at the 2,3-position and any other ring can be also condensed. The ring to be condensed includes an aromatic hydrocarbon ring, an aromatic hetero ring, an aliphatic hydrocarbon rind, and an aliphatic hetero ring. The aromatic hydrocarbon ring includes a benzene ring. The aromatic hetero ring includes a pyridine ring, a pyridazine ring, a pyrimidine ring, a pyrazine ring, a triazine ring, a pyrrole ring, a pyrazole ring, and an imidazole ring. The aliphatic hydrocarbon ring includes a cyclopentane ring, a cyclohexane ring, and a cycloheptane ring. The aliphatic hetero ring includes a piperidine ring, a pyrrolidine ring, and an imidazolidine ring. Specific examples of the condensed ring that constitutes an aromatic hydrocarbon ring include a naphthalene ring, an anthracene ring, a phenanthrene ring, a pyran ring, and a tetracene ring. Specific examples of a hetero atom-containing condensed ring include an indole ring, an isoindole ring, a benzimidazole ring, a benzotriazole ring, a quinoline ring, an isoquinoline ring, a quinazoline ring, a quinoxaline ring, and a cinnoline ring.

In the present invention, a substituted or unsubstituted benzofuro[2,3-a]carbazol-9-yl group can be employed

as the benzofuran-condensed carbazol-9-yl group. Also a substituted or unsubstituted benzofuro[3,2-a]carbazol-9-yl group can be employed. Also a substituted or unsubstituted benzofuro[2,3-b]carbazol-9-yl group can be employed. Also a substituted or unsubstituted benzofuro[3,2-b]carbazol-9-yl group can be employed. Also a substituted or unsubstituted benzofuro[2,3-c]carbazol-9-yl group can be employed. Also a substituted or unsubstituted benzofuro[3,2-c]carbazol-9-yl group can be employed.

A preferred benzofuran-condensed carbazol-9-yl group is a carbazol-9-yl group in which one benzofuran ring only is condensed at the 2,3-positions and any other ring is not condensed. Specifically, preferred are groups having any of the following structures in which the hydrogen atom can be substituted. For example, preferably exemplified are those in which a part of the hydrogen atoms in the following structures are substituted with a deuterium atom, and those where all the hydrogen atoms in the following structures are substituted with a deuterium atom. Unsubstituted ones are also preferably employable.

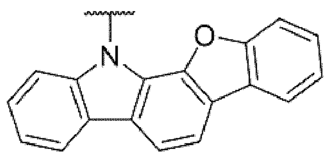
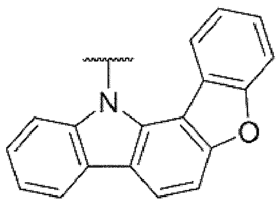
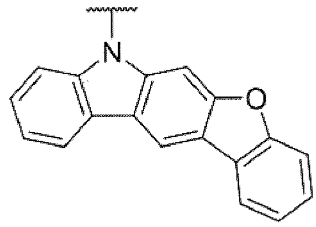

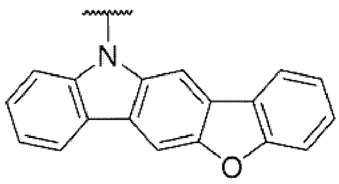
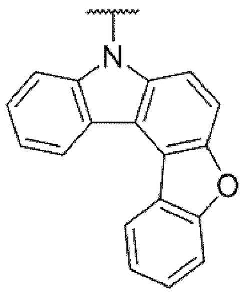
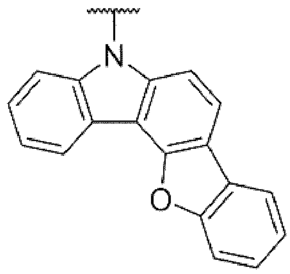

Also preferred is a carbazol-9-yl group in which two benzofuran rings are condensed at the 2,3-position and any other ring is not condensed. Specifically, preferred are groups having any of the following structures in which the hydrogen atom can be substituted. For example, preferably exemplified are those in which a part of the hydrogen atoms in the following structures are substituted with a deuterium atom, and those where all the hydrogen atoms in the following structures are substituted with a deuterium atom. Unsubstituted ones are also preferably employable.

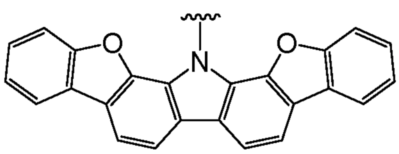
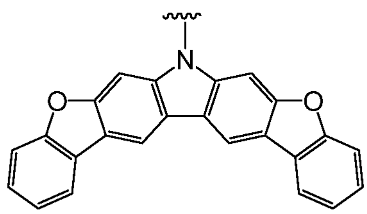
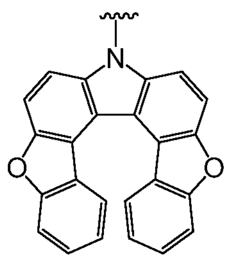

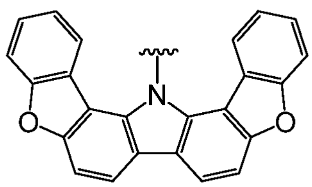
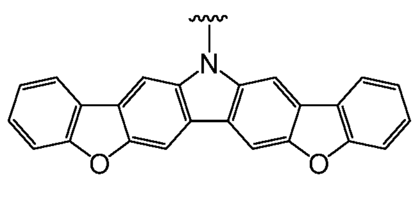
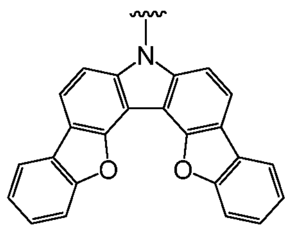

In the benzothiophene-condensed carbazol-9-yl group, only one benzothiophene ring can be condensed at the

2,3-positions, or two or more benzothiophene rings can be condensed. Also in the group, any other ring can be condensed along with the benzothiophene ring condensed at the 2,3-positions. Regarding the description and the specific examples of the ring to be condensed, reference can be made to the description and the specific examples of the ring to be condensed in the description of the benzofuran-condensed carbazol-9-yl group mentioned above. In the present invention, a substituted or unsubstituted benzothieno[2,3-a]carbazol-9-yl group can be employed as the benzothiophene-condensed carbazol-9-yl group. Also a substituted or unsubstituted benzothieno[3,2-a]carbazol-9-yl group can be employed. Also a substituted or unsubstituted benzothieno[2,3-b]carbazol-9-yl group can be employed. Also a substituted or unsubstituted benzothieno[3,2-b]carbazol-9-yl group can be employed. Also a substituted or unsubstituted benzothieno[2,3-c]carbazol-9-yl group can be employed. Also a substituted or unsubstituted benzothieno[3,2-c]carbazol-9-yl group can be employed.

A preferred benzothiophene-condensed carbazol-9-yl group is a carbazol-9-yl group in which one benzothiophene ring only is condensed at the 2,3-positions and any other ring is not condensed. Specifically, preferred are groups having any of the following structures in which the hydrogen atom can be substituted. For example, preferably exemplified are those in which a part of the hydrogen atoms in the following structures are substituted with a deuterium atom, and those where all the hydrogen atoms in the following structures are substituted with a deuterium atom. Unsubstituted ones are also preferably employable.

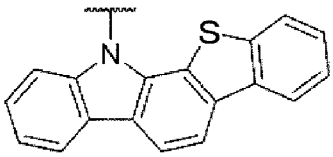
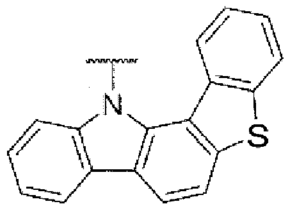
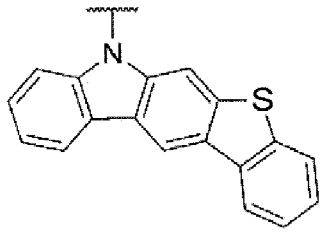

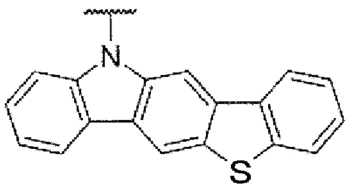
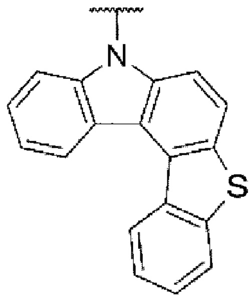
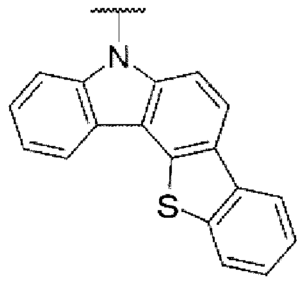

A carbazol-9-yl group is also preferred, in which two benzothiophene rings are condensed at the 2,3-positions, and any other ring is not condensed. Specifically, preferred are groups having any of the following structures in which the hydrogen atom can be substituted. For example, preferably exemplified are those in which a part of the hydrogen atoms in the following structures are substituted with a deuterium atom, and those where all the hydrogen atoms in the following structures are substituted with a deuterium atom. Unsubstituted ones are also preferably employable.

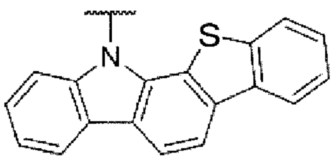
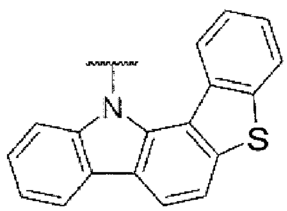
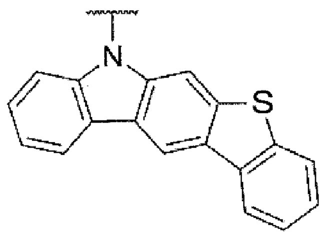

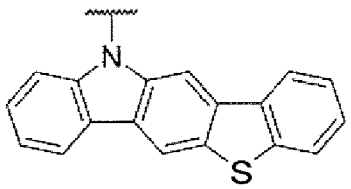
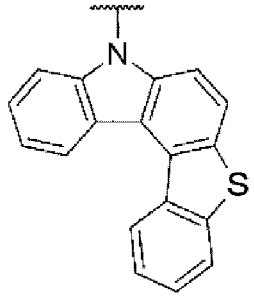
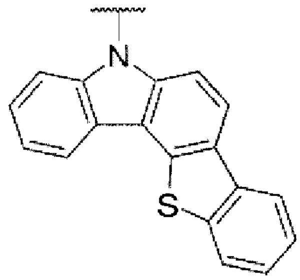

In the indole-condensed carbazol-9-yl group, only one indole ring can be condensed at the 2,3-positions, or two or more indole rings can be condensed. Also in the group, any other ring can be condensed along with the indole ring condensed at the 2,3-positions. Regarding the description and the specific examples of the ring to be condensed, reference can be made to the description and the specific examples of the ring to be condensed in the description of the benzofuran-condensed carbazol-9-yl group mentioned above. In the present invention, a substituted or unsubstituted indolo[2,3-a]carbazol-9-yl group can be employed as the indole-condensed carbazol-9-yl group. Also a substituted or unsubstituted indolo[3,2-a]carbazol-9-yl group can be employed. Also a substituted or unsubstituted indolo[2,3-b]carbazol-9-yl group can be employed. Also a substituted or unsubstituted indolo[3,2-b]carbazol-9-yl group can be employed. Also a substituted or unsubstituted indolo[2,3-c]carbazol-9-yl group can be employed. Also a substituted or unsubstituted indolo[3,2-c]carbazol-9-yl group can be employed.

A preferred indole-condensed carbazol-9-yl group is a carbazol-9-yl group in which one indole ring only is condensed at the 2,3-positions and any other ring is not condensed. Specifically, preferred are groups having any of the following structures in which R is a hydrogen atom, a deuterium atom or a substituent (preferably, R is a substituent). The hydrogen atoms in the following structures can be substituted. For example, preferably exemplified are those in which a part of the hydrogen atoms in the following structures are substituted with a deuterium atom, and those where all the hydrogen atoms in the following structures are substituted with a deuterium atom. Unsubstituted ones are also preferably employable.

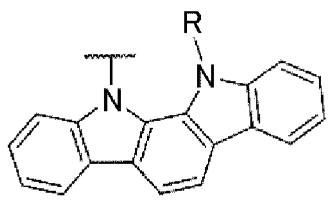
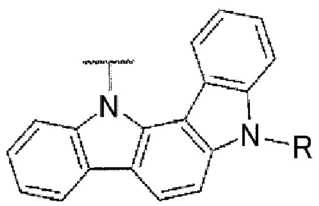
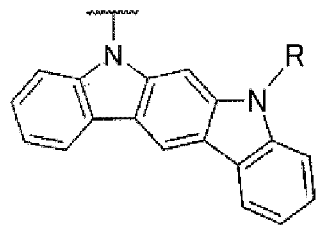

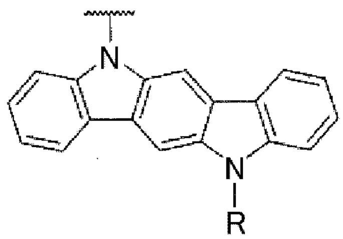
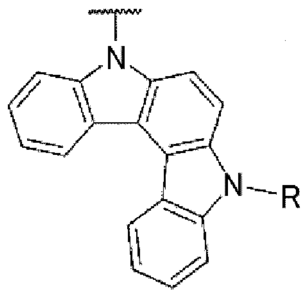
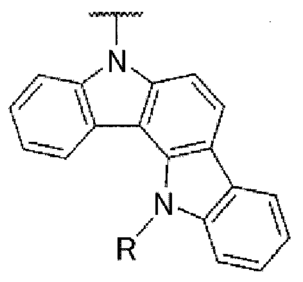

In the indene-condensed carbazol-9-yl group, only one indene ring can be condensed at the 2,3-positions, or two or more indene rings can be condensed. Also in the group, any other ring can be condensed along with the indene ring condensed at the 2,3-positions. Regarding the description and the specific examples of the ring to be condensed, reference can be made to the description and the specific examples of the ring to be condensed in the description of the benzofuran-condensed carbazol-9-yl group mentioned above. When referring to an indene ring in the present invention, the description will be given assuming 1H-indene (having a double bond between the 2-position and the 3-position). On the other hand, when indenocarbazole is defined, it is referred to as indeno[2,3-x]carbazole or indeno[3,2-x]carbazole (where x is a, b or c) according to the IUPAC nomenclature. In the present invention, a substituted or unsubstituted indeno[2,3-a]carbazol-9-yl group can be employed as the indene-condensed carbazol-9-yl group. Also a substituted or unsubstituted indeno[3,2-a]carbazol-9-yl group can be employed. Also a substituted or unsubstituted indeno[2,3-b]carbazol-9-yl group can be employed. Also a substituted or unsubstituted indeno[3,2-b]carbazol-9-yl group can be employed. Also a substituted or unsubstituted indeno[2,3-c]carbazol-9-yl group can be employed. Also a substituted or unsubstituted indeno[3,2-c]carbazol-9-yl group can be employed.

A preferred indene-condensed carbazol-9-yl group is a carbazol-9-yl group in which one indene ring only is condensed at the 2,3-positions and any other ring is not condensed. Specifically, preferred are groups having any of the following structures in which the hydrogen atoms can be substituted. For example, preferably exemplified are those in which a part of the hydrogen atoms in the following structures are substituted with a deuterium atom, and those where all the hydrogen atoms in the following structures are substituted with a deuterium atom. Unsubstituted ones are also preferably employable.

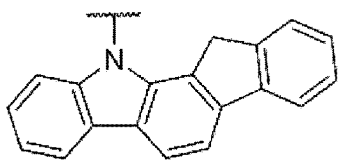
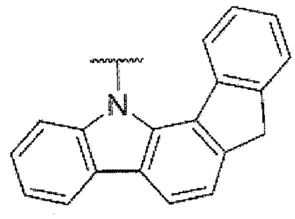
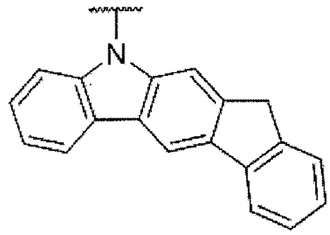
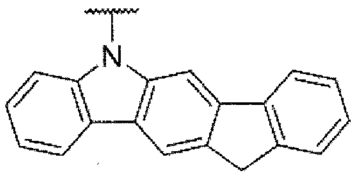
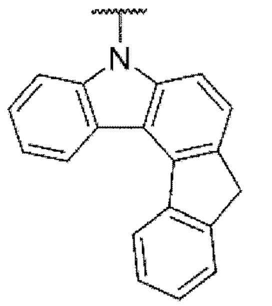
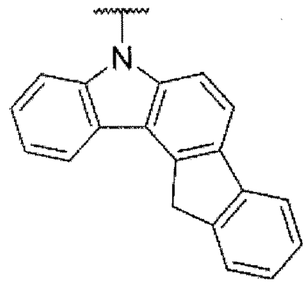

In the silaindene-condensed carbazol-9-yl group, only one silaindene ring can be condensed at the 2,3-positions, or two or more silaindene rings can be condensed. Also in the group, any other ring can be condensed along with the silaindene ring condensed at the 2,3-positions. Regarding the description and the specific examples of the ring to be condensed, reference can be made to the description and the specific examples of the ring to be condensed in the description of the benzofuran-condensed carbazol-9-yl group mentioned above. When referring to an silaindene ring in the present invention, the description will be given assuming 1H-silaindene (having a double bond between the 2-position and the 3-position). On the other hand, when silaindenocarbazole is defined, it is referred to as silaindeno[2,3-x]carbazole or silaindeno[3,2-x]carbazole (where x is a, b or c) according to the IUPAC nomenclature. In the present invention, a substituted or unsubstituted silaindeno[2,3-a]carbazol-9-yl group can be employed as the silaindene-condensed carbazol-9-yl group. Also a substituted or unsubstituted silaindeno[3,2-a]carbazol-9-yl group can be employed. Also a substituted or unsubstituted silaindeno[2,3-b]carbazol-9-yl group can be employed. Also a substituted or unsubstituted silaindeno[3,2-b]carbazol-9-yl group can be employed. Also a substituted or unsubstituted silaindeno[2,3-c]carbazol-9-yl group can be employed. Also a substituted or unsubstituted silaindeno[3,2-c]carbazol-9-yl group can be employed.

A preferred silaindene-condensed carbazol-9-yl group is a carbazol-9-yl group in which one silaindene ring only is condensed at the 2,3-positions and any other ring is not condensed. Specifically, preferred are groups having any of the following structures in which R and R' each independently represent a hydrogen atom, a deuterium atom or a substituent (preferably R and R' each are a substituent). The hydrogen atoms in the following structures can be substituted. For example, preferably exemplified are those in which a part of the hydrogen atoms in the following structures are substituted with a deuterium atom, and those where all the hydrogen atoms in the following structures are substituted with a deuterium atom. Unsubstituted ones are also preferably employable. R and R' can be the same or different, and can bond to each other to form a cyclic structure.

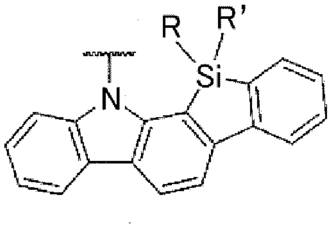
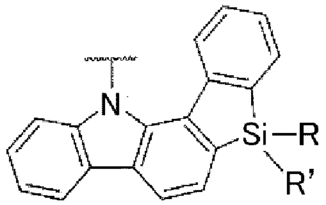
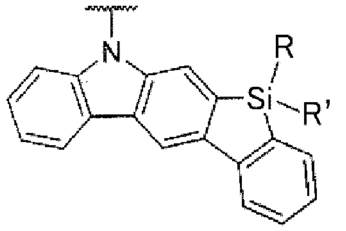

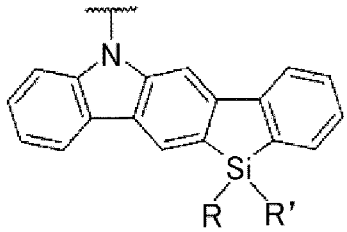
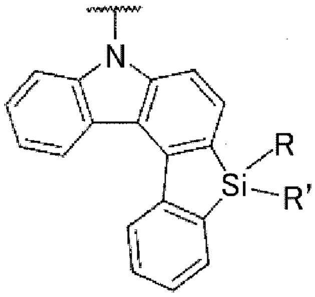
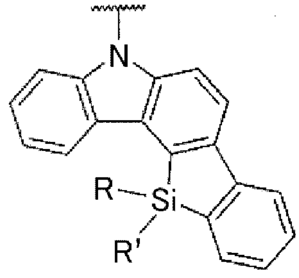

The benzofuran-condensed carbazol-9-yl group, the benzothiophene-condensed carbazol-9-yl group, the indole-condensed carbazol-9-yl group, the indene-condensed carbazol-9-yl group, and the silaindene-condensed carbazol-9-yl group employable in the general formula (1) can be substituted. Also these can be unsubstituted. In the case where the groups are substituted, they can be substituted with a deuterium atom, or can be substituted with any other substituent. The substituent as referred to herein includes an alkyl group, an alkenyl group, an aryl group, a heteroaryl group, an alkoxy group, an alkylthio group, an aryloxy group, an arylthio group, a heteroaryloxy group, a heteroarylthio group, and a cyano group. These substituent can be further substituted with any other substituent. For example, there are mentioned embodiments substituted with a deuterium atom, an alkyl group, an aryl group, an alkoxy group or an alkylthio group. In one embodiment of the present invention, the substituent is an aryl group that can be substituted with an alkyl group, or an alkyl group that can be substituted with an aryl group.

The "alkyl group" as referred to herein can be linear, branched or cyclic. Two or more of a linear moiety, a cyclic moiety and a branched moiety can exist therein as combined. Further, the alkyl group can have a cage structure, such as an adamantyl group. The carbon number of the alkyl group can be, for example 1 or more, 2 or more, or 4 or more. The carbon number can also be 30 or less, 20 or less, 10 or less, 6 or less, or 4 or less. Specific examples of the alkyl group include a methyl group, an ethyl group, an n-propyl group, an isopropyl group, an n-butyl group, an isobutyl group, a tert-butyl group, an n-pentyl group, an isopentyl group, an n-hexyl group, an isohexyl group, a 2-ethylhexyl group, an n-heptyl group, an isoheptyl group, an n-octyl group, an isooctyl group, an n-nonyl group, an isononyl group, an n-decanyl group, an isodecanyl group, a cyclopentyl group, a cyclohexyl group, a cycloheptyl group, a norbornyl group, and an adamantyl group. The alkyl group as a substituent can be further substituted with a deuterium atom, an aryl group, an alkoxy group, an aryloxy group or a halogen atom.

The "alkenyl group" can be linear, branched or cyclic. Two or more of a linear moiety, a cyclic moiety and a branched moiety can exist therein as combined. The carbon number of the alkenyl group can be, for example 2 or more, or 4 or more. The carbon number can also be 30 or less, 20 or less, 10 or less, 6 or less, or 4 or less. Specific examples of the alkyl group include an ethenyl group, an n-propenyl group, an isopropenyl group, an n-butenyl group, an isobutenyl group, an n-pentenyl group, an isopentenyl group, an n-hexenyl group, an isohexenyl group, and a 2-ethylhexenyl group. The alkenyl group as a substituent can be further substituted.

The "aryl group" and the "heteroaryl group" each may be a single ring or may be a condensed ring of two or more kinds of rings. In the case of a condensed ring, the number of the rings that are condensed is preferably 2 to 6, and, for example, can be selected from 2 to 4. Specific examples of the ring include a benzene ring, a pyridine ring, a pyrimidine ring, a triazine ring, a naphthalene ring, an anthracene ring, a phenanthrene ring, a triphenylene ring, a quinoline ring, a pyrazine ring, a quinoxaline ring, and a naphthyridine ring. Specific examples of the arylene ring or the heteroarylene ring include a phenyl group, a 1-naphthyl group, a 2-naphthyl group, a 1-anthracenyl group, a 2-anthracenyl group, a 9-anthracenyl group, a 2-pyridyl group, a 3-pyridyl group, and a 4-pyridyl group.

Regarding the alkyl moiety in the "alkoxy group" and the "alkylthio group", reference can be made to the description and the specific examples of the alkyl group mentioned above. Regarding the aryl moiety in the "aryloxy group" and the "arylthio group", reference can be made to the description and the specific examples of the aryl group mentioned above. Regarding the heteroaryl moiety in the "heteroaryloxy group" and the "heteroarylthio group", reference can be made to the description and the specific examples of the heteroaryl group mentioned above.

One or two of the three R's of donor groups can be any other donor groups than a benzofuran-condensed carbazol-9-yl group, a benzothiophene-condensed carbazol-9-yl group, an indole-condensed carbazol-9-yl group, an indene-condensed carbazol-9-yl group and a silaindene-condensed carbazol-9-yl group (hereinafter referred to as "other donor groups"). The other donor groups as referred to herein are groups having a negative Hammett's $\sigma_p$ value. Here, "Hammett's $\sigma_p$ value" is one propounded by L. P. Hammett, and is one to quantify the influence of a substituent on the reaction rate or the equilibrium of a para-substituted benzene derivative. Specifically, the value is a constant ($\sigma_p$) peculiar to the substituent in the following equation that is established between a substituent and a reaction rate constant or an equilibrium constant in a para-substituted benzene derivative:

$$\log(k/k_0) = \rho\sigma_p$$

or

$$\log(K/K_0) = \rho\sigma_p$$

In the above equations, k represents a rate constant of a benzene derivative not having a substituent; $k_0$ represents a rate constant of a benzene derivative substituted with a substituent; K represents an equilibrium constant of a benzene derivative not having a substituent; $K_0$ represents an equilibrium constant of a benzene derivative substituted with a substituent; $\rho$ represents a reaction constant to be determined by the kind and the condition of reaction. Regarding the description relating to the "Hammett's $\sigma_p$ value" and the numerical value of each substituent in the present invention, reference may be made to the description relating to $\sigma_p$ value in Hansch, C. et. al., Chem. Rev., 91, 165-195 (1991). A group having a negative Hammett's $\sigma_p$ value tends to exhibit an electron donor property, and a group having a positive Hammett's $\sigma_p$ value tends to exhibit an electron acceptor property.

The other donor group in the present invention is preferably a group containing a substituted amino group. The substituent to bond the nitrogen atom of the amino group is preferably a substituted or unsubstituted alkyl group, a substituted or unsubstituted alkenyl group, a substituted or unsubstituted aryl group, or a substituted or unsubstituted heteroaryl group, more preferably a substituted or unsubstituted aryl group, or a substituted or unsubstituted heteroaryl group. The substituted amino group is especially preferably a substituted or unsubstituted diarylamino group, or a substituted or unsubstituted diheteroarylamino group. In the present invention, the donor group can be a group bonding at the nitrogen atom of a substituted amino group, or can be a group bonding to the substituted amino group-bonding group. The substituted amino group-bonding group is preferably a $\pi$-conjugated group. More preferred is a group bonding at the nitrogen atom of the substituted amino group. Regarding the alkyl group, the alkenyl group, the aryl group and the heteroaryl group as referred to herein as substituents, reference can be made to the corresponding description relating to the substituents for the aromatic hydrocarbon cyclic group and the aromatic heterocyclic group mentioned hereinabove.

The other donor group especially preferred in the present invention is a substituted or unsubstituted carbazol-9-yl group. The carbazol-9-yl group can be further condensed with a benzene ring or a heteroring (but excepting a benzofuran ring, a benzothiophene ring, an indole ring, an indene ring and a silaindene ring). The substituent for the carbazol-9-yl group includes a deuterium atom, an alkyl group, an alkenyl group, an aryl group, a heteroaryl group, an alkoxy group, an alkylthio group, an aryloxy group, an arylthio group, a heteroaryloxy group, a heteroarylthio group, and a substituted amino group. Preferred substituents are a deuterium atom, an alkyl group, an aryl group and a substituted amino group. Regarding the description of the substituted amino group, reference can be made to the description in the preceding paragraph. The substituted amino group as referred to herein includes a substituted or unsubstituted carbazolyl group, and for example, includes a substituted or unsubstituted carbazol-3-yl group or a substituted or unsubstituted carbazol-9-yl group.

The number of the atoms except hydrogen atoms and deuterium atoms constituting the donor group in the present invention is preferably 8 or more, more preferably 12 or more, and, for example, can be 16 or more. The number is preferably 80 or less, more preferably 60 or less, even more preferably 40 or less.

All the three R's of donor groups in the general formula (1) can be the same or different. Also, two R's can be the same and one R can differ. In one preferred embodiment of the present invention, the three R's of donor groups are the same. In another preferred embodiment of the present invention, two of the three R's of donor groups are the same, and are any of a benzofuran-condensed carbazol-9-yl group, a benzothiophene-condensed carbazol-9-yl group, an indole-condensed carbazol-9-yl group, an indene-condensed carbazol-9-yl group and a silaindene-condensed carbazol-9-yl group (hereinafter referred to as "specific five types of condensed carbazol-9-yl groups"), and the remaining one is a donor group except the specific five types of condensed carbazol-9-yl groups. In another preferred embodiment of the present invention, one of the three R's of donor groups is any of the specific five types of condensed carbazol-9-yl

groups, and the remaining two are the same and are donor groups except the specific five types of condensed carbazol-9-yl groups.

In one preferred embodiment of the present invention, $R^1$ is a hydrogen atom or a deuterium atom, $R^2$, $R^3$ and $R^4$ are the same and are any of the specific five types of condensed carbazol-9-yl groups. In another preferred embodiment of the present invention, $R^1$ is a hydrogen atom or a deuterium atom, $R^2$ and $R^4$ are the same and are any of the specific five types of condensed carbazol-9-yl groups, and $R^3$ is an other donor group. In another preferred embodiment of the present invention, $R^1$ is a hydrogen atom or a deuterium atom, $R^3$ is any of the specific five types of condensed carbazol-9-yl groups and $R^2$ and $R^4$ are the same and an other donor group. In another embodiment of the present invention, $R^1$ is a hydrogen atom or a deuterium atom, $R^3$ and $R^4$ are the same and are any of the specific five types of condensed carbazol-9-yl groups, and $R^2$ is an other donor group.

Hereinunder shown are specific examples of the donor group that the three R's of donor groups can represent. D1 toD12, D109 to D113, D150 to D179, D192 to D197, D240 to D244, D299 and D433 are specific examples of other donor groups, D13 to D108, D114 to D149, D180 to D191, D198 to D239, D245 to D298, D300 to D432 and D434 to D839 are specific examples of a benzofuran-condensed carbazol-9-yl group, a benzothiophene-condensed carbazol-9-yl group, an indole-condensed carbazol-9-yl group, an indene-condensed carbazol-9-yl group and a silaindene-condensed carbazol-9-yl group. In the following structural formulae, Ph represents a phenyl group, D represents a deuterium atom, tBu represents a tert-butyl group, and iPro represents an isopropyl group.

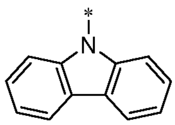
D1

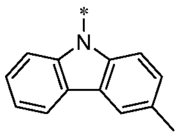
D2

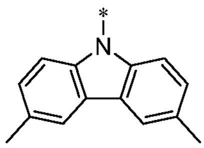
D3

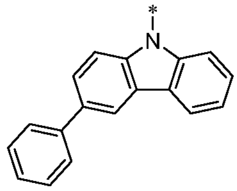
D4

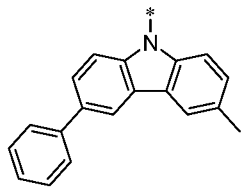
D5

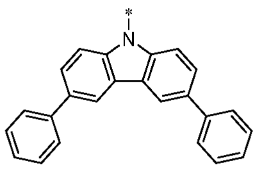
D6

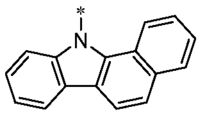
D7

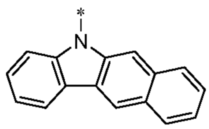
D8

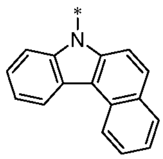
D9

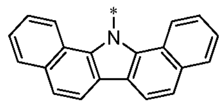
D10

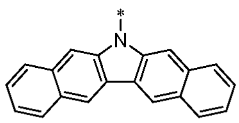
D11

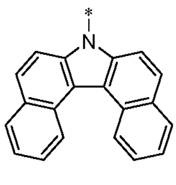
D12

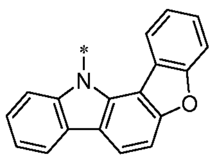
D13

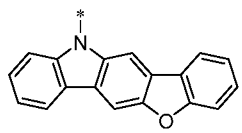
D14

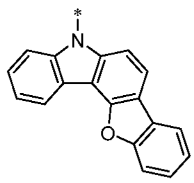
D15

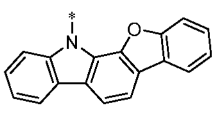
D16

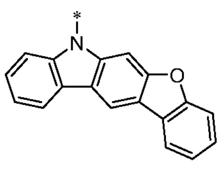
D17

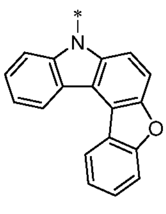
D18

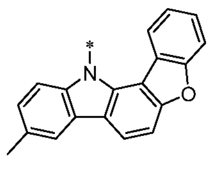
D19

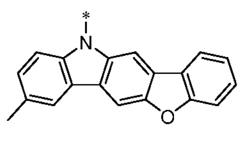
D20

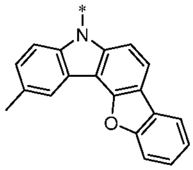
**D21**

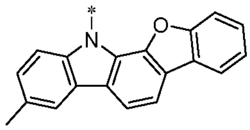
**D22**

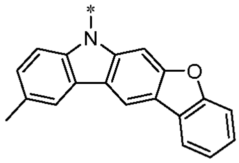
**D23**

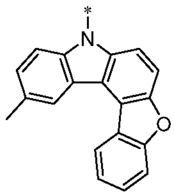
**D24**

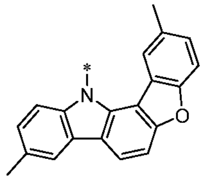
**D25**

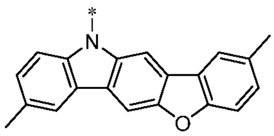
**D26**

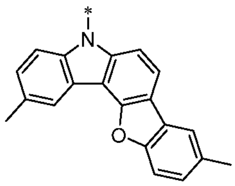
**D27**

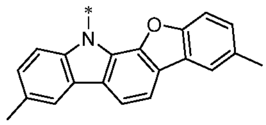
**D28**

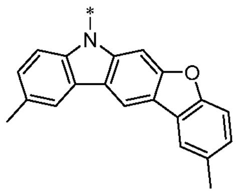
**D29**

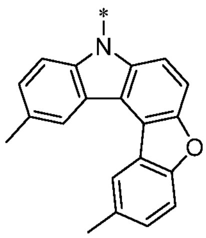
**D30**

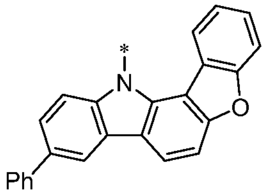
**D31**

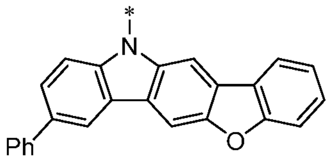
**D32**

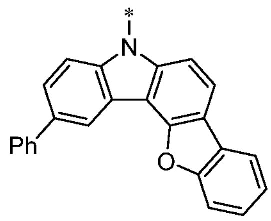
**D33**

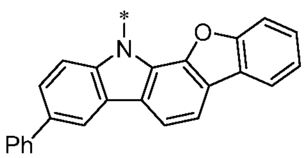
**D34**

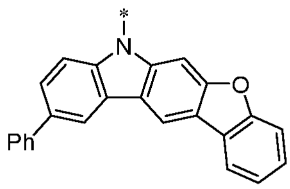
**D35**

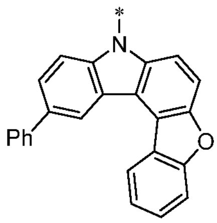
**D36**

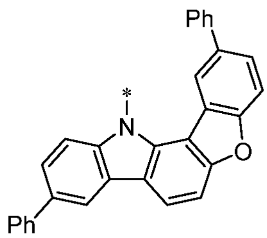
**D37**

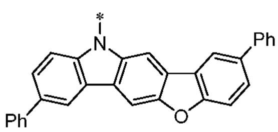
**D38**

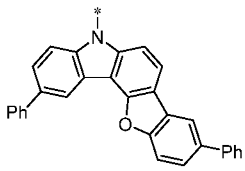
**D39**

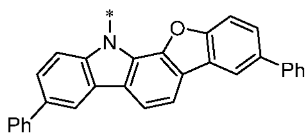
**D40**

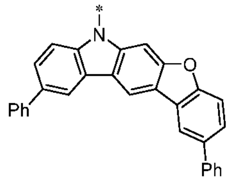
**D41**

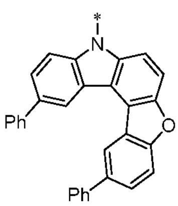
**D42**

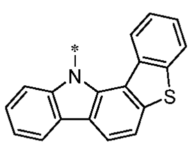
**D43**

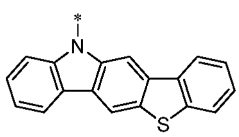
**D44**

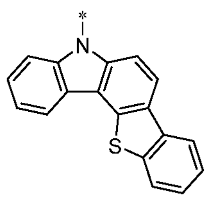
**D45**

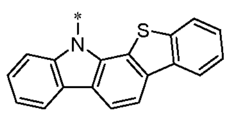
**D46**

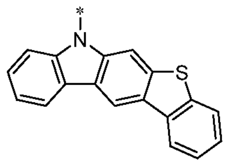
D47

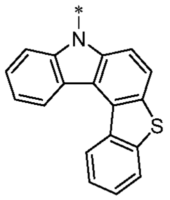
D48

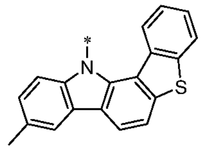
D49

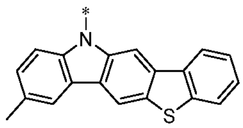
D50

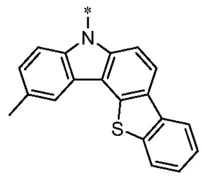
D51

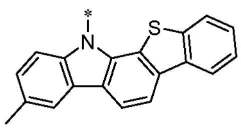
D52

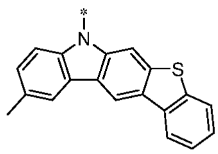
D53

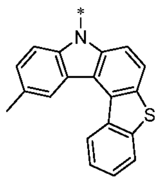
D54

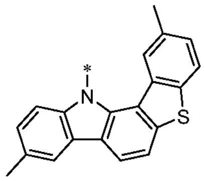
D55

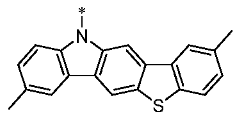
D56

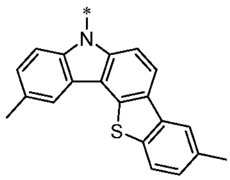
D57

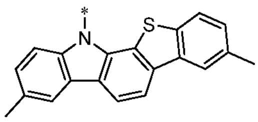
D58

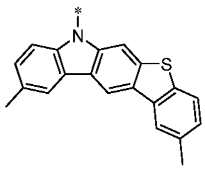
D59

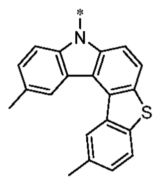
D60

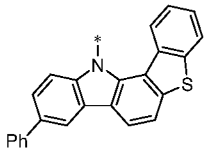
D61

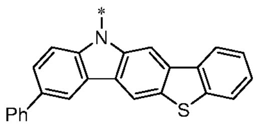
D62

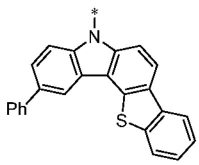
D63

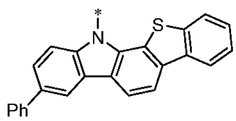
D64

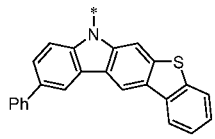
D65

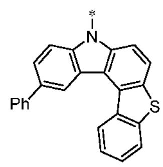
D66

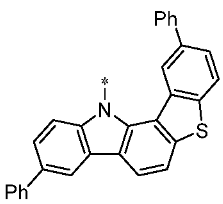
D67

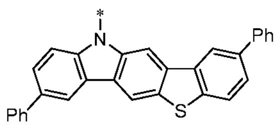
D68

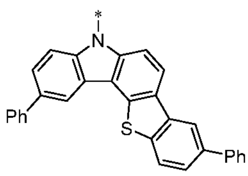
D69

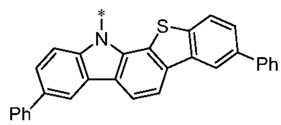
D70

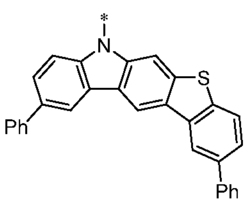
D71

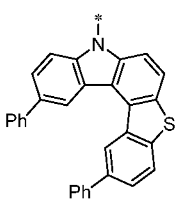
D72

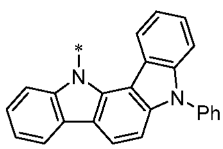
D73

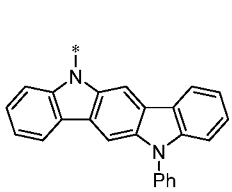
D74

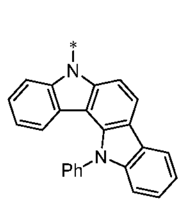
D75

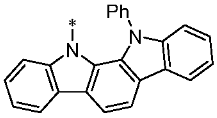
D76

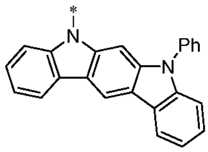
D77

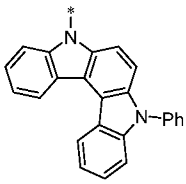
D78

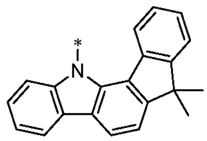
D79

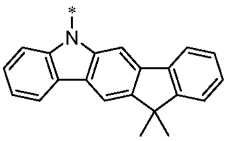
D80

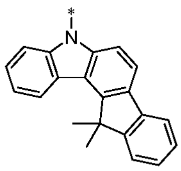
D81

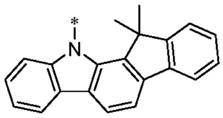
D82

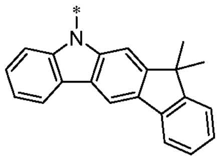
D83

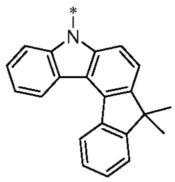
D84

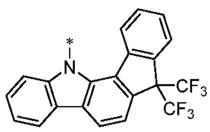
D85

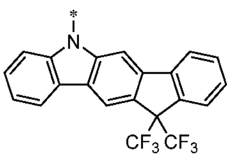
D86

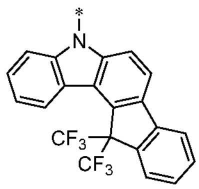
D87

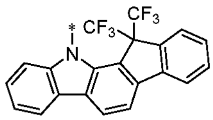
D88

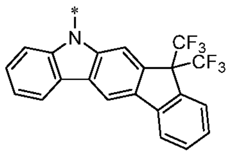
D89

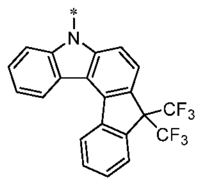
D90

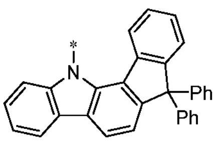
D91

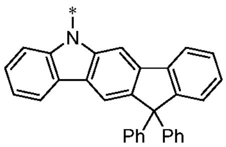
D92

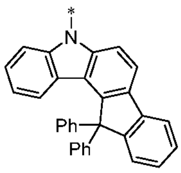
D93

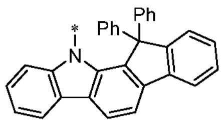
D94

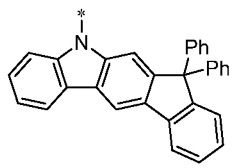
D95

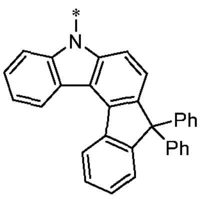
D96

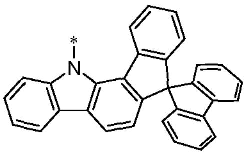
D97

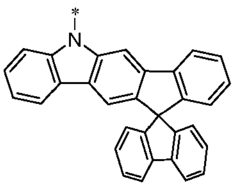
D98

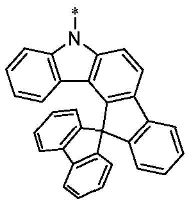
D99

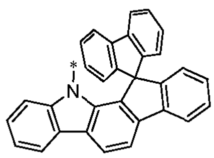
D100

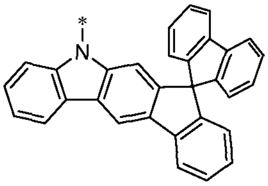
D101

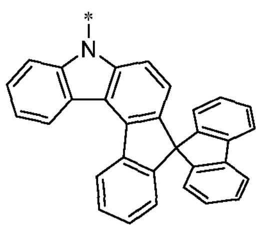
D102

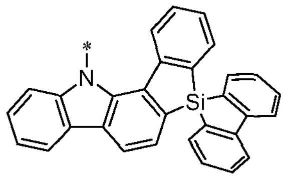
D103

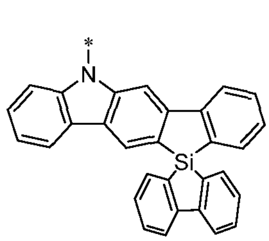
D104

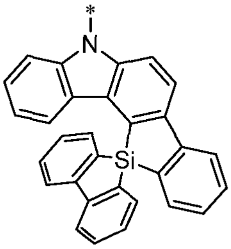
D105

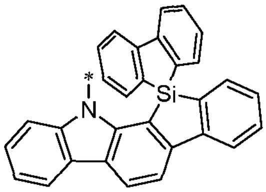
D106

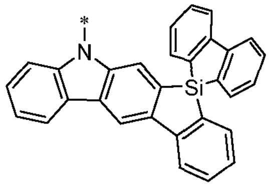
D107

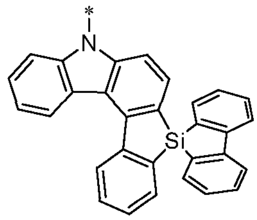
D108

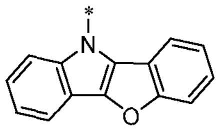
D109

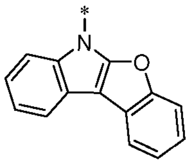
D110

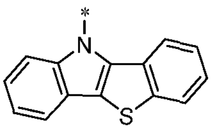
D111

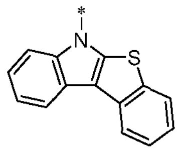
D112

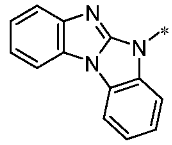
D113

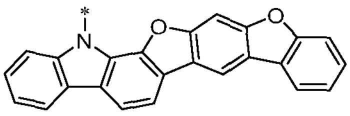
D114

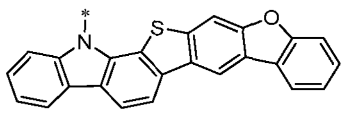
D115

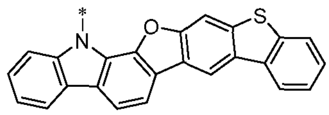
D116

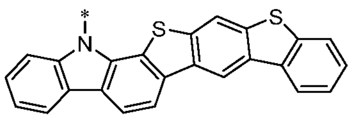
D117

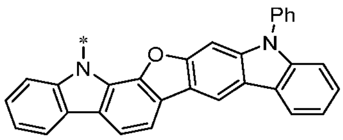
D118

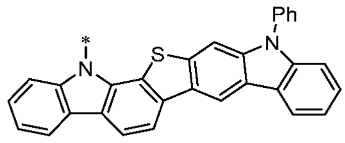
D119

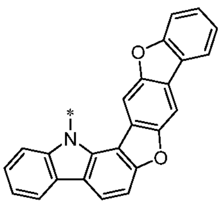
D120

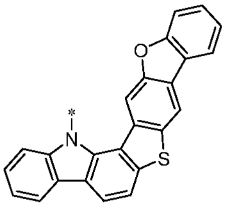
D121

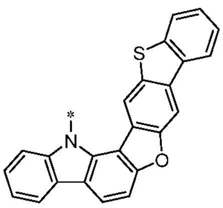
D122

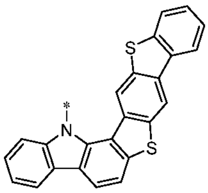
D123

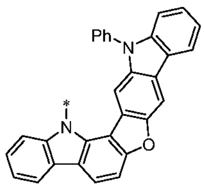
D124

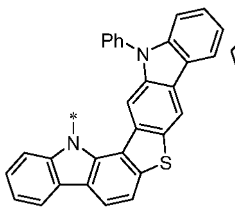
D125

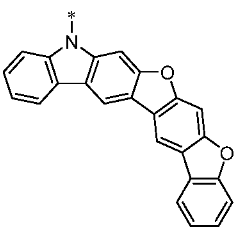
D126

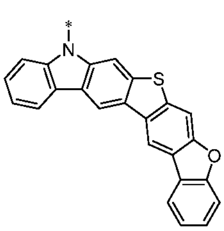
D127

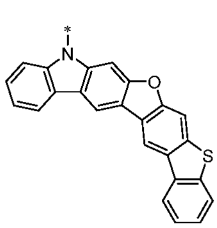
D128

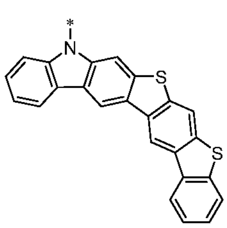
D129

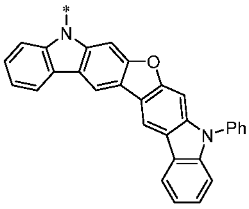
**D130**

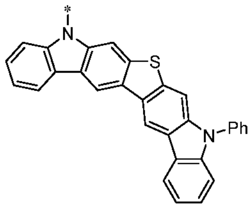
**D131**

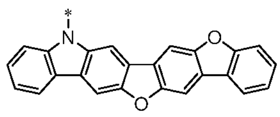
**D132**

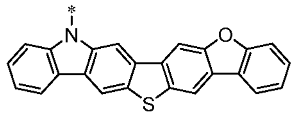
**D133**

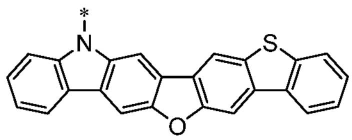
**D134**

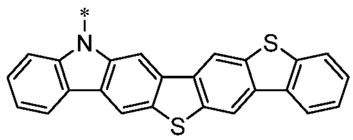
**D135**

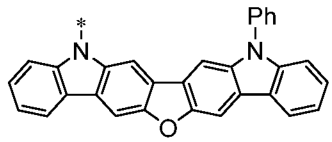
**D136**

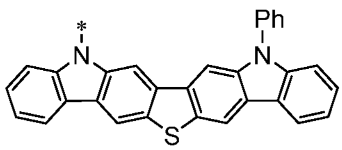
**D137**

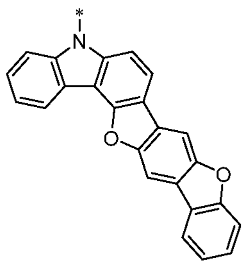
**D138**

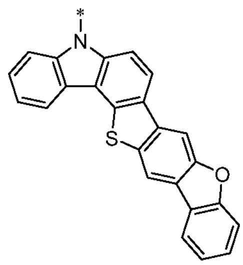
**D139**

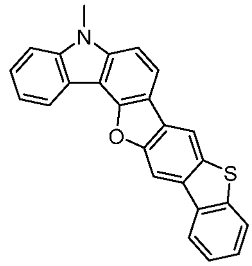
**D140**

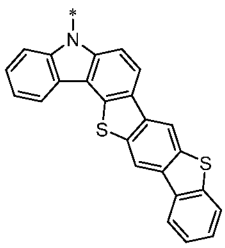
**D141**

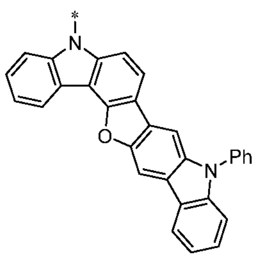
**D142**

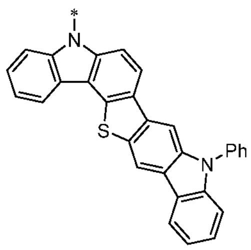
**D143**

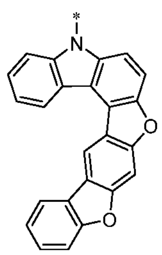
**D144**

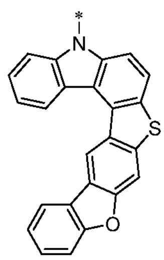
**D145**

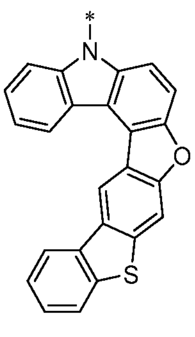
**D146**

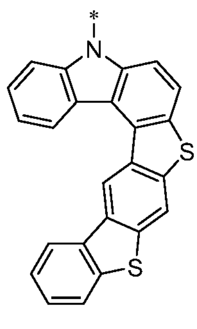
**D147**

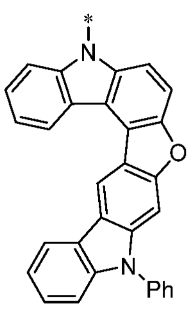
**D148**

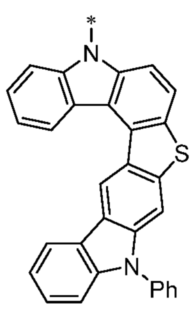
**D149**

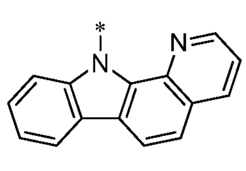
**D150**

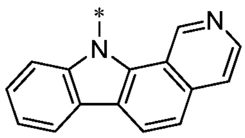
D151

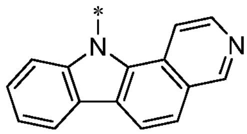
D152

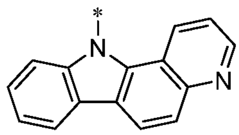
D153

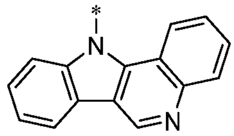
D154

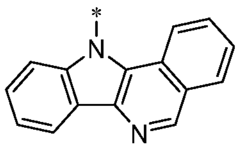
D155

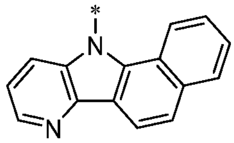
D156

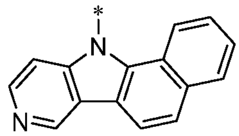
D157

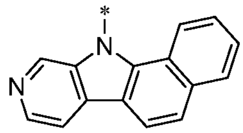
D158

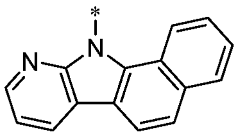
D159

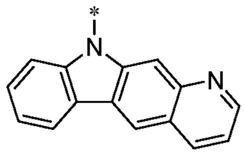
D160

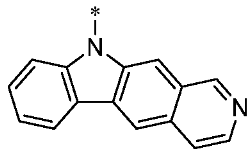
D161

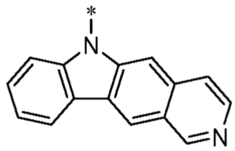
D162

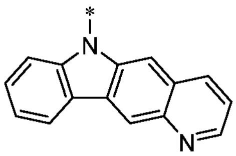
D163

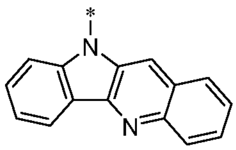
D164

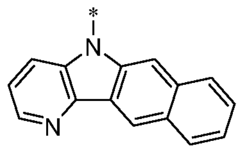
D165

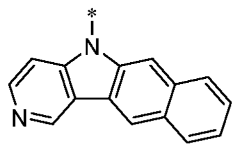
D166

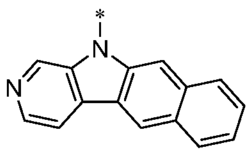
D167

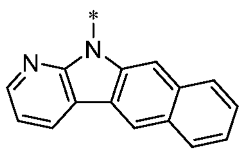
D168

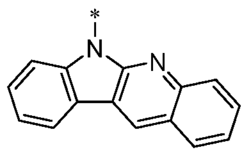
D169

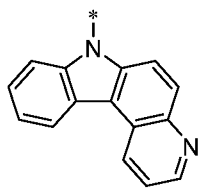
D170

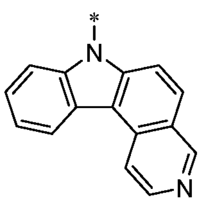
D171

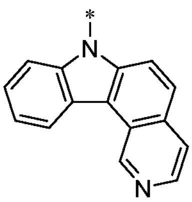
D172

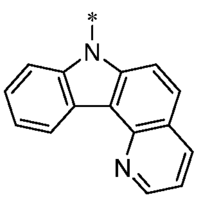
D173

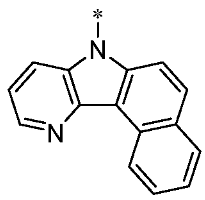
D174

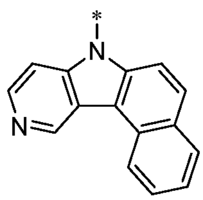
D175

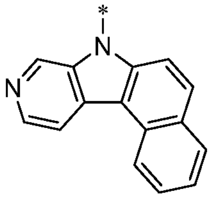
D176

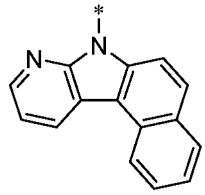
D177

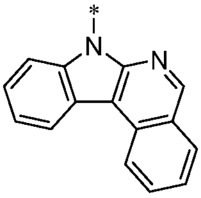
D178

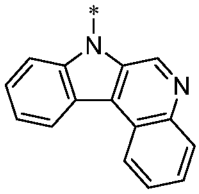
D179

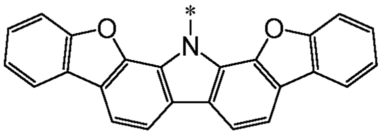
D180

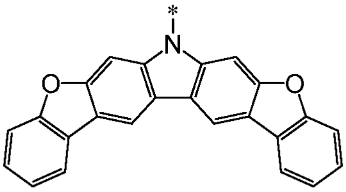
D181

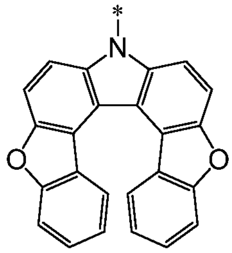
D182

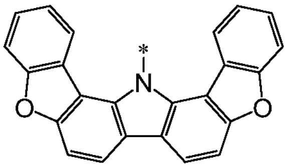
D183

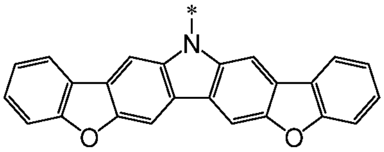
D184

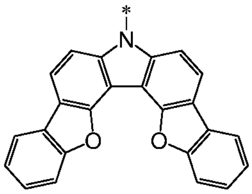
D185

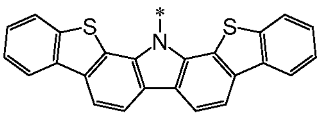
D186

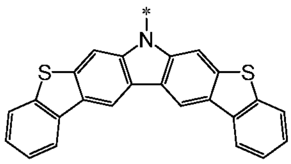
D187

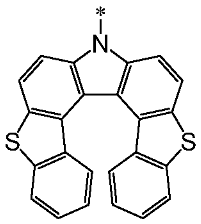
D188

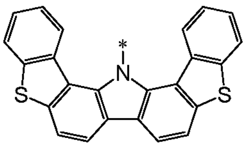
D189

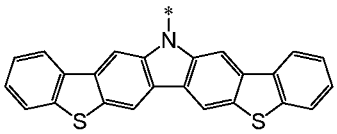
D190

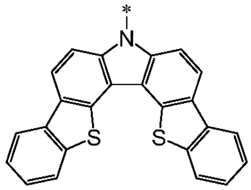
D191

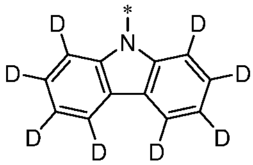
D192

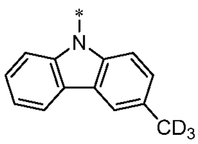
D193

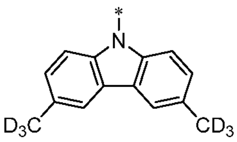
D194

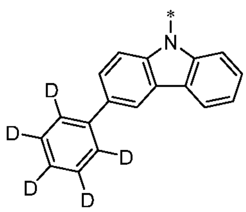
D195

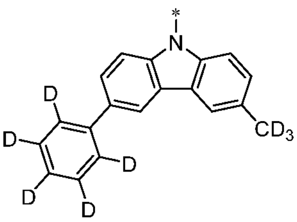
D196

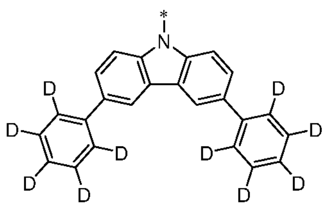
D197

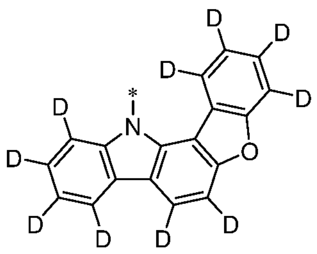

D198

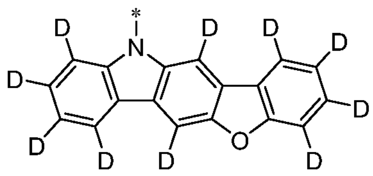

D199

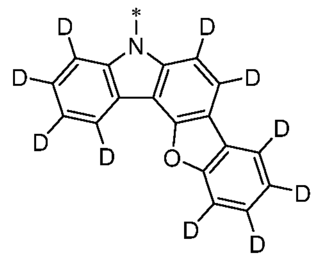

D200

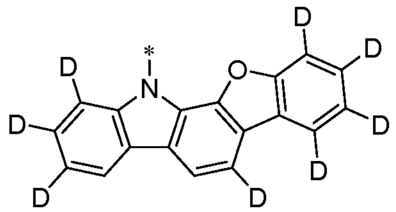

D201

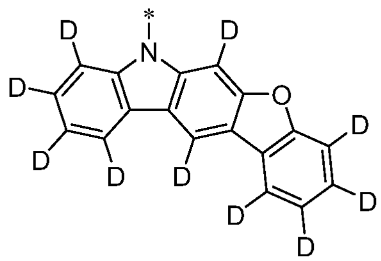

D202

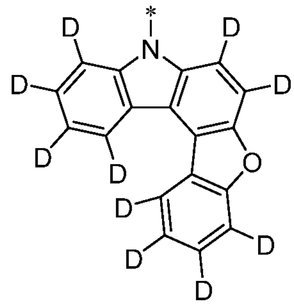

D203

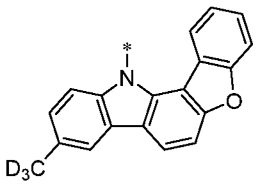

D204

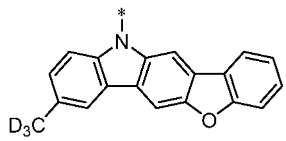

D205

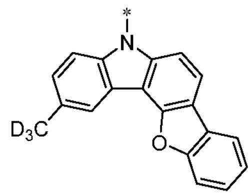

D206

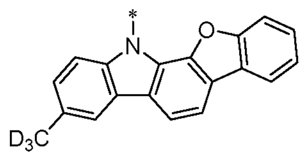

D207

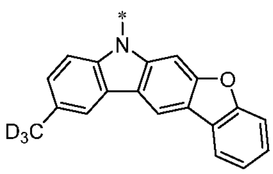

D208

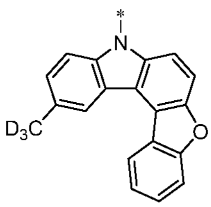

D209

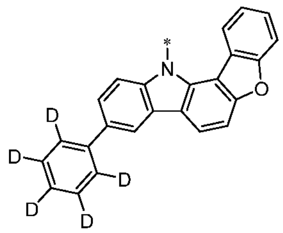

D210

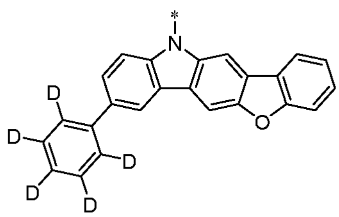

D211

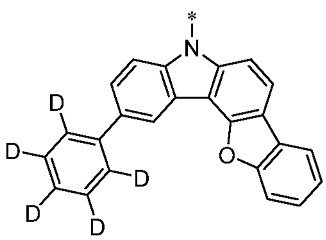

D212

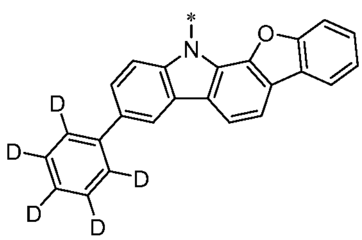

D213

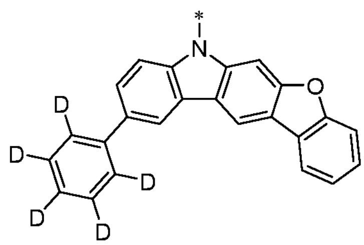

D214

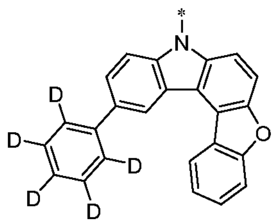

D215

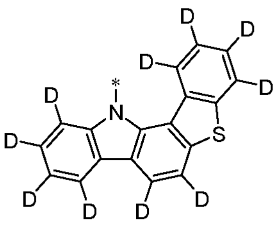

D216

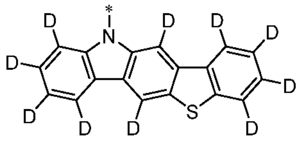

D217

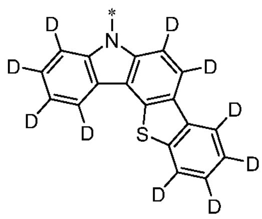

D218

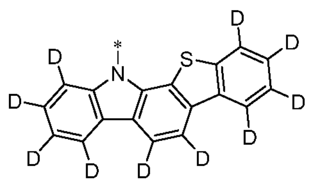

D219

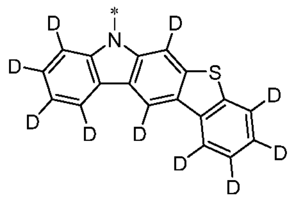

D220

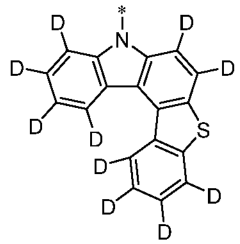

D221

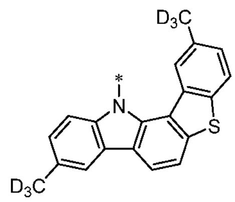

D222

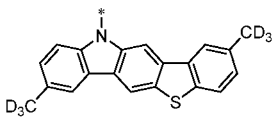

D223

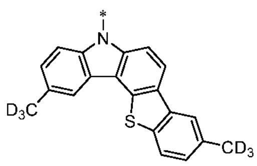

D224

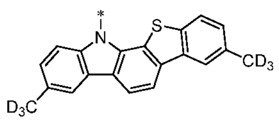

D225

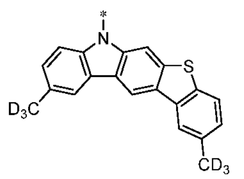

D226

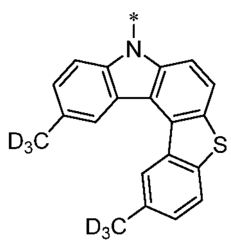

D227

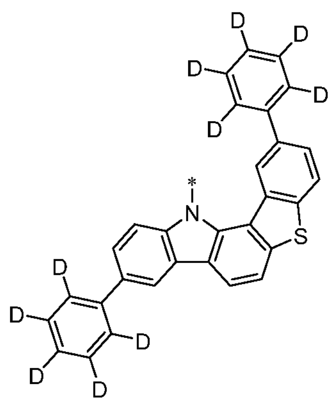

D228

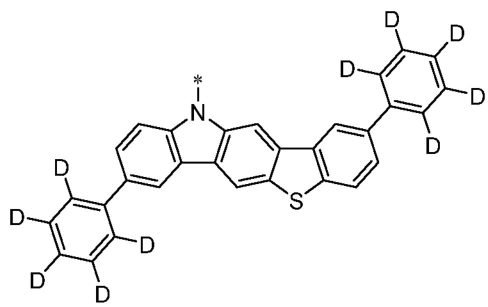

D229

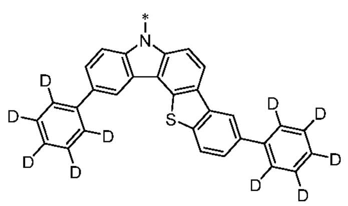

D230

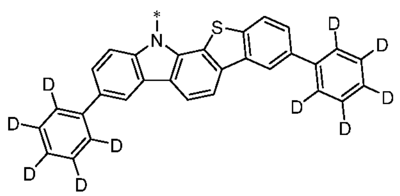

D231

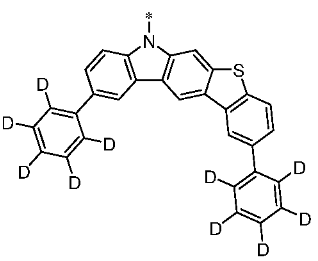

D232

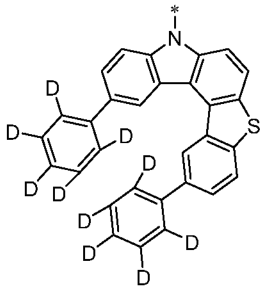
D233

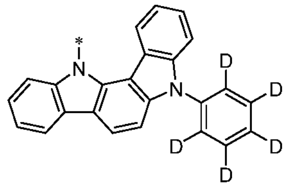
D234

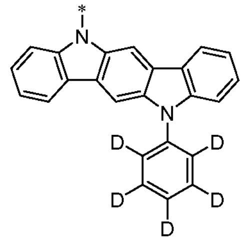
D235

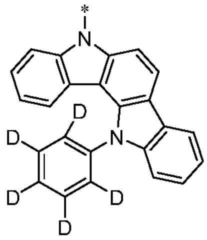
D236

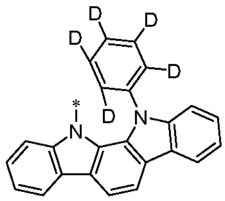
D237

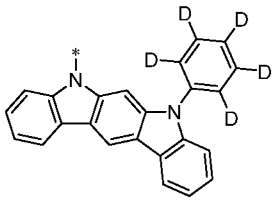
D238

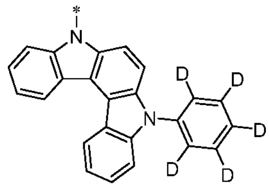
D239

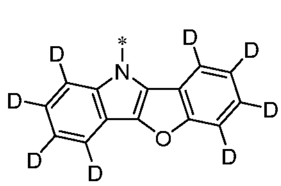
D240

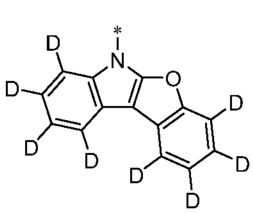
D241

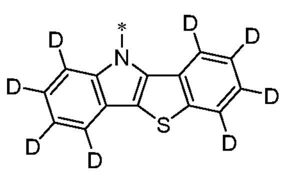
D242

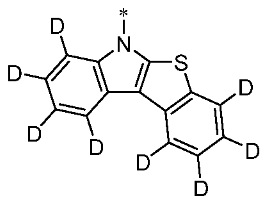
D243

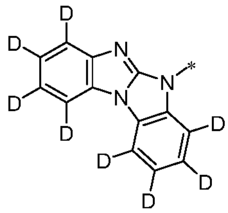
D244

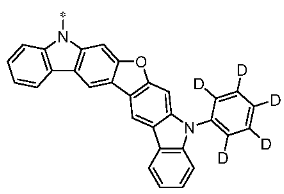
D245

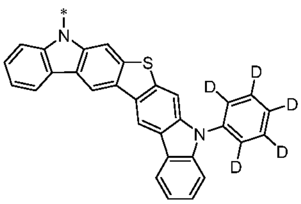
D246

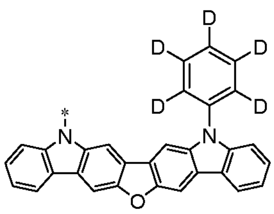
D247

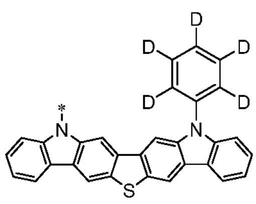
D248

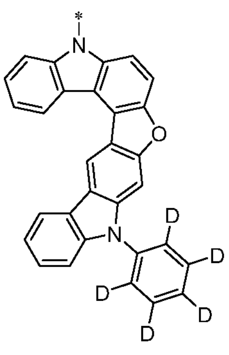
D249

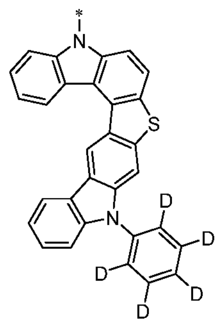
D250

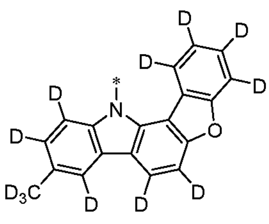
D251

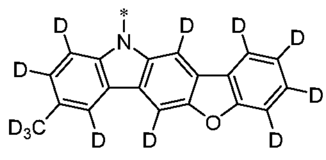
D252

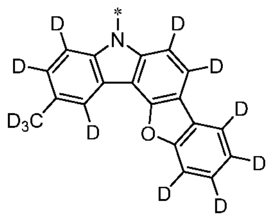
D253

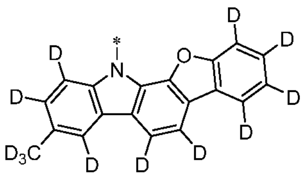
D254

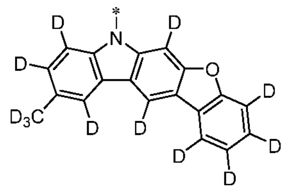
D255

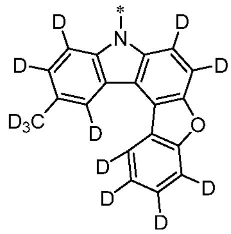
D256

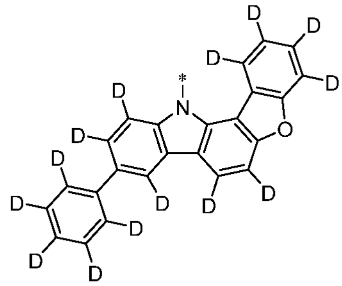
D257

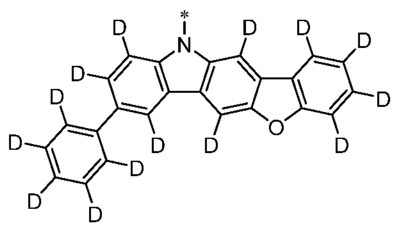
D258

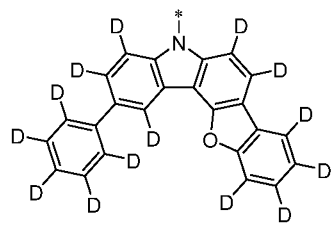
D259

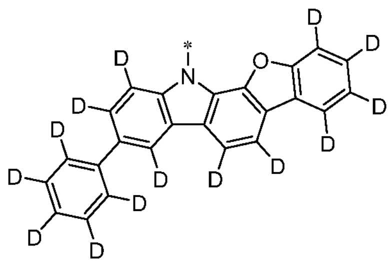
D260

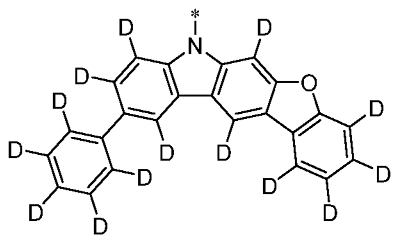
D261

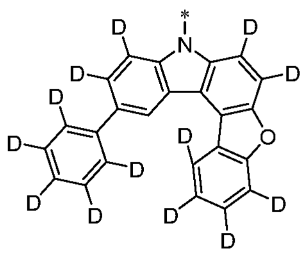
D262

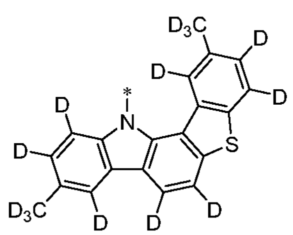
D263

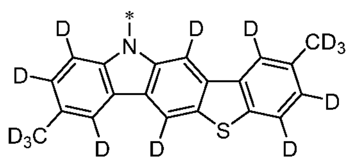
D264

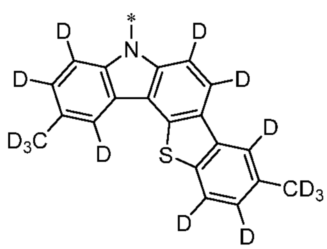
D265

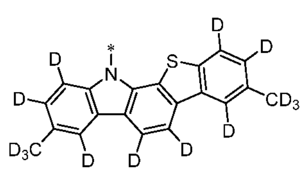
D266

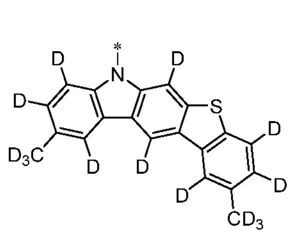
D267

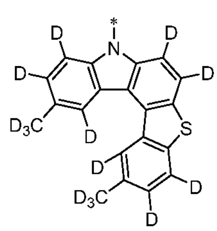
D268

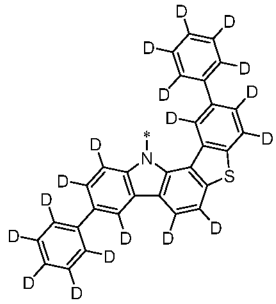
D269

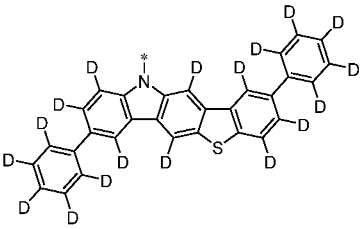

**D270**

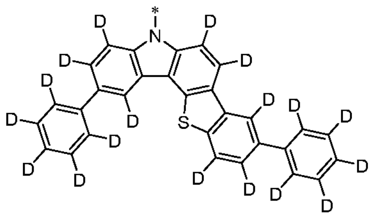

**D271**

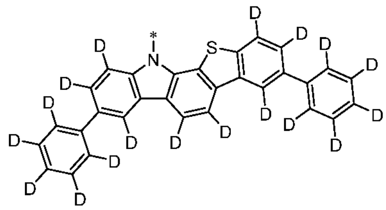

**D272**

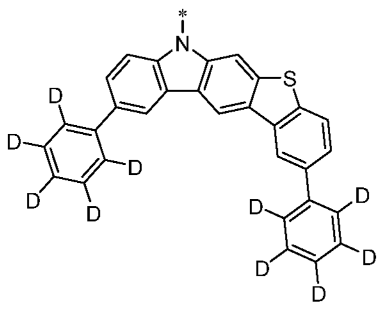

**D273**

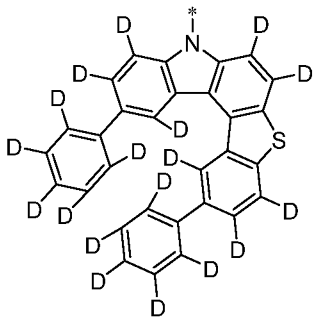

**D274**

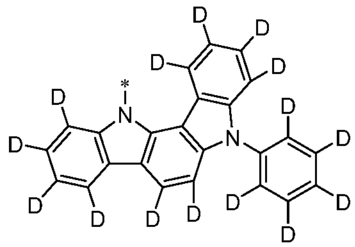

**D275**

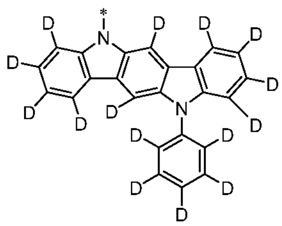

**D276**

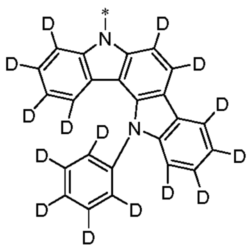

**D277**

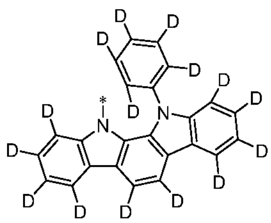

**D278**

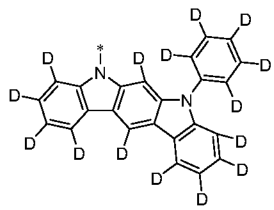

**D279**

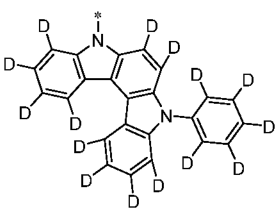

**D280**

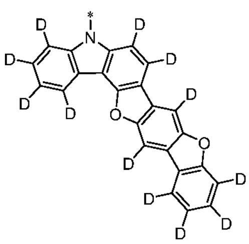

**D281**

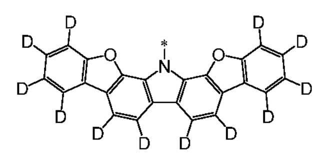

**D282**

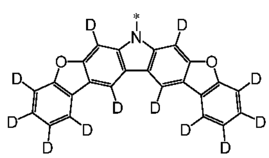

**D283**

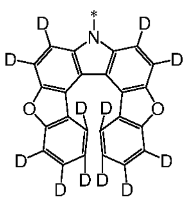

**D284**

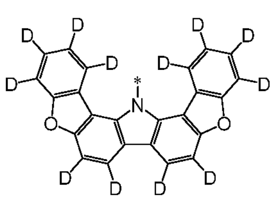

**D285**

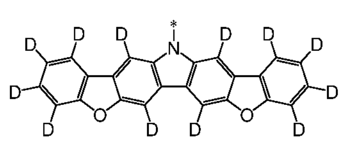

**D286**

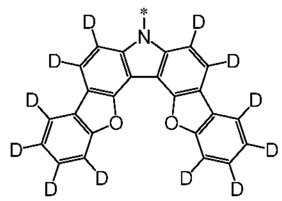

**D287**

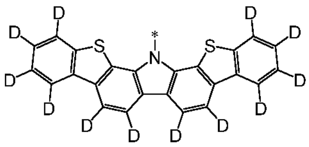
D288

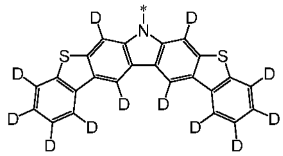
D289

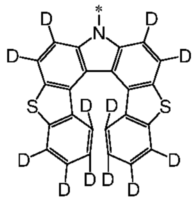
D290

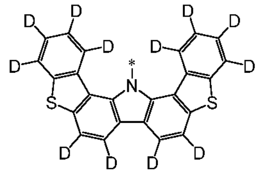
D291

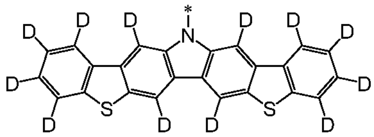
D292

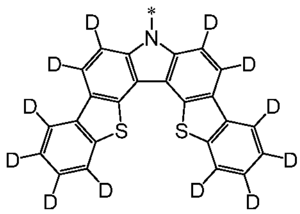
D293

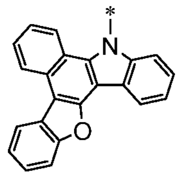
D294

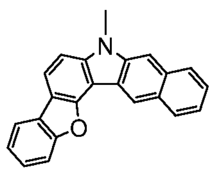
D295

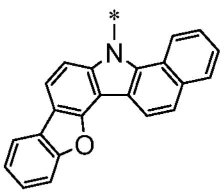
D296

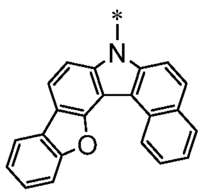
D297

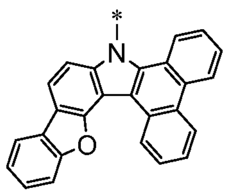
D298

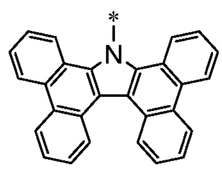
D299

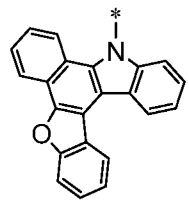
D300

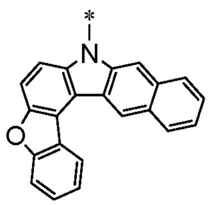
D301

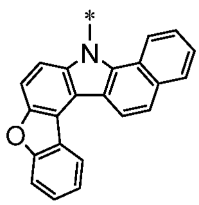
D302

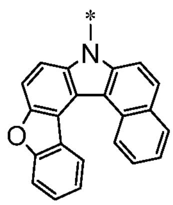
D303

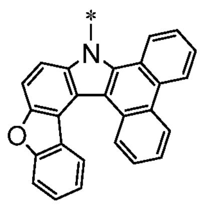
D304

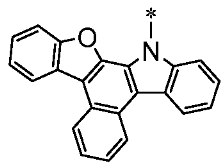
D305

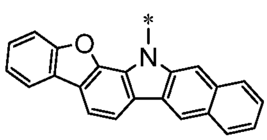
D306

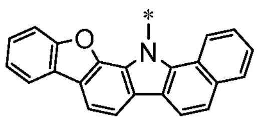
D307

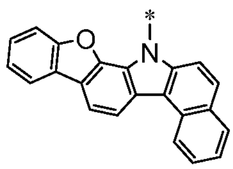
D308

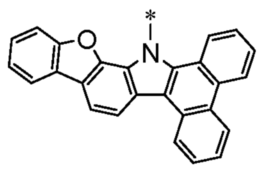
D309

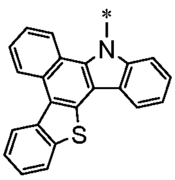
D310

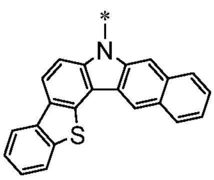
D311

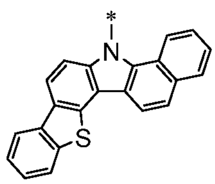
D312

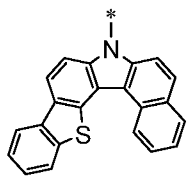
D313

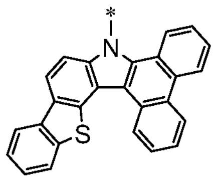
D314

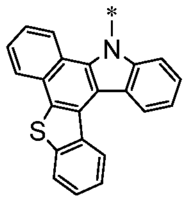
D315

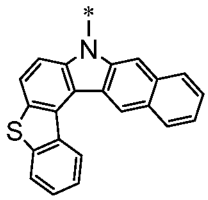
D316

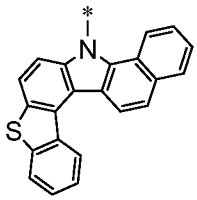
D317

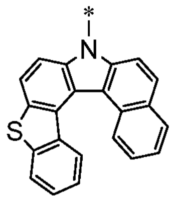
D318

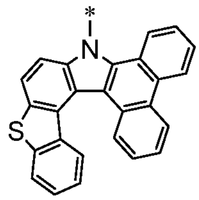
D319

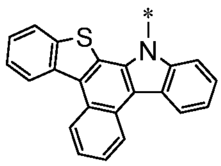
D320

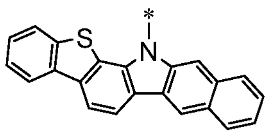
D321

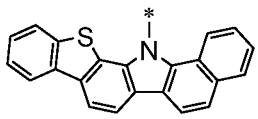
D322

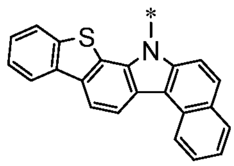
D323

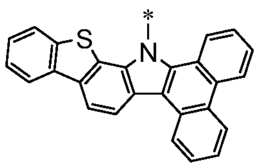
D324

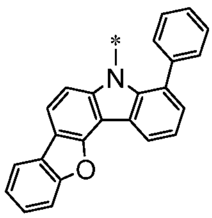
D325

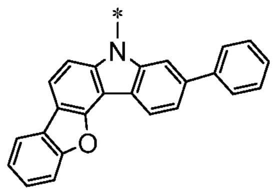
D326

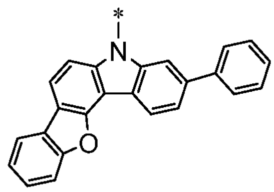
D327

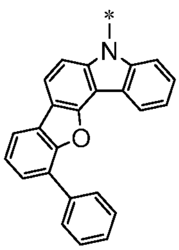
D328

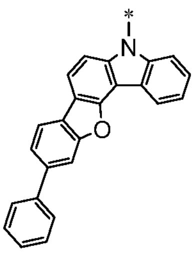
D329

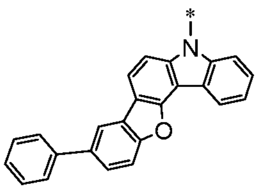
D330

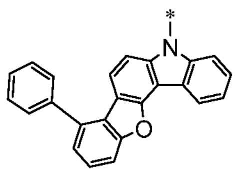
D331

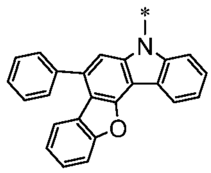
D332

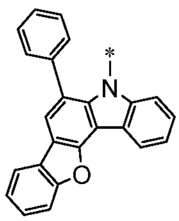
D333

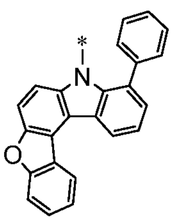
D334

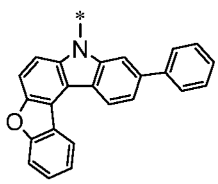
D335

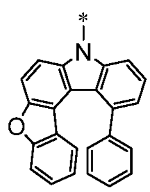
D336

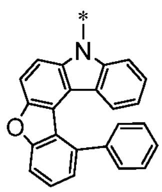
D337

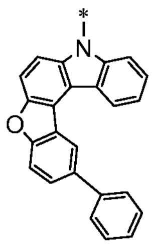
D338

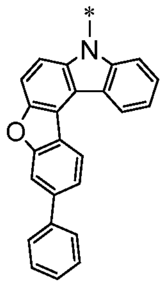
D339

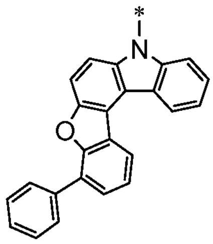
D340

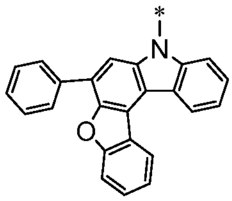
D341

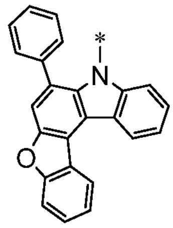
D342

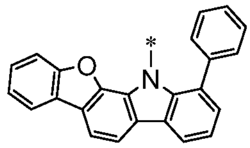
D343

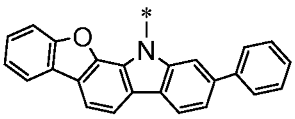
D344

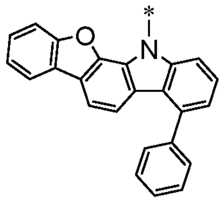
D345

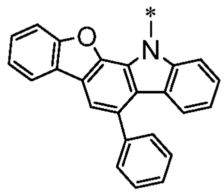
D346

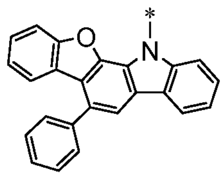
D347

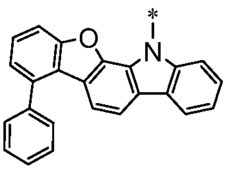
D348

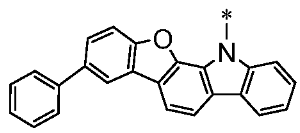
D349

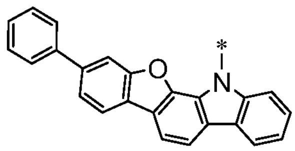
D350

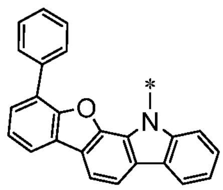
D351

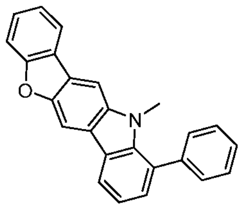
D352

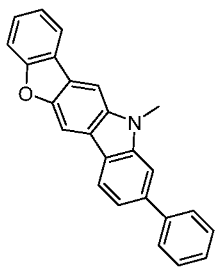
D353

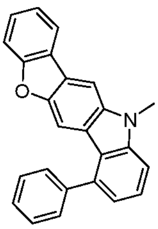
D354

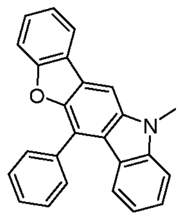
D355

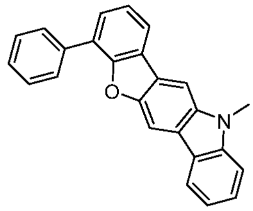
D356

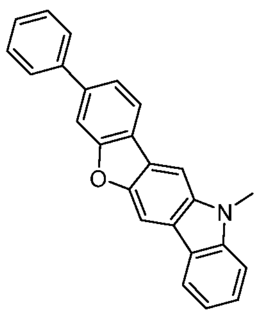
D357

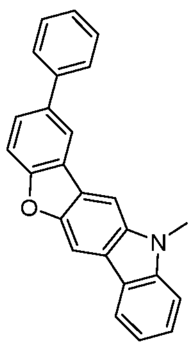
D358

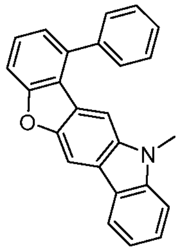
D359

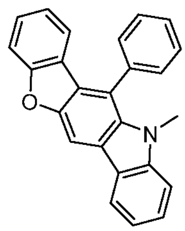
D360

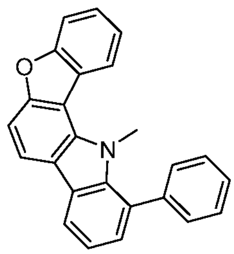
D361

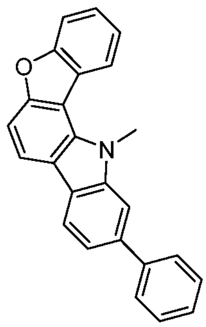
D362
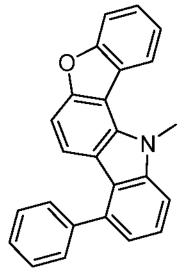
D363
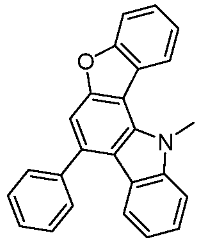
D364
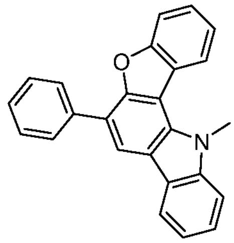
D365
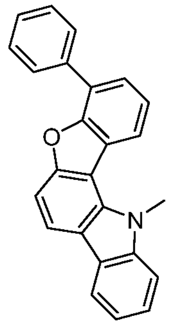
D366

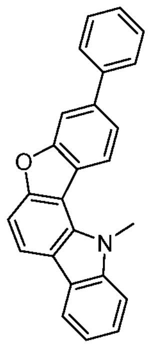
D367
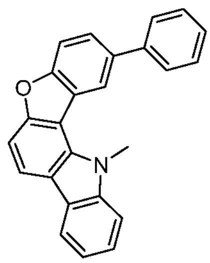
D368
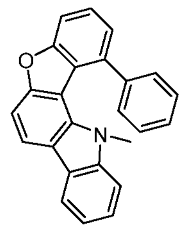
D369
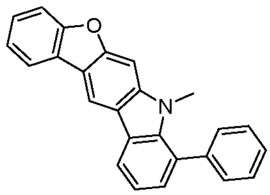
D370
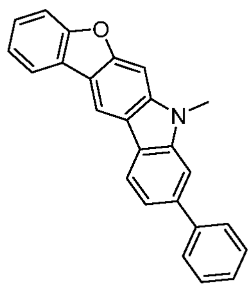
D371

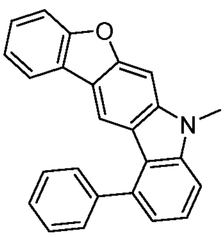
D372
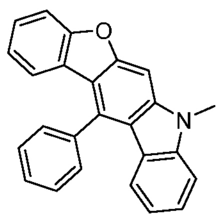
D373
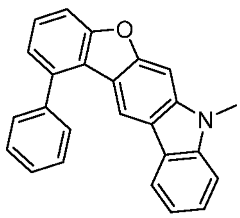
D374
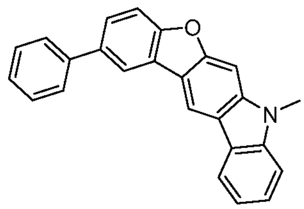
D375

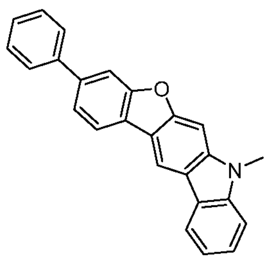
D376
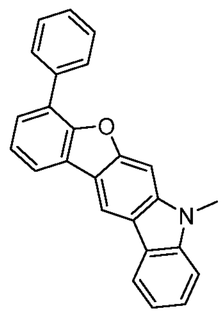
D377
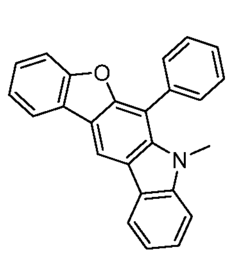
D378
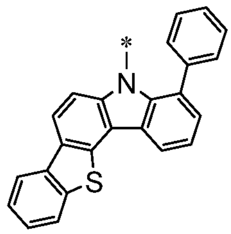
D379

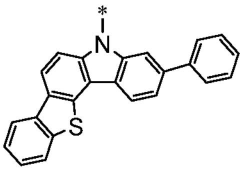
D380

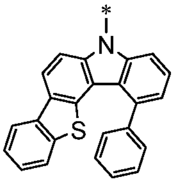
D381

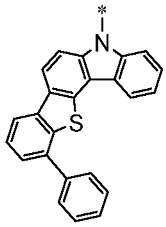
D382

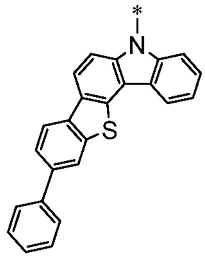
D383

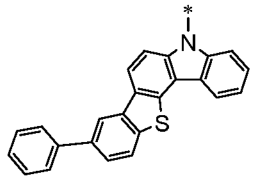
D384

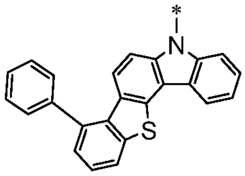
D385

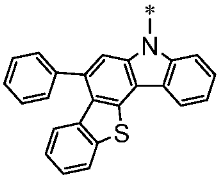
D386

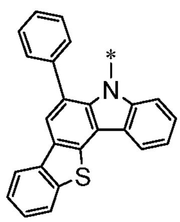
D387

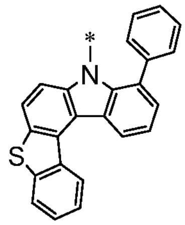
D388

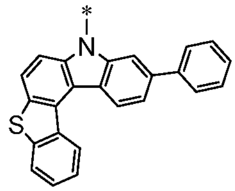
D389

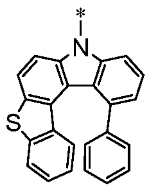
D390

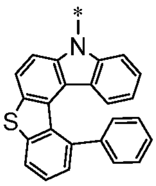
D391

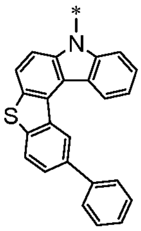
D392

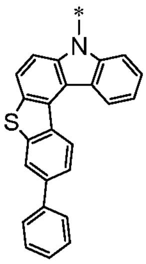
D393

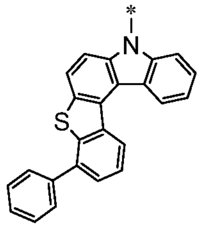
D394

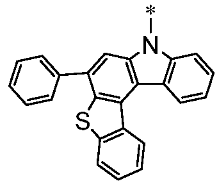
D395

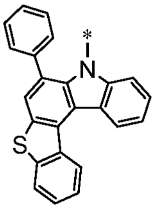
D396

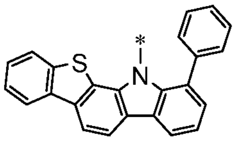
D397

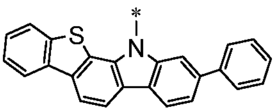
D398

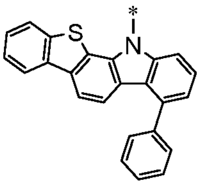
D399

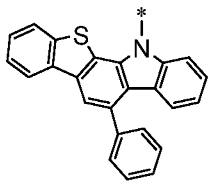
D400

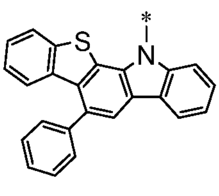
D401

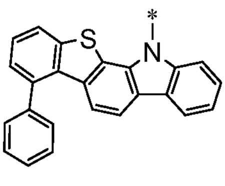
D402

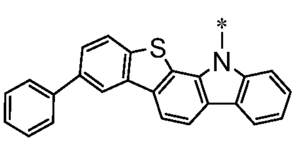
D403

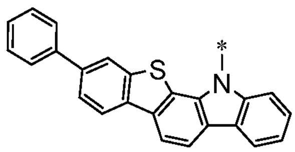
D404

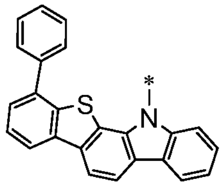

**D405**

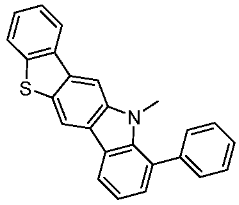

**D406**

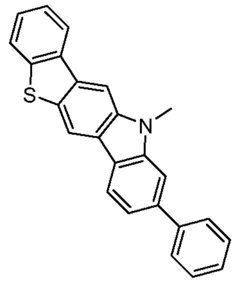

**D407**

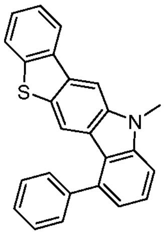

**D408**

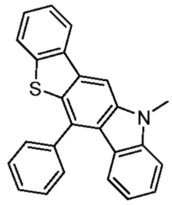

**D409**

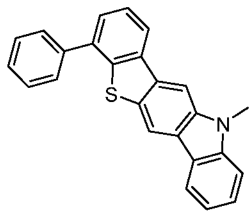

**D410**

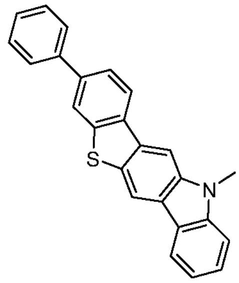

**D411**

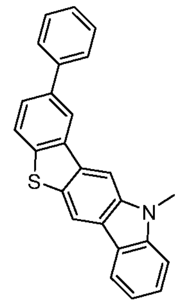

**D412**

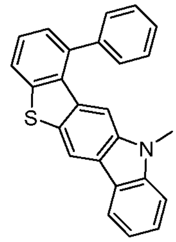

**D413**

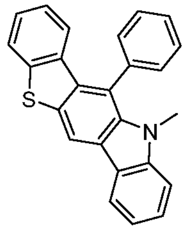

**D414**

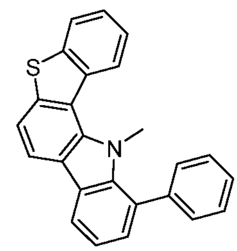

**D415**

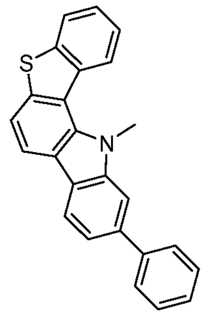

**D416**

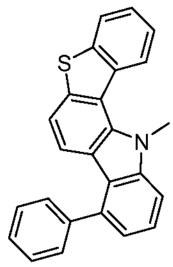

**D417**

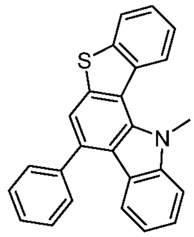

**D418**

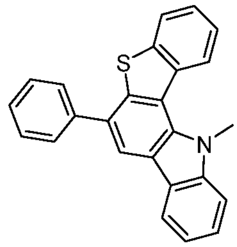

**D419**

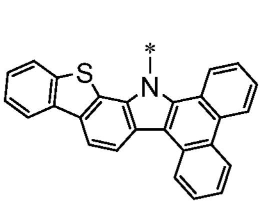

**D420**

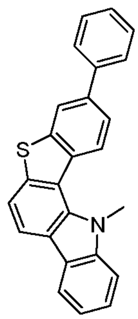

**D421**

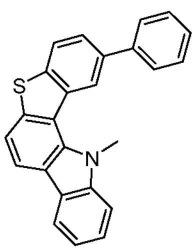

**D422**

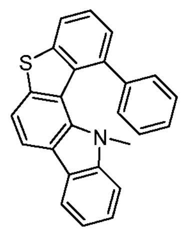

**D423**

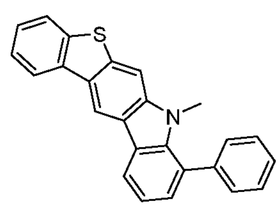

**D424**

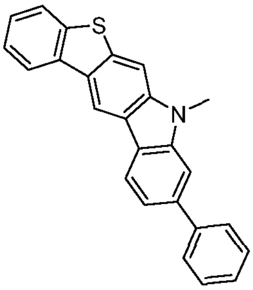
**D425**

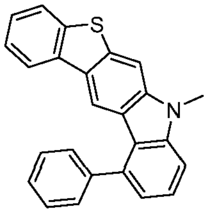
**D426**

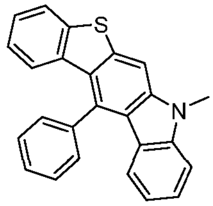
**D427**

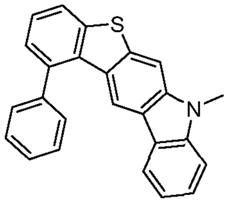
**D428**

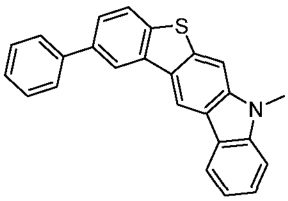
**D429**

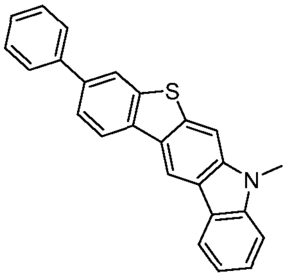
**D430**

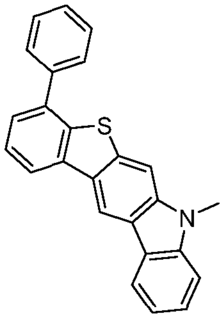
**D431**

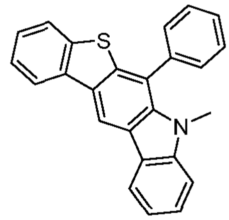
**D432**

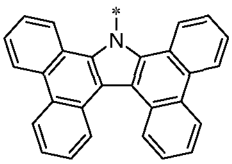
**D433**

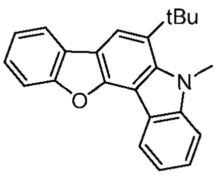
**D434**

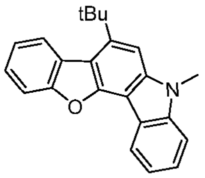
**D435**

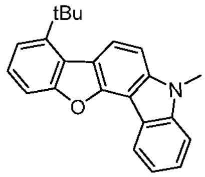
**D436**

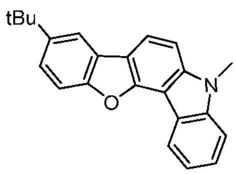
**D437**

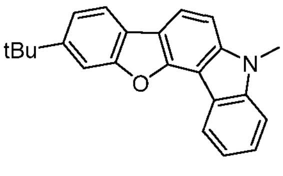
**D438**

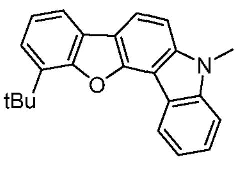
**D439**

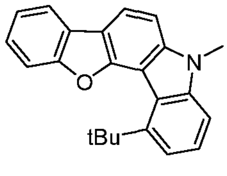
**D440**

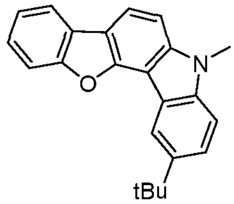
**D441**

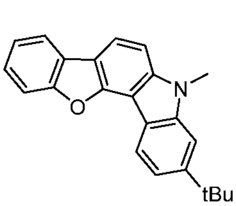
**D442**

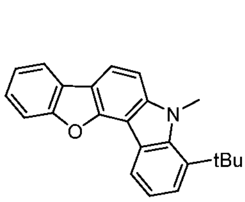
**D443**

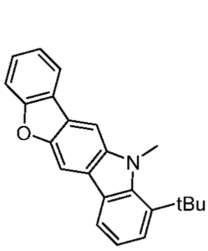
**D444**

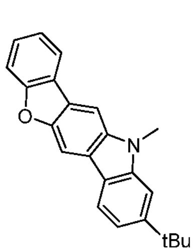
**D445**

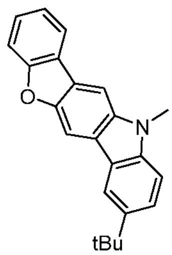
**D446**

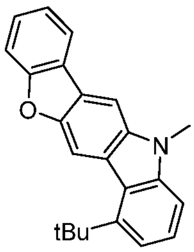
D447

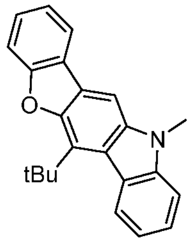
D448

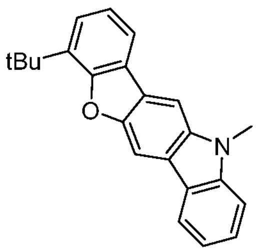
D449

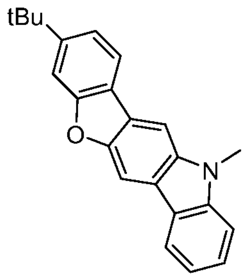
D450

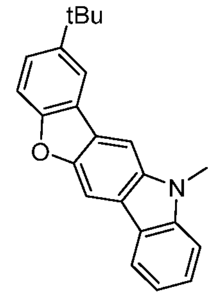
D451

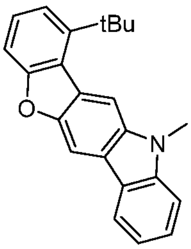
D452

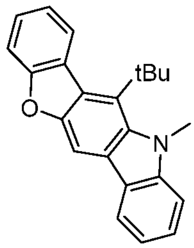
D453

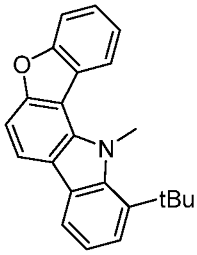
D454

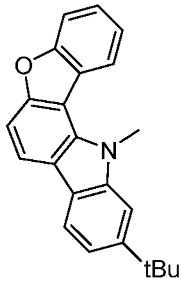
D455

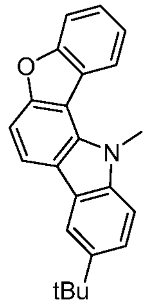
D456

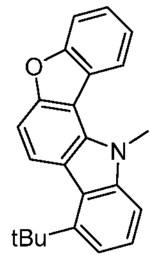
D457

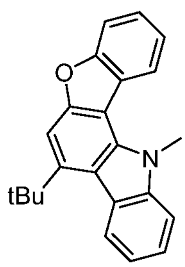
D458

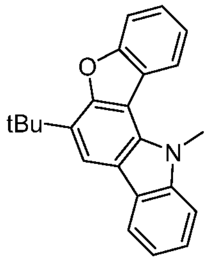
D459

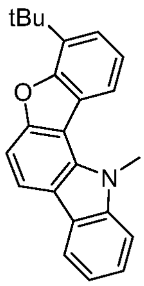
D460

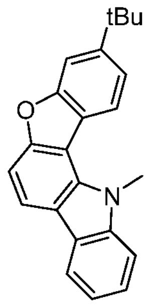
D461

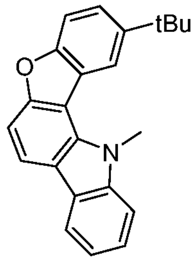
D462

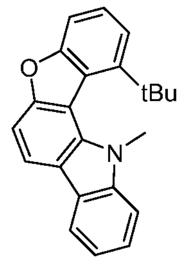
D463

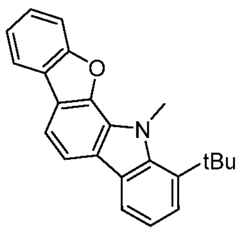
D464

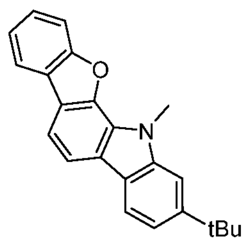
D465

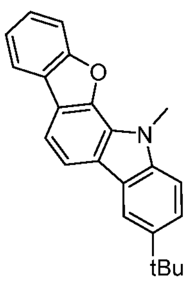
D466

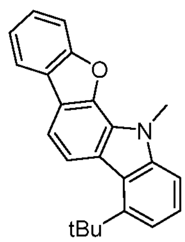
D467

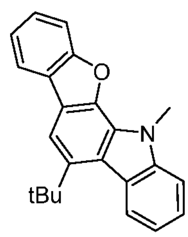
D468

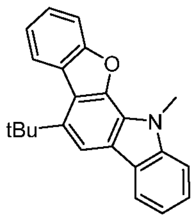
**D469**

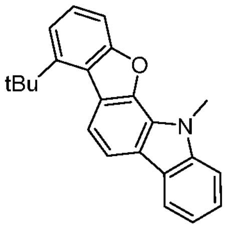
**D470**

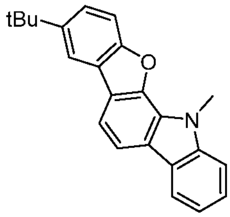
**D471**

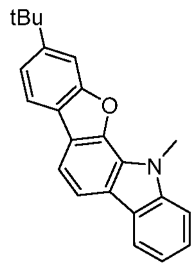
**D472**

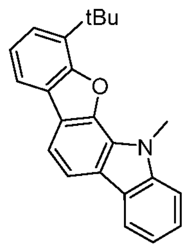
**D473**

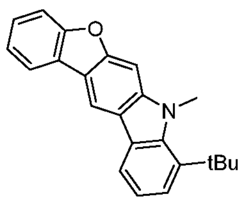
**D474**

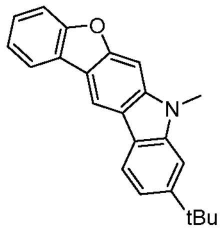
**D475**

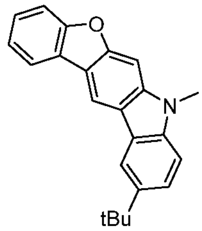
**D476**

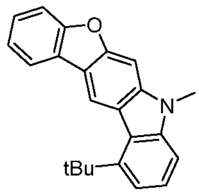
**D477**

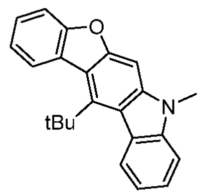
**D478**

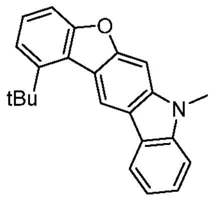
**D479**

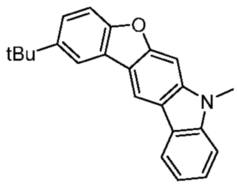
**D480**

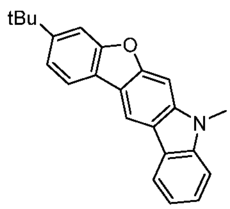
**D481**

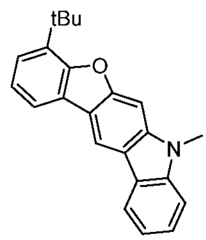
**D482**

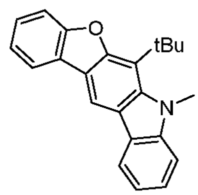
**D483**

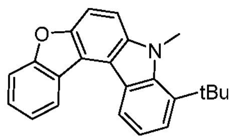
**D484**

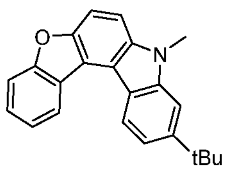
**D485**

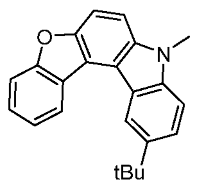
**D486**

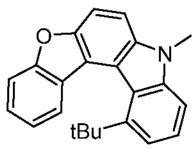
**D487**

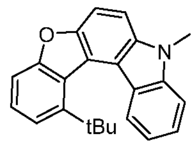
**D488**

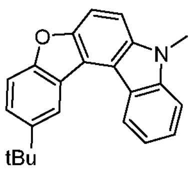
**D489**

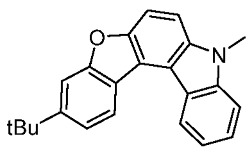
**D490**

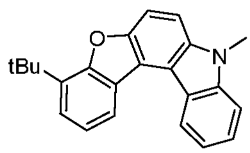
**D491**

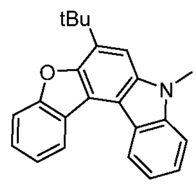
**D492**

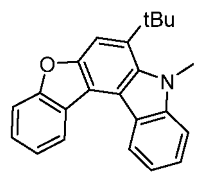
**D493**

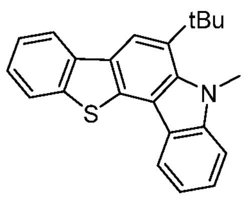
**D494**

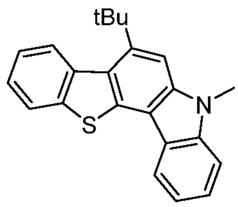
**D495**

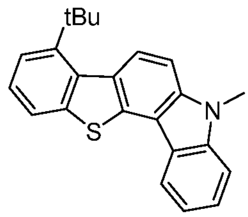
**D496**

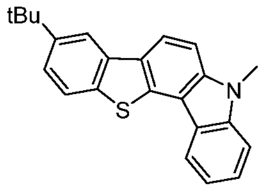
**D497**

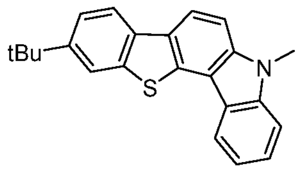
**D498**

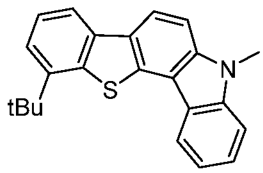
**D499**

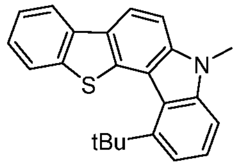
**D500**

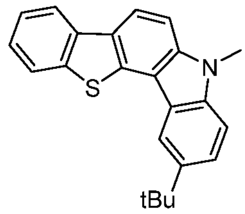
**D501**

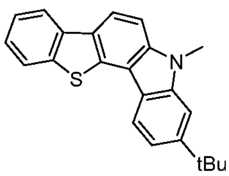
**D502**

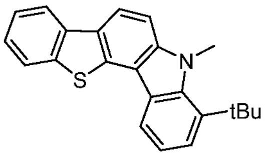
**D503**

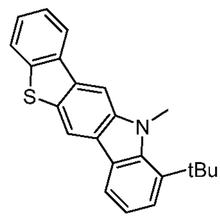
**D504**

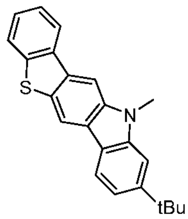
**D505**

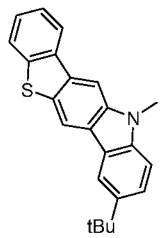
**D506**

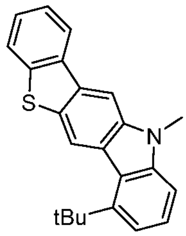
**D507**

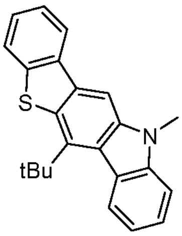
**D508**

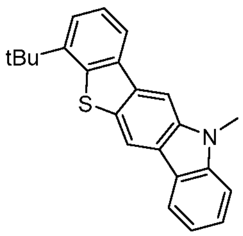
**D509**

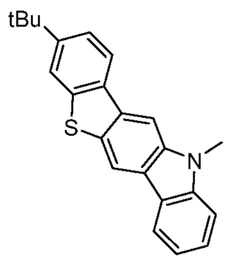
**D510**

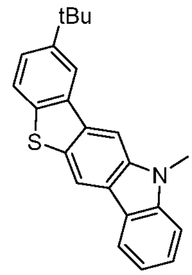
**D511**

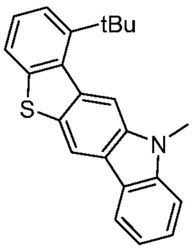
**D512**

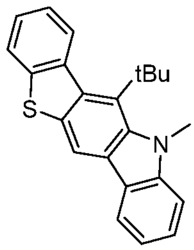
**D513**

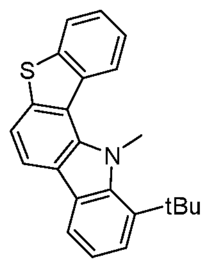
**D514**

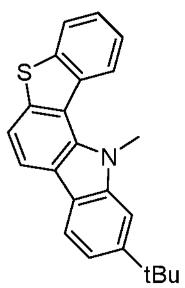
**D515**

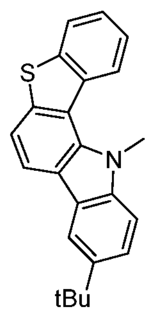
**D516**

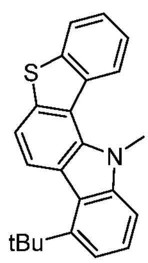
**D517**

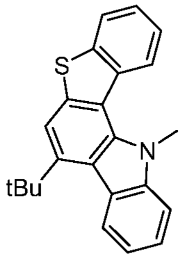

**D518**

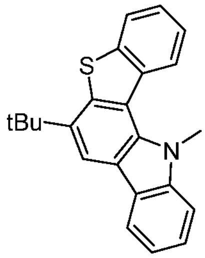

**D519**

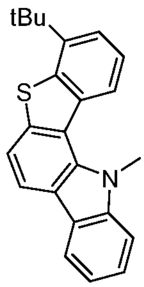

**D520**

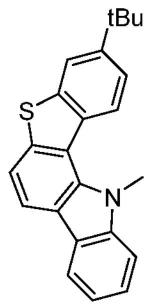

**D521**

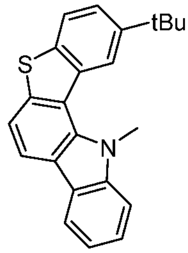

**D522**

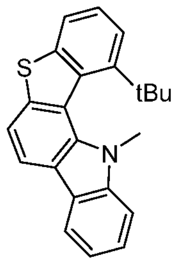

**D523**

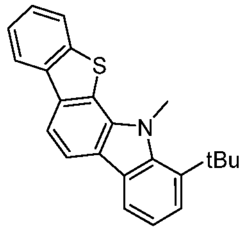

**D524**

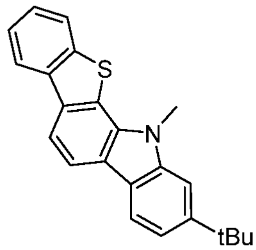

**D525**

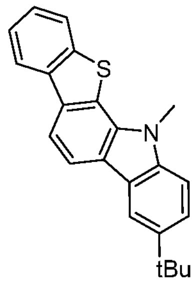

**D526**

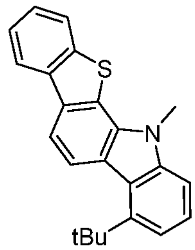

**D527**

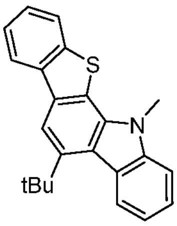

**D528**

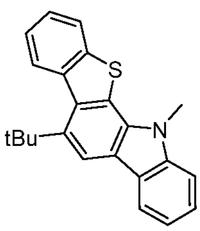

**D529**

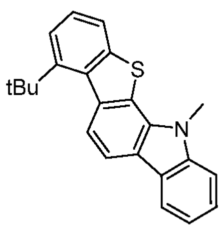

**D530**

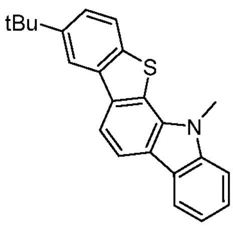

**D531**

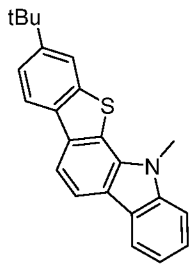

**D532**

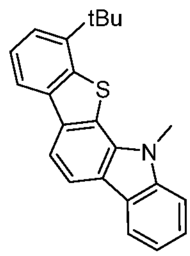

**D533**

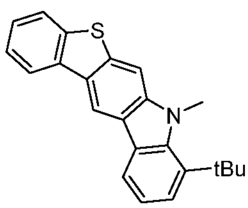

**D534**

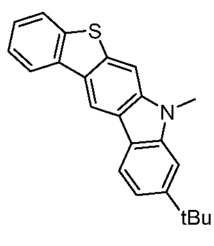

**D535**

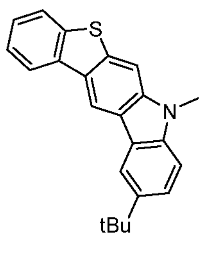

**D536**

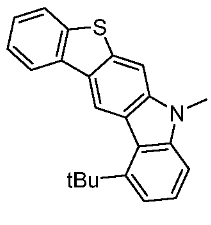

**D537**

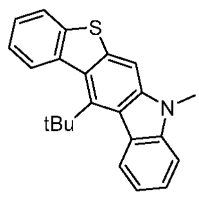

**D538**

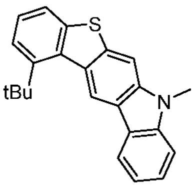
**D539**

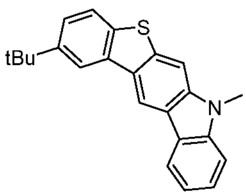
**D540**

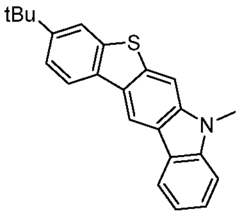
**D541**

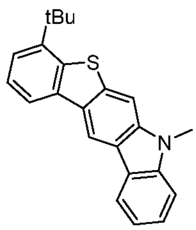
**D542**

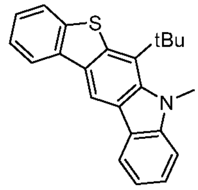
**D543**

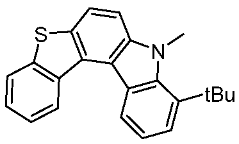
**D544**

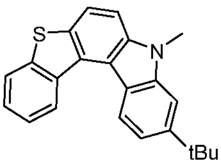
**D545**

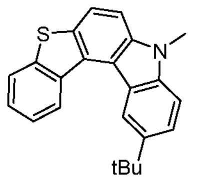
**D546**

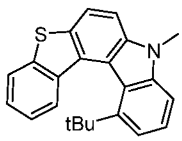
**D547**

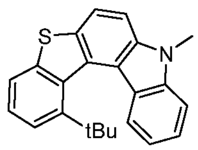
**D548**

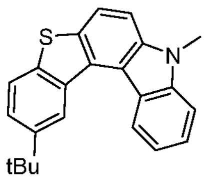
**D549**

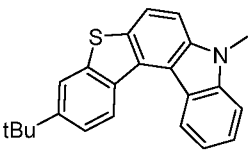
**D550**

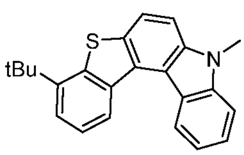
**D551**

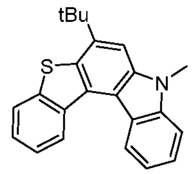
**D552**

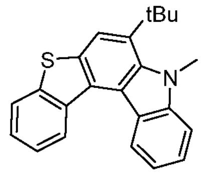
**D553**

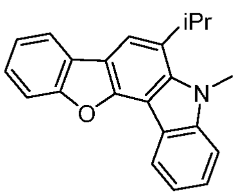
**D554**

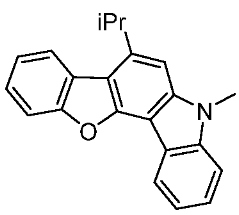
**D555**

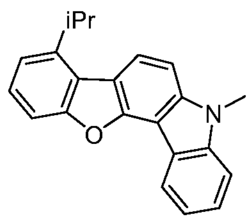
**D556**

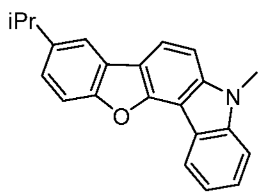
**D557**

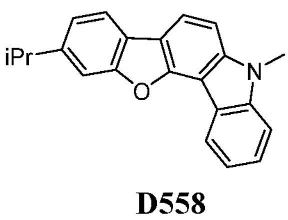
**D558**

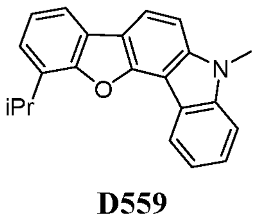
**D559**

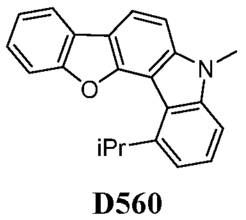
**D560**

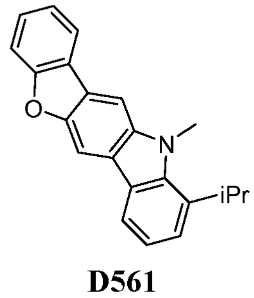
**D561**

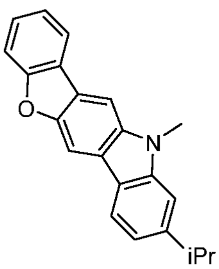
**D562**

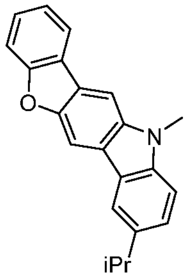
**D563**

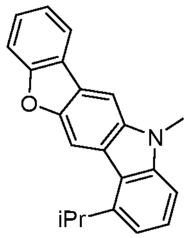
**D564**

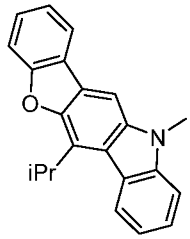
**D565**

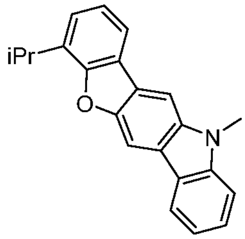
**D566**

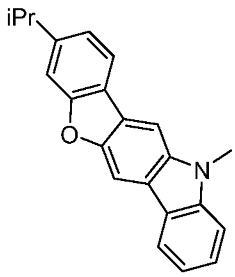
**D567**

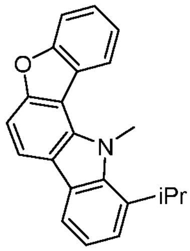
**D568**

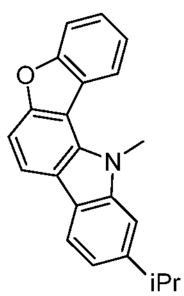
**D569**

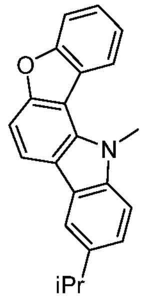
**D570**

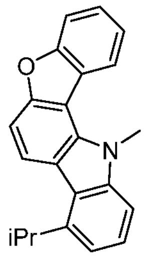
**D571**

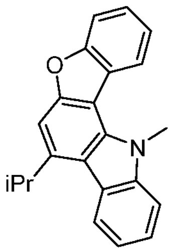
**D572**

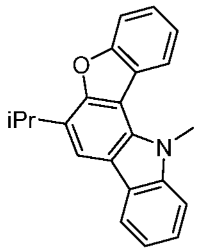
**D573**

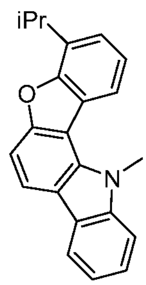
**D574**

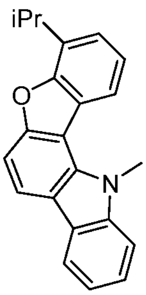
**D575**

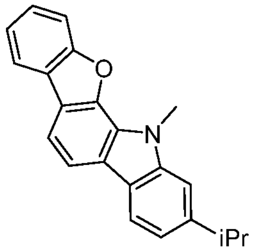
**D576**

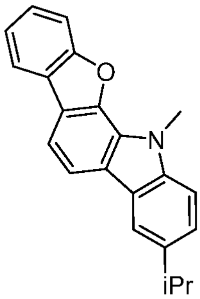
**D577**

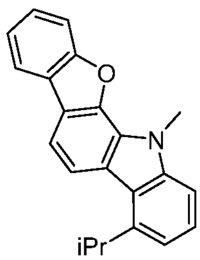
**D578**

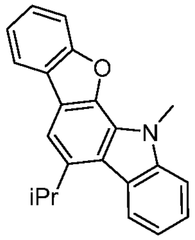
**D579**

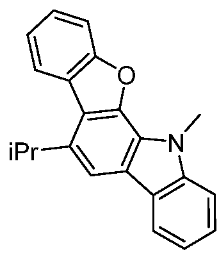
**D580**

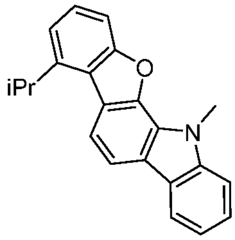
**D581**

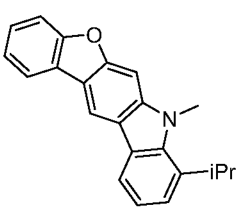
D582

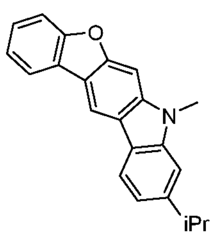
D583

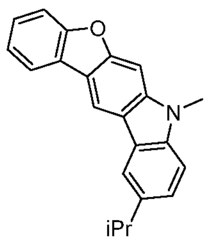
D584

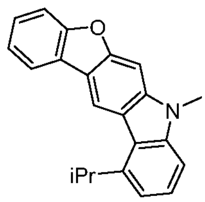
D585

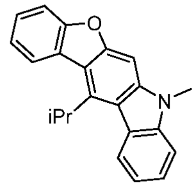
D586

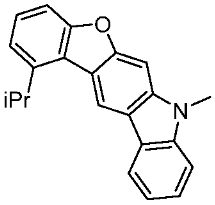
D587

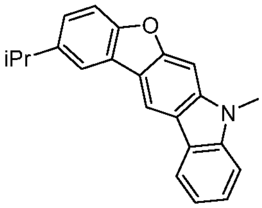
D588

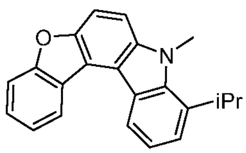
D589

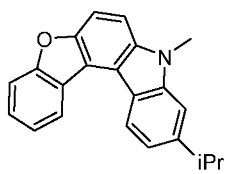
D590

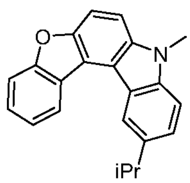
D591

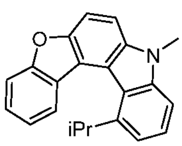
D592

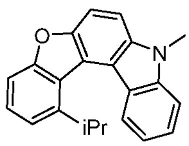
D593

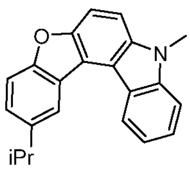
D594

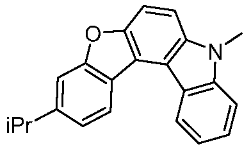
D595

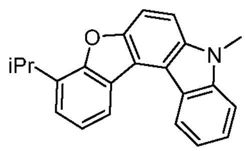
D596

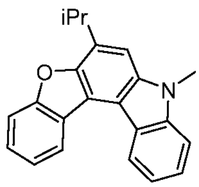
D597

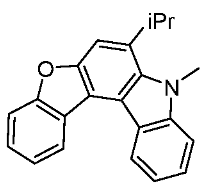
D598

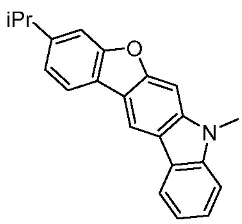
D599

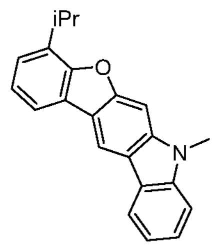
D600

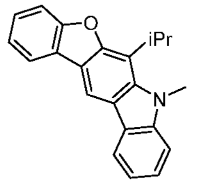
D601

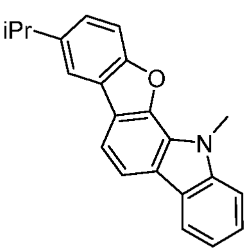
D602

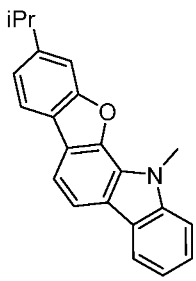
D603

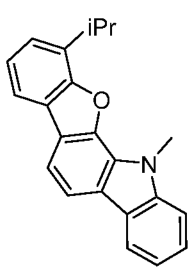
D604

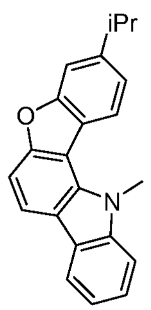
D605

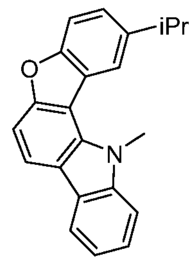
D606

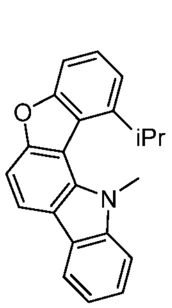
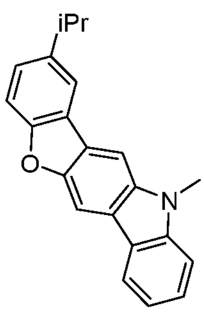
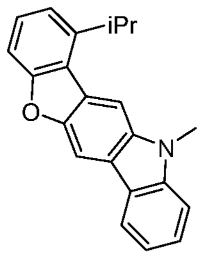
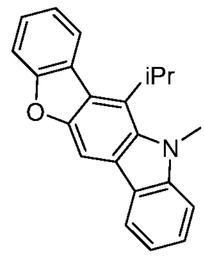
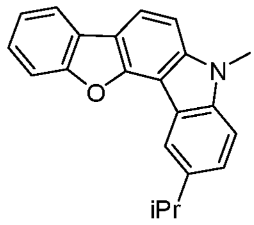

D607 D608 D609 D610 D611

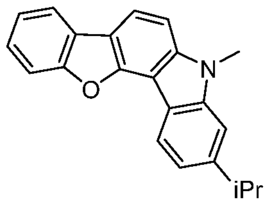
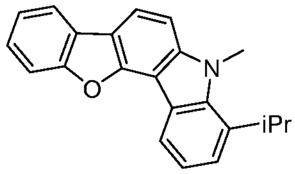
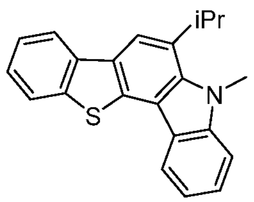
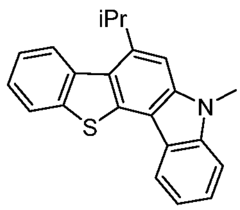

D612 D613 D614 D615

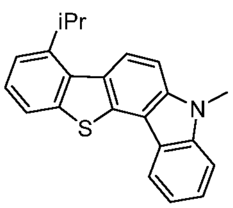
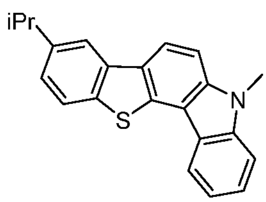
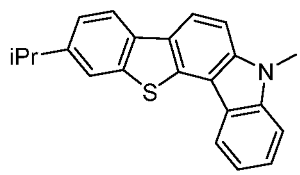
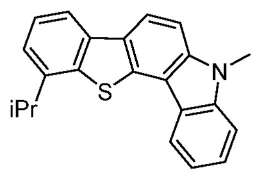

D616 D617 D618 D619

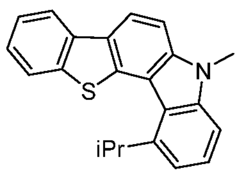
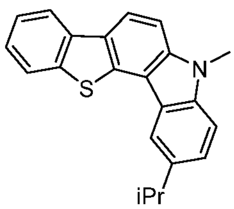
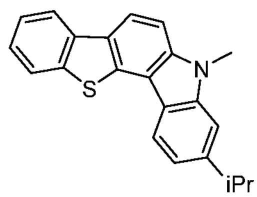
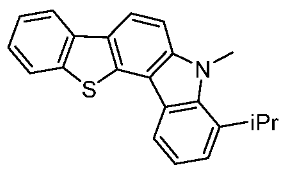

D620 D621 D622 D623

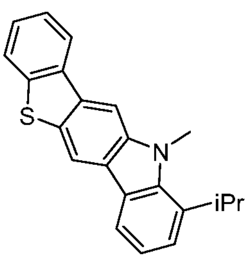
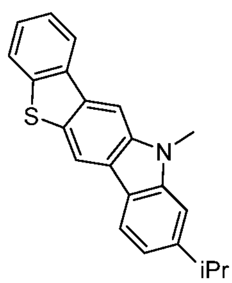
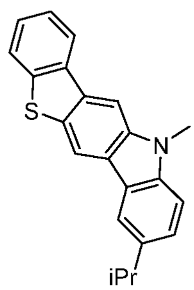
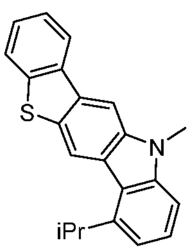
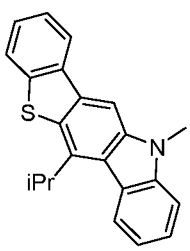

D624 D625 D626 D627 D628

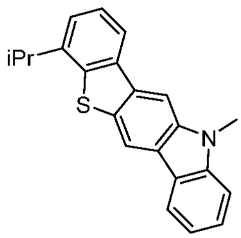
D629

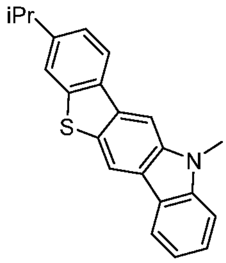
D630

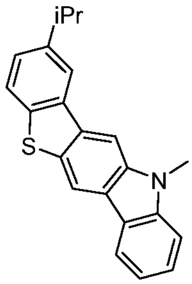
D631

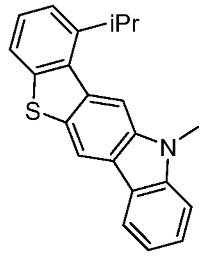
D632

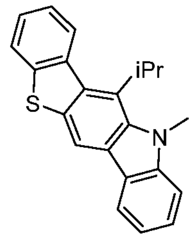
D633

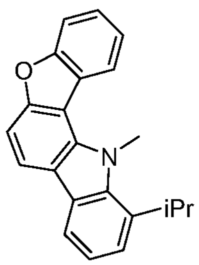
D634

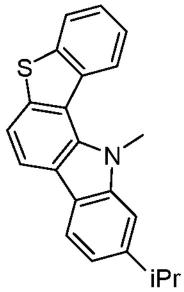
D635

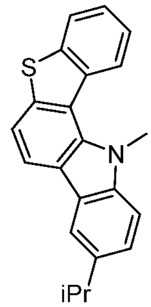
D636

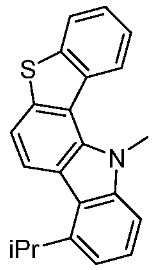
D637

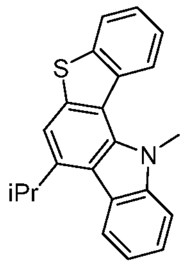
D638

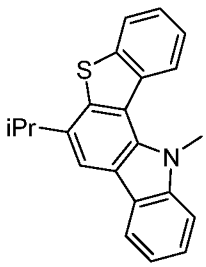
D639

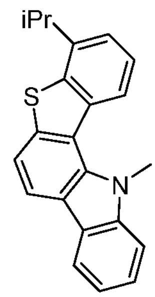
D640

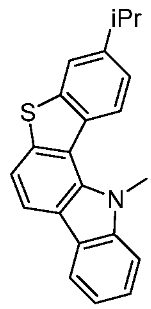
D641

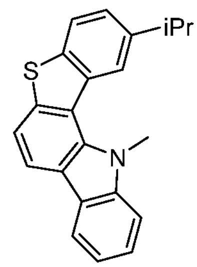
D642

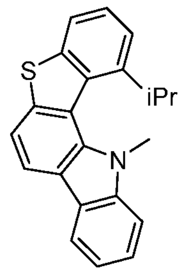
D643

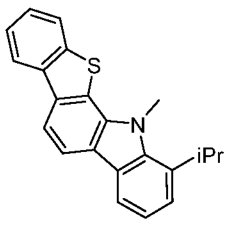
D644

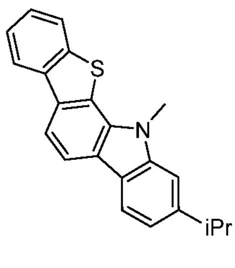
D645

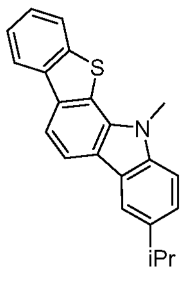
D646

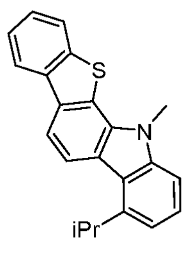
D647

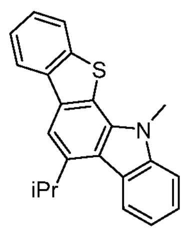
D648

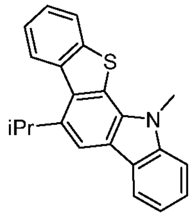
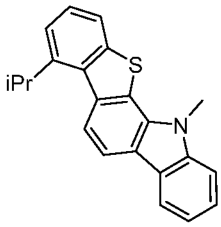
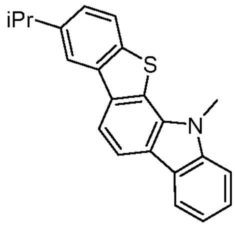
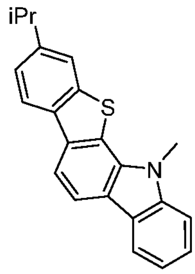
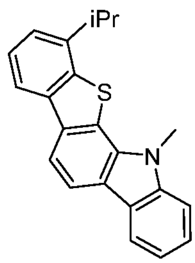

D649 D650 D651 D652 D653

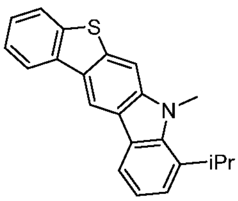
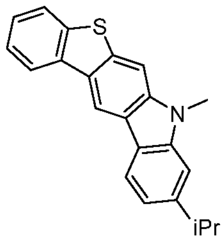
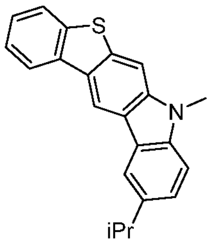
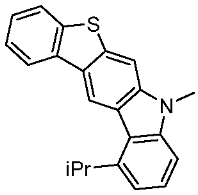
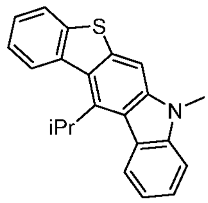

D654 D655 D656 D657 D658

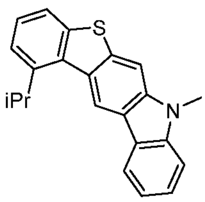
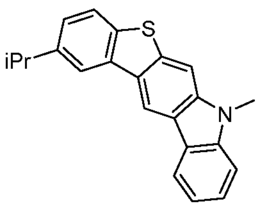
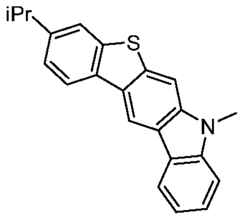
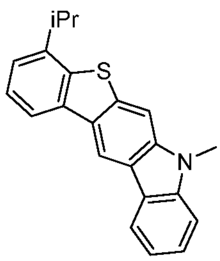
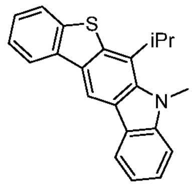

D659 D660 D661 D662 D663

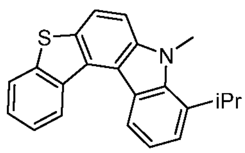
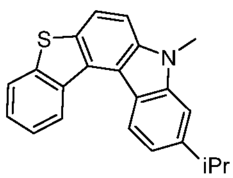
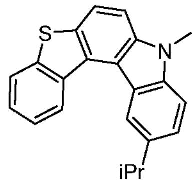
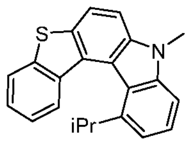
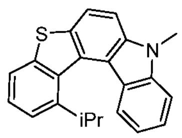

D664 D665 D666 D667 D668

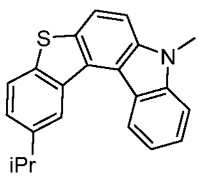
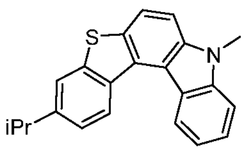
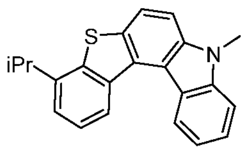
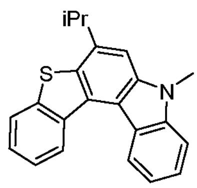
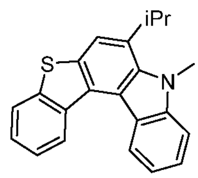

D669 D670 D671 D672 D673

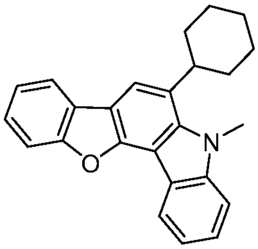
D674

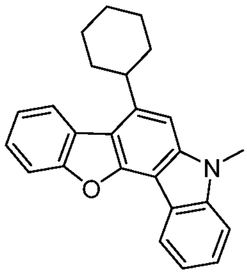
D675

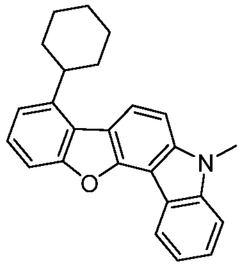
D676

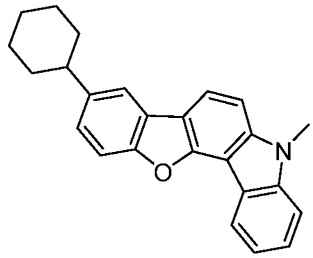
D677

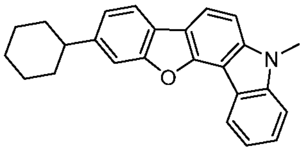
D678

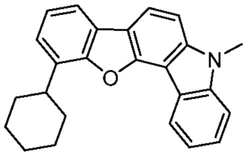
D679

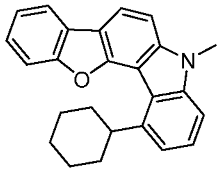
D680

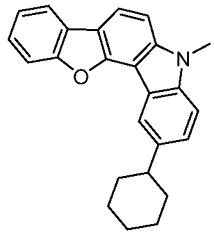
D681

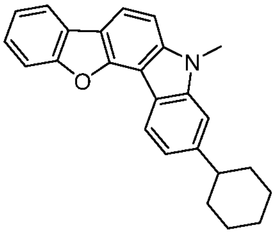
D682

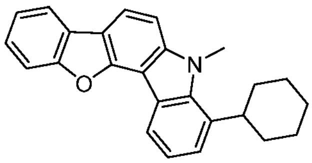
D683

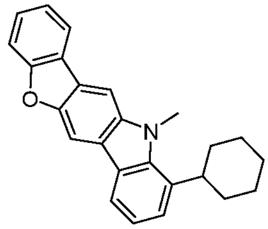
D684

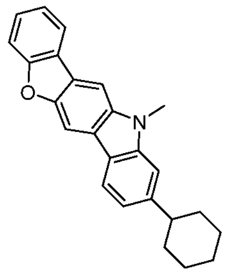
D685

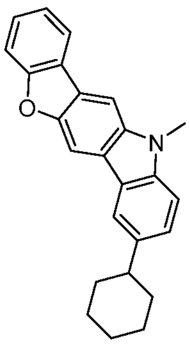
D686

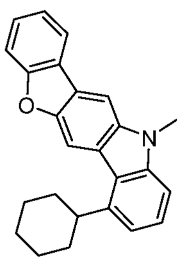
D687

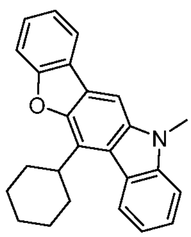
D688

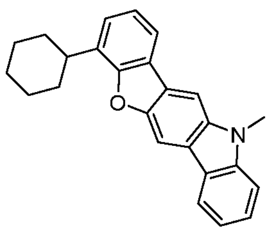
D689

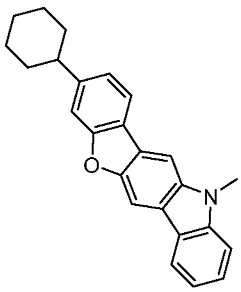
D690

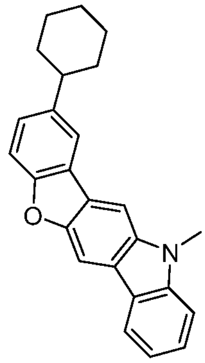
**D691**

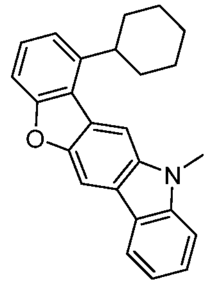
**D692**

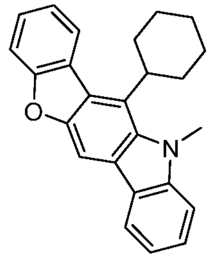
**D693**

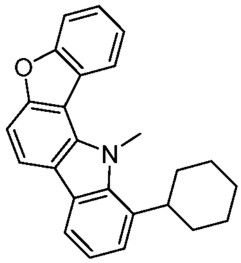
**D694**

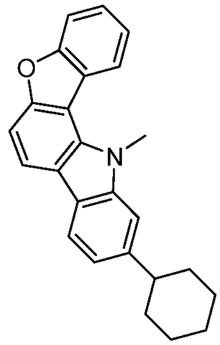
**D695**

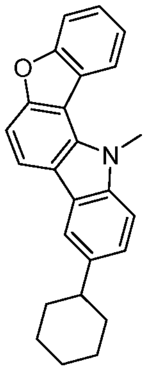
**D696**

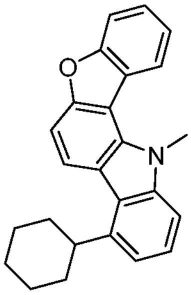
**D697**

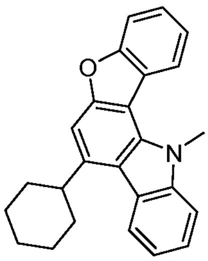
**D698**

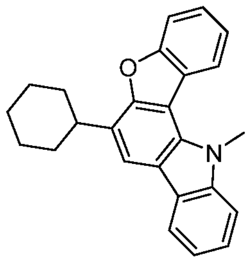
**D699**

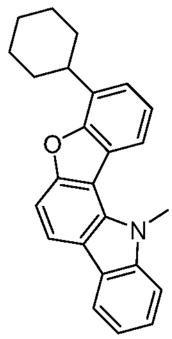
**D700**

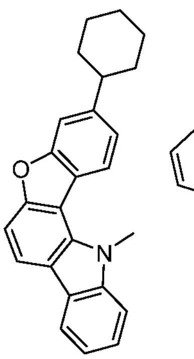
**D701**

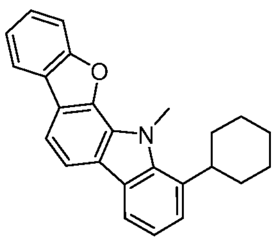
**D702**

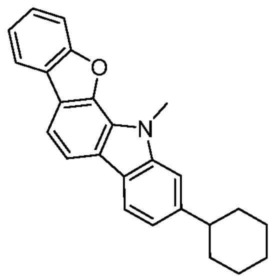
**D703**

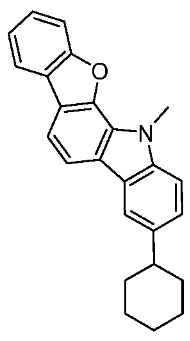
**D704**

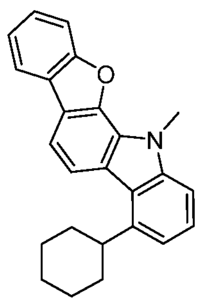
**D705**

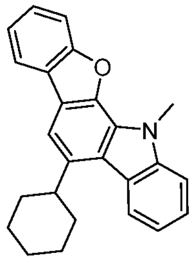
**D706**

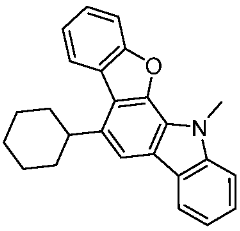
**D707**

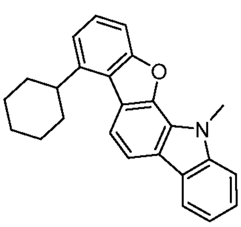
**D708**

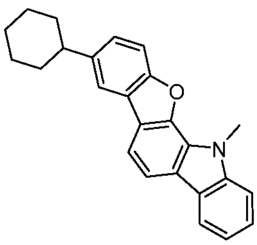
**D709**

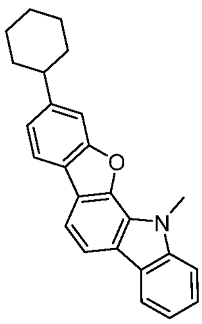
**D710**

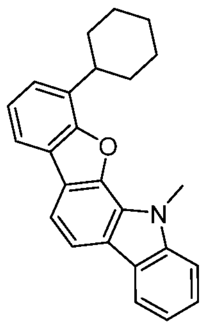
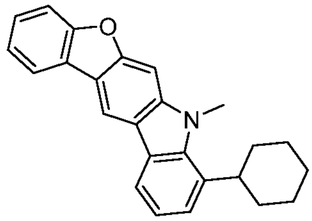
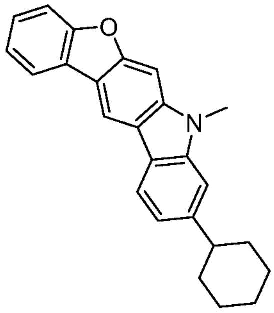
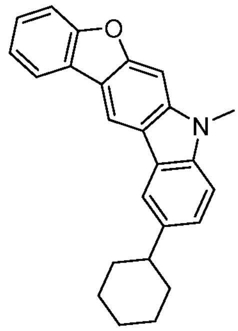

**D711** **D712** **D713** **D714**

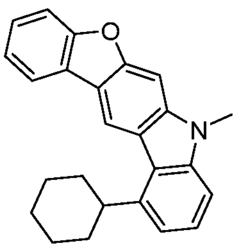
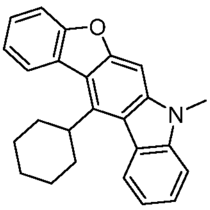
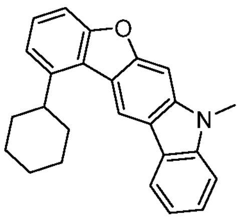
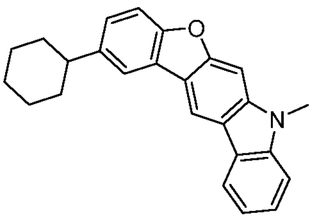

**D715** **D716** **D717** **D718**

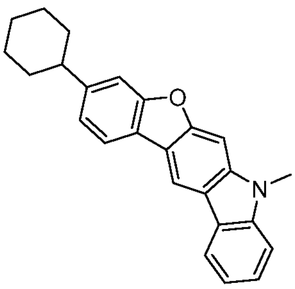
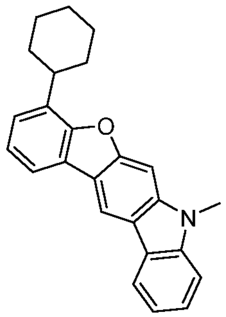
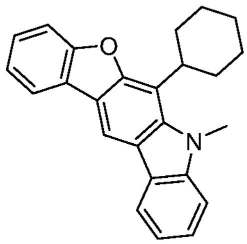
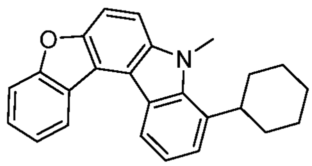

**D719** **D720** **D721** **D722**

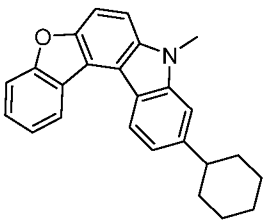
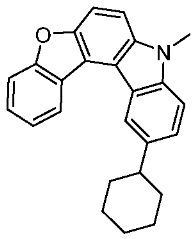
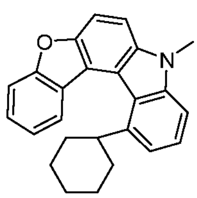
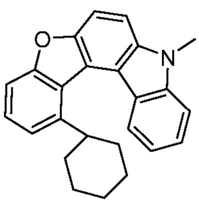
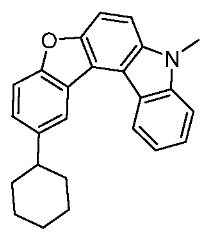

**D723** **D724** **D725** **D726** **D727**

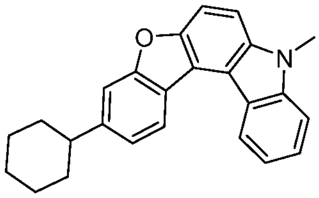

**D728**

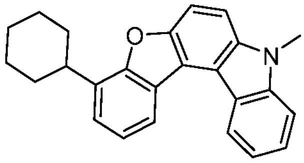

**D729**

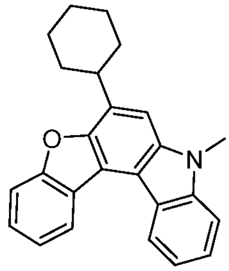

**D730**

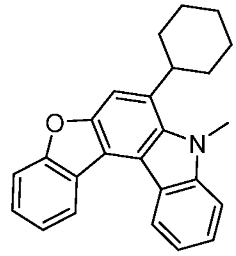

**D731**

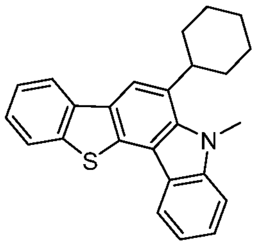

**D732**

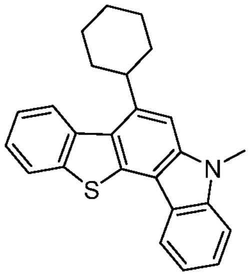

**D733**

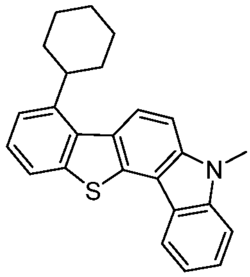

**D734**

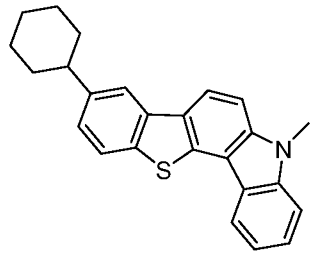

**D735**

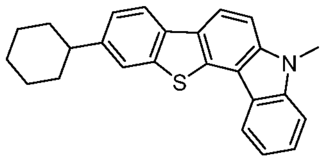

**D736**

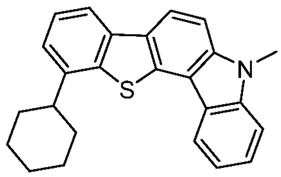

**D737**

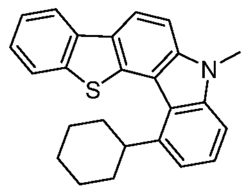

**D738**

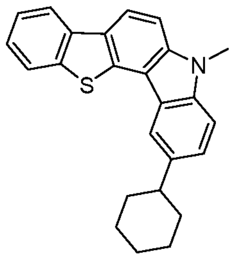

**D739**

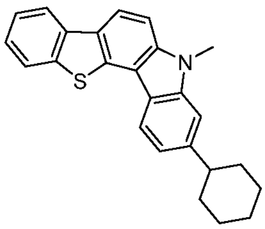

**D740**

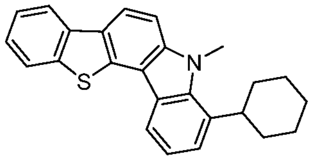

**D741**

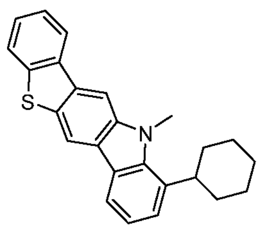

**D742**

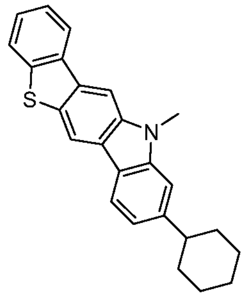

**D743**

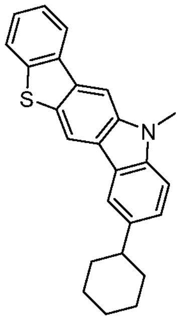

**D744**

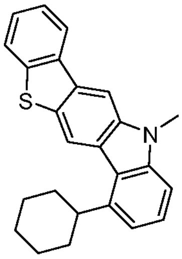

**D745**

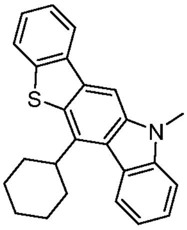

**D746**

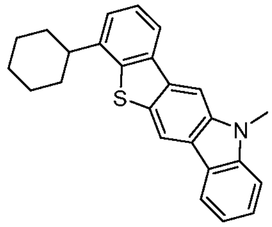

**D747**

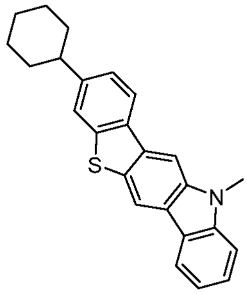

**D748**

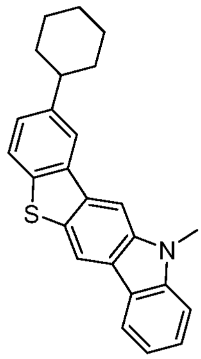

**D749**

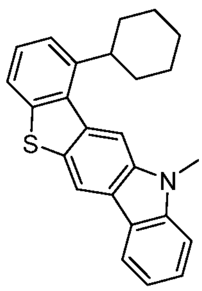

**D750**

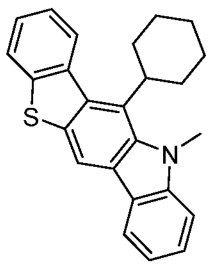

**D751**

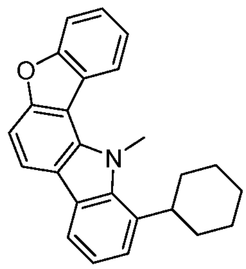

**D752**

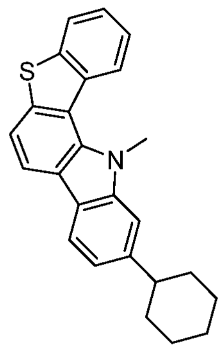

**D753**

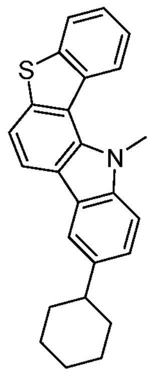

**D754**

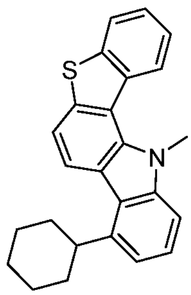

**D755**

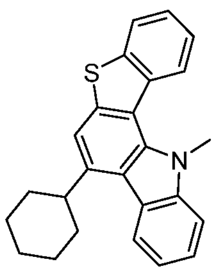

**D756**

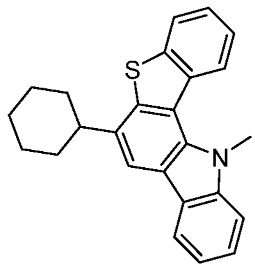

**D757**

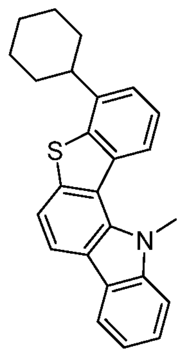

**D758**

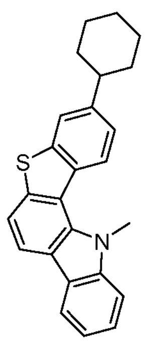

**D759**

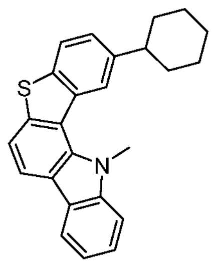

**D760**

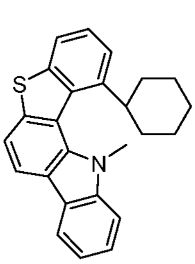

**D761**

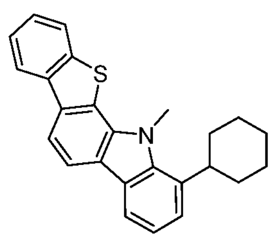

**D762**

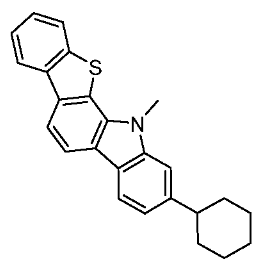

**D763**

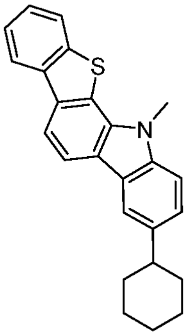
D764

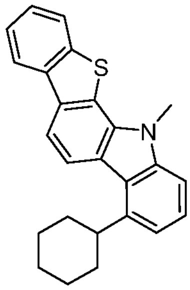
D765

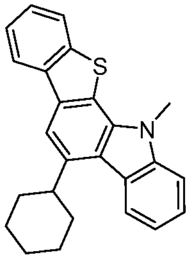
D766

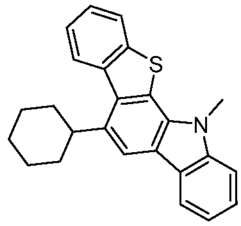
D767

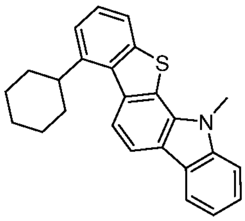
D768

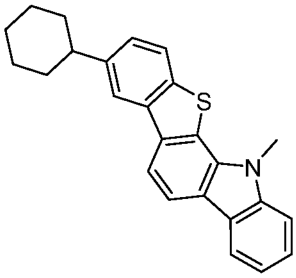
D769

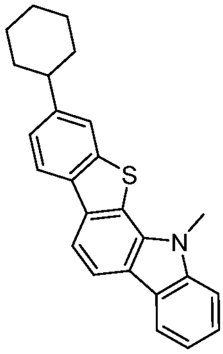
D770

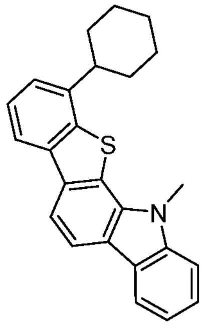
D771

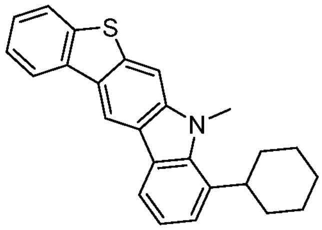
D772

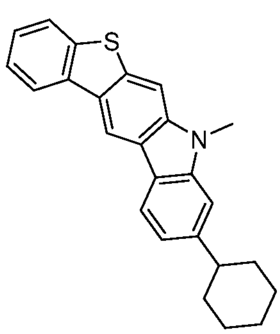
D773

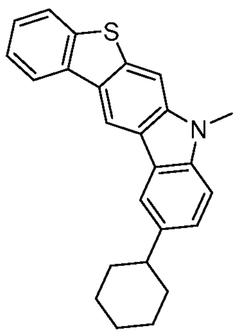
D774

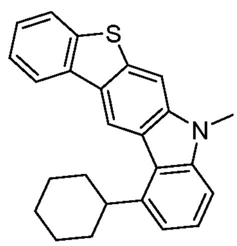
D775

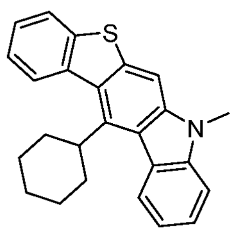
D776

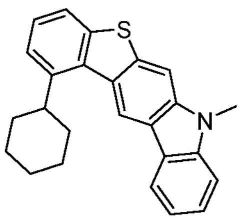
D777

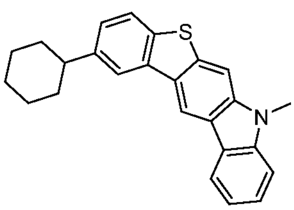
D778

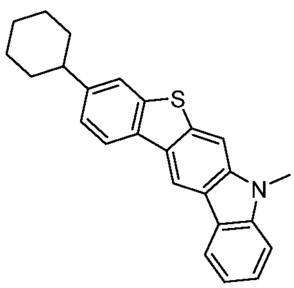
D779

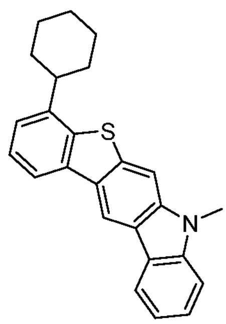
D780

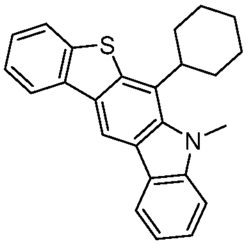
**D781**

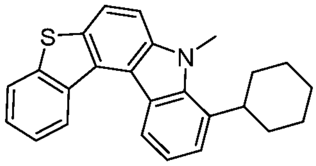
**D782**

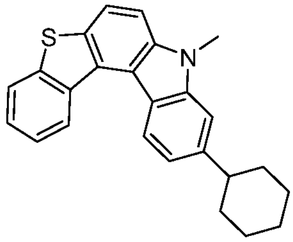
**D783**

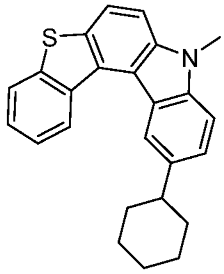
**D784**

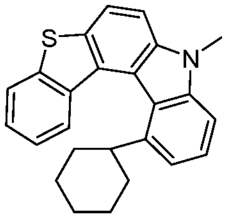
**D785**

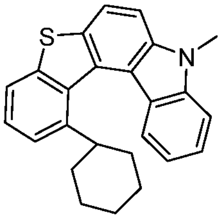
**D786**

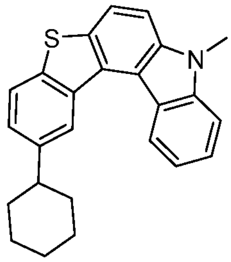
**D787**

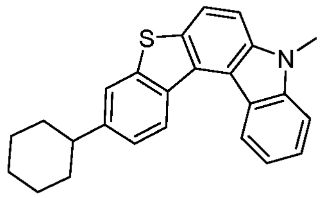
**D788**

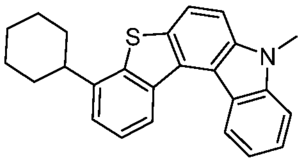
**D789**

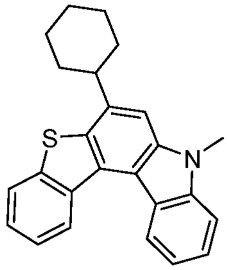
**D790**

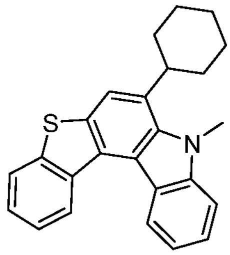
**D791**

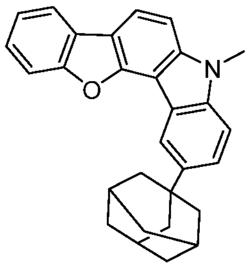
**D792**

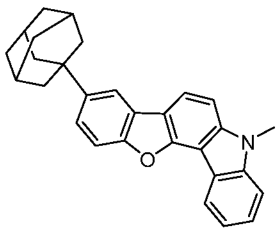
**D793**

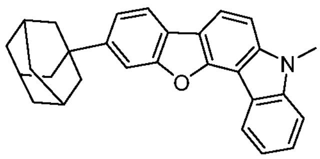
**D794**

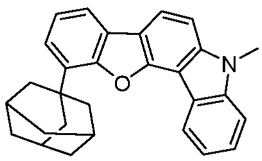
**D795**

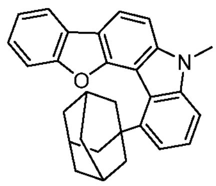
**D796**

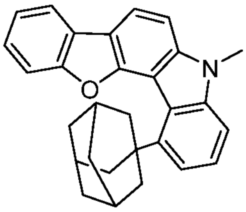

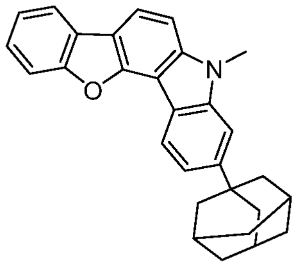

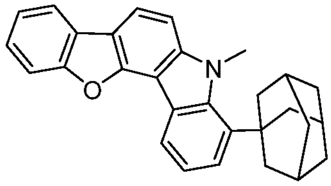

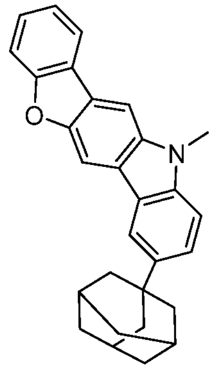

D797 D798 D799 D800

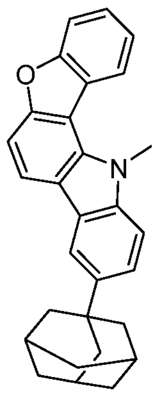

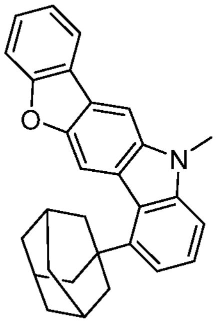

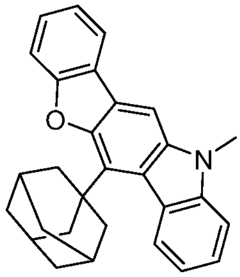

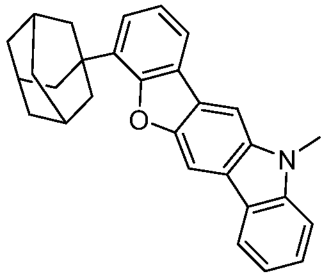

D801 D802 D803 D804

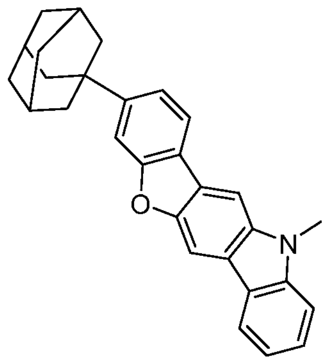

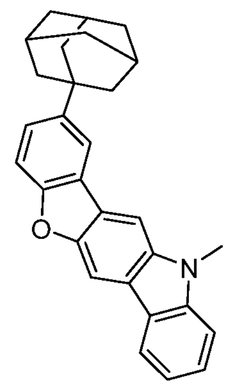

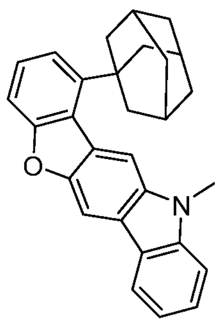

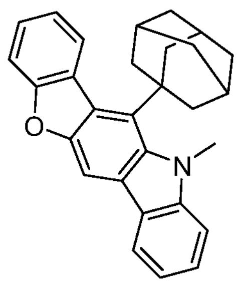

D805 D806 D807 D808

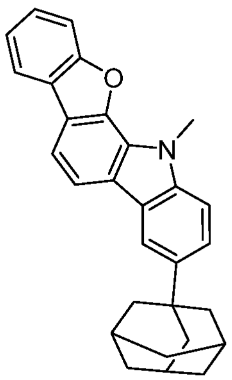

**D809**

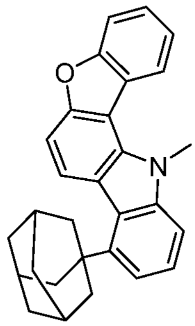

**D810**

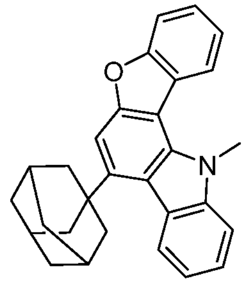

**D811**

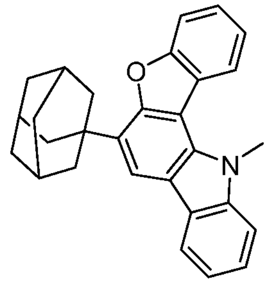

**D812**

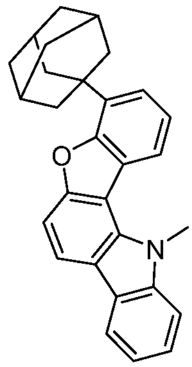

**D813**

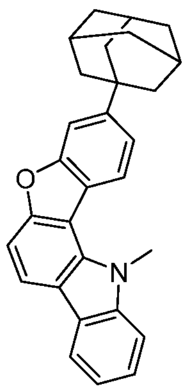

**D814**

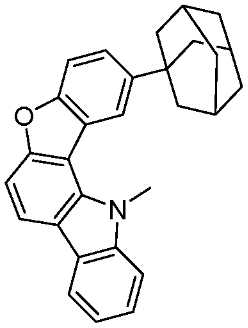

**D815**

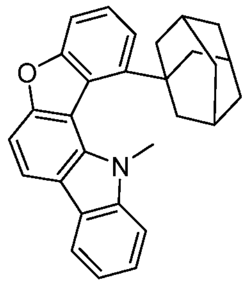

**D816**

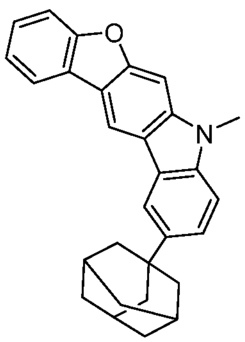

**D817**

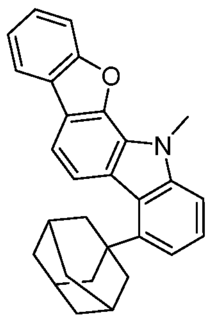

**D818**

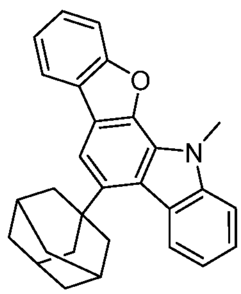

**D819**

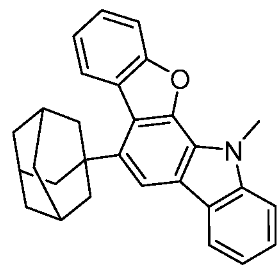

**D820**

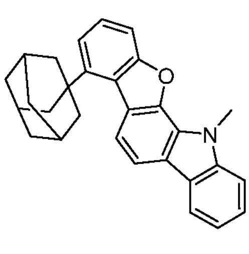

**D821**

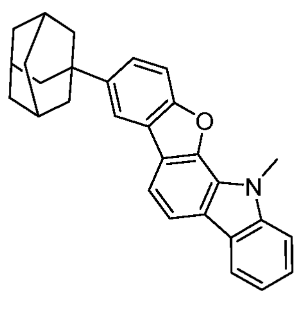

**D822**

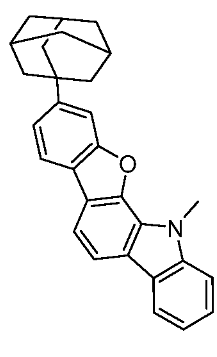

**D823**

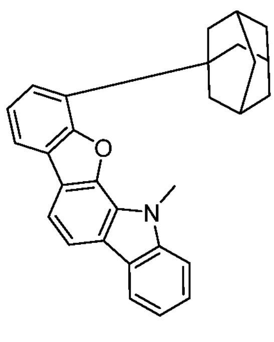

**D824**

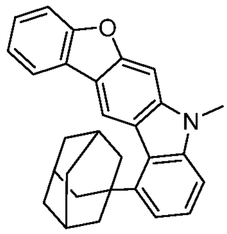
**D825**

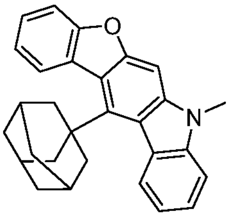
**D826**

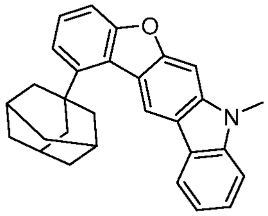
**D827**

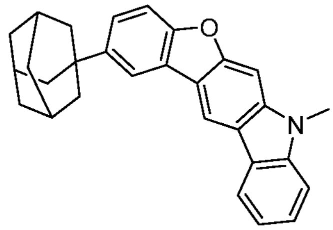
**D828**

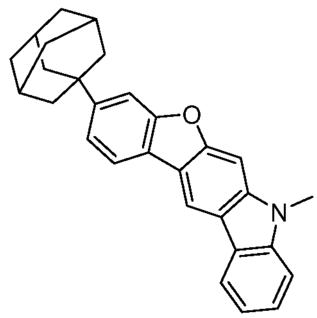
**D829**

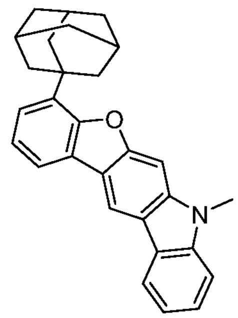
**D830**

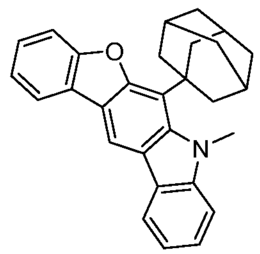
**D831**

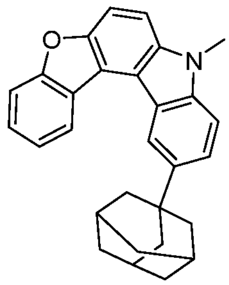
**D832**

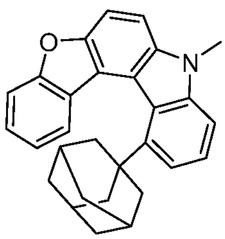
**D833**

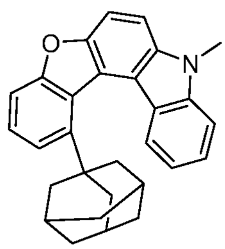
**D834**

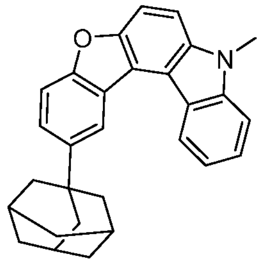
**D835**

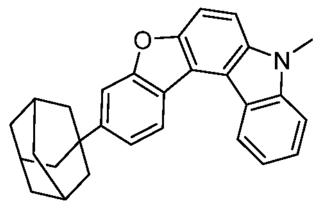
**D836**

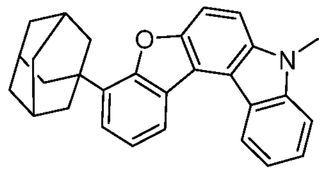
**D837**

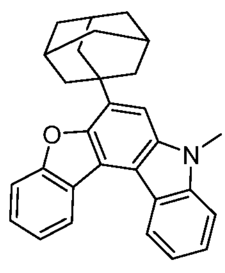
**D838**

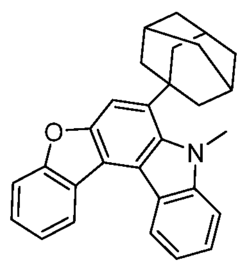
**D839**

The compound represented by the general formula (1) can be a compound composed of atoms alone selected from the group consisting of a carbon atom, a hydrogen atom, a deuterium atom, a nitrogen atom, an oxygen atom and a sulfur atom. In one preferred embodiment of the present invention, the compound represented by the general formula (1) is composed of atoms alone selected from the group consisting of a carbon atom, a hydrogen atom, a deuterium atom, a nitrogen atom and an oxygen atom. The compound represented by the general formula (1) can be a compound composed of atoms alone selected from the group consisting of a carbon atom, a hydrogen atom, a deuterium atom, a nitrogen atom and a sulfur atom. The compound represented by the general formula (1) can be a compound composed of atoms alone selected from the group consisting of a carbon atom, a hydrogen atom, a deuterium atom, and a nitrogen atom. The compound represented by the general formula (1) can be a compound composed of atoms alone selected from the group consisting of a carbon atom, a hydrogen atom, and a nitrogen atom. The compound represented by the

general formula (1) can be a compound containing a deuterium atom but not containing a hydrogen atom. For example, the compound represented by the general formula (1) can be a compound composed of atoms alone selected from the group consisting of a carbon atom, a deuterium atom and a nitrogen atom.

In one preferred embodiment of the present invention, the compound represented by the general formula (1) has a symmetric structure.

Specific examples of the compound represented by the general formula (1) are shown below. The following specific examples are specific examples of the compound represented by the general formula (1) where $R^1$ is a hydrogen atom. Table 1 shows a correlation between compound numbers and $R^2$, $R^3$ and $R^4$. In the case where the following compounds have rotational isomers, mixtures of rotational isomers and each separated rotational isomer are considered to be disclosed in the present specification.

[Table 1]

| No. | $R^2$ | $R^3$ | $R^4$ |
|---|---|---|---|
| 1 | D13 | D13 | D13 |
| 2 | D14 | D14 | D14 |
| 3 | D15 | D15 | D15 |
| 4 | D16 | D16 | D16 |
| 5 | D17 | D17 | D17 |
| 6 | D18 | D18 | D18 |
| 7 | D19 | D19 | D19 |
| 8 | D20 | D20 | D20 |
| 9 | D21 | D21 | D21 |
| 10 | D22 | D22 | D22 |
| 11 | D23 | D23 | D23 |
| 12 | D24 | D24 | D24 |
| 13 | D25 | D25 | D25 |
| 14 | D26 | D26 | D26 |
| 15 | D27 | D27 | D27 |
| 16 | D28 | D28 | D28 |
| 17 | D29 | D29 | D29 |
| 18 | D30 | D30 | D30 |
| 19 | D31 | D31 | D31 |
| 20 | D32 | D32 | D32 |
| 21 | D33 | D33 | D33 |
| 22 | D34 | D34 | D34 |
| 23 | D35 | D35 | D35 |
| 24 | D36 | D36 | D36 |
| 25 | D37 | D37 | D37 |
| 28 | D38 | D38 | D38 |
| 27 | D39 | D39 | D39 |
| 28 | D40 | D40 | D40 |
| 29 | D41 | D41 | D41 |
| 30 | D42 | D42 | D42 |

(continued)

| No. | $R^2$ | $R^3$ | $R^4$ |
|---|---|---|---|
| 31 | D43 | D43 | D43 |
| 32 | D44 | D44 | D44 |
| 33 | D45 | D45 | D45 |
| 34 | D46 | D46 | D46 |
| 35 | D47 | D47 | D47 |
| 38 | D48 | D48 | D48 |
| 37 | D49 | D49 | D49 |
| 38 | D50 | D50 | D50 |
| 39 | D51 | D51 | D51 |
| 40 | D52 | D52 | D52 |
| 41 | D53 | D53 | D53 |
| 42 | D54 | D54 | D54 |
| 43 | D55 | D55 | D55 |
| 44 | D56 | D56 | D56 |
| 45 | D57 | D57 | D57 |
| 48 | D58 | D58 | D58 |
| 47 | D59 | D59 | D59 |
| 48 | D60 | D60 | D60 |
| 49 | D61 | D81 | D61 |
| 50 | D62 | D62 | D62 |
| 51 | D63 | D63 | D63 |
| 52 | D64 | D64 | D64 |
| 53 | D65 | D65 | D65 |
| 54 | D66 | D66 | D66 |
| 55 | D67 | D67 | D67 |
| 56 | D68 | D68 | D68 |
| 57 | D69 | D69 | D69 |
| 58 | D70 | D70 | D70 |
| 59 | D71 | D71 | D71 |
| 60 | D72 | D72 | D72 |
| 61 | D73 | D73 | D73 |
| 62 | D74 | D74 | D74 |
| 63 | D75 | D75 | D75 |
| 64 | D76 | D76 | D76 |
| 65 | D77 | D77 | D77 |
| B8 | D78 | D78 | D78 |
| 67 | D79 | D79 | D79 |
| B8 | D80 | D80 | D80 |

(continued)

| No. | $R^2$ | $R^3$ | $R^4$ |
|---|---|---|---|
| 69 | D81 | D81 | D81 |
| 70 | D82 | D82 | D82 |
| 71 | D83 | D83 | D83 |
| 72 | D84 | D84 | D84 |
| 73 | D85 | D85 | D85 |
| 74 | D86 | D86 | D86 |
| 75 | D87 | D87 | D87 |
| 78 | D88 | D88 | D88 |
| 77 | D89 | D89 | D89 |
| 78 | D90 | D90 | D90 |
| 79 | D91 | D91 | D91 |
| 80 | D92 | D92 | D92 |
| 81 | D93 | D93 | D93 |
| 82 | D94 | D94 | D94 |
| 83 | D95 | D95 | D95 |
| 84 | D96 | D96 | D96 |
| 85 | D97 | D97 | D97 |
| 88 | D98 | D98 | D98 |
| 87 | D99 | D99 | D99 |
| 88 | D100 | D100 | D100 |
| 89 | D101 | D101 | D101 |
| 90 | D102 | D102 | D102 |
| 91 | D103 | D103 | D103 |
| 92 | D104 | D104 | D104 |
| 93 | D105 | D105 | D105 |
| 94 | D106 | D106 | D106 |
| 95 | D107 | D107 | D107 |
| 96 | D108 | D108 | D108 |
| 97 | D114 | D114 | D114 |
| 98 | D115 | D115 | D115 |
| 99 | D116 | D116 | D116 |
| 100 | D117 | D117 | D117 |
| 101 | D118 | D118 | D118 |
| 102 | D119 | D119 | D119 |
| 103 | D120 | D120 | D120 |
| 104 | D121 | D121 | D121 |
| 105 | D122 | D122 | D122 |
| 106 | D123 | D123 | D123 |

(continued)

| No. | $R^2$ | $R^3$ | $R^4$ |
|---|---|---|---|
| 107 | D124 | D124 | D124 |
| 108 | D125 | D125 | D125 |
| 109 | D126 | D126 | D126 |
| 110 | D127 | D127 | D127 |
| 111 | D128 | D128 | D128 |
| 112 | D129 | D129 | D129 |
| 113 | D130 | D130 | D130 |
| 114 | D131 | D131 | D131 |
| 115 | D132 | D132 | D132 |
| 116 | D133 | D133 | D133 |
| 117 | D134 | D134 | D134 |
| 118 | D135 | D135 | D135 |
| 119 | D136 | D136 | D136 |
| 120 | D137 | D137 | D137 |
| 121 | D138 | D138 | D138 |
| 122 | D139 | D139 | D139 |
| 123 | D140 | D140 | D140 |
| 124 | D141 | D141 | D141 |
| 125 | D142 | D142 | D142 |
| 128 | D143 | D143 | D143 |
| 127 | D144 | D144 | D144 |
| 128 | D145 | D145 | D145 |
| 129 | D146 | D146 | D146 |
| 130 | D147 | D147 | D147 |
| 131 | D148 | D148 | D148 |
| 132 | D149 | D149 | D149 |
| 133 | D180 | D180 | D180 |
| 134 | D181 | D181 | D181 |
| 135 | D182 | D182 | D182 |
| 136 | D183 | D183 | D183 |
| 137 | D184 | D184 | D184 |
| 138 | D185 | D185 | D185 |
| 139 | D186 | D186 | D186 |
| 140 | D187 | D187 | D187 |
| 141 | D188 | D188 | D188 |
| 142 | D189 | D189 | D189 |
| 143 | D190 | D190 | D190 |
| 144 | D191 | D191 | D191 |

(continued)

| No. | $R^2$ | $R^3$ | $R^4$ |
|---|---|---|---|
| 145 | D198 | D198 | D198 |
| 146 | D199 | D199 | D199 |
| 147 | D200 | D200 | D200 |
| 148 | D201 | D201 | D201 |
| 149 | D202 | D202 | D202 |
| 150 | D203 | D203 | D203 |
| 151 | D204 | D204 | D204 |
| 152 | D205 | D205 | D205 |
| 153 | D206 | D206 | D206 |
| 154 | D207 | D207 | D207 |
| 155 | D208 | D208 | D208 |
| 156 | D209 | D209 | D209 |
| 157 | D210 | D210 | D210 |
| 158 | D211 | D211 | D211 |
| 159 | D212 | D212 | D212 |
| 180 | D213 | D213 | D213 |
| 161 | D214 | D214 | D214 |
| 182 | D215 | D215 | D215 |
| 163 | D216 | D216 | D218 |
| 164 | D217 | D217 | D217 |
| 165 | D218 | D218 | D218 |
| 188 | D219 | D219 | D219 |
| 167 | D220 | D220 | D220 |
| 188 | D221 | D221 | D221 |
| 169 | D222 | D222 | D222 |
| 170 | D223 | D223 | D223 |
| 171 | D224 | D224 | D224 |
| 172 | D225 | D225 | D225 |
| 173 | D226 | D226 | D226 |
| 174 | D227 | D227 | D227 |
| 175 | D228 | D228 | D228 |
| 176 | D229 | D229 | D229 |
| 177 | D230 | D230 | D230 |
| 178 | D231 | D231 | D231 |
| 179 | D232 | D232 | D232 |
| 180 | D233 | D233 | D233 |
| 181 | D234 | D234 | D234 |
| 182 | D235 | D235 | D235 |

(continued)

| No. | $R^2$ | $R^3$ | $R^4$ |
|---|---|---|---|
| 183 | D236 | D236 | D236 |
| 184 | D237 | D237 | D237 |
| 185 | D238 | D238 | D238 |
| 188 | D239 | D239 | D239 |
| 187 | D245 | D245 | D245 |
| 188 | D246 | D246 | D246 |
| 189 | D247 | D247 | D247 |
| 190 | D248 | D248 | D248 |
| 191 | D249 | D249 | D249 |
| 192 | D250 | D250 | D250 |
| 193 | D251 | D251 | D251 |
| 194 | D252 | D252 | D252 |
| 195 | D253 | D253 | D253 |
| 196 | D254 | D254 | D254 |
| 197 | D255 | D255 | D255 |
| 198 | D256 | D256 | D258 |
| 199 | D257 | D257 | D257 |
| 200 | D258 | D258 | D258 |
| 201 | D259 | D259 | D259 |
| 202 | D260 | D260 | D260 |
| 203 | D261 | D261 | D261 |
| 204 | D262 | D262 | D262 |
| 205 | D263 | D263 | D263 |
| 208 | D264 | D264 | D264 |
| 207 | D265 | D265 | D265 |
| 208 | D266 | D266 | D266 |
| 209 | D267 | D267 | D267 |
| 210 | D268 | D268 | D268 |
| 211 | D269 | D269 | D269 |
| 212 | D270 | D270 | D270 |
| 213 | D271 | D271 | D271 |
| 214 | D272 | D272 | D272 |
| 215 | D273 | D273 | D273 |
| 216 | D274 | D274 | D274 |
| 217 | D275 | D275 | D275 |
| 218 | D276 | D276 | D276 |
| 219 | D277 | D277 | D277 |
| 220 | D278 | D278 | D278 |

(continued)

| No. | $R^2$ | $R^3$ | $R^4$ |
|---|---|---|---|
| 221 | D279 | D279 | D279 |
| 222 | D280 | D280 | D280 |
| 223 | D281 | D281 | D281 |
| 224 | D282 | D282 | D282 |
| 225 | D283 | D283 | D283 |
| 226 | D284 | D284 | D284 |
| 227 | D285 | D285 | D285 |
| 228 | D286 | D286 | D286 |
| 229 | D287 | D287 | D287 |
| 230 | D288 | D288 | D288 |
| 231 | D289 | D289 | D289 |
| 232 | D290 | D290 | D290 |
| 233 | D291 | D291 | D291 |
| 234 | D292 | D292 | D292 |
| 235 | D293 | D293 | D293 |
| 236 | D294 | D294 | D294 |
| 237 | D295 | D295 | D295 |
| 238 | D296 | D296 | D296 |
| 239 | D297 | D297 | D297 |
| 240 | D298 | D298 | D298 |
| 241 | D300 | D300 | D300 |
| 242 | D301 | D301 | D301 |
| 243 | D302 | D302 | D302 |
| 244 | D303 | D303 | D303 |
| 245 | D304 | D304 | D304 |
| 246 | D305 | D305 | D305 |
| 247 | D306 | D306 | D306 |
| 248 | D307 | D307 | D307 |
| 249 | D308 | D308 | D308 |
| 250 | D309 | D309 | D309 |
| 251 | D310 | D310 | D310 |
| 252 | D311 | D311 | D311 |
| 253 | D312 | D312 | D312 |
| 254 | D313 | D313 | D313 |
| 255 | D314 | D314 | D314 |
| 256 | D315 | D315 | D315 |
| 257 | D316 | D316 | D318 |
| 258 | D317 | D317 | D317 |

(continued)

| No. | $R^2$ | $R^3$ | $R^4$ |
|---|---|---|---|
| 259 | D318 | D318 | D318 |
| 260 | D319 | D319 | D319 |
| 261 | D320 | D320 | D320 |
| 262 | D321 | D321 | D321 |
| 263 | D322 | D322 | D322 |
| 264 | D323 | D323 | D323 |
| 265 | D324 | D324 | D324 |
| 266 | D325 | D325 | D325 |
| 267 | D326 | D326 | D326 |
| 268 | D327 | D327 | D327 |
| 269 | D328 | D328 | D328 |
| 270 | D329 | D329 | D329 |
| 271 | D330 | D330 | D330 |
| 272 | D331 | D331 | D331 |
| 273 | D332 | D332 | D332 |
| 274 | D333 | D333 | D333 |
| 275 | D334 | D334 | D334 |
| 276 | D335 | D335 | D335 |
| 277 | D336 | D336 | D336 |
| 278 | D337 | D337 | D337 |
| 279 | D338 | D338 | D338 |
| 280 | D339 | D339 | D339 |
| 281 | D340 | D340 | D340 |
| 282 | D341 | D341 | D341 |
| 283 | D342 | D342 | D342 |
| 284 | D343 | D343 | D343 |
| 285 | D344 | D344 | D344 |
| 286 | D345 | D345 | D345 |
| 287 | D346 | D346 | D346 |
| 288 | D347 | D347 | D347 |
| 289 | D348 | D348 | D348 |
| 290 | D349 | D349 | D349 |
| 291 | D350 | D350 | D350 |
| 292 | D351 | D351 | D351 |
| 293 | D352 | D352 | D352 |
| 294 | D353 | D353 | D353 |
| 295 | D354 | D354 | D354 |
| 296 | D355 | D355 | D355 |

(continued)

| No. | $R^2$ | $R^3$ | $R^4$ |
|---|---|---|---|
| 297 | D356 | D356 | D356 |
| 298 | D357 | D357 | D357 |
| 299 | D358 | D358 | D358 |
| 300 | D359 | D359 | D359 |
| 301 | D360 | D360 | D360 |
| 302 | D361 | D361 | D361 |
| 303 | D362 | D362 | D362 |
| 304 | D363 | D363 | D363 |
| 305 | D364 | D364 | D364 |
| 306 | D365 | D365 | D365 |
| 307 | D366 | D366 | D366 |
| 308 | D367 | D367 | D367 |
| 309 | D368 | D368 | D368 |
| 310 | D369 | D369 | D369 |
| 311 | D370 | D370 | D370 |
| 312 | D371 | D371 | D371 |
| 313 | D372 | D372 | D372 |
| 314 | D373 | D373 | D373 |
| 315 | D374 | D374 | D374 |
| 316 | D375 | D375 | D375 |
| 317 | D376 | D376 | D376 |
| 318 | D377 | D377 | D377 |
| 319 | D378 | D378 | D378 |
| 320 | D379 | D379 | D379 |
| 321 | D380 | D380 | D380 |
| 322 | D381 | D381 | D381 |
| 323 | D382 | D382 | D382 |
| 324 | D383 | D383 | D383 |
| 325 | D384 | D384 | D384 |
| 326 | D385 | D385 | D385 |
| 327 | D386 | D386 | D386 |
| 328 | D387 | D387 | D387 |
| 329 | D388 | D388 | D388 |
| 330 | D389 | D389 | D389 |
| 331 | D390 | D390 | D390 |
| 332 | D391 | D391 | D391 |
| 333 | D392 | D392 | D392 |
| 334 | D393 | D393 | D393 |

(continued)

| No. | $R^2$ | $R^3$ | $R^4$ |
|---|---|---|---|
| 335 | D394 | D394 | D394 |
| 336 | D395 | D395 | D395 |
| 337 | D396 | D396 | D396 |
| 338 | D397 | D397 | D397 |
| 339 | D398 | D398 | D398 |
| 340 | D399 | D399 | D399 |
| 341 | D400 | D400 | D400 |
| 342 | D401 | D401 | D401 |
| 343 | D402 | D402 | D402 |
| 344 | D403 | D403 | D403 |
| 345 | D404 | D404 | D404 |
| 346 | D405 | D405 | D405 |
| 347 | D406 | D406 | D406 |
| 348 | D407 | D407 | D407 |
| 349 | D408 | D408 | D408 |
| 350 | D409 | D409 | D409 |
| 351 | D410 | D410 | D410 |
| 352 | D411 | D411 | D411 |
| 353 | D412 | D412 | D412 |
| 354 | D413 | D413 | D413 |
| 355 | D414 | D414 | D414 |
| 356 | D415 | D415 | D415 |
| 357 | D416 | D416 | D416 |
| 358 | D417 | D417 | D417 |
| 359 | D418 | D418 | D418 |
| 360 | D419 | D419 | D419 |
| 361 | D420 | D420 | D420 |
| 362 | D421 | D421 | D421 |
| 363 | D422 | D422 | D422 |
| 364 | D423 | D423 | D423 |
| 365 | D424 | D424 | D424 |
| 366 | D425 | D425 | D425 |
| 367 | D426 | D426 | D426 |
| 368 | D427 | D427 | D427 |
| 369 | D428 | D428 | D428 |
| 370 | D429 | D429 | D429 |
| 371 | D430 | D430 | D430 |
| 372 | D431 | D431 | D431 |

(continued)

| No. | $R^2$ | $R^3$ | $R^4$ |
|---|---|---|---|
| 373 | D432 | D432 | D432 |
| 374 | D434 | D434 | D434 |
| 375 | D435 | D435 | D435 |
| 376 | D436 | D436 | D436 |
| 377 | D437 | D437 | D437 |
| 378 | D438 | D438 | D438 |
| 379 | D439 | D439 | D439 |
| 380 | D440 | D440 | D440 |
| 381 | D441 | D441 | D441 |
| 382 | D442 | D442 | D442 |
| 383 | D443 | D443 | D443 |
| 384 | D444 | D444 | D444 |
| 385 | D445 | D445 | D445 |
| 386 | D446 | D446 | D446 |
| 387 | D447 | D447 | D447 |
| 388 | D448 | D448 | D448 |
| 389 | D449 | D449 | D449 |
| 390 | D450 | D450 | D450 |
| 391 | D451 | D451 | D451 |
| 392 | D452 | D452 | D452 |
| 393 | D453 | D453 | D453 |
| 394 | D454 | D454 | D454 |
| 395 | D455 | D455 | D455 |
| 396 | D456 | D456 | D456 |
| 397 | D457 | D457 | D457 |
| 398 | D458 | D458 | D458 |
| 399 | D459 | D459 | D459 |
| 400 | D460 | D460 | D460 |
| 401 | D461 | D461 | D461 |
| 402 | D462 | D462 | D462 |
| 403 | D463 | D463 | D463 |
| 404 | D464 | D464 | D464 |
| 405 | D465 | D465 | D465 |
| 406 | D466 | D466 | D466 |
| 407 | D467 | D467 | D467 |
| 408 | D468 | D468 | D468 |
| 409 | D469 | D469 | D469 |
| 410 | D470 | D470 | D470 |

(continued)

| No. | $R^2$ | $R^3$ | $R^4$ |
|---|---|---|---|
| 411 | D471 | D471 | D471 |
| 412 | D472 | D472 | D472 |
| 413 | D473 | D473 | D473 |
| 414 | D474 | D474 | D474 |
| 415 | D475 | D475 | D475 |
| 416 | D476 | D476 | D476 |
| 417 | D477 | D477 | D477 |
| 418 | D478 | D478 | D478 |
| 419 | D479 | D479 | D479 |
| 420 | D480 | D480 | D480 |
| 421 | D481 | D481 | D481 |
| 422 | D482 | D482 | D482 |
| 423 | D483 | D483 | D483 |
| 424 | D484 | D484 | D484 |
| 425 | D485 | D485 | D485 |
| 426 | D486 | D486 | D486 |
| 427 | D487 | D487 | D487 |
| 428 | D488 | D488 | D488 |
| 429 | D489 | D489 | D489 |
| 430 | D490 | D490 | D490 |
| 431 | D491 | D491 | D491 |
| 432 | D492 | D492 | D492 |
| 433 | D493 | D493 | D493 |
| 434 | D494 | D494 | D494 |
| 435 | D495 | D495 | D495 |
| 436 | D496 | D496 | D496 |
| 437 | D497 | D497 | D497 |
| 438 | D498 | D498 | D498 |
| 439 | D499 | D499 | D499 |
| 440 | D500 | D500 | D500 |
| 441 | D501 | D501 | D501 |
| 442 | D502 | D502 | D502 |
| 443 | D503 | D503 | D503 |
| 444 | D504 | D504 | D504 |
| 445 | D505 | D505 | D505 |
| 446 | D506 | D506 | D506 |
| 447 | D507 | D507 | D507 |
| 448 | D508 | D508 | D508 |

(continued)

| No. | $R^2$ | $R^3$ | $R^4$ |
|---|---|---|---|
| 449 | D509 | D509 | D509 |
| 450 | D510 | D510 | D510 |
| 451 | D511 | D511 | D511 |
| 452 | D512 | D512 | D512 |
| 453 | D513 | D513 | D513 |
| 454 | D514 | D514 | D514 |
| 455 | D515 | D515 | D515 |
| 456 | D516 | D516 | D516 |
| 457 | D517 | D517 | D517 |
| 458 | D518 | D518 | D518 |
| 459 | D519 | D519 | D519 |
| 460 | D520 | D520 | D520 |
| 461 | D521 | D521 | D521 |
| 462 | D522 | D522 | D522 |
| 463 | D523 | D523 | D523 |
| 464 | D524 | D524 | D524 |
| 465 | D525 | D525 | D525 |
| 466 | D526 | D526 | D526 |
| 467 | D527 | D527 | D527 |
| 468 | D528 | D528 | D528 |
| 469 | D529 | D529 | D529 |
| 470 | D530 | D530 | D530 |
| 471 | D531 | D531 | D531 |
| 472 | D532 | D532 | D532 |
| 473 | D533 | D533 | D533 |
| 474 | D534 | D534 | D534 |
| 475 | D535 | D535 | D535 |
| 476 | D536 | D536 | D536 |
| 477 | D537 | D537 | D537 |
| 478 | D538 | D538 | D538 |
| 479 | D539 | D539 | D539 |
| 480 | D540 | D540 | D540 |
| 481 | D541 | D541 | D541 |
| 482 | D542 | D542 | D542 |
| 483 | D543 | D543 | D543 |
| 484 | D544 | D544 | D544 |
| 485 | D545 | D545 | D545 |
| 486 | D546 | D546 | D546 |

(continued)

| No. | $R^2$ | $R^3$ | $R^4$ |
|---|---|---|---|
| 487 | D547 | D547 | D547 |
| 488 | D548 | D548 | D548 |
| 489 | D549 | D549 | D549 |
| 490 | D550 | D550 | D550 |
| 491 | D551 | D551 | D551 |
| 492 | D552 | D552 | D552 |
| 493 | D553 | D553 | D553 |
| 494 | D554 | D554 | D554 |
| 495 | D555 | D555 | D555 |
| 496 | D556 | D55B | D55B |
| 497 | D557 | D557 | D557 |
| 498 | D558 | D558 | D558 |
| 499 | D559 | D559 | D559 |
| 500 | D560 | D560 | D560 |
| 501 | D561 | D561 | D561 |
| 502 | D562 | D562 | D562 |
| 503 | D563 | D563 | D563 |
| 504 | D564 | D564 | D564 |
| 505 | D565 | D565 | D565 |
| 508 | D566 | D566 | D566 |
| 507 | D567 | D567 | D567 |
| 508 | D568 | D568 | D568 |
| 509 | D569 | D569 | D569 |
| 510 | D570 | D570 | D570 |
| 511 | D571 | D571 | D571 |
| 512 | D572 | D572 | D572 |
| 513 | D573 | D573 | D573 |
| 514 | D574 | D574 | D574 |
| 515 | D575 | D575 | D575 |
| 516 | D576 | D576 | D576 |
| 517 | D577 | D577 | D577 |
| 518 | D578 | D578 | D578 |
| 519 | D579 | D579 | D579 |
| 520 | D580 | D580 | D580 |
| 521 | D581 | D581 | D581 |
| 522 | D582 | D582 | D582 |
| 523 | D583 | D583 | D583 |
| 524 | D584 | D584 | D584 |

(continued)

| No. | $R^2$ | $R^3$ | $R^4$ |
|---|---|---|---|
| 525 | D585 | D585 | D585 |
| 526 | D586 | D586 | D586 |
| 527 | D587 | D587 | D587 |
| 528 | D588 | D588 | D588 |
| 529 | D589 | D589 | D589 |
| 530 | D590 | D590 | D590 |
| 531 | D591 | D591 | D591 |
| 532 | D592 | D592 | D592 |
| 533 | D593 | D593 | D593 |
| 534 | D594 | D594 | D594 |
| 535 | D595 | D595 | D595 |
| 538 | D596 | D596 | D596 |
| 537 | D597 | D597 | D597 |
| 538 | D598 | D598 | D598 |
| 539 | D599 | D599 | D599 |
| 540 | D600 | D600 | D600 |
| 541 | D601 | D601 | D601 |
| 542 | D602 | D602 | D602 |
| 543 | D603 | D603 | D603 |
| 544 | D604 | D604 | D604 |
| 545 | D605 | D605 | D605 |
| 546 | D606 | D606 | D606 |
| 547 | D607 | D607 | D607 |
| 548 | D608 | D608 | D608 |
| 549 | D609 | D609 | D609 |
| 550 | D610 | D610 | D610 |
| 551 | D611 | DB11 | DB11 |
| 552 | D612 | DB12 | DB12 |
| 553 | D613 | DB13 | DB13 |
| 554 | D614 | DB14 | DB14 |
| 555 | D615 | DB15 | DB15 |
| 558 | D616 | DB18 | DB18 |
| 557 | D617 | DB17 | DB17 |
| 558 | D618 | DB18 | DB18 |
| 559 | D619 | DB19 | DB19 |
| 560 | D620 | D620 | D620 |
| 561 | D621 | DB21 | DB21 |
| 562 | D622 | DB22 | DB22 |

(continued)

| No. | $R^2$ | $R^3$ | $R^4$ |
|---|---|---|---|
| 563 | D623 | DB23 | DB23 |
| 564 | D624 | DB24 | DB24 |
| 565 | D625 | DB25 | DB25 |
| 566 | D626 | DB26 | DB26 |
| 567 | D627 | DB27 | DB27 |
| 568 | D628 | DB28 | DB28 |
| 569 | D629 | D629 | D629 |
| 570 | D630 | D630 | D630 |
| 571 | D631 | D631 | D631 |
| 572 | D632 | D632 | D632 |
| 573 | D633 | D633 | D633 |
| 574 | D634 | D634 | D634 |
| 575 | D635 | D635 | D635 |
| 576 | D636 | D636 | D636 |
| 577 | D637 | D637 | D637 |
| 578 | D638 | D638 | D638 |
| 579 | D639 | D639 | D639 |
| 580 | D640 | D640 | D640 |
| 581 | D641 | D641 | D641 |
| 582 | D642 | D642 | D642 |
| 583 | D643 | D643 | D643 |
| 584 | D644 | D644 | D644 |
| 585 | D645 | D645 | D645 |
| 588 | D646 | DB48 | D646 |
| 587 | D647 | DB47 | DB47 |
| 588 | D648 | DB48 | D648 |
| 589 | D649 | DB49 | DB49 |
| 590 | D650 | D650 | D650 |
| 591 | D651 | DB51 | DB51 |
| 592 | D652 | DB52 | DB52 |
| 593 | D653 | DB53 | DB53 |
| 594 | D654 | DB54 | DB54 |
| 595 | D655 | DB55 | DB55 |
| 596 | D656 | DB56 | DB56 |
| 597 | D657 | DB57 | DB57 |
| 598 | D658 | DB58 | DB58 |
| 599 | D659 | DB59 | DB59 |
| 600 | D660 | D660 | D660 |

(continued)

| No. | $R^2$ | $R^3$ | $R^4$ |
|---|---|---|---|
| 601 | DBB1 | D661 | D661 |
| 802 | DBB2 | D662 | D662 |
| 603 | DBB3 | D663 | D663 |
| 604 | D664 | D664 | D664 |
| 605 | D665 | D665 | D665 |
| 606 | D666 | DB88 | D666 |
| 607 | DBB7 | D667 | D667 |
| 608 | D668 | DB88 | D668 |
| 609 | D669 | D669 | D669 |
| 610 | D670 | D670 | D670 |
| 611 | DB71 | DB71 | D671 |
| 612 | D672 | DB72 | D672 |
| 613 | DB73 | DB73 | D673 |
| 614 | DB74 | DB74 | D674 |
| 615 | DB75 | DB75 | D675 |
| 616 | DB76 | D676 | D676 |
| 617 | DB77 | DB77 | D677 |
| 618 | DB78 | DB78 | D678 |
| 619 | D679 | D679 | D679 |
| 620 | D680 | D680 | D680 |
| 621 | D681 | D681 | D681 |
| 622 | D682 | D682 | D682 |
| 623 | D683 | D683 | D683 |
| 624 | D684 | D684 | D684 |
| 625 | D685 | D685 | D685 |
| 626 | D686 | D686 | D686 |
| 627 | D687 | D687 | D687 |
| 628 | D688 | D688 | D688 |
| 629 | D689 | D689 | D689 |
| 630 | D690 | D690 | D690 |
| 631 | D691 | D691 | D691 |
| 632 | D692 | D692 | D692 |
| 633 | D693 | D693 | D693 |
| 634 | D694 | D694 | D694 |
| 635 | D695 | D695 | D695 |
| 636 | DB98 | D696 | D696 |
| 637 | DB97 | DB97 | D697 |
| 638 | DB98 | D698 | D698 |

(continued)

| No. | $R^2$ | $R^3$ | $R^4$ |
|---|---|---|---|
| 639 | DB99 | D699 | D699 |
| 640 | D700 | D700 | D700 |
| 641 | D701 | D701 | D701 |
| 642 | D702 | D702 | D702 |
| 643 | D703 | D703 | D703 |
| 644 | D704 | D704 | D704 |
| 645 | D705 | D705 | D705 |
| 646 | D706 | D706 | D706 |
| 647 | D707 | D707 | D707 |
| 648 | D708 | D708 | D708 |
| 649 | D709 | D709 | D709 |
| 650 | D710 | D710 | D710 |
| 651 | D711 | D711 | D711 |
| 652 | D712 | D712 | D712 |
| 653 | D713 | D713 | D713 |
| 654 | D714 | D714 | D714 |
| 655 | D715 | D715 | D715 |
| 656 | D716 | D716 | D716 |
| 657 | D717 | D717 | D717 |
| 658 | D718 | D718 | D718 |
| 659 | D719 | D719 | D719 |
| 660 | D720 | D720 | D720 |
| 661 | D721 | D721 | D721 |
| 662 | D722 | D722 | D722 |
| 663 | D723 | D723 | D723 |
| 664 | D724 | D724 | D724 |
| 665 | D725 | D725 | D725 |
| 666 | D726 | D726 | D726 |
| 667 | D727 | D727 | D727 |
| 668 | D728 | D728 | D728 |
| 669 | D729 | D729 | D729 |
| 670 | D730 | D730 | D730 |
| 671 | D731 | D731 | D731 |
| 672 | D732 | D732 | D732 |
| 673 | D733 | D733 | D733 |
| 674 | D734 | D734 | D734 |
| 675 | D735 | D735 | D735 |
| 676 | D736 | D736 | D736 |

(continued)

| No. | $R^2$ | $R^3$ | $R^4$ |
|---|---|---|---|
| 677 | D737 | D737 | D737 |
| 678 | D738 | D738 | D738 |
| 679 | D739 | D739 | D739 |
| 680 | D740 | D740 | D740 |
| 681 | D741 | D741 | D741 |
| 682 | D742 | D742 | D742 |
| 683 | D743 | D743 | D743 |
| 684 | D744 | D744 | D744 |
| 685 | D745 | D745 | D745 |
| 686 | D748 | D746 | D746 |
| 687 | D747 | D747 | D747 |
| 688 | D748 | D748 | D748 |
| 689 | D749 | D749 | D749 |
| 690 | D750 | D750 | D750 |
| 691 | D751 | D751 | D751 |
| 692 | D752 | D752 | D752 |
| 693 | D753 | D753 | D753 |
| 694 | D754 | D754 | D754 |
| 695 | D755 | D755 | D755 |
| 696 | D756 | D756 | D756 |
| 697 | D757 | D757 | D757 |
| 698 | D758 | D758 | D758 |
| 699 | D759 | D759 | D759 |
| 700 | D760 | D760 | D760 |
| 701 | D761 | D761 | D761 |
| 702 | D762 | D762 | D762 |
| 703 | D763 | D763 | D763 |
| 704 | D764 | D764 | D764 |
| 705 | D765 | D765 | D765 |
| 708 | D766 | D766 | D766 |
| 707 | D767 | D767 | D767 |
| 708 | D768 | D768 | D768 |
| 709 | D769 | D769 | D769 |
| 710 | D770 | D770 | D770 |
| 711 | D771 | D771 | D771 |
| 712 | D772 | D772 | D772 |
| 713 | D773 | D773 | D773 |
| 714 | D774 | D774 | D774 |

(continued)

| No. | $R^2$ | $R^3$ | $R^4$ |
|---|---|---|---|
| 715 | D775 | D775 | D775 |
| 716 | D776 | D776 | D776 |
| 717 | D777 | D777 | D777 |
| 718 | D778 | D778 | D778 |
| 719 | D779 | D779 | D779 |
| 720 | D780 | D780 | D780 |

The following Table 2 and Table 3 further show specific examples of the compound represented by the general formula (1) in the form of tables. Additional compound numbers are given to the structures to be obtained by further substituting a part of the structures specific by compound numbers in Table 2 and Table 3. For example, in Table 2, compounds 721 to 1440 (expressed as No. 721 to No. 1440 in the Table) are compounds formed by substituting $R^2$ (expressed as R2 in tables) in compounds 1 to 720 with D1. Compound 721 is a compound formed by substituting $R^2$ in Compound 1 with D1, and compound 722 is a compound formed by substituting $R^2$ in compound 2 with D1. The structures of the compounds listed in Table 2 and the compounds listed in Table 3 are identified in such a manner. In Table 2 and Table 3, every numbered compound is individually identified for the structure thereof, and is specifically disclosed one by one in the present specification.

[Table 2]

| Group to be Substituted | | | Substituting Group | | | New Compound No. | | | |
|---|---|---|---|---|---|---|---|---|---|
| R2 of | No. | 1~720 | are substituted with | D1 | to form | No. | 721 | ~ | 1440 |
| R2 of | No. | 1~720 | are substituted with | D2 | to form | No. | 1441 | ~ | 2160 |
| R2 of | No. | 1~720 | are substituted with | D3 | to form | No. | 2161 | ~ | 2880 |
| R2 of | No. | 1~720 | are substituted with | D4 | to form | No. | 2881 | ~ | 3600 |
| R2 of | No. | 1~720 | are substituted with | D5 | to form | No. | 3601 | ~ | 4320 |
| R2 of | No. | 1~720 | are substituted with | D6 | to form | No. | 4321 | ~ | 5040 |
| R2 of | No. | 1~720 | are substituted with | D7 | to form | No. | 5041 | ~ | 5760 |
| R2 of | No. | 1~720 | are substituted with | D8 | to form | No. | 5761 | ~ | 6480 |
| R2 of | No. | 1~720 | are substituted with | D9 | to form | No. | 6481 | ~ | 7200 |
| R2 of | No. | 1~720 | are substituted with | D10 | to form | No. | 7201 | ~ | 7920 |
| R2 of | No. | 1~720 | are substituted with | D11 | to form | No. | 7921 | ~ | 8640 |
| R2 of | No. | 1~720 | are substituted with | D12 | to form | No. | 8641 | ~ | 9360 |
| R2 of | No. | 2~720 | are substituted with | D13 | to form | No. | 9361 | ~ | 10079 |
| R2 of | No. | 1, 3~720 | are substituted with | D14 | to form | No. | 0080 | ~ | 10798 |
| R2 of | No. | 1, 2, 4~720 | are substituted with | D15 | to form | No. | 10799 | ~ | 11517 |
| R2 of | No. | 1~3, 5~720 | are substituted with | D16 | to form | No. | 11518 | ~ | 12236 |
| R2 of | No. | 1~4, 6~720 | are substituted with | D17 | to form | No. | 12237 | ~ | 12955 |
| R2 of | No. | 1~5, 7~720 | are substituted with | D18 | to form | No. | 12956 | ~ | 13674 |
| R2 of | No. | 1~6, 8~720 | are substituted with | D19 | to form | No. | 13675 | ~ | 14393 |
| R2 of | No. | 1~7, 9~720 | are substituted with | D20 | to form | No. | 14394 | ~ | 15112 |
| R2 of | No. | 1~8, 10~720 | are substituted with | D21 | to form | No. | 15113 | ~ | 15831 |
| R2 of | No. | 1~9 11~720 | are substituted with | D22 | to form | No. | 15832 | ~ | 16550 |

(continued)

| Group to be Substituted | | | Substituting Group | | | New Compound No. | | | |
|---|---|---|---|---|---|---|---|---|---|
| R2 of | No. | 1~10, 12~720 | are substituted with | D23 | to form | No. | 16551 | ~ | 17269 |
| R2 of | No. | 1~11, 13~720 | are substituted with | D24 | to form | No. | 17270 | ~ | 17988 |
| R2 of | No. | 1~12, 14~720 | are substituted with | D25 | to form | No. | 17989 | ~ | 18707 |
| R2 of | No. | 1~13, 15~720 | are substituted with | D26 | to form | No. | 18708 | ~ | 19426 |
| R2 of | No. | 1~14, 16~720 | are substituted with | D27 | to form | No. | 19427 | ~ | 20145 |
| R2 of | No. | 1~15, 17~720 | are substituted with | D28 | to form | No. | 20146 | ~ | 20864 |
| R2 of | No. | 1~16, 18~720 | are substituted with | D29 | to form | No. | 20865 | ~ | 21583 |
| R2 of | No. | 1~17, 19~720 | are substituted with | D30 | to form | No. | 21584 | ~ | 22302 |
| R2 of | No. | 1~18, 20~720 | are substituted with | D31 | to form | No. | 22303 | ~ | 23021 |
| R2 of | No. | 1~19, 21~720 | are substituted with | D32 | to form | No. | 23022 | ~ | 23740 |
| R2 of | No. | 1~20, 22~720 | are substituted with | D33 | to form | No. | 23741 | ~ | 24459 |
| R2 of | No. | 1~21, 23~720 | are substituted with | D34 | to form | No. | 24460 | ~ | 25178 |
| R2 of | No. | 1~22, 24~720 | are substituted with | D35 | to form | No. | 25179 | ~ | 25897 |
| R2 of | No. | 1~23, 25~720 | are substituted with | D36 | to form | No. | 25898 | ~ | 26616 |
| R2 of | No. | 1~24, 26~720 | are substituted with | D37 | to form | No. | 26617 | ~ | 27335 |
| R2 of | No. | 1~25, 27~720 | are substituted with | D38 | to form | No. | 27336 | ~ | 28054 |
| R2 of | No. | 1~26, 28~720 | are substituted with | D39 | to form | No. | 28055 | ~ | 28773 |
| R2 of | No. | 1~27, 29~720 | are substituted with | D40 | to form | No. | 28774 | ~ | 29492 |
| R2 of | No. | 1~28, 30~720 | are substituted with | D41 | to form | No. | 29493 | ~ | 30211 |
| R2 of | No. | 1~29, 31~720 | are substituted with | D42 | to form | No. | 30212 | ~ | 30930 |
| R2 of | No. | 1~30, 32~720 | are substituted with | D43 | to form | No. | 30931 | ~ | 31649 |
| R2 of | No. | 1~31, 33~720 | are substituted with | D44 | to form | No. | 31650 | ~ | 32368 |
| R2 of | No. | 1~32, 34~720 | are substituted with | D45 | to form | No. | 32369 | ~ | 33087 |
| R2 of | No. | 1~33, 35~720 | are substituted with | D46 | to form | No. | 33088 | ~ | 33806 |
| R2 of | No. | 1~34, 36~720 | are substituted with | D47 | to form | No. | 33807 | ~ | 34525 |
| R2 of | No. | 1~35, 37~720 | are substituted with | D48 | to form | No. | 34526 | ~ | 35244 |
| R2 of | No. | 1~36, 38~720 | are substituted with | D49 | to form | No. | 35245 | ~ | 35963 |
| R2 of | No. | 1~37, 39~720 | are substituted with | D50 | to form | No. | 35964 | ~ | 36682 |
| R2 of | No. | 1~38, 40~720 | are substituted with | D51 | to form | No. | 36683 | ~ | 37401 |
| R2 of | No. | 1~39, 41~720 | are substituted with | D52 | to form | No. | 37402 | ~ | 38120 |
| R2 of | No. | 1~40, 42~720 | are substituted with | D53 | to form | No. | 38121 | ~ | 38839 |
| R2 of | No. | 1~41, 43~720 | are substituted with | D54 | to form | No. | 38840 | ~ | 39558 |
| R2 of | No. | 1~42, 44~720 | are substituted with | D55 | to form | No. | 39559 | ~ | 40277 |
| R2 of | No. | 1~43, 45~720 | are substituted with | D56 | to form | No. | 40278 | ~ | 40996 |
| R2 of | No. | 1~44, 46~720 | are substituted with | D57 | to form | No. | 40997 | ~ | 41715 |
| R2 of | No. | 1~45, 47~720 | are substituted with | D58 | to form | No. | 41716 | ~ | 42434 |
| R2 of | No. | 1~46, 48~720 | are substituted with | D59 | to form | No. | 42435 | ~ | 43153 |
| R2 of | No. | 1~47, 49~720 | are substituted with | D60 | to form | No. | 43154 | ~ | 43872 |

(continued)

| Group to be Substituted | | | Substituting Group | | | New Compound No. | | | |
|---|---|---|---|---|---|---|---|---|---|
| R2 of | No. | 1~48, 50~720 | are substituted with | D61 | to form | No. | 43873 | ~ | 44591 |
| R2 of | No. | 1~49, 51~720 | are substituted with | D62 | to form | No. | 44592 | ~ | 45310 |
| R2 of | No. | 1~50, 52~720 | are substituted with | D63 | to form | No. | 45311 | ~ | 46029 |
| R2 of | No. | 1~51, 53~720 | are substituted with | D64 | to form | No. | 46030 | ~ | 46748 |
| R2 of | No. | 1~52, 54~720 | are substituted with | D65 | to form | No. | 46749 | ~ | 47467 |
| R2 of | No. | 1~53, 55~720 | are substituted with | D66 | to form | No. | 47468 | ~ | 48186 |
| R2 of | No. | 1~54, 56~720 | are substituted with | D67 | to form | No. | 48187 | ~ | 48905 |
| R2 of | No. | 1~55, 57~720 | are substituted with | D68 | to form | No. | 48906 | ~ | 49624 |
| R2 of | No. | 1~56, 58~720 | are substituted with | D69 | to form | No. | 49625 | ~ | 50343 |
| R2 of | No. | 1~57, 59~720 | are substituted with | D70 | to form | No. | 50344 | ~ | 51062 |
| R2 of | No. | 1~58, 60~720 | are substituted with | D71 | to form | No. | 51063 | ~ | 51781 |
| R2 of | No. | 1~59, 61~720 | are substituted with | D72 | to form | No. | 51782 | ~ | 52500 |
| R2 of | No. | 1~60, 62~720 | are substituted with | D73 | to form | No. | 52501 | ~ | 53219 |
| R2 of | No. | 1~61, 63~720 | are substituted with | D74 | to form | No. | 53220 | ~ | 53938 |
| R2 of | No. | 1~62, 64~720 | are substituted with | D75 | to form | No. | 53939 | ~ | 54657 |
| R2 of | No. | 1~63, 65~720 | are substituted with | D76 | to form | No. | 54658 | ~ | 55376 |
| R2 of | No. | 1~64, 66~720 | are substituted with | D77 | to form | No. | 55377 | ~ | 56095 |
| R2 of | No. | 1~65, 67~720 | are substituted with | D78 | to form | No. | 56096 | ~ | 56814 |
| R2 of | No. | 1~66, 68~720 | are substituted with | D79 | to form | No. | 56815 | ~ | 57533 |
| R2 of | No. | 1~67, 69~720 | are substituted with | D80 | to form | No. | 57534 | ~ | 58252 |
| R2 of | No. | 1~68, 70~720 | are substituted with | D81 | to form | No. | 58253 | ~ | 58971 |
| R2 of | No. | 1~69, 71~720 | are substituted with | D82 | to form | No. | 58972 | ~ | 59690 |
| R2 of | No. | 1~70, 72~720 | are substituted with | D83 | to form | No. | 59691 | ~ | 60409 |
| R2 of | No. | 1~71, 73~720 | are substituted with | D84 | to form | No. | 60410 | ~ | 61128 |
| R2 of | No. | 1~72, 74~720 | are substituted with | D85 | to form | No. | 61129 | ~ | 61847 |
| R2 of | No. | 1~73, 75~720 | are substituted with | D86 | to form | No. | 61848 | ~ | 62566 |
| R2 of | No. | 1~74, 76~720 | are substituted with | D87 | to form | No. | 62567 | ~ | 63285 |
| R2 of | No. | 1~75, 77~720 | are substituted with | D88 | to form | No. | 63286 | ~ | 64004 |
| R2 of | No. | 1~76, 78~720 | are substituted with | D89 | to form | No. | 64005 | ~ | 64723 |
| R2 of | No. | 1~77, 79~720 | are substituted with | D90 | to form | No. | 64724 | ~ | 65442 |
| R2 of | No. | 1~78, 80~720 | are substituted with | D91 | to form | No. | 65443 | ~ | 66161 |
| R2 of | No. | 1~79, 81~720 | are substituted with | D92 | to form | No. | 66162 | ~ | 66880 |
| R2 of | No. | 1~80, 82~720 | are substituted with | D93 | to form | No. | 66881 | ~ | 67599 |
| R2 of | No. | 1~81, 83~720 | are substituted with | D94 | to form | No. | 67600 | ~ | 68318 |
| R2 of | No. | 1~82, 84~720 | are substituted with | D95 | to form | No. | 68319 | ~ | 69037 |
| R2 of | No. | 1~83, 85~720 | are substituted with | D96 | to form | No. | 69038 | ~ | 69756 |
| R2 of | No. | 1~84, 86~720 | are substituted with | D97 | to form | No. | 69757 | ~ | 70475 |
| R2 of | No. | 1~85, 87~720 | are substituted with | D98 | to form | No. | 70476 | ~ | 71194 |

(continued)

| Group to be Substituted | | | Substituting Group | | | New Compound No. | | | |
|---|---|---|---|---|---|---|---|---|---|
| R2 of | No. | 1~86, 88~720 | are substituted with | D99 | to form | No. | 71195 | ~ | 71913 |
| R2 of | No. | 1~87, 89~720 | are substituted with | D100 | to form | No. | 71914 | ~ | 72632 |
| R2 of | No. | 1~88, 90~720 | are substituted with | D101 | to form | No. | 72633 | ~ | 73351 |
| R2 of | No. | 1~89, 91~720 | are substituted with | D102 | to form | No. | 73352 | ~ | 74070 |
| R2 of | No. | 1~90, 92~720 | are substituted with | D103 | to form | No. | 74071 | ~ | 74789 |
| R2 of | No. | 1~91, 93~720 | are substituted with | D104 | to form | No. | 74790 | ~ | 75508 |
| R2 of | No. | 1~92, 94~720 | are substituted with | D105 | to form | No. | 75509 | ~ | 76227 |
| R2 of | No. | 1~93, 95~720 | are substituted with | D106 | to form | No. | 76228 | ~ | 76946 |
| R2 of | No. | 1~94, 96~720 | are substituted with | D107 | to form | No. | 76947 | ~ | 77665 |
| R2 of | No. | 1~95, 97~720 | are substituted with | D108 | to form | No. | 77666 | ~ | 78384 |
| R2 of | No. | 1~720 | are substituted with | D109 | to form | No. | 78385 | ~ | 79104 |
| R2 of | No. | 1~720 | are substituted with | D110 | to form | No. | 79105 | ~ | 79824 |
| R2 of | No. | 1~720 | are substituted with | D111 | to form | No. | 79825 | ~ | 80544 |
| R2 of | No. | 1~720 | are substituted with | D112 | to form | No. | 80545 | ~ | 81264 |
| R2 of | No. | 1~720 | are substituted with | D113 | to form | No. | 81265 | ~ | 81984 |
| R2 of | No. | 1~96, 98~720 | are substituted with | D114 | to form | No. | 81985 | ~ | 82703 |
| R2 of | No. | 1~97, 99~720 | are substituted with | D115 | to form | No. | 82704 | ~ | 83422 |
| R2 of | No. | 1~98, 100~720 | are substituted with | D116 | to form | No. | 83423 | ~ | 84141 |
| R2 of | No. | 1~99, 101~720 | are substituted with | D117 | to form | No. | 84142 | ~ | 84860 |
| R2 of | No. | 1~100, 102~720 | are substituted with | D118 | to form | No. | 84861 | ~ | 85579 |
| R2 of | No. | 1~101, 103~720 | are substituted with | D119 | to form | No. | 85580 | ~ | 86298 |
| R2 of | No. | 1~102, 104~720 | are substituted with | D120 | to form | No. | 86299 | ~ | 87017 |
| R2 of | No. | 1~103, 105~720 | are substituted with | D121 | to form | No. | 87018 | ~ | 87736 |
| R2 of | No. | 1~104, 106~720 | are substituted with | D122 | to form | No. | 87737 | ~ | 88455 |
| R2 of | No. | 1~105, 107~720 | are substituted with | D123 | to form | No. | 88456 | ~ | 89174 |
| R2 of | No. | 1~106, 108~720 | are substituted with | D124 | to form | No. | 89175 | ~ | 89893 |
| R2 of | No. | 1~107, 109~720 | are substituted with | D125 | to form | No. | 89894 | ~ | 90612 |
| R2 of | No. | 1~108, 110~720 | are substituted with | D126 | to form | No. | 90613 | ~ | 91331 |
| R2 of | No. | 1~109, 111~720 | are substituted with | D127 | to form | No. | 91332 | ~ | 92050 |
| R2 of | No. | 1~110, 112~720 | are substituted with | D128 | to form | No. | 92051 | ~ | 92769 |
| R2 of | No. | 1~111, 113~720 | are substituted with | D129 | to form | No. | 92770 | ~ | 93488 |
| R2 of | No. | 1~112, 114~720 | are substituted with | D130 | to form | No. | 93489 | ~ | 94207 |
| R2 of | No. | 1~113, 115~720 | are substituted with | D131 | to form | No. | 94208 | ~ | 94926 |
| R2 of | No. | 1~114, 116~720 | are substituted with | D132 | to form | No. | 94927 | ~ | 95645 |
| R2 of | No. | 1~115, 117~720 | are substituted with | D133 | to form | No. | 95646 | ~ | 96364 |
| R2 of | No. | 1~116, 118~720 | are substituted with | D134 | to form | No. | 96365 | ~ | 97083 |
| R2 of | No. | 1~117, 119~720 | are substituted with | D135 | to form | No. | 97084 | ~ | 97802 |
| R2 of | No. | 1~118, 120~720 | are substituted with | D136 | to form | No. | 97803 | ~ | 98521 |

(continued)

| Group to be Substituted | | | Substituting Group | | | New Compound No. | | | |
|---|---|---|---|---|---|---|---|---|---|
| R2 of | No. | 1~119, 121~720 | are substituted with | D137 | to form | No. | 98522 | ~ | 99240 |
| R2 of | No. | 1~120, 122~720 | are substituted with | D138 | to form | No. | 99241 | ~ | 99959 |
| R2 of | No. | 1~121, 123~720 | are substituted with | D139 | to form | No. | 99960 | ~ | 100678 |
| R2 of | No. | 1~122, 124~720 | are substituted with | D140 | to form | No. | 100679 | ~ | 101397 |
| R2 of | No. | 1~123, 125~720 | are substituted with | D141 | to form | No. | 101398 | ~ | 102116 |
| R2 of | No. | 1~124, 126~720 | are substituted with | D142 | to form | No. | 102117 | ~ | 102835 |
| R2 of | No. | 1~125, 127~720 | are substituted with | D143 | to form | No. | 102836 | ~ | 103554 |
| R2 of | No. | 1~126, 128~720 | are substituted with | D144 | to form | No. | 103555 | ~ | 104273 |
| R2 of | No. | 1~127, 129~720 | are substituted with | D145 | to form | No. | 104274 | ~ | 104992 |
| R2 of | No. | 1~128, 130~720 | are substituted with | D146 | to form | No. | 104993 | ~ | 105711 |
| R2 of | No. | 1~129, 131~720 | are substituted with | D147 | to form | No. | 105712 | ~ | 106430 |
| R2 of | No. | 1~130, 132~720 | are substituted with | D148 | to form | No. | 106431 | ~ | 107149 |
| R2 of | No. | 1~131, 133~720 | are substituted with | D149 | to form | No. | 107150 | ~ | 107868 |
| R2 of | No. | 1~720 | are substituted with | D150 | to form | No. | 107869 | ~ | 108588 |
| R2 of | No. | 1~720 | are substituted with | D151 | to form | No. | 108589 | ~ | 109308 |
| R2 of | No. | 1~720 | are substituted with | D152 | to form | No. | 109309 | ~ | 110028 |
| R2 of | No. | 1~720 | are substituted with | D153 | to form | No. | 110029 | ~ | 110748 |
| R2 of | No. | 1~720 | are substituted with | D154 | to form | No. | 110749 | ~ | 111468 |
| R2 of | No. | 1~720 | are substituted with | D155 | to form | No. | 111469 | ~ | 112188 |
| R2 of | No. | 1~720 | are substituted with | D156 | to form | No. | 112189 | ~ | 112908 |
| R2 of | No. | 1~720 | are substituted with | D157 | to form | No. | 112909 | ~ | 113628 |
| R2 of | No. | 1~720 | are substituted with | D158 | to form | No. | 113629 | ~ | 114348 |
| R2 of | No. | 1~720 | are substituted with | D159 | to form | No. | 114349 | ~ | 115068 |
| R2 of | No. | 1~720 | are substituted with | D160 | to form | No. | 115069 | ~ | 115788 |
| R2 of | No. | 1~720 | are substituted with | D161 | to form | No. | 115789 | ~ | 116508 |
| R2 of | No. | 1~720 | are substituted with | D162 | to form | No. | 116509 | ~ | 117228 |
| R2 of | No. | 1~720 | are substituted with | D163 | to form | No. | 117229 | ~ | 117948 |
| R2 of | No. | 1~720 | are substituted with | D164 | to form | No. | 117949 | ~ | 118668 |
| R2 of | No. | 1~720 | are substituted with | D165 | to form | No. | 118669 | ~ | 119388 |
| R2 of | No. | 1~720 | are substituted with | D166 | to form | No. | 119389 | ~ | 120108 |
| R2 of | No. | 1~720 | are substituted with | D167 | to form | No. | 120109 | ~ | 120828 |
| R2 of | No. | 1~720 | are substituted with | D168 | to form | No. | 120829 | ~ | 121548 |
| R2 of | No. | 1~720 | are substituted with | D169 | to form | No. | 121549 | ~ | 122268 |
| R2 of | No. | 1~720 | are substituted with | D170 | to form | No. | 122269 | ~ | 122988 |
| R2 of | No. | 1~720 | are substituted with | D171 | to form | No. | 122989 | ~ | 123708 |
| R2 of | No. | 1~720 | are substituted with | D172 | to form | No. | 123709 | ~ | 124428 |
| R2 of | No. | 1~720 | are substituted with | D173 | to form | No. | 124429 | ~ | 125148 |
| R2 of | No. | 1~720 | are substituted with | D174 | to form | No. | 125149 | ~ | 125868 |

(continued)

| Group to be Substituted | | | Substituting Group | | | New Compound No. | | | |
|---|---|---|---|---|---|---|---|---|---|
| R2 of | No. | 1~720 | are substituted with | D175 | to form | No. | 125869 | ~ | 126588 |
| R2 of | No. | 1~720 | are substituted with | D176 | to form | No. | 126589 | ~ | 127308 |
| R2 of | No. | 1~720 | are substituted with | D177 | to form | No. | 127309 | ~ | 128028 |
| R2 of | No. | 1~720 | are substituted with | D178 | to form | No. | 128029 | ~ | 128748 |
| R2 of | No. | 1~720 | are substituted with | D179 | to form | No. | 128749 | ~ | 129468 |
| R2 of | No. | 1~132, 134~720 | are substituted with | D180 | to form | No. | 129469 | ~ | 130187 |
| R2 of | No. | 1~133, 135~720 | are substituted with | D181 | to form | No. | 130188 | ~ | 130906 |
| R2 of | No. | 1~134, 136~720 | are substituted with | D182 | to form | No. | 130907 | ~ | 131625 |
| R2 of | No. | 1~135, 135~720 | are substituted with | D183 | to form | No. | 131626 | ~ | 132344 |
| R2 of | No. | 1~136, 138~720 | are substituted with | D184 | to form | No. | 132345 | ~ | 133063 |
| R2 of | No. | 1~137, 139~720 | are substituted with | D185 | to form | No. | 133064 | ~ | 133782 |
| R2 of | No. | 1~138, 140~720 | are substituted with | D186 | to form | No. | 133783 | ~ | 134501 |
| R2 of | No. | 1~139, 141~720 | are substituted with | D187 | to form | No. | 134502 | ~ | 135220 |
| R2 of | No. | 1~140, 142~720 | are substituted with | D188 | to form | No. | 135221 | ~ | 135939 |
| R2 of | No. | 1~141, 143~720 | are substituted with | D189 | to form | No. | 135940 | ~ | 136658 |
| R2 of | No. | 1~142, 144~720 | are substituted with | D190 | to form | No. | 136659 | ~ | 137377 |
| R2 of | No. | 1~143, 145~720 | are substituted with | D191 | to form | No. | 137378 | ~ | 138096 |
| R2 of | No. | 1~720 | are substituted with | D192 | to form | No. | 138097 | ~ | 138816 |
| R2 of | No. | 1~720 | are substituted with | D193 | to form | No. | 138817 | ~ | 139536 |
| R2 of | No. | 1~720 | are substituted with | D194 | to form | No. | 139537 | ~ | 140256 |
| R2 of | No. | 1~720 | are substituted with | D195 | to form | No. | 140257 | ~ | 140976 |
| R2 of | No. | 1~720 | are substituted with | D196 | to form | No. | 140977 | ~ | 141696 |
| R2 of | No. | 1~720 | are substituted with | D197 | to form | No. | 141697 | ~ | 142416 |
| R2 of | No. | 1~144, 146~720 | are substituted with | D198 | to form | No. | 142417 | ~ | 143135 |
| R2 of | No. | 1~145, 147~720 | are substituted with | D199 | to form | No. | 143136 | ~ | 143854 |
| R2 of | No. | 1~146, 148~720 | are substituted with | D200 | to form | No. | 143855 | ~ | 144573 |
| R2 of | No. | 1~147, 149~720 | are substituted with | D201 | to form | No. | 144574 | ~ | 145292 |
| R2 of | No. | 1~148, 150~720 | are substituted with | D202 | to form | No. | 145293 | ~ | 146011 |
| R2 of | No. | 1~149, 151~720 | are substituted with | D203 | to form | No. | 146012 | ~ | 146730 |
| R2 of | No. | 1~150, 152~720 | are substituted with | D204 | to form | No. | 146731 | ~ | 147449 |
| R2 of | No. | 1~151, 153~720 | are substituted with | D205 | to form | No. | 147450 | ~ | 148168 |
| R2 of | No. | 1~152, 154~720 | are substituted with | D206 | to form | No. | 148169 | ~ | 148887 |
| R2 of | No. | 1~153, 155~720 | are substituted with | D207 | to form | No. | 148888 | ~ | 149606 |
| R2 of | No. | 1~154, 156~720 | are substituted with | D208 | to form | No. | 149607 | ~ | 150325 |
| R2 of | No. | 1~155, 157~720 | are substituted with | D209 | to form | No. | 150326 | ~ | 151044 |
| R2 of | No. | 1~156, 158~720 | are substituted with | D210 | to form | No. | 151045 | ~ | 151763 |
| R2 of | No. | 1~157, 159~720 | are substituted with | D211 | to form | No. | 151764 | ~ | 152482 |
| R2 of | No. | 1~158, 160~720 | are substituted with | D212 | to form | No. | 152483 | ~ | 153201 |

(continued)

| Group to be Substituted | | | Substituting Group | | | New Compound No. | | | |
|---|---|---|---|---|---|---|---|---|---|
| R2 of | No. | 1~159, 161~720 | are substituted with | D213 | to form | No. | 153202 | ~ | 153920 |
| R2 of | No. | 1~160, 162~720 | are substituted with | D214 | to form | No. | 153921 | ~ | 154639 |
| R2 of | No. | 1~161, 163~720 | are substituted with | D215 | to form | No. | 154640 | ~ | 155358 |
| R2 of | No. | 1~162, 164~720 | are substituted with | D216 | to form | No. | 155359 | ~ | 156077 |
| R2 of | No. | 1~163, 165~720 | are substituted with | D217 | to form | No. | 156078 | ~ | 156796 |
| R2 of | No. | 1~164, 166~720 | are substituted with | D218 | to form | No. | 156797 | ~ | 157515 |
| R2 of | No. | 1~165, 167~720 | are substituted with | D219 | to form | No. | 157516 | ~ | 158234 |
| R2 of | No. | 1~166, 168~720 | are substituted with | D220 | to form | No. | 158235 | ~ | 158953 |
| R2 of | No. | 1~167, 169~720 | are substituted with | D221 | to form | No. | 158954 | ~ | 159672 |
| R2 of | No. | 1~168, 170~720 | are substituted with | D222 | to form | No. | 159673 | ~ | 160391 |
| R2 of | No. | 1~169, 171~720 | are substituted with | D223 | to form | No. | 160392 | ~ | 161110 |
| R2 of | No. | 1~170, 172~720 | are substituted with | D224 | to form | No. | 161111 | ~ | 161829 |
| R2 of | No. | 1~171, 173~720 | are substituted with | D225 | to form | No. | 161830 | ~ | 162548 |
| R2 of | No. | 1~172, 174~720 | are substituted with | D226 | to form | No. | 162549 | ~ | 163267 |
| R2 of | No. | 1~173, 175~720 | are substituted with | D227 | to form | No. | 163268 | ~ | 163986 |
| R2 of | No. | 1~174, 176~720 | are substituted with | D228 | to form | No. | 163987 | ~ | 164705 |
| R2 of | No. | 1~175, 177~720 | are substituted with | D229 | to form | No. | 164706 | ~ | 165424 |
| R2 of | No. | 1~176, 178~720 | are substituted with | D230 | to form | No. | 165425 | ~ | 166143 |
| R2 of | No. | 1~177, 179~720 | are substituted with | D231 | to form | No. | 166144 | ~ | 166862 |
| R2 of | No. | 1~178, 180~720 | are substituted with | D232 | to form | No. | 166863 | ~ | 167581 |
| R2 of | No. | 1~179, 181~720 | are substituted with | D233 | to form | No. | 167582 | ~ | 168300 |
| R2 of | No. | 1~180, 182~720 | are substituted with | D234 | to form | No. | 168301 | ~ | 169019 |
| R2 of | No. | 1~181, 183~720 | are substituted with | D235 | to form | No. | 169020 | ~ | 169738 |
| R2 of | No. | 1~182, 184~720 | are substituted with | D236 | to form | No. | 169739 | ~ | 170457 |
| R2 of | No. | 1~183, 185~720 | are substituted with | D237 | to form | No. | 170458 | ~ | 171176 |
| R2 of | No. | 1~184, 186~720 | are substituted with | D238 | to form | No. | 171177 | ~ | 171895 |
| R2 of | No. | 1~185, 187~720 | are substituted with | D239 | to form | No. | 171896 | ~ | 172614 |
| R2 of | No. | 1~720 | are substituted with | D240 | to form | No. | 172615 | ~ | 173334 |
| R2 of | No. | 1~720 | are substituted with | D241 | to form | No. | 173335 | ~ | 174054 |
| R2 of | No. | 1~720 | are substituted with | D242 | to form | No. | 174055 | ~ | 174774 |
| R2 of | No. | 1~720 | are substituted with | D243 | to form | No. | 174775 | ~ | 175494 |
| R2 of | No. | 1~720 | are substituted with | D244 | to form | No. | 175495 | ~ | 176214 |
| R2 of | No. | 1~186, 188~720 | are substituted with | D245 | to form | No. | 176215 | ~ | 176933 |
| R2 of | No. | 1~187, 189~720 | are substituted with | D246 | to form | No. | 176934 | ~ | 177652 |
| R2 of | No. | 1~188, 190~720 | are substituted with | D247 | to form | No. | 177653 | ~ | 178371 |
| R2 of | No. | 1~189, 191~720 | are substituted with | D248 | to form | No. | 178372 | ~ | 179090 |
| R2 of | No. | 1~190, 192~720 | are substituted with | D249 | to form | No. | 179091 | ~ | 179809 |
| R2 of | No. | 1~191, 193~720 | are substituted with | D250 | to form | No. | 179810 | ~ | 180528 |

(continued)

| Group to be Substituted | | | Substituting Group | | | New Compound No. | | | |
|---|---|---|---|---|---|---|---|---|---|
| R2 of | No. | 1~192, 194~720 | are substituted with | D251 | to form | No. | 180529 | ~ | 181247 |
| R2 of | No. | 1~193, 195~720 | are substituted with | D252 | to form | No. | 181248 | ~ | 181966 |
| R2 of | No. | 1~194, 196~720 | are substituted with | D253 | to form | No. | 181967 | ~ | 182685 |
| R2 of | No. | 1~195, 197~720 | are substituted with | D254 | to form | No. | 182686 | ~ | 183404 |
| R2 of | No. | 1~196, 198~720 | are substituted with | D255 | to form | No. | 183405 | ~ | 184123 |
| R2 of | No. | 1~197, 199~720 | are substituted with | D256 | to form | No. | 184124 | ~ | 184842 |
| R2 of | No. | 1~198, 200~720 | are substituted with | D257 | to form | No. | 184843 | ~ | 185561 |
| R2 of | No. | 1~199, 201~720 | are substituted with | D258 | to form | No. | 185562 | ~ | 186280 |
| R2 of | No. | 1~200, 202~720 | are substituted with | D259 | to form | No. | 186281 | ~ | 186999 |
| R2 of | No. | 1~201, 203~720 | are substituted with | D260 | to form | No. | 187000 | ~ | 187718 |
| R2 of | No. | 1~202, 204~720 | are substituted with | D261 | to form | No. | 187719 | ~ | 188437 |
| R2 of | No. | 1~203, 205~720 | are substituted with | D262 | to form | No. | 188438 | ~ | 189156 |
| R2 of | No. | 1~204, 206~720 | are substituted with | D263 | to form | No. | 189157 | ~ | 189875 |
| R2 of | No. | 1~205, 207~720 | are substituted with | D264 | to form | No. | 189876 | ~ | 190594 |
| R2 of | No. | 1~206, 208~720 | are substituted with | D265 | to form | No. | 190595 | ~ | 191313 |
| R2 of | No. | 1~207, 209~720 | are substituted with | D266 | to form | No. | 191314 | ~ | 192032 |
| R2 of | No. | 1~208, 210~720 | are substituted with | D267 | to form | No. | 192033 | ~ | 192751 |
| R2 of | No. | 1~209, 211~720 | are substituted with | D268 | to form | No. | 192752 | ~ | 193470 |
| R2 of | No. | 1~210, 212~720 | are substituted with | D269 | to form | No. | 193471 | ~ | 194189 |
| R2 of | No. | 1~211, 213~720 | are substituted with | D270 | to form | No. | 194190 | ~ | 194908 |
| R2 of | No. | 1~212, 214~720 | are substituted with | D271 | to form | No. | 194909 | ~ | 195627 |
| R2 of | No. | 1~213, 215~720 | are substituted with | D272 | to form | No. | 195628 | ~ | 196346 |
| R2 of | No. | 1~214, 216~720 | are substituted with | D273 | to form | No. | 196347 | ~ | 197065 |
| R2 of | No. | 1~215, 217~720 | are substituted with | D274 | to form | No. | 197066 | ~ | 197784 |
| R2 of | No. | 1~216, 218~720 | are substituted with | D275 | to form | No. | 197785 | ~ | 198503 |
| R2 of | No. | 1~217, 219~720 | are substituted with | D276 | to form | No. | 198504 | ~ | 199222 |
| R2 of | No. | 1~218, 220~720 | are substituted with | D277 | to form | No. | 199223 | ~ | 199941 |
| R2 of | No. | 1~219, 221~720 | are substituted with | D278 | to form | No. | 199942 | ~ | 200660 |
| R2 of | No. | 1~220, 222~720 | are substituted with | D279 | to form | No. | 200661 | ~ | 201379 |
| R2 of | No. | 1~221, 223~720 | are substituted with | D280 | to form | No. | 201380 | ~ | 202098 |
| R2 of | No. | 1~222, 224~720 | are substituted with | D281 | to form | No. | 202099 | ~ | 202817 |
| R2 of | No. | 1~223, 225~720 | are substituted with | D282 | to form | No. | 202818 | ~ | 203536 |
| R2 of | No. | 1~224, 226~720 | are substituted with | D283 | to form | No. | 203537 | ~ | 204255 |
| R2 of | No. | 1~225, 227~720 | are substituted with | D284 | to form | No. | 204256 | ~ | 204974 |
| R2 of | No. | 1~226, 228~720 | are substituted with | D285 | to form | No. | 204975 | ~ | 205693 |
| R2 of | No. | 1~227, 229~720 | are substituted with | D286 | to form | No. | 205694 | ~ | 206412 |
| R2 of | No. | 1~228, 230~720 | are substituted with | D287 | to form | No. | 206413 | ~ | 207131 |
| R2 of | No. | 1~229, 231~720 | are substituted with | D288 | to form | No. | 207132 | ~ | 207850 |

(continued)

| Group to be Substituted | | | Substituting Group | | | New Compound No. | | | |
|---|---|---|---|---|---|---|---|---|---|
| R2 of | No. | 1~230, 232~720 | are substituted with | D289 | to form | No. | 207851 | ~ | 208569 |
| R2 of | No. | 1~231, 233~720 | are substituted with | D290 | to form | No. | 208570 | ~ | 209288 |
| R2 of | No. | 1~232, 234~720 | are substituted with | D291 | to form | No. | 209289 | ~ | 210007 |
| R2 of | No. | 1~233, 235~720 | are substituted with | D292 | to form | No. | 210008 | ~ | 210726 |
| R2 of | No. | 1~234, 236~720 | are substituted with | D293 | to form | No. | 210727 | ~ | 211445 |
| R2 of | No. | 1~235, 237~720 | are substituted with | D294 | to form | No. | 211446 | ~ | 212164 |
| R2 of | No. | 1~236, 238~720 | are substituted with | D295 | to form | No. | 212165 | ~ | 212883 |
| R2 of | No. | 1~237, 239~720 | are substituted with | D296 | to form | No. | 212884 | ~ | 213602 |
| R2 of | No. | 1~238, 240~720 | are substituted with | D297 | to form | No. | 213603 | ~ | 214321 |
| R2 of | No. | 1~239, 241~720 | are substituted with | D298 | to form | No. | 214322 | ~ | 215040 |
| R2 of | No. | 1~720 | are substituted with | D299 | to form | No. | 215041 | ~ | 215760 |
| R2 of | No. | 1~240, 242~720 | are substituted with | D300 | to form | No. | 215761 | ~ | 216479 |
| R2 of | No. | 1~241, 243~720 | are substituted with | D301 | to form | No. | 216480 | ~ | 217198 |
| R2 of | No. | 1~242, 244~720 | are substituted with | D302 | to form | No. | 217199 | ~ | 217917 |
| R2 of | No. | 1~243, 245~720 | are substituted with | D303 | to form | No. | 217918 | ~ | 218636 |
| R2 of | No. | 1~244, 246~720 | are substituted with | D304 | to form | No. | 218637 | ~ | 219355 |
| R2 of | No. | 1~245, 247~720 | are substituted with | D305 | to form | No. | 219356 | ~ | 220074 |
| R2 of | No. | 1~246, 248~720 | are substituted with | D306 | to form | No. | 220075 | ~ | 220793 |
| R2 of | No. | 1~247, 249~720 | are substituted with | D307 | to form | No. | 220794 | ~ | 221512 |
| R2 of | No. | 1~248, 250~720 | are substituted with | D308 | to form | No. | 221513 | ~ | 222231 |
| R2 of | No. | 1~249, 251~720 | are substituted with | D309 | to form | No. | 222232 | ~ | 222950 |
| R2 of | No. | 1~250, 252~720 | are substituted with | D310 | to form | No. | 222951 | ~ | 223669 |
| R2 of | No. | 1~251, 253~720 | are substituted with | D311 | to form | No. | 223670 | ~ | 224388 |
| R2 of | No. | 1~252, 254~720 | are substituted with | D312 | to form | No. | 224389 | ~ | 225107 |
| R2 of | No. | 1~253, 255~720 | are substituted with | D313 | to form | No. | 225108 | ~ | 225826 |
| R2 of | No. | 1~254, 256~720 | are substituted with | D314 | to form | No. | 225827 | ~ | 226545 |
| R2 of | No. | 1~255, 257~720 | are substituted with | D315 | to form | No. | 226546 | ~ | 227264 |
| R2 of | No. | 1~256, 258~720 | are substituted with | D316 | to form | No. | 227265 | ~ | 227983 |
| R2 of | No. | 1~257, 259~720 | are substituted with | D317 | to form | No. | 227984 | ~ | 228702 |
| R2 of | No. | 1~258, 260~720 | are substituted with | D318 | to form | No. | 228703 | ~ | 229421 |
| R2 of | No. | 1~259, 261~720 | are substituted with | D319 | to form | No. | 229422 | ~ | 230140 |
| R2 of | No. | 1~260, 262~720 | are substituted with | D320 | to form | No. | 230141 | ~ | 230859 |
| R2 of | No. | 1~261, 263~720 | are substituted with | D321 | to form | No. | 230860 | ~ | 231578 |
| R2 of | No. | 1~262, 264~720 | are substituted with | D322 | to form | No. | 231579 | ~ | 232297 |
| R2 of | No. | 1~263, 265~720 | are substituted with | D323 | to form | No. | 232298 | ~ | 233016 |
| R2 of | No. | 1~264, 266~720 | are substituted with | D324 | to form | No. | 233017 | ~ | 233735 |
| R2 of | No. | 1~265, 267~720 | are substituted with | D325 | to form | No. | 233736 | ~ | 234454 |
| R2 of | No. | 1~266, 268~720 | are substituted with | D326 | to form | No. | 234455 | ~ | 235173 |

(continued)

| Group to be Substituted | | | Substituting Group | | | New Compound No. | | | |
|---|---|---|---|---|---|---|---|---|---|
| R2 of | No. | 1~267, 269~720 | are substituted with | D327 | to form | No. | 235174 | ~ | 235892 |
| R2 of | No. | 1~268, 270~720 | are substituted with | D328 | to form | No. | 235893 | ~ | 236611 |
| R2 of | No. | 1~269, 271~720 | are substituted with | D329 | to form | No. | 236612 | ~ | 237330 |
| R2 of | No. | 1~270, 272~720 | are substituted with | D330 | to form | No. | 237331 | ~ | 238049 |
| R2 of | No. | 1~271, 273~720 | are substituted with | D331 | to form | No. | 238050 | ~ | 238768 |
| R2 of | No. | 1~272, 274~720 | are substituted with | D332 | to form | No. | 238769 | ~ | 239487 |
| R2 of | No. | 1~273, 275~720 | are substituted with | D333 | to form | No. | 239488 | ~ | 240206 |
| R2 of | No. | 1~274, 276~720 | are substituted with | D334 | to form | No. | 240207 | ~ | 240925 |
| R2 of | No. | 1~275, 277~720 | are substituted with | D335 | to form | No. | 240926 | ~ | 241644 |
| R2 of | No. | 1~276, 278~720 | are substituted with | D336 | to form | No. | 241645 | ~ | 242363 |
| R2 of | No. | 1~277, 279~720 | are substituted with | D337 | to form | No. | 242364 | ~ | 243082 |
| R2 of | No. | 1~278, 280~720 | are substituted with | D338 | to form | No. | 243083 | ~ | 243801 |
| R2 of | No. | 1~279, 281~720 | are substituted with | D339 | to form | No. | 243802 | ~ | 244520 |
| R2 of | No. | 1~280, 282~720 | are substituted with | D340 | to form | No. | 244521 | ~ | 245239 |
| R2 of | No. | 1~281, 283~720 | are substituted with | D341 | to form | No. | 245240 | ~ | 245958 |
| R2 of | No. | 1~282, 284~720 | are substituted with | D342 | to form | No. | 245959 | ~ | 246677 |
| R2 of | No. | 1~283, 285~720 | are substituted with | D343 | to form | No. | 246678 | ~ | 247396 |
| R2 of | No. | 1~284, 286~720 | are substituted with | D344 | to form | No. | 247397 | ~ | 248115 |
| R2 of | No. | 1~285, 287~720 | are substituted with | D345 | to form | No. | 248116 | ~ | 248834 |
| R2 of | No. | 1~286, 288~720 | are substituted with | D346 | to form | No. | 248835 | ~ | 249553 |
| R2 of | No. | 1~287, 289~720 | are substituted with | D347 | to form | No. | 249554 | ~ | 250272 |
| R2 of | No. | 1~288, 290~720 | are substituted with | D348 | to form | No. | 250273 | ~ | 250991 |
| R2 of | No. | 1~289, 291~720 | are substituted with | D349 | to form | No. | 250992 | ~ | 251710 |
| R2 of | No. | 1~290, 292~720 | are substituted with | D350 | to form | No. | 251711 | ~ | 252429 |
| R2 of | No. | 1~291, 293~720 | are substituted with | D351 | to form | No. | 252430 | ~ | 253148 |
| R2 of | No. | 1~292, 294~720 | are substituted with | D352 | to form | No. | 253149 | ~ | 253867 |
| R2 of | No. | 1~293, 295~720 | are substituted with | D353 | to form | No. | 253868 | ~ | 254586 |
| R2 of | No. | 1~294, 296~720 | are substituted with | D354 | to form | No. | 254587 | ~ | 255305 |
| R2 of | No. | 1~295, 297~720 | are substituted with | D355 | to form | No. | 255306 | ~ | 256024 |
| R2 of | No. | 1~296, 298~720 | are substituted with | D356 | to form | No. | 256025 | ~ | 256743 |
| R2 of | No. | 1~297, 299~720 | are substituted with | D357 | to form | No. | 256744 | ~ | 257462 |
| R2 of | No. | 1~298, 300~720 | are substituted with | D358 | to form | No. | 257463 | ~ | 258181 |
| R2 of | No. | 1~299, 301~720 | are substituted with | D359 | to form | No. | 258182 | ~ | 258900 |
| R2 of | No. | 1~300, 302~720 | are substituted with | D360 | to form | No. | 258901 | ~ | 259619 |
| R2 of | No. | 1~301, 303~720 | are substituted with | D361 | to form | No. | 259620 | ~ | 260338 |
| R2 of | No. | 1~302, 304~720 | are substituted with | D362 | to form | No. | 260339 | ~ | 261057 |
| R2 of | No. | 1~303, 305~720 | are substituted with | D363 | to form | No. | 261058 | ~ | 261776 |
| R2 of | No. | 1~304, 306~720 | are substituted with | D364 | to form | No. | 261777 | ~ | 262495 |

(continued)

| Group to be Substituted | | | Substituting Group | | | New Compound No. | | | |
|---|---|---|---|---|---|---|---|---|---|
| R2 of | No. | 1~305, 307~720 | are substituted with | D365 | to form | No. | 262496 | ~ | 263214 |
| R2 of | No. | 1~306, 308~720 | are substituted with | D366 | to form | No. | 263215 | ~ | 263933 |
| R2 of | No. | 1~307, 309~720 | are substituted with | D367 | to form | No. | 263934 | ~ | 264652 |
| R2 of | No. | 1~308, 310~720 | are substituted with | D368 | to form | No. | 264653 | ~ | 265371 |
| R2 of | No. | 1~309, 311~720 | are substituted with | D369 | to form | No. | 265372 | ~ | 266090 |
| R2 of | No. | 1~310, 312~720 | are substituted with | D370 | to form | No. | 266091 | ~ | 266809 |
| R2 of | No. | 1~311, 313~720 | are substituted with | D371 | to form | No. | 266810 | ~ | 267528 |
| R2 of | No. | 1~312, 314~720 | are substituted with | D372 | to form | No. | 267529 | ~ | 268247 |
| R2 of | No. | 1~313, 315~720 | are substituted with | D373 | to form | No. | 268248 | ~ | 268966 |
| R2 of | No. | 1~314, 316~720 | are substituted with | D374 | to form | No. | 268967 | ~ | 269685 |
| R2 of | No. | 1~315, 317~720 | are substituted with | D375 | to form | No. | 269686 | ~ | 270404 |
| R2 of | No. | 1~316, 318~720 | are substituted with | D376 | to form | No. | 270405 | ~ | 271123 |
| R2 of | No. | 1~317, 319~720 | are substituted with | D377 | to form | No. | 271124 | ~ | 271842 |
| R2 of | No. | 1~318, 320~720 | are substituted with | D378 | to form | No. | 271843 | ~ | 272561 |
| R2 of | No. | 1~319, 321~720 | are substituted with | D379 | to form | No. | 272562 | ~ | 273280 |
| R2 of | No. | 1~320, 322~720 | are substituted with | D380 | to form | No. | 273281 | ~ | 273999 |
| R2 of | No. | 1~321, 323~720 | are substituted with | D381 | to form | No. | 274000 | ~ | 274718 |
| R2 of | No. | 1~322, 324~720 | are substituted with | D382 | to form | No. | 274719 | ~ | 275437 |
| R2 of | No. | 1~323, 325~720 | are substituted with | D383 | to form | No. | 275438 | ~ | 276156 |
| R2 of | No. | 1~324, 326~720 | are substituted with | D384 | to form | No. | 276157 | ~ | 276875 |
| R2 of | No. | 1~325, 327~720 | are substituted with | D385 | to form | No. | 276876 | ~ | 277594 |
| R2 of | No. | 1~326, 328~720 | are substituted with | D386 | to form | No. | 277595 | ~ | 278313 |
| R2 of | No. | 1~327, 329~720 | are substituted with | D387 | to form | No. | 278314 | ~ | 279032 |
| R2 of | No. | 1~328, 330~720 | are substituted with | D388 | to form | No. | 279033 | ~ | 279751 |
| R2 of | No. | 1~329, 331~720 | are substituted with | D389 | to form | No. | 279752 | ~ | 280470 |
| R2 of | No. | 1~330, 332~720 | are substituted with | D390 | to form | No. | 280471 | ~ | 281189 |
| R2 of | No. | 1~331, 333~720 | are substituted with | D391 | to form | No. | 281190 | ~ | 281908 |
| R2 of | No. | 1~332, 334~720 | are substituted with | D392 | to form | No. | 281909 | ~ | 282627 |
| R2 of | No. | 1~333, 335~720 | are substituted with | D393 | to form | No. | 282628 | ~ | 283346 |
| R2 of | No. | 1~334, 336~720 | are substituted with | D394 | to form | No. | 283347 | ~ | 284065 |
| R2 of | No. | 1~335, 337~720 | are substituted with | D395 | to form | No. | 284066 | ~ | 284784 |
| R2 of | No. | 1~336, 338~720 | are substituted with | D396 | to form | No. | 284785 | ~ | 285503 |
| R2 of | No. | 1~337, 339~720 | are substituted with | D397 | to form | No. | 285504 | ~ | 286222 |
| R2 of | No. | 1~338, 340~720 | are substituted with | D398 | to form | No. | 286223 | ~ | 286941 |
| R2 of | No. | 1~339, 341~720 | are substituted with | D399 | to form | No. | 286942 | ~ | 287660 |
| R2 of | No. | 1~340, 342~720 | are substituted with | D400 | to form | No. | 287661 | ~ | 288379 |
| R2 of | No. | 1~341, 343~720 | are substituted with | D401 | to form | No. | 288380 | ~ | 289098 |
| R2 of | No. | 1~342, 344~720 | are substituted with | D402 | to form | No. | 289099 | ~ | 289817 |

(continued)

| Group to be Substituted | | | Substituting Group | | | New Compound No. | | | |
|---|---|---|---|---|---|---|---|---|---|
| R2 of | No. | 1~343, 345~720 | are substituted with | D403 | to form | No. | 289818 | ~ | 290536 |
| R2 of | No. | 1~344, 346~720 | are substituted with | D404 | to form | No. | 290537 | ~ | 291255 |
| R2 of | No. | 1~345, 347~720 | are substituted with | D405 | to form | No. | 291256 | ~ | 291974 |
| R2 of | No. | 1~346, 348~720 | are substituted with | D406 | to form | No. | 291975 | ~ | 292693 |
| R2 of | No. | 1~347, 349~720 | are substituted with | D407 | to form | No. | 292694 | ~ | 293412 |
| R2 of | No. | 1~348, 350~720 | are substituted with | D408 | to form | No. | 293413 | ~ | 294131 |
| R2 of | No. | 1~349, 351~720 | are substituted with | D409 | to form | No. | 294132 | ~ | 294850 |
| R2 of | No. | 1~350, 352~720 | are substituted with | D410 | to form | No. | 294851 | ~ | 295569 |
| R2 of | No. | 1~351, 353~720 | are substituted with | D411 | to form | No. | 295570 | ~ | 296288 |
| R2 of | No. | 1~352, 354~720 | are substituted with | D412 | to form | No. | 296289 | ~ | 297007 |
| R2 of | No. | 1~353, 355~720 | are substituted with | D413 | to form | No. | 297008 | ~ | 297726 |
| R2 of | No. | 1~354, 356~720 | are substituted with | D414 | to form | No. | 297727 | ~ | 298445 |
| R2 of | No. | 1~355, 357~720 | are substituted with | D415 | to form | No. | 298446 | ~ | 299164 |
| R2 of | No. | 1~356, 358~720 | are substituted with | D416 | to form | No. | 299165 | ~ | 299883 |
| R2 of | No. | 1~357, 359~720 | are substituted with | D417 | to form | No. | 299884 | ~ | 300602 |
| R2 of | No. | 1~358, 360~720 | are substituted with | D418 | to form | No. | 300603 | ~ | 301321 |
| R2 of | No. | 1~359, 361~720 | are substituted with | D419 | to form | No. | 301322 | ~ | 302040 |
| R2 of | No. | 1~360, 362~720 | are substituted with | D420 | to form | No. | 302041 | ~ | 302759 |
| R2 of | No. | 1~361, 363~720 | are substituted with | D421 | to form | No. | 302760 | ~ | 303478 |
| R2 of | No. | 1~362, 364~720 | are substituted with | D422 | to form | No. | 303479 | ~ | 304197 |
| R2 of | No. | 1~363, 365~720 | are substituted with | D423 | to form | No. | 304198 | ~ | 304916 |
| R2 of | No. | 1~364, 366~720 | are substituted with | D424 | to form | No. | 304917 | ~ | 305635 |
| R2 of | No. | 1~365, 367~720 | are substituted with | D425 | to form | No. | 305636 | ~ | 306354 |
| R2 of | No. | 1~366, 368~720 | are substituted with | D426 | to form | No. | 306355 | ~ | 307073 |
| R2 of | No. | 1~367, 369~720 | are substituted with | D427 | to form | No. | 307074 | ~ | 307792 |
| R2 of | No. | 1~368, 370~720 | are substituted with | D428 | to form | No. | 307793 | ~ | 308511 |
| R2 of | No. | 1~369, 371~720 | are substituted with | D429 | to form | No. | 308512 | ~ | 309230 |
| R2 of | No. | 1~370, 372~720 | are substituted with | D430 | to form | No. | 309231 | ~ | 309949 |
| R2 of | No. | 1~371, 373~720 | are substituted with | D431 | to form | No. | 309950 | ~ | 310668 |
| R2 of | No. | 1~372, 374~720 | are substituted with | D432 | to form | No. | 310669 | ~ | 311387 |
| R2 of | No. | 1~720 | are substituted with | D433 | to form | No. | 311388 | ~ | 312107 |
| R2 of | No. | 1~373, 375~720 | are substituted with | D434 | to form | No. | 312108 | ~ | 312826 |
| R2 of | No. | 1~374, 376~720 | are substituted with | D435 | to form | No. | 312827 | ~ | 313545 |
| R2 of | No. | 1~375, 377~720 | are substituted with | D436 | to form | No. | 313546 | ~ | 314264 |
| R2 of | No. | 1~376, 378~720 | are substituted with | D437 | to form | No. | 314265 | ~ | 314983 |
| R2 of | No. | 1~377, 379~720 | are substituted with | D438 | to form | No. | 314984 | ~ | 315702 |
| R2 of | No. | 1~378, 380~720 | are substituted with | D439 | to form | No. | 315703 | ~ | 316421 |
| R2 of | No. | 1~379, 381~720 | are substituted with | D440 | to form | No. | 316422 | ~ | 317140 |

(continued)

| Group to be Substituted | | | Substituting Group | | | New Compound No. | | | |
|---|---|---|---|---|---|---|---|---|---|
| R2 of | No. | 1~380, 382~720 | are substituted with | D441 | to form | No. | 317141 | ~ | 317859 |
| R2 of | No. | 1~381, 383~720 | are substituted with | D442 | to form | No. | 317860 | ~ | 318578 |
| R2 of | No. | 1~382, 384~720 | are substituted with | D443 | to form | No. | 318579 | ~ | 319297 |
| R2 of | No. | 1~383, 385~720 | are substituted with | D444 | to form | No. | 319298 | ~ | 320016 |
| R2 of | No. | 1~384, 386~720 | are substituted with | D445 | to form | No. | 320017 | ~ | 320735 |
| R2 of | No. | 1~385, 387~720 | are substituted with | D446 | to form | No. | 320736 | ~ | 321454 |
| R2 of | No. | 1~386, 388~720 | are substituted with | D447 | to form | No. | 321455 | ~ | 322173 |
| R2 of | No. | 1~387, 389~720 | are substituted with | D448 | to form | No. | 322174 | ~ | 322892 |
| R2 of | No. | 1~388, 390~720 | are substituted with | D449 | to form | No. | 322893 | ~ | 323611 |
| R2 of | No. | 1~389, 391~720 | are substituted with | D450 | to form | No. | 323612 | ~ | 324330 |
| R2 of | No. | 1~390, 392~720 | are substituted with | D451 | to form | No. | 324331 | ~ | 325049 |
| R2 of | No. | 1~391, 393~720 | are substituted with | D452 | to form | No. | 325050 | ~ | 325768 |
| R2 of | No. | 1~392, 394~720 | are substituted with | D453 | to form | No. | 325769 | ~ | 326487 |
| R2 of | No. | 1~393, 395~720 | are substituted with | D454 | to form | No. | 326488 | ~ | 327206 |
| R2 of | No. | 1~394, 396~720 | are substituted with | D455 | to form | No. | 327207 | ~ | 327925 |
| R2 of | No. | 1~395, 397~720 | are substituted with | D456 | to form | No. | 327926 | ~ | 328644 |
| R2 of | No. | 1~396, 398~720 | are substituted with | D457 | to form | No. | 328645 | ~ | 329363 |
| R2 of | No. | 1~397, 399~720 | are substituted with | D458 | to form | No. | 329364 | ~ | 330082 |
| R2 of | No. | 1~398, 400~720 | are substituted with | D459 | to form | No. | 330083 | ~ | 330801 |
| R2 of | No. | 1~389, 401~720 | are substituted with | D460 | to form | No. | 330802 | ~ | 331520 |
| R2 of | No. | 1~400, 402~720 | are substituted with | D461 | to form | No. | 331521 | ~ | 332239 |
| R2 of | No. | 1~401, 403~720 | are substituted with | D462 | to form | No. | 332240 | ~ | 332958 |
| R2 of | No. | 1~402, 404~720 | are substituted with | D463 | to form | No. | 332959 | ~ | 333677 |
| R2 of | No. | 1~403, 405~720 | are substituted with | D464 | to form | No. | 333678 | ~ | 334396 |
| R2 of | No. | 1~404, 406~720 | are substituted with | D465 | to form | No. | 334397 | ~ | 335115 |
| R2 of | No. | 1~405, 407~720 | are substituted with | D466 | to form | No. | 335116 | ~ | 335834 |
| R2 of | No. | 1~406, 408~720 | are substituted with | D467 | to form | No. | 335835 | ~ | 336553 |
| R2 of | No. | 1~407, 409~720 | are substituted with | D468 | to form | No. | 336554 | ~ | 337272 |
| R2 of | No. | 1~408, 410~720 | are substituted with | D469 | to form | No. | 337273 | ~ | 337991 |
| R2 of | No. | 1~409, 411~720 | are substituted with | D470 | to form | No. | 337992 | ~ | 338710 |
| R2 of | No. | 1~410, 412~720 | are substituted with | D471 | to form | No. | 338711 | ~ | 339429 |
| R2 of | No. | 1~411, 413~720 | are substituted with | D472 | to form | No. | 339430 | ~ | 340148 |
| R2 of | No. | 1~412, 414~720 | are substituted with | D473 | to form | No. | 340149 | ~ | 340867 |
| R2 of | No. | 1~413, 415~720 | are substituted with | D474 | to form | No. | 340868 | ~ | 341586 |
| R2 of | No. | 1~414, 416~720 | are substituted with | D475 | to form | No. | 341587 | ~ | 342305 |
| R2 of | No. | 1~415, 417~720 | are substituted with | D476 | to form | No. | 342306 | ~ | 343024 |
| R2 of | No. | 1~416, 418~720 | are substituted with | D477 | to form | No. | 343025 | ~ | 343743 |
| R2 of | No. | 1~417, 419~720 | are substituted with | D478 | to form | No. | 343744 | ~ | 344462 |

(continued)

| Group to be Substituted | | | Substituting Group | | | New Compound No. | | | |
|---|---|---|---|---|---|---|---|---|---|
| R2 of | No. | 1~418, 420~720 | are substituted with | D479 | to form | No. | 344463 | ~ | 345181 |
| R2 of | No. | 1~419, 421~720 | are substituted with | D480 | to form | No. | 345182 | ~ | 345900 |
| R2 of | No. | 1~420, 422~720 | are substituted with | D481 | to form | No. | 345901 | ~ | 346619 |
| R2 of | No. | 1~421, 423~720 | are substituted with | D482 | to form | No. | 346620 | ~ | 347338 |
| R2 of | No. | 1~422, 424~720 | are substituted with | D483 | to form | No. | 347339 | ~ | 348057 |
| R2 of | No. | 1~423, 425~720 | are substituted with | D484 | to form | No. | 348058 | ~ | 348776 |
| R2 of | No. | 1~424, 426~720 | are substituted with | D485 | to form | No. | 348777 | ~ | 349495 |
| R2 of | No. | 1~425, 427~720 | are substituted with | D486 | to form | No. | 349496 | ~ | 350214 |
| R2 of | No. | 1~426, 428~720 | are substituted with | D487 | to form | No. | 350215 | ~ | 350933 |
| R2 of | No. | 1~427, 429~720 | are substituted with | D488 | to form | No. | 350934 | ~ | 351652 |
| R2 of | No. | 1~428, 430~720 | are substituted with | D489 | to form | No. | 351653 | ~ | 352371 |
| R2 of | No. | 1~429, 431~720 | are substituted with | D490 | to form | No. | 352372 | ~ | 353090 |
| R2 of | No. | 1~430, 432~720 | are substituted with | D491 | to form | No. | 353091 | ~ | 353809 |
| R2 of | No. | 1~431, 433~720 | are substituted with | D492 | to form | No. | 353810 | ~ | 354528 |
| R2 of | No. | 1~432, 434~720 | are substituted with | D493 | to form | No. | 354529 | ~ | 355247 |
| R2 of | No. | 1~433, 435~720 | are substituted with | D494 | to form | No. | 355248 | ~ | 355966 |
| R2 of | No. | 1~434, 436~720 | are substituted with | D495 | to form | No. | 355967 | ~ | 356685 |
| R2 of | No. | 1~435, 437~720 | are substituted with | D496 | to form | No. | 356686 | ~ | 357404 |
| R2 of | No. | 1~436, 438~720 | are substituted with | D497 | to form | No. | 357405 | ~ | 358123 |
| R2 of | No. | 1~437, 439~720 | are substituted with | D498 | to form | No. | 358124 | ~ | 358842 |
| R2 of | No. | 1~438, 440~720 | are substituted with | D499 | to form | No. | 358843 | ~ | 359561 |
| R2 of | No. | 1~439, 441~720 | are substituted with | D500 | to form | No. | 359562 | ~ | 360280 |
| R2 of | No. | 1~440, 442~720 | are substituted with | D501 | to form | No. | 360281 | ~ | 360999 |
| R2 of | No. | 1~441, 443~720 | are substituted with | D502 | to form | No. | 361000 | ~ | 361718 |
| R2 of | No. | 1~442, 444~720 | are substituted with | D503 | to form | No. | 361719 | ~ | 362437 |
| R2 of | No. | 1~443, 445~720 | are substituted with | D504 | to form | No. | 362438 | ~ | 363156 |
| R2 of | No. | 1~444, 446~720 | are substituted with | D505 | to form | No. | 363157 | ~ | 363875 |
| R2 of | No. | 1~445, 447~720 | are substituted with | D506 | to form | No. | 363876 | ~ | 364594 |
| R2 of | No. | 1~446, 448~720 | are substituted with | D507 | to form | No. | 364595 | ~ | 365313 |
| R2 of | No. | 1~447, 449~720 | are substituted with | D508 | to form | No. | 365314 | ~ | 366032 |
| R2 of | No. | 1~448, 450~720 | are substituted with | D509 | to form | No. | 366033 | ~ | 366751 |
| R2 of | No. | 1~449, 451~720 | are substituted with | D510 | to form | No. | 366752 | ~ | 367470 |
| R2 of | No. | 1~450, 452~720 | are substituted with | D511 | to form | No. | 367471 | ~ | 368189 |
| R2 of | No. | 1~451, 453~720 | are substituted with | D512 | to form | No. | 368190 | ~ | 368908 |
| R2 of | No. | 1~452, 454~720 | are substituted with | D513 | to form | No. | 368909 | ~ | 369627 |
| R2 of | No. | 1~453, 455~720 | are substituted with | D514 | to form | No. | 369628 | ~ | 370346 |
| R2 of | No. | 1~454, 456~720 | are substituted with | D515 | to form | No. | 370347 | ~ | 371065 |
| R2 of | No. | 1~455, 457~720 | are substituted with | D516 | to form | No. | 371066 | ~ | 371784 |

(continued)

| Group to be Substituted | | | Substituting Group | | | New Compound No. | | | |
|---|---|---|---|---|---|---|---|---|---|
| R2 of | No. | 1~456, 458~720 | are substituted with | D517 | to form | No. | 371785 | ~ | 372503 |
| R2 of | No. | 1~457, 459~720 | are substituted with | D518 | to form | No. | 372504 | ~ | 373222 |
| R2 of | No. | 1~458, 460~720 | are substituted with | D519 | to form | No. | 373223 | ~ | 373941 |
| R2 of | No. | 1~459, 461~720 | are substituted with | D520 | to form | No. | 373942 | ~ | 374660 |
| R2 of | No. | 1~460, 462~720 | are substituted with | D521 | to form | No. | 374661 | ~ | 375379 |
| R2 of | No. | 1~461, 463~720 | are substituted with | D522 | to form | No. | 375380 | ~ | 376098 |
| R2 of | No. | 1~462, 464~720 | are substituted with | D523 | to form | No. | 376099 | ~ | 376817 |
| R2 of | No. | 1~463, 465~720 | are substituted with | D524 | to form | No. | 376818 | ~ | 377536 |
| R2 of | No. | 1~464, 466~720 | are substituted with | D525 | to form | No. | 377537 | ~ | 378255 |
| R2 of | No. | 1~465, 467~720 | are substituted with | D526 | to form | No. | 378256 | ~ | 378974 |
| R2 of | No. | 1~466, 468~720 | are substituted with | D527 | to form | No. | 378975 | ~ | 379693 |
| R2 of | No. | 1~467, 469~720 | are substituted with | D528 | to form | No. | 379694 | ~ | 380412 |
| R2 of | No. | 1~468, 470~720 | are substituted with | D529 | to form | No. | 380413 | ~ | 381131 |
| R2 of | No. | 1~469, 471~720 | are substituted with | D530 | to form | No. | 381132 | ~ | 381850 |
| R2 of | No. | 1~470, 472~720 | are substituted with | D531 | to form | No. | 381851 | ~ | 382569 |
| R2 of | No. | 1~471, 473~720 | are substituted with | D532 | to form | No. | 382570 | ~ | 383288 |
| R2 of | No. | 1~472, 474~720 | are substituted with | D533 | to form | No. | 383289 | ~ | 384007 |
| R2 of | No. | 1~473, 475~720 | are substituted with | D534 | to form | No. | 384008 | ~ | 384726 |
| R2 of | No. | 1~474, 476~720 | are substituted with | D535 | to form | No. | 384727 | ~ | 385445 |
| R2 of | No. | 1~475, 477~720 | are substituted with | D536 | to form | No. | 385446 | ~ | 386164 |
| R2 of | No. | 1~476, 478~720 | are substituted with | D537 | to form | No. | 386165 | ~ | 386883 |
| R2 of | No. | 1~477, 479~720 | are substituted with | D538 | to form | No. | 386884 | ~ | 387602 |
| R2 of | No. | 1~478, 480~720 | are substituted with | D539 | to form | No. | 387603 | ~ | 388321 |
| R2 of | No. | 1~479, 481~720 | are substituted with | D540 | to form | No. | 388322 | ~ | 389040 |
| R2 of | No. | 1~480, 482~720 | are substituted with | D541 | to form | No. | 389041 | ~ | 389759 |
| R2 of | No. | 1~481, 483~720 | are substituted with | D542 | to form | No. | 389760 | ~ | 390478 |
| R2 of | No. | 1~482, 484~720 | are substituted with | D543 | to form | No. | 390479 | ~ | 391197 |
| R2 of | No. | 1~483, 485~720 | are substituted with | D544 | to form | No. | 391198 | ~ | 391916 |
| R2 of | No. | 1~484, 486~720 | are substituted with | D545 | to form | No. | 391917 | ~ | 392635 |
| R2 of | No. | 1~485, 487~720 | are substituted with | D546 | to form | No. | 392636 | ~ | 393354 |
| R2 of | No. | 1~486, 488~720 | are substituted with | D547 | to form | No. | 393355 | ~ | 394073 |
| R2 of | No. | 1~487, 489~720 | are substituted with | D548 | to form | No. | 394074 | ~ | 394792 |
| R2 of | No. | 1~488, 490~720 | are substituted with | D549 | to form | No. | 394793 | ~ | 395511 |
| R2 of | No. | 1~489, 491~720 | are substituted with | D550 | to form | No. | 395512 | ~ | 396230 |
| R2 of | No. | 1~490, 492~720 | are substituted with | D551 | to form | No. | 396231 | ~ | 396949 |
| R2 of | No. | 1~491, 493~720 | are substituted with | D552 | to form | No. | 396950 | ~ | 397668 |
| R2 of | No. | 1~492, 494~720 | are substituted with | D553 | to form | No. | 397669 | ~ | 398387 |
| R2 of | No. | 1~493, 495~720 | are substituted with | D554 | to form | No. | 398388 | ~ | 399106 |

(continued)

| Group to be Substituted | | | Substituting Group | | | New Compound No. | | | |
|---|---|---|---|---|---|---|---|---|---|
| R2 of | No. | 1~494, 496~720 | are substituted with | D555 | to form | No. | 399107 | ~ | 399825 |
| R2 of | No. | 1~495, 497~720 | are substituted with | D556 | to form | No. | 399826 | ~ | 400544 |
| R2 of | No. | 1~496, 498~720 | are substituted with | D557 | to form | No. | 400545 | ~ | 401263 |
| R2 of | No. | 1~497, 499~720 | are substituted with | D558 | to form | No. | 401264 | ~ | 401982 |
| R2 of | No. | 1~498, 500~720 | are substituted with | D559 | to form | No. | 401983 | ~ | 402701 |
| R2 of | No. | 1~499, 501~720 | are substituted with | D560 | to form | No. | 402702 | ~ | 403420 |
| R2 of | No. | 1~500, 502~720 | are substituted with | D561 | to form | No. | 403421 | ~ | 404139 |
| R2 of | No. | 1~501, 503~720 | are substituted with | D562 | to form | No. | 404140 | ~ | 404858 |
| R2 of | No. | 1~502, 504~720 | are substituted with | D563 | to form | No. | 404859 | ~ | 405577 |
| R2 of | No. | 1~503, 505~720 | are substituted with | D564 | to form | No. | 405578 | ~ | 406296 |
| R2 of | No. | 1~504, 506~720 | are substituted with | D565 | to form | No. | 406297 | ~ | 407015 |
| R2 of | No. | 1~505, 507~720 | are substituted with | D566 | to form | No. | 407016 | ~ | 407734 |
| R2 of | No. | 1~506, 508~720 | are substituted with | D567 | to form | No. | 407735 | ~ | 408453 |
| R2 of | No. | 1~507, 509~720 | are substituted with | D568 | to form | No. | 408454 | ~ | 409172 |
| R2 of | No. | 1~508, 510~720 | are substituted with | D569 | to form | No. | 409173 | ~ | 409891 |
| R2 of | No. | 1~509, 511~720 | are substituted with | D570 | to form | No. | 409892 | ~ | 410610 |
| R2 of | No. | 1~510, 512~720 | are substituted with | D571 | to form | No. | 410611 | ~ | 411329 |
| R2 of | No. | 1~511, 513~720 | are substituted with | D572 | to form | No. | 411330 | ~ | 412048 |
| R2 of | No. | 1~512, 514~720 | are substituted with | D573 | to form | No. | 412049 | ~ | 412767 |
| R2 of | No. | 1~513, 515~720 | are substituted with | D574 | to form | No. | 412768 | ~ | 413486 |
| R2 of | No. | 1~514, 516~720 | are substituted with | D575 | to form | No. | 413487 | ~ | 414205 |
| R2 of | No. | 1~515, 517~720 | are substituted with | D576 | to form | No. | 414206 | ~ | 414924 |
| R2 of | No. | 1~516, 518~720 | are substituted with | D577 | to form | No. | 414925 | ~ | 415643 |
| R2 of | No. | 1~517, 519~720 | are substituted with | D578 | to form | No. | 415644 | ~ | 416362 |
| R2 of | No. | 1~518, 520~720 | are substituted with | D579 | to form | No. | 416363 | ~ | 417081 |
| R2 of | No. | 1~519, 521~720 | are substituted with | D580 | to form | No. | 417082 | ~ | 417800 |
| R2 of | No. | 1~520, 522~720 | are substituted with | D581 | to form | No. | 417801 | ~ | 418519 |
| R2 of | No. | 1~521, 523~720 | are substituted with | D582 | to form | No. | 418520 | ~ | 419238 |
| R2 of | No. | 1~522, 524~720 | are substituted with | D583 | to form | No. | 419239 | ~ | 419957 |
| R2 of | No. | 1~523, 525~720 | are substituted with | D584 | to form | No. | 419958 | ~ | 420676 |
| R2 of | No. | 1~524, 526~720 | are substituted with | D585 | to form | No. | 420677 | ~ | 421395 |
| R2 of | No. | 1~525, 527~720 | are substituted with | D586 | to form | No. | 421396 | ~ | 422114 |
| R2 of | No. | 1~526, 528~720 | are substituted with | D587 | to form | No. | 422115 | ~ | 422833 |
| R2 of | No. | 1~527, 529~720 | are substituted with | D588 | to form | No. | 422834 | ~ | 423552 |
| R2 of | No. | 1~528, 530~720 | are substituted with | D589 | to form | No. | 423553 | ~ | 424271 |
| R2 of | No. | 1~529, 531~720 | are substituted with | D590 | to form | No. | 424272 | ~ | 424990 |
| R2 of | No. | 1~530, 532~720 | are substituted with | D591 | to form | No. | 424991 | ~ | 425709 |
| R2 of | No. | 1~531, 533~720 | are substituted with | D592 | to form | No. | 425710 | ~ | 426428 |

(continued)

| Group to be Substituted | | | Substituting Group | | | New Compound No. | | | |
|---|---|---|---|---|---|---|---|---|---|
| R2 of | No. | 1~532, 534~720 | are substituted with | D593 | to form | No. | 426429 | ~ | 427147 |
| R2 of | No. | 1~533, 535~720 | are substituted with | D594 | to form | No. | 427148 | ~ | 427866 |
| R2 of | No. | 1~534, 536~720 | are substituted with | D595 | to form | No. | 427867 | ~ | 428585 |
| R2 of | No. | 1~535, 537~720 | are substituted with | D596 | to form | No. | 428586 | ~ | 429304 |
| R2 of | No. | 1~536, 538~720 | are substituted with | D597 | to form | No. | 429305 | ~ | 430023 |
| R2 of | No. | 1~537, 539~720 | are substituted with | D598 | to form | No. | 430024 | ~ | 430742 |
| R2 of | No. | 1~538, 540~720 | are substituted with | D599 | to form | No. | 430743 | ~ | 431461 |
| R2 of | No. | 1~539, 541~720 | are substituted with | D600 | to form | No. | 431462 | ~ | 432180 |
| R2 of | No. | 1~540, 542~720 | are substituted with | D601 | to form | No. | 432181 | ~ | 432899 |
| R2 of | No. | 1~541, 543~720 | are substituted with | D602 | to form | No. | 432900 | ~ | 433618 |
| R2 of | No. | 1~542, 544~720 | are substituted with | D603 | to form | No. | 433619 | ~ | 434337 |
| R2 of | No. | 1~543, 545~720 | are substituted with | D604 | to form | No. | 434338 | ~ | 435056 |
| R2 of | No. | 1~544, 546~720 | are substituted with | D605 | to form | No. | 435057 | ~ | 435775 |
| R2 of | No. | 1~545, 547~720 | are substituted with | D606 | to form | No. | 435776 | ~ | 436494 |
| R2 of | No. | 1~546, 548~720 | are substituted with | D607 | to form | No. | 436495 | ~ | 437213 |
| R2 of | No. | 1~547, 549~720 | are substituted with | D608 | to form | No. | 437214 | ~ | 437932 |
| R2 of | No. | 1~548, 550~720 | are substituted with | D609 | to form | No. | 437933 | ~ | 438651 |
| R2 of | No. | 1~549, 551~720 | are substituted with | D610 | to form | No. | 438652 | ~ | 439370 |
| R2 of | No. | 1~550, 552~720 | are substituted with | D611 | to form | No. | 439371 | ~ | 440089 |
| R2 of | No. | 1~551, 553~720 | are substituted with | D612 | to form | No. | 440090 | ~ | 440808 |
| R2 of | No. | 1~552, 554~720 | are substituted with | D613 | to form | No. | 440809 | ~ | 441527 |
| R2 of | No. | 1~553, 555~720 | are substituted with | D614 | to form | No. | 441528 | ~ | 442246 |
| R2 of | No. | 1~554, 556~720 | are substituted with | D615 | to form | No. | 442247 | ~ | 442965 |
| R2 of | No. | 1~555, 557~720 | are substituted with | D616 | to form | No. | 442966 | ~ | 443684 |
| R2 of | No. | 1~556, 558~720 | are substituted with | D617 | to form | No. | 443685 | ~ | 444403 |
| R2 of | No. | 1~557, 559~720 | are substituted with | D618 | to form | No. | 444404 | ~ | 445122 |
| R2 of | No. | 1~558, 560~720 | are substituted with | D619 | to form | No. | 445123 | ~ | 445841 |
| R2 of | No. | 1~559, 561~720 | are substituted with | D620 | to form | No. | 445842 | ~ | 446560 |
| R2 of | No. | 1~560, 562~720 | are substituted with | D621 | to form | No. | 446561 | ~ | 447279 |
| R2 of | No. | 1~561, 563~720 | are substituted with | D622 | to form | No. | 447280 | ~ | 447998 |
| R2 of | No. | 1~562, 564~720 | are substituted with | D623 | to form | No. | 447999 | ~ | 448717 |
| R2 of | No. | 1~563, 565~720 | are substituted with | D624 | to form | No. | 448718 | ~ | 449436 |
| R2 of | No. | 1~564, 566~720 | are substituted with | D625 | to form | No. | 449437 | ~ | 450155 |
| R2 of | No. | 1~565, 567~720 | are substituted with | D626 | to form | No. | 450156 | ~ | 450874 |
| R2 of | No. | 1~566, 568~720 | are substituted with | D627 | to form | No. | 450875 | ~ | 451593 |
| R2 of | No. | 1~567, 569~720 | are substituted with | D628 | to form | No. | 451594 | ~ | 452312 |
| R2 of | No. | 1~568, 570~720 | are substituted with | D629 | to form | No. | 452313 | ~ | 453031 |
| R2 of | No. | 1~569, 571~720 | are substituted with | D630 | to form | No. | 453032 | ~ | 453750 |

(continued)

| Group to be Substituted | | | Substituting Group | | | New Compound No. | | | |
|---|---|---|---|---|---|---|---|---|---|
| R2 of | No. | 1~570, 572~720 | are substituted with | D631 | to form | No. | 453751 | ~ | 454469 |
| R2 of | No. | 1~571, 573~720 | are substituted with | D632 | to form | No. | 454470 | ~ | 455188 |
| R2 of | No. | 1~572, 574~720 | are substituted with | D633 | to form | No. | 455189 | ~ | 455907 |
| R2 of | No. | 1~573, 575~720 | are substituted with | D634 | to form | No. | 455908 | ~ | 456626 |
| R2 of | No. | 1~574, 576~720 | are substituted with | D635 | to form | No. | 456627 | ~ | 457345 |
| R2 of | No. | 1~575, 577~720 | are substituted with | D636 | to form | No. | 457346 | ~ | 458064 |
| R2 of | No. | 1~576, 578~720 | are substituted with | D637 | to form | No. | 458065 | ~ | 458783 |
| R2 of | No. | 1~577, 579~720 | are substituted with | D638 | to form | No. | 458784 | ~ | 459502 |
| R2 of | No. | 1~578, 580~720 | are substituted with | D639 | to form | No. | 459503 | ~ | 460221 |
| R2 of | No. | 1~579, 581~720 | are substituted with | D640 | to form | No. | 460222 | ~ | 460940 |
| R2 of | No. | 1~580, 582~720 | are substituted with | D641 | to form | No. | 460941 | ~ | 461659 |
| R2 of | No. | 1~581, 583~720 | are substituted with | D642 | to form | No. | 461660 | ~ | 462378 |
| R2 of | No. | 1~582, 584~720 | are substituted with | D643 | to form | No. | 462379 | ~ | 463097 |
| R2 of | No. | 1~583, 585~720 | are substituted with | D644 | to form | No. | 463098 | ~ | 463816 |
| R2 of | No. | 1~584, 586~720 | are substituted with | D645 | to form | No. | 463817 | ~ | 464535 |
| R2 of | No. | 1~585, 587~720 | are substituted with | D646 | to form | No. | 464536 | ~ | 465254 |
| R2 of | No. | 1~586, 588~720 | are substituted with | D647 | to form | No. | 465255 | ~ | 465973 |
| R2 of | No. | 1~587, 589~720 | are substituted with | D648 | to form | No. | 465974 | ~ | 466692 |
| R2 of | No. | 1~588, 590~720 | are substituted with | D649 | to form | No. | 466693 | ~ | 467411 |
| R2 of | No. | 1~589, 591~720 | are substituted with | D650 | to form | No. | 467412 | ~ | 468130 |
| R2 of | No. | 1~590, 592~720 | are substituted with | D651 | to form | No. | 468131 | ~ | 468849 |
| R2 of | No. | 1~591, 593~720 | are substituted with | D652 | to form | No. | 468850 | ~ | 469568 |
| R2 of | No. | 1~592, 594~720 | are substituted with | D653 | to form | No. | 469569 | ~ | 470287 |
| R2 of | No. | 1~593, 595~720 | are substituted with | D654 | to form | No. | 470288 | ~ | 471006 |
| R2 of | No. | 1~594, 596~720 | are substituted with | D655 | to form | No. | 471007 | ~ | 471725 |
| R2 of | No. | 1~595, 597~720 | are substituted with | D656 | to form | No. | 471726 | ~ | 472444 |
| R2 of | No. | 1~596, 598~720 | are substituted with | D657 | to form | No. | 472445 | ~ | 473163 |
| R2 of | No. | 1~597, 599~720 | are substituted with | D658 | to form | No. | 473164 | ~ | 473882 |
| R2 of | No. | 1~598, 600~720 | are substituted with | D659 | to form | No. | 473883 | ~ | 474601 |
| R2 of | No. | 1~599, 601~720 | are substituted with | D660 | to form | No. | 474602 | ~ | 475320 |
| R2 of | No. | 1~600, 602~720 | are substituted with | D661 | to form | No. | 475321 | ~ | 476039 |
| R2 of | No. | 1~601, 603~720 | are substituted with | D662 | to form | No. | 476040 | ~ | 476758 |
| R2 of | No. | 1~602, 604~720 | are substituted with | D663 | to form | No. | 476759 | ~ | 477477 |
| R2 of | No. | 1~603, 605~720 | are substituted with | D664 | to form | No. | 477478 | ~ | 478196 |
| R2 of | No. | 1~604, 606~720 | are substituted with | D665 | to form | No. | 478197 | ~ | 478915 |
| R2 of | No. | 1~605, 607~720 | are substituted with | D666 | to form | No. | 478916 | ~ | 479634 |
| R2 of | No. | 1~606, 608~720 | are substituted with | D667 | to form | No. | 479635 | ~ | 480353 |
| R2 of | No. | 1~607, 609~720 | are substituted with | D668 | to form | No. | 480354 | ~ | 481072 |

(continued)

| Group to be Substituted | | | Substituting Group | | | New Compound No. | | | |
|---|---|---|---|---|---|---|---|---|---|
| R2 of | No. | 1~608, 610~720 | are substituted with | D669 | to form | No. | 481073 | ~ | 481791 |
| R2 of | No. | 1~609, 611~720 | are substituted with | D670 | to form | No. | 481792 | ~ | 482510 |
| R2 of | No. | 1~610, 612~720 | are substituted with | D671 | to form | No. | 482511 | ~ | 483229 |
| R2 of | No. | 1~611, 613~720 | are substituted with | D672 | to form | No. | 483230 | ~ | 483948 |
| R2 of | No. | 1~612, 614~720 | are substituted with | D673 | to form | No. | 483949 | ~ | 484667 |
| R2 of | No. | 1~613, 615~720 | are substituted with | D674 | to form | No. | 484668 | ~ | 485386 |
| R2 of | No. | 1~614, 616~720 | are substituted with | D675 | to form | No. | 485387 | ~ | 486105 |
| R2 of | No. | 1~615, 617~720 | are substituted with | D676 | to form | No. | 486106 | ~ | 486824 |
| R2 of | No. | 1~616, 618~720 | are substituted with | D677 | to form | No. | 486825 | ~ | 487543 |
| R2 of | No. | 1~617, 619~720 | are substituted with | D678 | to form | No. | 487544 | ~ | 488262 |
| R2 of | No. | 1~618, 620~720 | are substituted with | D679 | to form | No. | 488263 | ~ | 488981 |
| R2 of | No. | 1~619, 621~720 | are substituted with | D680 | to form | No. | 488982 | ~ | 489700 |
| R2 of | No. | 1~620, 622~720 | are substituted with | D681 | to form | No. | 489701 | ~ | 490419 |
| R2 of | No. | 1~621, 623~720 | are substituted with | D682 | to form | No. | 490420 | ~ | 491138 |
| R2 of | No. | 1~622, 624~720 | are substituted with | D683 | to form | No. | 491139 | ~ | 491857 |
| R2 of | No. | 1~623, 625~720 | are substituted with | D684 | to form | No. | 491858 | ~ | 492576 |
| R2 of | No. | 1~624, 626~720 | are substituted with | D685 | to form | No. | 492577 | ~ | 493295 |
| R2 of | No. | 1~625, 627~720 | are substituted with | D686 | to form | No. | 493296 | ~ | 494014 |
| R2 of | No. | 1~626, 628~720 | are substituted with | D687 | to form | No. | 494015 | ~ | 494733 |
| R2 of | No. | 1~627, 629~720 | are substituted with | D688 | to form | No. | 494734 | ~ | 495452 |
| R2 of | No. | 1~628, 630~720 | are substituted with | D689 | to form | No. | 495453 | ~ | 496171 |
| R2 of | No. | 1~629, 631~720 | are substituted with | D690 | to form | No. | 496172 | ~ | 496890 |
| R2 of | No. | 1~630, 632~720 | are substituted with | D691 | to form | No. | 496891 | ~ | 497609 |
| R2 of | No. | 1~631, 633~720 | are substituted with | D692 | to form | No. | 497610 | ~ | 498328 |
| R2 of | No. | 1~632, 634~720 | are substituted with | D693 | to form | No. | 498329 | ~ | 499047 |
| R2 of | No. | 1~633, 635~720 | are substituted with | D694 | to form | No. | 499048 | ~ | 499766 |
| R2 of | No. | 1~634, 636~720 | are substituted with | D695 | to form | No. | 499767 | ~ | 500485 |
| R2 of | No. | 1~635, 637~720 | are substituted with | D696 | to form | No. | 500486 | ~ | 501204 |
| R2 of | No. | 1~636, 638~720 | are substituted with | D697 | to form | No. | 501205 | ~ | 501923 |
| R2 of | No. | 1~637, 639~720 | are substituted with | D698 | to form | No. | 501924 | ~ | 502642 |
| R2 of | No. | 1~638, 640~720 | are substituted with | D699 | to form | No. | 502643 | ~ | 503361 |
| R2 of | No. | 1~639, 641~720 | are substituted with | D700 | to form | No. | 503362 | ~ | 504080 |
| R2 of | No. | 1~640, 642~720 | are substituted with | D701 | to form | No. | 504081 | ~ | 504799 |
| R2 of | No. | 1~641, 643~720 | are substituted with | D702 | to form | No. | 504800 | ~ | 505518 |
| R2 of | No. | 1~642, 644~720 | are substituted with | D703 | to form | No. | 505519 | ~ | 506237 |
| R2 of | No. | 1~643, 645~720 | are substituted with | D704 | to form | No. | 506238 | ~ | 506956 |
| R2 of | No. | 1~644, 646~720 | are substituted with | D705 | to form | No. | 506957 | ~ | 507675 |
| R2 of | No. | 1~645, 647~720 | are substituted with | D706 | to form | No. | 507676 | ~ | 508394 |

(continued)

| Group to be Substituted | | | Substituting Group | | | New Compound No. | | | |
|---|---|---|---|---|---|---|---|---|---|
| R2 of | No. | 1~646, 648~720 | are substituted with | D707 | to form | No. | 508395 | ~ | 509113 |
| R2 of | No. | 1~647, 649~720 | are substituted with | D708 | to form | No. | 509114 | ~ | 509832 |
| R2 of | No. | 1~648, 650~720 | are substituted with | D709 | to form | No. | 509833 | ~ | 510551 |
| R2 of | No. | 1~649, 651~720 | are substituted with | D710 | to form | No. | 510552 | ~ | 511270 |
| R2 of | No. | 1~650, 652~720 | are substituted with | D711 | to form | No. | 511271 | ~ | 511989 |
| R2 of | No. | 1~651, 653~720 | are substituted with | D712 | to form | No. | 511990 | ~ | 512708 |
| R2 of | No. | 1~652, 654~720 | are substituted with | D713 | to form | No. | 512709 | ~ | 513427 |
| R2 of | No. | 1~653, 655~720 | are substituted with | D714 | to form | No. | 513428 | ~ | 514146 |
| R2 of | No. | 1~654, 656~720 | are substituted with | D715 | to form | No. | 514147 | ~ | 514865 |
| R2 of | No. | 1~655, 657~720 | are substituted with | D716 | to form | No. | 514866 | ~ | 515584 |
| R2 of | No. | 1~656, 658~720 | are substituted with | D717 | to form | No. | 515585 | ~ | 516303 |
| R2 of | No. | 1~657, 659~720 | are substituted with | D718 | to form | No. | 516304 | ~ | 517022 |
| R2 of | No. | 1~658, 660~720 | are substituted with | D719 | to form | No. | 517023 | ~ | 517741 |
| R2 of | No. | 1~659, 661~720 | are substituted with | D720 | to form | No. | 517742 | ~ | 518460 |
| R2 of | No. | 1~660, 662~720 | are substituted with | D721 | to form | No. | 518461 | ~ | 519179 |
| R2 of | No. | 1~661, 663~720 | are substituted with | D722 | to form | No. | 519180 | ~ | 519898 |
| R2 of | No. | 1~662, 664~720 | are substituted with | D723 | to form | No. | 519899 | ~ | 520617 |
| R2 of | No. | 1~663, 665~720 | are substituted with | D724 | to form | No. | 520618 | ~ | 521336 |
| R2 of | No. | 1~664, 666~720 | are substituted with | D725 | to form | No. | 521337 | ~ | 522055 |
| R2 of | No. | 1~665, 667~720 | are substituted with | D726 | to form | No. | 522056 | ~ | 522774 |
| R2 of | No. | 1~666, 668~720 | are substituted with | D727 | to form | No. | 522775 | ~ | 523493 |
| R2 of | No. | 1~667, 669~720 | are substituted with | D728 | to form | No. | 523494 | ~ | 524212 |
| R2 of | No. | 1~668, 670~720 | are substituted with | D729 | to form | No. | 524213 | ~ | 524931 |
| R2 of | No. | 1~669, 671~720 | are substituted with | D730 | to form | No. | 524932 | ~ | 525650 |
| R2 of | No. | 1~670, 672~720 | are substituted with | D731 | to form | No. | 525651 | ~ | 526369 |
| R2 of | No. | 1~671, 673~720 | are substituted with | D732 | to form | No. | 526370 | ~ | 527088 |
| R2 of | No. | 1~672, 674~720 | are substituted with | D733 | to form | No. | 527089 | ~ | 527807 |
| R2 of | No. | 1~673, 675~720 | are substituted with | D734 | to form | No. | 527808 | ~ | 528526 |
| R2 of | No. | 1~674, 676~720 | are substituted with | D735 | to form | No. | 528527 | ~ | 529245 |
| R2 of | No. | 1~675, 677~720 | are substituted with | D736 | to form | No. | 529246 | ~ | 529964 |
| R2 of | No. | 1~676, 678~720 | are substituted with | D737 | to form | No. | 529965 | ~ | 530683 |
| R2 of | No. | 1~677, 679~720 | are substituted with | D738 | to form | No. | 530684 | ~ | 531402 |
| R2 of | No. | 1~678, 680~720 | are substituted with | D739 | to form | No. | 531403 | ~ | 532121 |
| R2 of | No. | 1~679, 681~720 | are substituted with | D740 | to form | No. | 532122 | ~ | 532840 |
| R2 of | No. | 1~680, 682~720 | are substituted with | D741 | to form | No. | 532841 | ~ | 533559 |
| R2 of | No. | 1-681, 683~720 | are substituted with | D742 | to form | No. | 533560 | ~ | 534278 |
| R2 of | No. | 1~682, 684~720 | are substituted with | D743 | to form | No. | 534279 | ~ | 534997 |
| R2 of | No. | 1~683, 685~720 | are substituted with | D744 | to form | No. | 534998 | ~ | 535716 |

(continued)

| Group to be Substituted | | | Substituting Group | | | New Compound No. | | | |
|---|---|---|---|---|---|---|---|---|---|
| R2 of | No. | 1~684, 686~720 | are substituted with | D745 | to form | No. | 535717 | ~ | 536435 |
| R2 of | No. | 1~685, 687~720 | are substituted with | D746 | to form | No. | 536436 | ~ | 537154 |
| R2 of | No. | 1~686, 688~720 | are substituted with | D747 | to form | No. | 537155 | ~ | 537873 |
| R2 of | No. | 1~687, 689~720 | are substituted with | D748 | to form | No. | 537874 | ~ | 538592 |
| R2 of | No. | 1~688, 690~720 | are substituted with | D749 | to form | No. | 538593 | ~ | 539311 |
| R2 of | No. | 1~689, 691~720 | are substituted with | D750 | to form | No. | 539312 | ~ | 540030 |
| R2 of | No. | 1~690, 692~720 | are substituted with | D751 | to form | No. | 540031 | ~ | 540749 |
| R2 of | No. | 1~691, 693~720 | are substituted with | D752 | to form | No. | 540750 | ~ | 541468 |
| R2 of | No. | 1~692, 694~720 | are substituted with | D753 | to form | No. | 541469 | ~ | 542187 |
| R2 of | No. | 1~693, 695~720 | are substituted with | D754 | to form | No. | 542188 | ~ | 542906 |
| R2 of | No. | 1~694, 696~720 | are substituted with | D755 | to form | No. | 542907 | ~ | 543625 |
| R2 of | No. | 1~695, 697~720 | are substituted with | D756 | to form | No. | 543626 | ~ | 544344 |
| R2 of | No. | 1~696, 698~720 | are substituted with | D757 | to form | No. | 544345 | ~ | 545063 |
| R2 of | No. | 1~697, 699~720 | are substituted with | D758 | to form | No. | 545064 | ~ | 545782 |
| R2 of | No. | 1~698, 700~720 | are substituted with | D759 | to form | No. | 545783 | ~ | 546501 |
| R2 of | No. | 1~699, 701~720 | are substituted with | D760 | to form | No. | 546502 | ~ | 547220 |
| R2 of | No. | 1~700, 702~720 | are substituted with | D761 | to form | No. | 547221 | ~ | 547939 |
| R2 of | No. | 1~701, 703~720 | are substituted with | D762 | to form | No. | 547940 | ~ | 548658 |
| R2 of | No. | 1~702, 704~720 | are substituted with | D763 | to form | No. | 548659 | ~ | 549377 |
| R2 of | No. | 1~703, 705~720 | are substituted with | D764 | to form | No. | 549378 | ~ | 550096 |
| R2 of | No. | 1~704, 706~720 | are substituted with | D765 | to form | No. | 550097 | ~ | 550815 |
| R2 of | No. | 1~705, 707~720 | are substituted with | D766 | to form | No. | 550816 | ~ | 551534 |
| R2 of | No. | 1~706, 708~720 | are substituted with | D767 | to form | No. | 551535 | ~ | 552253 |
| R2 of | No. | 1~707, 709~720 | are substituted with | D768 | to form | No. | 552254 | ~ | 552972 |
| R2 of | No. | 1~708, 710~720 | are substituted with | D769 | to form | No. | 552973 | ~ | 553691 |
| R2 of | No. | 1~709, 711~720 | are substituted with | D770 | to form | No. | 553692 | ~ | 554410 |
| R2 of | No. | 1~710, 712~720 | are substituted with | D771 | to form | No. | 554411 | ~ | 555129 |
| R2 of | No. | 1~711, 713~720 | are substituted with | D772 | to form | No. | 555130 | ~ | 555848 |
| R2 of | No. | 1~712, 714~720 | are substituted with | D773 | to form | No. | 555849 | ~ | 556567 |
| R2 of | No. | 1~713, 715~720 | are substituted with | D774 | to form | No. | 556568 | ~ | 557286 |
| R2 of | No. | 1~714, 716~720 | are substituted with | D775 | to form | No. | 557287 | ~ | 558005 |
| R2 of | No. | 1~715, 717~720 | are substituted with | D776 | to form | No. | 558006 | ~ | 558724 |
| R2 of | No. | 1~716, 718~720 | are substituted with | D777 | to form | No. | 558725 | ~ | 559443 |
| R2 of | No. | 1~717, 719~720 | are substituted with | D778 | to form | No. | 559444 | ~ | 560162 |
| R2 of | No. | 1~718, 720~720 | are substituted with | D779 | to form | No. | 560163 | ~ | 560881 |
| R2 of | No. | 1~719, 721~720 | are substituted with | D780 | to form | No. | 560882 | ~ | 561600 |
| R2 of | No. | 1~720 | are substituted with | D781 | to form | No. | 561601 | ~ | 562320 |
| R2 of | No. | 1~720 | are substituted with | D782 | to form | No. | 562321 | ~ | 563040 |

(continued)

| Group to be Substituted | | | Substituting Group | | | New Compound No. | | | |
|---|---|---|---|---|---|---|---|---|---|
| R2 of | No. | 1~720 | are substituted with | D783 | to form | No. | 563041 | ~ | 563760 |
| R2 of | No. | 1~720 | are substituted with | D784 | to form | No. | 563761 | ~ | 564480 |
| R2 of | No. | 1~720 | are substituted with | D785 | to form | No. | 564481 | ~ | 565200 |
| R2 of | No. | 1~720 | are substituted with | D786 | to form | No. | 565201 | ~ | 565920 |
| R2 of | No. | 1~720 | are substituted with | D787 | to form | No. | 565921 | ~ | 566640 |
| R2 of | No. | 1~720 | are substituted with | D788 | to form | No. | 566641 | ~ | 567360 |
| R2 of | No. | 1~720 | are substituted with | D789 | to form | No. | 567361 | ~ | 568080 |
| R2 of | No. | 1~720 | are substituted with | D790 | to form | No. | 568081 | ~ | 568800 |
| R2 of | No. | 1~720 | are substituted with | D791 | to form | No. | 568801 | ~ | 569520 |
| R2 of | No. | 1~720 | are substituted with | D792 | to form | No. | 569521 | ~ | 570240 |

[Table 3] ~

| Group to be Substituted | | | Substituting Group | | | New Compound No. | | | |
|---|---|---|---|---|---|---|---|---|---|
| R3 of | No. | 1~720 | are substituted with | D1 | to form | No. | 570241 | ~ | 570960 |
| R3 of | No. | 1~720 | are substituted with | D2 | to form | No. | 570961 | ~ | 571680 |
| R3 of | No. | 1~720 | are substituted with | D3 | to form | No. | 571681 | ~ | 572400 |
| R3 of | No. | 1~720 | are substituted with | D4 | to form | No. | 572401 | ~ | 573120 |
| R3 of | No. | 1~720 | are substituted with | D5 | to form | No. | 573121 | ~ | 573840 |
| R3 of | No. | 1~720 | are substituted with | D6 | to form | No. | 573841 | ~ | 574560 |
| R3 of | No. | 1~720 | are substituted with | D7 | to form | No. | 574561 | ~ | 575280 |
| R3 of | No. | 1~720 | are substituted with | D8 | to form | No. | 575281 | ~ | 576000 |
| R3 of | No. | 1~720 | are substituted with | D9 | to form | No. | 576001 | ~ | 576720 |
| R3 of | No. | 1~720 | are substituted with | D10 | to form | No. | 576721 | ~ | 577440 |
| R3 of | No. | 1~720 | are substituted with | D11 | to form | No. | 577441 | ~ | 578160 |
| R3 of | No. | 1~720 | are substituted with | D12 | to form | No. | 578161 | ~ | 578880 |
| R3 of | No. | 2~720 | are substituted with | D13 | to form | No. | 578881 | ~ | 579599 |
| R3 of | No. | 1, 3~720 | are substituted with | D14 | to form | No. | 579600 | ~ | 580318 |
| R3 of | No. | 1, 2, 4~720 | are substituted with | D15 | to form | No. | 580319 | ~ | 581037 |
| R3 of | No. | 1~3, 5~720 | are substituted with | D16 | to form | No. | 581038 | ~ | 581756 |
| R3 of | No. | 1~4, 6~720 | are substituted with | D17 | to form | No. | 581757 | ~ | 582475 |
| R3 of | No. | 1~5, 7~720 | are substituted with | D18 | to form | No. | 582476 | ~ | 583194 |
| R3 of | No. | 1~6, 8~720 | are substituted with | D19 | to form | No. | 583195 | ~ | 583913 |
| R3 of | No. | 1~7, 9~720 | are substituted with | D20 | to form | No. | 583914 | ~ | 584632 |
| R3 of | No. | 1~8, 10~720 | are substituted with | D21 | to form | No. | 584633 | ~ | 585351 |
| R3 of | No. | 1~9 11~720 | are substituted with | D22 | to form | No. | 585352 | ~ | 586070 |
| R3 of | No. | 1~10, 12~720 | are substituted with | D23 | to form | No. | 586071 | ~ | 586789 |
| R3 of | No. | 1~11, 13~720 | are substituted with | D24 | to form | No. | 586790 | ~ | 587508 |

(continued)

| Group to be Substituted | | | Substituting Group | | | New Compound No. | | | |
|---|---|---|---|---|---|---|---|---|---|
| R3 of | No. | 1~12, 14~720 | are substituted with | D25 | to form | No. | 587509 | ~ | 588227 |
| R3 of | No. | 1~13, 15~720 | are substituted with | D26 | to form | No. | 588228 | ~ | 588946 |
| R3 of | No. | 1~14, 16~720 | are substituted with | D27 | to form | No. | 588947 | ~ | 589665 |
| R3 of | No. | 1~15, 17~720 | are substituted with | D28 | to form | No. | 589666 | ~ | 590384 |
| R3 of | No. | 1~16, 18~720 | are substituted with | D29 | to form | No. | 590385 | ~ | 591103 |
| R3 of | No. | 1~17, 19~720 | are substituted with | D30 | to form | No. | 591104 | ~ | 591822 |
| R3 of | No. | 1~18, 20~720 | are substituted with | D31 | to form | No. | 591823 | ~ | 592541 |
| R3 of | No. | 1~19, 21 -720 | are substituted with | D32 | to form | No. | 592542 | ~ | 593260 |
| R3 of | No. | 1~20, 22~720 | are substituted with | D33 | to form | No. | 593261 | ~ | 593979 |
| R3 of | No. | 1~21, 23~720 | are substituted with | D34 | to form | No. | 593980 | ~ | 594698 |
| R3 of | No. | 1~22, 24~720 | are substituted with | D35 | to form | No. | 594699 | ~ | 595417 |
| R3 of | No. | 1~23, 25~720 | are substituted with | D36 | to form | No. | 595418 | ~ | 596136 |
| R3 of | No. | 1~24, 26~720 | are substituted with | D37 | to form | No. | 596137 | ~ | 596855 |
| R3 of | No. | 1~25, 27~720 | are substituted with | D38 | to form | No. | 596856 | ~ | 597574 |
| R3 of | No. | 1~26, 28~720 | are substituted with | D39 | to form | No. | 597575 | ~ | 598293 |
| R3 of | No. | 1~27, 29~720 | are substituted with | D40 | to form | No. | 598294 | ~ | 599012 |
| R3 of | No. | 1~28, 30~720 | are substituted with | D41 | to form | No. | 599013 | ~ | 599731 |
| R3 of | No. | 1~29, 31~720 | are substituted with | D42 | to form | No. | 599732 | ~ | 600450 |
| R3 of | No. | 1~30, 32~720 | are substituted with | D43 | to form | No. | 600451 | ~ | 601169 |
| R3 of | No. | 1~31, 33~720 | are substituted with | D44 | to form | No. | 601170 | ~ | 601888 |
| R3 of | No. | 1~32, 34~720 | are substituted with | D45 | to form | No. | 601889 | ~ | 602607 |
| R3 of | No. | 1~33, 35~720 | are substituted with | D46 | to form | No. | 602608 | ~ | 603326 |
| R3 of | No. | 1~34, 36~720 | are substituted with | D47 | to form | No. | 603327 | ~ | 604045 |
| R3 of | No. | 1~35, 37~720 | are substituted with | D48 | to form | No. | 604046 | ~ | 604764 |
| R3 of | No. | 1~36, 38~720 | are substituted with | D49 | to form | No. | 604765 | ~ | 605483 |
| R3 of | No. | 1~37, 39~720 | are substituted with | D50 | to form | No. | 605484 | ~ | 606202 |
| R3 of | No. | 1~38, 40~720 | are substituted with | D51 | to form | No. | 606203 | ~ | 606921 |
| R3 of | No. | 1~39, 41~720 | are substituted with | D52 | to form | No. | 606922 | ~ | 607640 |
| R3 of | No. | 1~40, 42~720 | are substituted with | D53 | to form | No. | 607641 | ~ | 608359 |
| R3 of | No. | 1~41, 43~720 | are substituted with | D54 | to form | No. | 608360 | ~ | 609078 |
| R3 of | No. | 1~42, 44~720 | are substituted with | D55 | to form | No. | 609079 | ~ | 609797 |
| R3 of | No. | 1~43, 45~720 | are substituted with | D56 | to form | No. | 609798 | ~ | 610516 |
| R3 of | No. | 1~44, 46~720 | are substituted with | D57 | to form | No. | 610517 | ~ | 611235 |
| R3 of | No. | 1~45, 47~720 | are substituted with | D58 | to form | No. | 611236 | ~ | 611954 |
| R3 of | No. | 1~46, 48~720 | are substituted with | D59 | to form | No. | 611955 | ~ | 612673 |
| R3 of | No. | 1~47, 49~720 | are substituted with | D60 | to form | No. | 612674 | ~ | 613392 |
| R3 of | No. | 1~48, 50~720 | are substituted with | D61 | to form | No. | 613393 | ~ | 614111 |
| R3 of | No. | 1~49, 51~720 | are substituted with | D62 | to form | No. | 614112 | ~ | 614830 |

(continued)

| Group to be Substituted | | | Substituting Group | | | New Compound No. | | | |
|---|---|---|---|---|---|---|---|---|---|
| R3 of | No. | 1~50, 52~720 | are substituted with | D63 | to form | No. | 614831 | ~ | 615549 |
| R3 of | No. | 1~51, 53~720 | are substituted with | D64 | to form | No. | 615550 | ~ | 616268 |
| R3 of | No. | 1~52, 54~720 | are substituted with | D65 | to form | No. | 616269 | ~ | 616987 |
| R3 of | No. | 1~53, 55~720 | are substituted with | D66 | to form | No. | 616988 | ~ | 617706 |
| R3 of | No. | 1~54, 56~720 | are substituted with | D67 | to form | No. | 617707 | ~ | 618425 |
| R3 of | No. | 1~55, 57~720 | are substituted with | D68 | to form | No. | 618426 | ~ | 619144 |
| R3 of | No. | 1~56, 58~720 | are substituted with | D69 | to form | No. | 619145 | ~ | 619863 |
| R3 of | No. | 1~57, 59~720 | are substituted with | D70 | to form | No. | 619864 | ~ | 620582 |
| R3 of | No. | 1~58, 60~720 | are substituted with | D71 | to form | No. | 620583 | ~ | 621301 |
| R3 of | No. | 1~59, 61~720 | are substituted with | D72 | to form | No. | 621302 | ~ | 622020 |
| R3 of | No. | 1~60, 62~720 | are substituted with | D73 | to form | No. | 622021 | ~ | 622739 |
| R3 of | No. | 1~61, 63~720 | are substituted with | D74 | to form | No. | 622740 | ~ | 623458 |
| R3 of | No. | 1~62, 64~720 | are substituted with | D75 | to form | No. | 623459 | ~ | 624177 |
| R3 of | No. | 1~63, 65~720 | are substituted with | D76 | to form | No. | 624178 | ~ | 624896 |
| R3 of | No. | 1~64, 66~720 | are substituted with | D77 | to form | No. | 624897 | ~ | 625615 |
| R3 of | No. | 1~65, 67~720 | are substituted with | D78 | to form | No. | 625616 | ~ | 626334 |
| R3 of | No. | 1~66, 68~720 | are substituted with | D79 | to form | No. | 626335 | ~ | 627053 |
| R3 of | No. | 1~67, 69~720 | are substituted with | D80 | to form | No. | 627054 | ~ | 627772 |
| R3 of | No. | 1~68, 70~720 | are substituted with | D81 | to form | No. | 627773 | ~ | 628491 |
| R3 of | No. | 1~69, 71~720 | are substituted with | D82 | to form | No. | 628492 | ~ | 629210 |
| R3 of | No. | 1~70, 72~720 | are substituted with | D83 | to form | No. | 629211 | ~ | 629929 |
| R3 of | No. | 1~71,73~720 | are substituted with | D84 | to form | No. | 629930 | ~ | 630648 |
| R3 of | No. | 1~72, 74~720 | are substituted with | D85 | to form | No. | 630649 | ~ | 631367 |
| R3 of | No. | 1~73, 75~720 | are substituted with | D86 | to form | No. | 631368 | ~ | 632086 |
| R3 of | No. | 1~74, 76~720 | are substituted with | D87 | to form | No. | 632087 | ~ | 632805 |
| R3 of | No. | 1~75, 77~720 | are substituted with | D88 | to form | No. | 632806 | ~ | 633524 |
| R3 of | No. | 1~76, 78~720 | are substituted with | D89 | to form | No. | 633525 | ~ | 634243 |
| R3 of | No. | 1~77, 79~720 | are substituted with | D90 | to form | No. | 634244 | ~ | 634962 |
| R3 of | No. | 1~78, 80~720 | are substituted with | D91 | to form | No. | 634963 | ~ | 635681 |
| R3 of | No. | 1~79, 81~720 | are substituted with | D92 | to form | No. | 635682 | ~ | 636400 |
| R3 of | No. | 1~80, 82~720 | are substituted with | D93 | to form | No. | 636401 | ~ | 637119 |
| R3 of | No. | 1~81, 83~720 | are substituted with | D94 | to form | No. | 637120 | ~ | 637838 |
| R3 of | No. | 1~82, 84~720 | are substituted with | D95 | to form | No. | 637839 | ~ | 638557 |
| R3 of | No. | 1~83, 85~720 | are substituted with | D96 | to form | No. | 638558 | ~ | 639276 |
| R3 of | No. | 1~84, 86~720 | are substituted with | D97 | to form | No. | 639277 | ~ | 639995 |
| R3 of | No. | 1~85, 87~720 | are substituted with | D98 | to form | No. | 639996 | ~ | 640714 |
| R3 of | No. | 1~86, 88~720 | are substituted with | D99 | to form | No. | 640715 | ~ | 641433 |
| R3 of | No. | 1~87, 89~720 | are substituted with | D100 | to form | No. | 641434 | ~ | 642152 |

(continued)

| Group to be Substituted | | | Substituting Group | | | New Compound No. | | | |
|---|---|---|---|---|---|---|---|---|---|
| R3 of | No. | 1~88, 90~720 | are substituted with | D101 | to form | No. | 642153 | ~ | 642871 |
| R3 of | No. | 1~89, 91~720 | are substituted with | D102 | to form | No. | 642872 | ~ | 643590 |
| R3 of | No. | 1~90, 92~720 | are substituted with | D103 | to form | No. | 643591 | ~ | 644309 |
| R3 of | No. | 1~91, 93~720 | are substituted with | D104 | to form | No. | 644310 | ~ | 645028 |
| R3 of | No. | 1~92, 94~720 | are substituted with | D105 | to form | No. | 645029 | ~ | 645747 |
| R3 of | No. | 1~93, 95~720 | are substituted with | D106 | to form | No. | 645748 | ~ | 646466 |
| R3 of | No. | 1~94, 96~720 | are substituted with | D107 | to form | No. | 646467 | ~ | 647185 |
| R3 of | No. | 1~95, 97~720 | are substituted with | D108 | to form | No. | 647186 | ~ | 647904 |
| R3 of | No. | 1~720 | are substituted with | D109 | to form | No. | 647905 | ~ | 648624 |
| R3 of | No. | 1~720 | are substituted with | D110 | to form | No. | 648625 | ~ | 649344 |
| R3 of | No. | 1~720 | are substituted with | D111 | to form | No. | 649345 | ~ | 650064 |
| R3 of | No. | 1~720 | are substituted with | D112 | to form | No. | 650065 | ~ | 650784 |
| R3 of | No. | 1~720 | are substituted with | D113 | to form | No. | 650785 | ~ | 651504 |
| R3 of | No. | 1~96, 98~720 | are substituted with | D114 | to form | No. | 651505 | ~ | 652223 |
| R3 of | No. | 1~97, 99~720 | are substituted with | D115 | to form | No. | 652224 | ~ | 652942 |
| R3 of | No. | 1~98, 100~720 | are substituted with | D116 | to form | No. | 652943 | ~ | 653661 |
| R3 of | No. | 1~99, 101~720 | are substituted with | D117 | to form | No. | 653662 | ~ | 654380 |
| R3 of | No. | 1~100, 102~720 | are substituted with | D118 | to form | No. | 654381 | ~ | 655099 |
| R3 of | No. | 1~101, 103~720 | are substituted with | D119 | to form | No. | 655100 | ~ | 655818 |
| R3 of | No. | 1~102, 104~720 | are substituted with | D120 | to form | No. | 655819 | ~ | 656537 |
| R3 of | No. | 1~103, 105~720 | are substituted with | D121 | to form | No. | 656538 | ~ | 657256 |
| R3 of | No. | 1~104, 106~720 | are substituted with | D122 | to form | No. | 657257 | ~ | 657975 |
| R3 of | No. | 1~105, 107~720 | are substituted with | D123 | to form | No. | 657976 | ~ | 658694 |
| R3 of | No. | 1~106, 108~720 | are substituted with | D124 | to form | No. | 658695 | ~ | 659413 |
| R3 of | No. | 1~107, 109~720 | are substituted with | D125 | to form | No. | 659414 | ~ | 660132 |
| R3 of | No. | 1~108, 110~720 | are substituted with | D126 | to form | No. | 660133 | ~ | 660851 |
| R3 of | No. | 1~109, 111~720 | are substituted with | D127 | to form | No. | 660852 | ~ | 661570 |
| R3 of | No. | 1~110, 112~720 | are substituted with | D128 | to form | No. | 661571 | ~ | 662289 |
| R3 of | No. | 1~111, 113~720 | are substituted with | D129 | to form | No. | 662290 | ~ | 663008 |
| R3 of | No. | 1~112, 114~720 | are substituted with | D130 | to form | No. | 663009 | ~ | 663727 |
| R3 of | No. | 1~113, 115~720 | are substituted with | D131 | to form | No. | 663728 | ~ | 664446 |
| R3 of | No. | 1~114, 116~720 | are substituted with | D132 | to form | No. | 664447 | ~ | 665165 |
| R3 of | No. | 1~115, 117~720 | are substituted with | D133 | to form | No. | 665166 | ~ | 665884 |
| R3 of | No. | 1~116, 118~720 | are substituted with | D134 | to form | No. | 665885 | ~ | 666603 |
| R3 of | No. | 1~117, 119~720 | are substituted with | D135 | to form | No. | 666604 | ~ | 667322 |
| R3 of | No. | 1~118, 120~720 | are substituted with | D136 | to form | No. | 667323 | ~ | 668041 |
| R3 of | No. | 1~119, 121~720 | are substituted with | D137 | to form | No. | 668042 | ~ | 668760 |
| R3 of | No. | 1~120, 122~720 | are substituted with | D138 | to form | No. | 668761 | ~ | 669479 |

(continued)

| Group to be Substituted | | | Substituting Group | | | New Compound No. | | | |
|---|---|---|---|---|---|---|---|---|---|
| R3 of | No. | 1~121, 123~720 | are substituted with | D139 | to form | No. | 669480 | ~ | 670198 |
| R3 of | No. | 1~122, 124~720 | are substituted with | D140 | to form | No. | 670199 | ~ | 670917 |
| R3 of | No. | 1~123, 125~720 | are substituted with | D141 | to form | No. | 670918 | ~ | 671636 |
| R3 of | No. | 1~124, 126~720 | are substituted with | D142 | to form | No. | 671637 | ~ | 672355 |
| R3 of | No. | 1~125, 127~720 | are substituted with | D143 | to form | No. | 672356 | ~ | 673074 |
| R3 of | No. | 1~126, 128~720 | are substituted with | D144 | to form | No. | 673075 | ~ | 673793 |
| R3 of | No. | 1~127, 129~720 | are substituted with | D145 | to form | No. | 673794 | ~ | 674512 |
| R3 of | No. | 1~128, 130~720 | are substituted with | D146 | to form | No. | 674513 | ~ | 675231 |
| R3 of | No. | 1~129, 131~720 | are substituted with | D147 | to form | No. | 675232 | ~ | 675950 |
| R3 of | No. | 1~130, 132~720 | are substituted with | D148 | to form | No. | 675951 | ~ | 676669 |
| R3 of | No. | 1~131, 133~720 | are substituted with | D149 | to form | No. | 676670 | ~ | 677388 |
| R3 of | No. | 1~720 | are substituted with | D150 | to form | No. | 677389 | ~ | 678108 |
| R3 of | No. | 1~720 | are substituted with | D151 | to form | No. | 678109 | ~ | 678828 |
| R3 of | No. | 1~720 | are substituted with | D152 | to form | No. | 678829 | ~ | 679548 |
| R3 of | No. | 1~720 | are substituted with | D153 | to form | No. | 679549 | ~ | 680268 |
| R3 of | No. | 1~720 | are substituted with | D154 | to form | No. | 680269 | ~ | 680988 |
| R3 of | No. | 1~720 | are substituted with | D155 | to form | No. | 680989 | ~ | 681708 |
| R3 of | No. | 1~720 | are substituted with | D156 | to form | No. | 681709 | ~ | 682428 |
| R3 of | No. | 1~720 | are substituted with | D157 | to form | No. | 682429 | ~ | 683148 |
| R3 of | No. | 1~720 | are substituted with | D158 | to form | No. | 683149 | ~ | 683868 |
| R3 of | No. | 1~720 | are substituted with | D159 | to form | No. | 683869 | ~ | 684588 |
| R3 of | No. | 1~720 | are substituted with | D160 | to form | No. | 684589 | ~ | 685308 |
| R3 of | No. | 1~720 | are substituted with | D161 | to form | No. | 685309 | ~ | 686028 |
| R3 of | No. | 1~720 | are substituted with | D162 | to form | No. | 686029 | ~ | 686748 |
| R3 of | No. | 1~720 | are substituted with | D163 | to form | No. | 686749 | ~ | 687468 |
| R3 of | No. | 1~720 | are substituted with | D164 | to form | No. | 687469 | ~ | 688188 |
| R3 of | No. | 1~720 | are substituted with | D165 | to form | No. | 688189 | ~ | 688908 |
| R3 of | No. | 1~720 | are substituted with | D166 | to form | No. | 688909 | ~ | 689628 |
| R3 of | No. | 1~720 | are substituted with | D167 | to form | No. | 689629 | ~ | 690348 |
| R3 of | No. | 1~720 | are substituted with | D168 | to form | No. | 690349 | ~ | 691068 |
| R3 of | No. | 1~720 | are substituted with | D169 | to form | No. | 691069 | ~ | 691788 |
| R3 of | No. | 1~720 | are substituted with | D170 | to form | No. | 691789 | ~ | 692508 |
| R3 of | No. | 1~720 | are substituted with | D171 | to form | No. | 692509 | ~ | 693228 |
| R3 of | No. | 1~720 | are substituted with | D172 | to form | No. | 693229 | ~ | 693948 |
| R3 of | No. | 1~720 | are substituted with | D173 | to form | No. | 693949 | ~ | 694668 |
| R3 of | No. | 1~720 | are substituted with | D174 | to form | No. | 694669 | ~ | 695388 |
| R3 of | No. | 1~720 | are substituted with | D175 | to form | No. | 695389 | ~ | 696108 |
| R3 of | No. | 1~720 | are substituted with | D176 | to form | No. | 696109 | ~ | 696828 |

(continued)

| Group to be Substituted | | | Substituting Group | | | New Compound No. | | | |
|---|---|---|---|---|---|---|---|---|---|
| R3 of | No. | 1~720 | are substituted with | D177 | to form | No. | 696829 | ~ | 697548 |
| R3 of | No. | 1~720 | are substituted with | D178 | to form | No. | 697549 | ~ | 698268 |
| R3 of | No. | 1~720 | are substituted with | D179 | to form | No. | 698269 | ~ | 698988 |
| R3 of | No. | 1~132, 134~720 | are substituted with | D180 | to form | No. | 698989 | ~ | 699707 |
| R3 of | No. | 1~133, 135~720 | are substituted with | D181 | to form | No. | 699708 | ~ | 700426 |
| R3 of | No. | 1~134, 136~720 | are substituted with | D182 | to form | No. | 700427 | ~ | 701145 |
| R3 of | No. | 1~135, 137~720 | are substituted with | D183 | to form | No. | 701146 | ~ | 701864 |
| R3 of | No. | 1~136, 138~720 | are substituted with | D184 | to form | No. | 701865 | ~ | 702583 |
| R3 of | No. | 1~137, 139~720 | are substituted with | D185 | to form | No. | 702584 | ~ | 703302 |
| R3 of | No. | 1~138, 140~720 | are substituted with | D186 | to form | No. | 703303 | ~ | 704021 |
| R3 of | No. | 1~139, 141~720 | are substituted with | D187 | to form | No. | 704022 | ~ | 704740 |
| R3 of | No. | 1~140, 142~720 | are substituted with | D188 | to form | No. | 704741 | ~ | 705459 |
| R3 of | No. | 1~141, 143~720 | are substituted with | D189 | to form | No. | 705460 | ~ | 706178 |
| R3 of | No. | 1~142, 144~720 | are substituted with | D190 | to form | No. | 706179 | ~ | 706897 |
| R3 of | No. | 1~143, 145~720 | are substituted with | D191 | to form | No. | 706898 | ~ | 707616 |
| R3 of | No. | 1~720 | are substituted with | D192 | to form | No. | 707617 | ~ | 708336 |
| R3 of | No. | 1~720 | are substituted with | D193 | to form | No. | 708337 | ~ | 709056 |
| R3 of | No. | 1~720 | are substituted with | D194 | to form | No. | 709057 | ~ | 709776 |
| R3 of | No. | 1~720 | are substituted with | D195 | to form | No. | 709777 | ~ | 710496 |
| R3 of | No. | 1~720 | are substituted with | D196 | to form | No. | 710497 | ~ | 711216 |
| R3 of | No. | 1~720 | are substituted with | D197 | to form | No. | 711217 | ~ | 711936 |
| R3 of | No. | 1~144, 146~720 | are substituted with | D198 | to form | No. | 711937 | ~ | 712655 |
| R3 of | No. | 1~145, 147~720 | are substituted with | D199 | to form | No. | 712656 | ~ | 713374 |
| R3 of | No. | 1~146, 148~720 | are substituted with | D200 | to form | No. | 713375 | ~ | 714093 |
| R3 of | No. | 1~147, 149~720 | are substituted with | D201 | to form | No. | 714094 | ~ | 714812 |
| R3 of | No. | 1~148, 150~720 | are substituted with | D202 | to form | No. | 714813 | ~ | 715531 |
| R3 of | No. | 1~149, 151~720 | are substituted with | D203 | to form | No. | 715532 | ~ | 716250 |
| R3 of | No. | 1~150, 152~720 | are substituted with | D204 | to form | No. | 716251 | ~ | 716969 |
| R3 of | No. | 1~151, 153~720 | are substituted with | D205 | to form | No. | 716970 | ~ | 717688 |
| R3 of | No. | 1~152, 154~720 | are substituted with | D206 | to form | No. | 717689 | ~ | 718407 |
| R3 of | No. | 1~153, 155~720 | are substituted with | D207 | to form | No. | 718408 | ~ | 719126 |
| R3 of | No. | 1~154, 156~720 | are substituted with | D208 | to form | No. | 719127 | ~ | 719845 |
| R3 of | No. | 1~155, 157~720 | are substituted with | D209 | to form | No. | 719846 | ~ | 720564 |
| R3 of | No. | 1~156, 158~720 | are substituted with | D210 | to form | No. | 720565 | ~ | 721283 |
| R3 of | No. | 1~157, 159~720 | are substituted with | D211 | to form | No. | 721284 | ~ | 722002 |
| R3 of | No. | 1~158, 160~720 | are substituted with | D212 | to form | No. | 722003 | ~ | 722721 |
| R3 of | No. | 1~159, 161~720 | are substituted with | D213 | to form | No. | 722722 | ~ | 723440 |
| R3 of | No. | 1~160, 162~720 | are substituted with | D214 | to form | No. | 723441 | ~ | 724159 |

(continued)

| Group to be Substituted | | | Substituting Group | | | New Compound No. | | | |
|---|---|---|---|---|---|---|---|---|---|
| R3 of | No. | 1~161, 163~720 | are substituted with | D215 | to form | No. | 724160 | ~ | 724878 |
| R3 of | No. | 1~162, 164~720 | are substituted with | D216 | to form | No. | 724879 | ~ | 725597 |
| R3 of | No. | 1~163, 165~720 | are substituted with | D217 | to form | No. | 725598 | ~ | 726316 |
| R3 of | No. | 1~164, 166~720 | are substituted with | D218 | to form | No. | 726317 | ~ | 727035 |
| R3 of | No. | 1~165, 167~720 | are substituted with | D219 | to form | No. | 727036 | ~ | 727754 |
| R3 of | No. | 1~166, 168~720 | are substituted with | D220 | to form | No. | 727755 | ~ | 728473 |
| R3 of | No. | 1~167, 169~720 | are substituted with | D221 | to form | No. | 728474 | ~ | 729192 |
| R3 of | No. | 1~168, 170~720 | are substituted with | D222 | to form | No. | 729193 | ~ | 729911 |
| R3 of | No. | 1~169, 171~720 | are substituted with | D223 | to form | No. | 729912 | ~ | 730630 |
| R3 of | No. | 1~170, 172~720 | are substituted with | D224 | to form | No. | 730631 | ~ | 731349 |
| R3 of | No. | 1~171, 173~720 | are substituted with | D225 | to form | No. | 731350 | ~ | 732068 |
| R3 of | No. | 1~172, 174~720 | are substituted with | D226 | to form | No. | 732069 | ~ | 732787 |
| R3 of | No. | 1~173, 175~720 | are substituted with | D227 | to form | No. | 732788 | ~ | 733506 |
| R3 of | No. | 1~174, 176~720 | are substituted with | D228 | to form | No. | 733507 | ~ | 734225 |
| R3 of | No. | 1~175, 177~720 | are substituted with | D229 | to form | No. | 734226 | ~ | 734944 |
| R3 of | No. | 1~176, 178~720 | are substituted with | D230 | to form | No. | 734945 | ~ | 735663 |
| R3 of | No. | 1~177, 179~720 | are substituted with | D231 | to form | No. | 735664 | ~ | 736382 |
| R3 of | No. | 1~178, 180~720 | are substituted with | D232 | to form | No. | 736383 | ~ | 737101 |
| R3 of | No. | 1~179, 181~720 | are substituted with | D233 | to form | No. | 737102 | ~ | 737820 |
| R3 of | No. | 1~180, 182~720 | are substituted with | D234 | to form | No. | 737821 | ~ | 738539 |
| R3 of | No. | 1~181, 183~720 | are substituted with | D235 | to form | No. | 738540 | ~ | 739258 |
| R3 of | No. | 1~182, 184~720 | are substituted with | D236 | to form | No. | 739259 | ~ | 739977 |
| R3 of | No. | 1~183, 185~720 | are substituted with | D237 | to form | No. | 739978 | ~ | 740696 |
| R3 of | No. | 1~184, 186~720 | are substituted with | D238 | to form | No. | 740697 | ~ | 741415 |
| R3 of | No. | 1~185, 187~720 | are substituted with | D239 | to form | No. | 741416 | ~ | 742134 |
| R3 of | No. | 1 ~720 | are substituted with | D240 | to form | No. | 742135 | ~ | 742854 |
| R3 of | No. | 1~720 | are substituted with | D241 | to form | No. | 742855 | ~ | 743574 |
| R3 of | No. | 1 ~720 | are substituted with | D242 | to form | No. | 743575 | ~ | 744294 |
| R3 of | No. | 1~720 | are substituted with | D243 | to form | No. | 744295 | ~ | 745014 |
| R3 of | No. | 1~720 | are substituted with | D244 | to form | No. | 745015 | ~ | 745734 |
| R3 of | No. | 1~186, 188~720 | are substituted with | D245 | to form | No. | 745735 | ~ | 746453 |
| R3 of | No. | 1~187, 189~720 | are substituted with | D246 | to form | No. | 746454 | ~ | 747172 |
| R3 of | No. | 1~188, 190~720 | are substituted with | D247 | to form | No. | 747173 | ~ | 747891 |
| R3 of | No. | 1~189, 191~720 | are substituted with | D248 | to form | No. | 747892 | ~ | 748610 |
| R3 of | No. | 1~190, 192~720 | are substituted with | D249 | to form | No. | 748611 | ~ | 749329 |
| R3 of | No. | 1~191, 193~720 | are substituted with | D250 | to form | No. | 749330 | ~ | 750048 |
| R3 of | No. | 1~192, 194~720 | are substituted with | D251 | to form | No. | 750049 | ~ | 750767 |
| R3 of | No. | 1~193, 195~720 | are substituted with | D252 | to form | No. | 750768 | ~ | 751486 |

(continued)

| Group to be Substituted | | | Substituting Group | | | New Compound No. | | | |
|---|---|---|---|---|---|---|---|---|---|
| R3 of | No. | 1~194, 196~720 | are substituted with | D253 | to form | No. | 751487 | ~ | 752205 |
| R3 of | No. | 1~195, 197~720 | are substituted with | D254 | to form | No. | 752206 | ~ | 752924 |
| R3 of | No. | 1~196, 198~720 | are substituted with | D255 | to form | No. | 752925 | ~ | 753643 |
| R3 of | No. | 1~197, 199~720 | are substituted with | D256 | to form | No. | 753644 | ~ | 754362 |
| R3 of | No. | 1~198, 200~720 | are substituted with | D257 | to form | No. | 754363 | ~ | 755081 |
| R3 of | No. | 1~199, 201 ~720 | are substituted with | D258 | to form | No. | 755082 | ~ | 755800 |
| R3 of | No. | 1~200, 202~720 | are substituted with | D259 | to form | No. | 755801 | ~ | 756519 |
| R3 of | No. | 1~201, 203~720 | are substituted with | D260 | to form | No. | 756520 | ~ | 757238 |
| R3 of | No. | 1~202, 204~720 | are substituted with | D261 | to form | No. | 757239 | ~ | 757957 |
| R3 of | No. | 1~203, 205~720 | are substituted with | D262 | to form | No. | 757958 | ~ | 758676 |
| R3 of | No. | 1~204, 206~720 | are substituted with | D263 | to form | No. | 758677 | ~ | 759395 |
| R3 of | No. | 1~205, 207~720 | are substituted with | D264 | to form | No. | 759396 | ~ | 760114 |
| R3 of | No. | 1~206, 208~720 | are substituted with | D265 | to form | No. | 760115 | ~ | 760833 |
| R3 of | No. | 1~207, 209~720 | are substituted with | D266 | to form | No. | 760834 | ~ | 761552 |
| R3 of | No. | 1~208, 210~720 | are substituted with | D267 | to form | No. | 761553 | ~ | 762271 |
| R3 of | No. | 1~209, 211~720 | are substituted with | D268 | to form | No. | 762272 | ~ | 762990 |
| R3 of | No. | 1~210, 212~720 | are substituted with | D269 | to form | No. | 762991 | ~ | 763709 |
| R3 of | No. | 1~211, 213~720 | are substituted with | D270 | to form | No. | 763710 | ~ | 764428 |
| R3 of | No. | 1~212, 214~720 | are substituted with | D271 | to form | No. | 764429 | ~ | 765147 |
| R3 of | No. | 1~213, 215~720 | are substituted with | D272 | to form | No. | 765148 | ~ | 765866 |
| R3 of | No. | 1~214, 216~720 | are substituted with | D273 | to form | No. | 765867 | ~ | 766585 |
| R3 of | No. | 1~215, 217~720 | are substituted with | D274 | to form | No. | 766586 | ~ | 767304 |
| R3 of | No. | 1~216, 218~720 | are substituted with | D275 | to form | No. | 767305 | ~ | 768023 |
| R3 of | No. | 1~217, 219~720 | are substituted with | D276 | to form | No. | 768024 | ~ | 768742 |
| R3 of | No. | 1~218, 220~720 | are substituted with | D277 | to form | No. | 768743 | ~ | 769461 |
| R3 of | No. | 1~219, 221~720 | are substituted with | D278 | to form | No. | 769462 | ~ | 770180 |
| R3 of | No. | 1~220, 222~720 | are substituted with | D279 | to form | No. | 770181 | ~ | 770899 |
| R3 of | No. | 1~221, 223~720 | are substituted with | D280 | to form | No. | 770900 | ~ | 771618 |
| R3 of | No. | 1~222, 224~720 | are substituted with | D281 | to form | No. | 771619 | ~ | 772337 |
| R3 of | No. | 1~223, 225~720 | are substituted with | D282 | to form | No. | 772338 | ~ | 773056 |
| R3 of | No. | 1~224, 226~720 | are substituted with | D283 | to form | No. | 773057 | ~ | 773775 |
| R3 of | No. | 1~225, 227~720 | are substituted with | D284 | to form | No. | 773776 | ~ | 774494 |
| R3 of | No. | 1~226, 228~720 | are substituted with | D285 | to form | No. | 774495 | ~ | 775213 |
| R3 of | No. | 1~227, 229~720 | are substituted with | D286 | to form | No. | 775214 | ~ | 775932 |
| R3 of | No. | 1~228, 230~720 | are substituted with | D287 | to form | No. | 775933 | ~ | 776651 |
| R3 of | No. | 1~229, 231~720 | are substituted with | D288 | to form | No. | 776652 | ~ | 777370 |
| R3 of | No. | 1~230, 232~720 | are substituted with | D289 | to form | No. | 777371 | ~ | 778089 |
| R3 of | No. | 1~231, 233~720 | are substituted with | D290 | to form | No. | 778090 | ~ | 778808 |

(continued)

| Group to be Substituted | | | Substituting Group | | | New Compound No. | | | |
|---|---|---|---|---|---|---|---|---|---|
| R3 of | No. | 1~232, 234~720 | are substituted with | D291 | to form | No. | 778809 | ~ | 779527 |
| R3 of | No. | 1~233, 235~720 | are substituted with | D292 | to form | No. | 779528 | ~ | 780246 |
| R3 of | No. | 1~234, 236~720 | are substituted with | D293 | to form | No. | 780247 | ~ | 780965 |
| R3 of | No. | 1~235, 237~720 | are substituted with | D294 | to form | No. | 780966 | ~ | 781684 |
| R3 of | No. | 1~236, 238~720 | are substituted with | D295 | to form | No. | 781685 | ~ | 782403 |
| R3 of | No. | 1~237, 239~720 | are substituted with | D296 | to form | No. | 782404 | ~ | 783122 |
| R3 of | No. | 1~238, 240~720 | are substituted with | D297 | to form | No. | 783123 | ~ | 783841 |
| R3 of | No. | 1~239, 241 ~720 | are substituted with | D298 | to form | No. | 783842 | ~ | 784560 |
| R3 of | No. | 1~720 | are substituted with | D299 | to form | No. | 784561 | ~ | 785280 |
| R3 of | No. | 1~240, 242~720 | are substituted with | D300 | to form | No. | 785281 | ~ | 785999 |
| R3 of | No. | 1~241, 243~720 | are substituted with | D301 | to form | No. | 786000 | ~ | 786718 |
| R3 of | No. | 1~242, 244~720 | are substituted with | D302 | to form | No. | 786719 | ~ | 787437 |
| R3 of | No. | 1~243, 245~720 | are substituted with | D303 | to form | No. | 787438 | ~ | 788156 |
| R3 of | No. | 1~244, 246~720 | are substituted with | D304 | to form | No. | 788157 | ~ | 788875 |
| R3 of | No. | 1~245, 247~720 | are substituted with | D305 | to form | No. | 788876 | ~ | 789594 |
| R3 of | No. | 1~246, 248~720 | are substituted with | D306 | to form | No. | 789595 | ~ | 790313 |
| R3 of | No. | 1~247, 249~720 | are substituted with | D307 | to form | No. | 790314 | ~ | 791032 |
| R3 of | No. | 1~248, 250~720 | are substituted with | D308 | to form | No. | 791033 | ~ | 791751 |
| R3 of | No. | 1~249, 251~720 | are substituted with | D309 | to form | No. | 791752 | ~ | 792470 |
| R3 of | No. | 1~250, 252~720 | are substituted with | D310 | to form | No. | 792471 | ~ | 793189 |
| R3 of | No. | 1~251, 253~720 | are substituted with | D311 | to form | No. | 793190 | ~ | 793908 |
| R3 of | No. | 1~252, 254~720 | are substituted with | D312 | to form | No. | 793909 | ~ | 794627 |
| R3 of | No. | 1~253, 255~720 | are substituted with | D313 | to form | No. | 794628 | ~ | 795346 |
| R3 of | No. | 1~254, 256~720 | are substituted with | D314 | to form | No. | 795347 | ~ | 796065 |
| R3 of | No. | 1~255, 257~720 | are substituted with | D315 | to form | No. | 796066 | ~ | 796784 |
| R3 of | No. | 1~256, 258~720 | are substituted with | D316 | to form | No. | 796785 | ~ | 797503 |
| R3 of | No. | 1~257, 259~720 | are substituted with | D317 | to form | No. | 797504 | ~ | 798222 |
| R3 of | No. | 1~258, 260~720 | are substituted with | D318 | to form | No. | 798223 | ~ | 798941 |
| R3 of | No. | 1~259, 261~720 | are substituted with | D319 | to form | No. | 798942 | ~ | 799660 |
| R3 of | No. | 1~260, 262~720 | are substituted with | D320 | to form | No. | 799661 | ~ | 800379 |
| R3 of | No. | 1~261, 263~720 | are substituted with | D321 | to form | No. | 800380 | ~ | 801098 |
| R3 of | No. | 1~262, 264~720 | are substituted with | D322 | to form | No. | 801099 | ~ | 801817 |
| R3 of | No. | 1~263, 265~720 | are substituted with | D323 | to form | No. | 801818 | ~ | 802536 |
| R3 of | No. | 1~264, 266~720 | are substituted with | D324 | to form | No. | 802537 | ~ | 803255 |
| R3 of | No. | 1~265, 267~720 | are substituted with | D325 | to form | No. | 803256 | ~ | 803974 |
| R3 of | No. | 1~266, 268~720 | are substituted with | D326 | to form | No. | 803975 | ~ | 804693 |
| R3 of | No. | 1~267, 269~720 | are substituted with | D327 | to form | No. | 804694 | ~ | 805412 |
| R3 of | No. | 1~268, 270~720 | are substituted with | D328 | to form | No. | 805413 | ~ | 806131 |

(continued)

| Group to be Substituted | | | Substituting Group | | | New Compound No. | | | |
|---|---|---|---|---|---|---|---|---|---|
| R3 of | No. | 1~269, 271~720 | are substituted with | D329 | to form | No. | 806132 | ~ | 806850 |
| R3 of | No. | 1~270, 272~720 | are substituted with | D330 | to form | No. | 806851 | ~ | 807569 |
| R3 of | No. | 1~271, 273~720 | are substituted with | D331 | to form | No. | 807570 | ~ | 808288 |
| R3 of | No. | 1~272, 274~720 | are substituted with | D332 | to form | No. | 808289 | ~ | 809007 |
| R3 of | No. | 1~273, 275~720 | are substituted with | D333 | to form | No. | 809008 | - | 809726 |
| R3 of | No. | 1~274, 276~720 | are substituted with | D334 | to form | No. | 809727 | ~ | 810445 |
| R3 of | No. | 1~275, 277~720 | are substituted with | D335 | to form | No. | 810446 | ~ | 811164 |
| R3 of | No. | 1~276, 278~720 | are substituted with | D336 | to form | No. | 811165 | ~ | 811883 |
| R3 of | No. | 1~277, 279~720 | are substituted with | D337 | to form | No. | 811884 | ~ | 812602 |
| R3 of | No. | 1~278, 280~720 | are substituted with | D338 | to form | No. | 812603 | ~ | 813321 |
| R3 of | No. | 1~279, 281~720 | are substituted with | D339 | to form | No. | 813322 | - | 814040 |
| R3 of | No. | 1~280, 282~720 | are substituted with | D340 | to form | No. | 814041 | ~ | 814759 |
| R3 of | No. | 1~281, 283~720 | are substituted with | D341 | to form | No. | 814760 | ~ | 815478 |
| R3 of | No. | 1~282, 284~720 | are substituted with | D342 | to form | No. | 815479 | ~ | 816197 |
| R3 of | No. | 1~283, 285~720 | are substituted with | D343 | to form | No. | 816198 | ~ | 816916 |
| R3 of | No. | 1~284, 286~720 | are substituted with | D344 | to form | No. | 816917 | ~ | 817635 |
| R3 of | No. | 1~285, 287~720 | are substituted with | D345 | to form | No. | 817636 | ~ | 818354 |
| R3 of | No. | 1~286, 288~720 | are substituted with | D346 | to form | No. | 818355 | ~ | 819073 |
| R3 of | No. | 1~287, 289~720 | are substituted with | D347 | to form | No. | 819074 | ~ | 819792 |
| R3 of | No. | 1~288, 290~720 | are substituted with | D348 | to form | No. | 819793 | ~ | 820511 |
| R3 of | No. | 1~289, 291~720 | are substituted with | D349 | to form | No. | 820512 | ~ | 821230 |
| R3 of | No. | 1~290, 292~720 | are substituted with | D350 | to form | No. | 821231 | - | 821949 |
| R3 of | No. | 1~291,293~720 | are substituted with | D351 | to form | No. | 821950 | ~ | 822668 |
| R3 of | No. | 1~292, 294~720 | are substituted with | D352 | to form | No. | 822669 | ~ | 823387 |
| R3 of | No. | 1~293, 295~720 | are substituted with | D353 | to form | No. | 823388 | ~ | 824106 |
| R3 of | No. | 1~294, 296~720 | are substituted with | D354 | to form | No. | 824107 | ~ | 824825 |
| R3 of | No. | 1~295, 297~720 | are substituted with | D355 | to form | No. | 824826 | ~ | 825544 |
| R3 of | No. | 1~296, 298~720 | are substituted with | D356 | to form | No. | 825545 | ~ | 826263 |
| R3 of | No. | 1~297, 299~720 | are substituted with | D357 | to form | No. | 826264 | ~ | 826982 |
| R3 of | No. | 1~298, 300~720 | are substituted with | D358 | to form | No. | 826983 | ~ | 827701 |
| R3 of | No. | 1~299, 301~720 | are substituted with | D359 | to form | No. | 827702 | ~ | 828420 |
| R3 of | No. | 1~300, 302~720 | are substituted with | D360 | to form | No. | 828421 | ~ | 829139 |
| R3 of | No. | 1~301, 303~720 | are substituted with | D361 | to form | No. | 829140 | ~ | 829858 |
| R3 of | No. | 1~302, 304~720 | are substituted with | D362 | to form | No. | 829859 | ~ | 830577 |
| R3 of | No. | 1~303, 305~720 | are substituted with | D363 | to form | No. | 830578 | ~ | 831296 |
| R3 of | No. | 1~304, 306~720 | are substituted with | D364 | to form | No. | 831297 | ~ | 832015 |
| R3 of | No. | 1~305, 307~720 | are substituted with | D365 | to form | No. | 832016 | ~ | 832734 |
| R3 of | No. | 1~306, 308~720 | are substituted with | D366 | to form | No. | 832735 | ~ | 833453 |

(continued)

| Group to be Substituted | | | Substituting Group | | | New Compound No. | | | |
|---|---|---|---|---|---|---|---|---|---|
| R3 of | No. | 1~307, 309~720 | are substituted with | D367 | to form | No. | 833454 | ~ | 834172 |
| R3 of | No. | 1~308, 310~720 | are substituted with | D368 | to form | No. | 834173 | ~ | 834891 |
| R3 of | No. | 1~309, 311~720 | are substituted with | D369 | to form | No. | 834892 | ~ | 835610 |
| R3 of | No. | 1~310, 312~720 | are substituted with | D370 | to form | No. | 835611 | ~ | 836329 |
| R3 of | No. | 1~311, 313~720 | are substituted with | D371 | to form | No. | 836330 | ~ | 837048 |
| R3 of | No. | 1~312, 314~720 | are substituted with | D372 | to form | No. | 837049 | ~ | 837767 |
| R3 of | No. | 1~313, 315~720 | are substituted with | D373 | to form | No. | 837768 | ~ | 838486 |
| R3 of | No. | 1~314, 316~720 | are substituted with | D374 | to form | No. | 838487 | ~ | 839205 |
| R3 of | No. | 1~315, 317~720 | are substituted with | D375 | to form | No. | 839206 | ~ | 839924 |
| R3 of | No. | 1~316, 318~720 | are substituted with | D376 | to form | No. | 839925 | ~ | 840643 |
| R3 of | No. | 1~317, 319~720 | are substituted with | D377 | to form | No. | 840644 | ~ | 841362 |
| R3 of | No. | 1~318, 320~720 | are substituted with | D378 | to form | No. | 841363 | ~ | 842081 |
| R3 of | No. | 1~319, 321~720 | are substituted with | D379 | to form | No. | 842082 | ~ | 842800 |
| R3 of | No. | 1~320, 322~720 | are substituted with | D380 | to form | No. | 842801 | ~ | 843519 |
| R3 of | No. | 1~321, 323~720 | are substituted with | D381 | to form | No. | 843520 | ~ | 844238 |
| R3 of | No. | 1~322, 324~720 | are substituted with | D382 | to form | No. | 844239 | ~ | 844957 |
| R3 of | No. | 1~323, 325~720 | are substituted with | D383 | to form | No. | 844958 | ~ | 845676 |
| R3 of | No. | 1~324, 326~720 | are substituted with | D384 | to form | No. | 845677 | ~ | 846395 |
| R3 of | No. | 1~325, 327~720 | are substituted with | D385 | to form | No. | 846396 | ~ | 847114 |
| R3 of | No. | 1~326, 328~720 | are substituted with | D386 | to form | No. | 847115 | ~ | 847833 |
| R3 of | No. | 1~327, 329~720 | are substituted with | D387 | to form | No. | 847834 | ~ | 848552 |
| R3 of | No. | 1~328, 330~720 | are substituted with | D388 | to form | No. | 848553 | ~ | 849271 |
| R3 of | No. | 1~329, 331~720 | are substituted with | D389 | to form | No. | 849272 | ~ | 849990 |
| R3 of | No. | 1~330, 332~720 | are substituted with | D390 | to form | No. | 849991 | ~ | 850709 |
| R3 of | No. | 1~331, 333~720 | are substituted with | D391 | to form | No. | 85071 0 | ~ | 851428 |
| R3 of | No. | 1~332, 334~720 | are substituted with | D392 | to form | No. | 851429 | ~ | 852147 |
| R3 of | No. | 1~333, 335~720 | are substituted with | D393 | to form | No. | 852148 | ~ | 852866 |
| R3 of | No. | 1~334, 336~720 | are substituted with | D394 | to form | No. | 852867 | ~ | 853585 |
| R3 of | No. | 1~335, 337~720 | are substituted with | D395 | to form | No. | 853586 | ~ | 854304 |
| R3 of | No. | 1~336, 338~720 | are substituted with | D396 | to form | No. | 854305 | ~ | 855023 |
| R3 of | No. | 1~337, 339~720 | are substituted with | D397 | to form | No. | 855024 | ~ | 855742 |
| R3 of | No. | 1~338, 340~720 | are substituted with | D398 | to form | No. | 855743 | ~ | 856461 |
| R3 of | No. | 1~339, 341~720 | are substituted with | D399 | to form | No. | 856462 | ~ | 857180 |
| R3 of | No. | 1~340, 342~720 | are substituted with | D400 | to form | No. | 857181 | ~ | 857899 |
| R3 of | No. | 1~341, 343~720 | are substituted with | D401 | to form | No. | 857900 | ~ | 858618 |
| R3 of | No. | 1~342, 344~720 | are substituted with | D402 | to form | No. | 858619 | ~ | 859337 |
| R3 of | No. | 1~343, 345~720 | are substituted with | D403 | to form | No. | 859338 | ~ | 860056 |
| R3 of | No. | 1~344, 346~720 | are substituted with | D404 | to form | No. | 860057 | ~ | 860775 |

(continued)

| Group to be Substituted | | | Substituting Group | | | New Compound No. | | | |
|---|---|---|---|---|---|---|---|---|---|
| R3 of | No. | 1~345, 347~720 | are substituted with | D405 | to form | No. | 860776 | ~ | 861494 |
| R3 of | No. | 1~346, 348~720 | are substituted with | D406 | to form | No. | 861495 | ~ | 862213 |
| R3 of | No. | 1~347, 349~720 | are substituted with | D407 | to form | No. | 862214 | ~ | 862932 |
| R3 of | No. | 1~348, 350~720 | are substituted with | D408 | to form | No. | 862933 | ~ | 863651 |
| R3 of | No. | 1~349, 351~720 | are substituted with | D409 | to form | No. | 863652 | ~ | 864370 |
| R3 of | No. | 1~350, 352~720 | are substituted with | D410 | to form | No. | 864371 | ~ | 865089 |
| R3 of | No. | 1~351, 353~720 | are substituted with | D411 | to form | No. | 865090 | ~ | 865808 |
| R3 of | No. | 1~352, 354~720 | are substituted with | D412 | to form | No. | 865809 | ~ | 866527 |
| R3 of | No. | 1~353, 355~720 | are substituted with | D413 | to form | No. | 866528 | ~ | 867246 |
| R3 of | No. | 1~354, 356~720 | are substituted with | D414 | to form | No. | 867247 | ~ | 867965 |
| R3 of | No. | 1~355, 357~720 | are substituted with | D415 | to form | No. | 867966 | ~ | 868684 |
| R3 of | No. | 1~356, 358~720 | are substituted with | D416 | to form | No. | 868685 | ~ | 869403 |
| R3 of | No. | 1~357, 359~720 | are substituted with | D417 | to form | No. | 869404 | ~ | 870122 |
| R3 of | No. | 1~358, 360~720 | are substituted with | D418 | to form | No. | 870123 | ~ | 870841 |
| R3 of | No. | 1~359, 361~720 | are substituted with | D419 | to form | No. | 870842 | ~ | 871560 |
| R3 of | No. | 1~360, 362~720 | are substituted with | D420 | to form | No. | 871561 | ~ | 872279 |
| R3 of | No. | 1~361, 363~720 | are substituted with | D421 | to form | No. | 872280 | ~ | 872998 |
| R3 of | No. | 1~362, 364~720 | are substituted with | D422 | to form | No. | 872999 | ~ | 873717 |
| R3 of | No. | 1~363, 365~720 | are substituted with | D423 | to form | No. | 873718 | ~ | 874436 |
| R3 of | No. | 1~364, 366~720 | are substituted with | D424 | to form | No. | 874437 | ~ | 875155 |
| R3 of | No. | 1~365, 367~720 | are substituted with | D425 | to form | No. | 875156 | ~ | 875874 |
| R3 of | No. | 1~366, 368~720 | are substituted with | D426 | to form | No. | 875875 | ~ | 876593 |
| R3 of | No. | 1~367, 369~720 | are substituted with | D427 | to form | No. | 876594 | ~ | 877312 |
| R3 of | No. | 1~368, 370~720 | are substituted with | D428 | to form | No. | 877313 | ~ | 878031 |
| R3 of | No. | 1~369, 371~720 | are substituted with | D429 | to form | No. | 878032 | ~ | 878750 |
| R3 of | No. | 1~370, 372~720 | are substituted with | D430 | to form | No. | 878751 | ~ | 879469 |
| R3 of | No. | 1~371, 373~720 | are substituted with | D431 | to form | No. | 879470 | ~ | 880188 |
| R3 of | No. | 1~372, 374~720 | are substituted with | D432 | to form | No. | 880189 | ~ | 880907 |
| R3 of | No. | 1~720 | are substituted with | D433 | to form | No. | 880908 | ~ | 881627 |
| R3 of | No. | 1~373, 375~720 | are substituted with | D434 | to form | No. | 881628 | ~ | 882346 |
| R3 of | No. | 1~374, 376~720 | are substituted with | D435 | to form | No. | 882347 | ~ | 883065 |
| R3 of | No. | 1~375, 377~720 | are substituted with | D436 | to form | No. | 883066 | ~ | 883784 |
| R3 of | No. | 1~376, 378~720 | are substituted with | D437 | to form | No. | 883785 | ~ | 884503 |
| R3 of | No. | 1~377, 379~720 | are substituted with | D438 | to form | No. | 884504 | ~ | 885222 |
| R3 of | No. | 1~378, 380~720 | are substituted with | D439 | to form | No. | 885223 | ~ | 885941 |
| R3 of | No. | 1~379, 381~720 | are substituted with | D440 | to form | No. | 885942 | ~ | 886660 |
| R3 of | No. | 1~380, 382~720 | are substituted with | D441 | to form | No. | 886661 | ~ | 887379 |
| R3 of | No. | 1~381, 383~720 | are substituted with | D442 | to form | No. | 887380 | ~ | 888098 |

(continued)

| Group to be Substituted | | | Substituting Group | | | New Compound No. | | | |
|---|---|---|---|---|---|---|---|---|---|
| R3 of | No. | 1~382, 384~720 | are substituted with | D443 | to form | No. | 888099 | ~ | 888817 |
| R3 of | No. | 1~383, 385~720 | are substituted with | D444 | to form | No. | 888818 | ~ | 889536 |
| R3 of | No. | 1~384, 386~720 | are substituted with | D445 | to form | No. | 889537 | ~ | 890255 |
| R3 of | No. | 1~385, 387~720 | are substituted with | D446 | to form | No. | 890256 | ~ | 890974 |
| R3 of | No. | 1~386, 388~720 | are substituted with | D447 | to form | No. | 890975 | ~ | 891693 |
| R3 of | No. | 1~387, 389~720 | are substituted with | D448 | to form | No. | 891694 | ~ | 892412 |
| R3 of | No. | 1~388, 390~720 | are substituted with | D449 | to form | No. | 892413 | ~ | 893131 |
| R3 of | No. | 1~389, 391~720 | are substituted with | D450 | to form | No. | 893132 | ~ | 893850 |
| R3 of | No. | 1~390, 392~720 | are substituted with | D451 | to form | No. | 893851 | ~ | 894569 |
| R3 of | No. | 1~391, 393~720 | are substituted with | D452 | to form | No. | 894570 | ~ | 895288 |
| R3 of | No. | 1~392, 394~720 | are substituted with | D453 | to form | No. | 895289 | ~ | 896007 |
| R3 of | No. | 1~393, 395~720 | are substituted with | D454 | to form | No. | 896008 | ~ | 896726 |
| R3 of | No. | 1~394, 396~720 | are substituted with | D455 | to form | No. | 896727 | ~ | 897445 |
| R3 of | No. | 1~395, 397~720 | are substituted with | D456 | to form | No. | 897446 | ~ | 898164 |
| R3 of | No. | 1~396, 398~720 | are substituted with | D457 | to form | No. | 898165 | ~ | 898883 |
| R3 of | No. | 1~397, 399~720 | are substituted with | D458 | to form | No. | 898884 | ~ | 899602 |
| R3 of | No. | 1~398, 400~720 | are substituted with | D459 | to form | No. | 899603 | ~ | 900321 |
| R3 of | No. | 1~389, 401~720 | are substituted with | D460 | to form | No. | 900322 | ~ | 901040 |
| R3 of | No. | 1~400, 402~720 | are substituted with | D461 | to form | No. | 901041 | ~ | 901759 |
| R3 of | No. | 1~401, 403~720 | are substituted with | D462 | to form | No. | 901760 | ~ | 902478 |
| R3 of | No. | 1~402, 404~720 | are substituted with | D463 | to form | No. | 902479 | ~ | 903197 |
| R3 of | No. | 1~403, 405~720 | are substituted with | D464 | to form | No. | 903198 | ~ | 903916 |
| R3 of | No. | 1~404, 406~720 | are substituted with | D465 | to form | No. | 903917 | ~ | 904635 |
| R3 of | No. | 1~405, 407~720 | are substituted with | D466 | to form | No. | 904636 | ~ | 905354 |
| R3 of | No. | 1~406, 408~720 | are substituted with | D467 | to form | No. | 905355 | ~ | 906073 |
| R3 of | No. | 1~407, 409~720 | are substituted with | D468 | to form | No. | 906074 | ~ | 906792 |
| R3 of | No. | 1~408, 410~720 | are substituted with | D469 | to form | No. | 906793 | ~ | 907511 |
| R3 of | No. | 1~409, 411~720 | are substituted with | D470 | to form | No. | 907512 | ~ | 908230 |
| R3 of | No. | 1~410, 412~720 | are substituted with | D471 | to form | No. | 908231 | ~ | 908949 |
| R3 of | No. | 1~411, 413~720 | are substituted with | D472 | to form | No. | 908950 | ~ | 909668 |
| R3 of | No. | 1~412, 414~720 | are substituted with | D473 | to form | No. | 909669 | ~ | 910387 |
| R3 of | No. | 1~413, 415~720 | are substituted with | D474 | to form | No. | 910388 | ~ | 911106 |
| R3 of | No. | 1~414, 416~720 | are substituted with | D475 | to form | No. | 911107 | ~ | 911825 |
| R3 of | No. | 1~415, 417~720 | are substituted with | D476 | to form | No. | 911826 | ~ | 912544 |
| R3 of | No. | 1~416, 418~720 | are substituted with | D477 | to form | No. | 912545 | ~ | 913263 |
| R3 of | No. | 1~417, 419~720 | are substituted with | D478 | to form | No. | 913264 | ~ | 913982 |
| R3 of | No. | 1~418, 420~720 | are substituted with | D479 | to form | No. | 913983 | ~ | 914701 |
| R3 of | No. | 1~419, 421~720 | are substituted with | D480 | to form | No. | 914702 | ~ | 915420 |

(continued)

| Group to be Substituted | | | Substituting Group | | | New Compound No. | | | |
|---|---|---|---|---|---|---|---|---|---|
| R3 of | No. | 1~420, 422~720 | are substituted with | D481 | to form | No. | 915421 | ~ | 916139 |
| R3 of | No. | 1~421, 423~720 | are substituted with | D482 | to form | No. | 916140 | ~ | 916858 |
| R3 of | No. | 1~422, 424~720 | are substituted with | D483 | to form | No. | 916859 | ~ | 917577 |
| R3 of | No. | 1~423, 425~720 | are substituted with | D484 | to form | No. | 917578 | ~ | 918296 |
| R3 of | No. | 1~424, 426~720 | are substituted with | D485 | to form | No. | 918297 | ~ | 919015 |
| R3 of | No. | 1~425, 427~720 | are substituted with | D486 | to form | No. | 919016 | ~ | 919734 |
| R3 of | No. | 1~426, 428~720 | are substituted with | D487 | to form | No. | 919735 | ~ | 920453 |
| R3 of | No. | 1~427, 429~720 | are substituted with | D488 | to form | No. | 920454 | ~ | 921172 |
| R3 of | No. | 1~428, 430~720 | are substituted with | D489 | to form | No. | 921173 | ~ | 921891 |
| R3 of | No. | 1~429, 431~720 | are substituted with | D490 | to form | No. | 921892 | ~ | 922610 |
| R3 of | No. | 1~430, 432~720 | are substituted with | D491 | to form | No. | 922611 | ~ | 923329 |
| R3 of | No. | 1~431, 433~720 | are substituted with | D492 | to form | No. | 923330 | ~ | 924048 |
| R3 of | No. | 1~432, 434~720 | are substituted with | D493 | to form | No. | 924049 | ~ | 924767 |
| R3 of | No. | 1~433, 435~720 | are substituted with | D494 | to form | No. | 924768 | ~ | 925486 |
| R3 of | No. | 1~434, 436~720 | are substituted with | D495 | to form | No. | 925487 | ~ | 926205 |
| R3 of | No. | 1~435, 437~720 | are substituted with | D496 | to form | No. | 926206 | ~ | 926924 |
| R3 of | No. | 1~436, 438~720 | are substituted with | D497 | to form | No. | 926925 | ~ | 927643 |
| R3 of | No. | 1~437, 439~720 | are substituted with | D498 | to form | No. | 927644 | ~ | 928362 |
| R3 of | No. | 1~438, 440~720 | are substituted with | D499 | to form | No. | 928363 | ~ | 929081 |
| R3 of | No. | 1~439, 441~720 | are substituted with | D500 | to form | No. | 929082 | ~ | 929800 |
| R3 of | No. | 1~440, 442~720 | are substituted with | D501 | to form | No. | 929801 | ~ | 930519 |
| R3 of | No. | 1~441, 443~720 | are substituted with | D502 | to form | No. | 930520 | ~ | 931238 |
| R3 of | No. | 1~442, 444~720 | are substituted with | D503 | to form | No. | 931239 | ~ | 931957 |
| R3 of | No. | 1~443, 445~720 | are substituted with | D504 | to form | No. | 931958 | ~ | 932676 |
| R3 of | No. | 1~444, 446~720 | are substituted with | D505 | to form | No. | 932677 | ~ | 933395 |
| R3 of | No. | 1~445, 447~720 | are substituted with | D506 | to form | No. | 933396 | ~ | 934114 |
| R3 of | No. | 1~446, 448~720 | are substituted with | D507 | to form | No. | 934115 | ~ | 934833 |
| R3 of | No. | 1~447, 449~720 | are substituted with | D508 | to form | No. | 934834 | ~ | 935552 |
| R3 of | No. | 1~448, 450~720 | are substituted with | D509 | to form | No. | 935553 | ~ | 936271 |
| R3 of | No. | 1~449, 451~720 | are substituted with | D510 | to form | No. | 936272 | ~ | 936990 |
| R3 of | No. | 1~450, 452~720 | are substituted with | D511 | to form | No. | 936991 | ~ | 937709 |
| R3 of | No. | 1~451, 453~720 | are substituted with | D512 | to form | No. | 937710 | ~ | 938428 |
| R3 of | No. | 1~452, 454~720 | are substituted with | D513 | to form | No. | 938429 | ~ | 939147 |
| R3 of | No. | 1~453, 455~720 | are substituted with | D514 | to form | No. | 939148 | ~ | 939866 |
| R3 of | No. | 1~454, 456~720 | are substituted with | D515 | to form | No. | 939867 | ~ | 940585 |
| R3 of | No. | 1~455, 457~720 | are substituted with | D516 | to form | No. | 940586 | ~ | 941304 |
| R3 of | No. | 1~456, 458~720 | are substituted with | D517 | to form | No. | 941305 | ~ | 942023 |
| R3 of | No. | 1~457, 459~720 | are substituted with | D518 | to form | No. | 942024 | ~ | 942742 |

(continued)

| Group to be Substituted | | | Substituting Group | | | New Compound No. | | | |
|---|---|---|---|---|---|---|---|---|---|
| R3 of | No. | 1~458, 460~720 | are substituted with | D519 | to form | No. | 942743 | ~ | 943461 |
| R3 of | No. | 1~459, 461~720 | are substituted with | D520 | to form | No. | 943462 | ~ | 944180 |
| R3 of | No. | 1~460, 462~720 | are substituted with | D521 | to form | No. | 944181 | ~ | 944899 |
| R3 of | No. | 1~461, 463~720 | are substituted with | D522 | to form | No. | 944900 | ~ | 945618 |
| R3 of | No. | 1~462, 464~720 | are substituted with | D523 | to form | No. | 945619 | ~ | 946337 |
| R3 of | No. | 1~463, 465~720 | are substituted with | D524 | to form | No. | 946338 | ~ | 947056 |
| R3 of | No. | 1~464, 466~720 | are substituted with | D525 | to form | No. | 947057 | ~ | 947775 |
| R3 of | No. | 1~465, 467~720 | are substituted with | D526 | to form | No. | 947776 | ~ | 948494 |
| R3 of | No. | 1~466, 468~720 | are substituted with | D527 | to form | No. | 948495 | ~ | 949213 |
| R3 of | No. | 1~467, 469~720 | are substituted with | D528 | to form | No. | 949214 | ~ | 949932 |
| R3 of | No. | 1~468, 470~720 | are substituted with | D529 | to form | No. | 949933 | ~ | 950651 |
| R3 of | No. | 1~469, 471~720 | are substituted with | D530 | to form | No. | 950652 | ~ | 951370 |
| R3 of | No. | 1~470, 472~720 | are substituted with | D531 | to form | No. | 951371 | ~ | 952089 |
| R3 of | No. | 1~471, 473~720 | are substituted with | D532 | to form | No. | 952090 | ~ | 952808 |
| R3 of | No. | 1~472, 474~720 | are substituted with | D533 | to form | No. | 952809 | ~ | 953527 |
| R3 of | No. | 1~473, 475~720 | are substituted with | D534 | to form | No. | 953528 | ~ | 954246 |
| R3 of | No. | 1~474, 476~720 | are substituted with | D535 | to form | No. | 954247 | ~ | 954965 |
| R3 of | No. | 1~475, 477~720 | are substituted with | D536 | to form | No. | 954966 | ~ | 955684 |
| R3 of | No. | 1~476, 478~720 | are substituted with | D537 | to form | No. | 955685 | ~ | 956403 |
| R3 of | No. | 1~477, 479~720 | are substituted with | D538 | to form | No. | 956404 | ~ | 957122 |
| R3 of | No. | 1~478, 480~720 | are substituted with | D539 | to form | No. | 957123 | ~ | 957841 |
| R3 of | No. | 1~479, 481~720 | are substituted with | D540 | to form | No. | 957842 | ~ | 958560 |
| R3 of | No. | 1~480, 482~720 | are substituted with | D541 | to form | No. | 958561 | ~ | 959279 |
| R3 of | No. | 1~481, 483~720 | are substituted with | D542 | to form | No. | 959280 | ~ | 959998 |
| R3 of | No. | 1~482, 484~720 | are substituted with | D543 | to form | No. | 959999 | ~ | 960717 |
| R3 of | No. | 1~483, 485~720 | are substituted with | D544 | to form | No. | 960718 | ~ | 961436 |
| R3 of | No. | 1~484, 486~720 | are substituted with | D545 | to form | No. | 961437 | ~ | 962155 |
| R3 of | No. | 1~485, 487~720 | are substituted with | D546 | to form | No. | 962156 | ~ | 962874 |
| R3 of | No. | 1~486, 488~720 | are substituted with | D547 | to form | No. | 962875 | ~ | 963593 |
| R3 of | No. | 1~487, 489~720 | are substituted with | D548 | to form | No. | 963594 | ~ | 964312 |
| R3 of | No. | 1~488, 490~720 | are substituted with | D549 | to form | No. | 964313 | ~ | 965031 |
| R3 of | No. | 1~489, 491~720 | are substituted with | D550 | to form | No. | 965032 | ~ | 965750 |
| R3 of | No. | 1~490, 492~720 | are substituted with | D551 | to form | No. | 965751 | ~ | 966469 |
| R3 of | No. | 1~491, 493~720 | are substituted with | D552 | to form | No. | 966470 | ~ | 967188 |
| R3 of | No. | 1~492, 494~720 | are substituted with | D553 | to form | No. | 967189 | ~ | 967907 |
| R3 of | No. | 1~493, 495~720 | are substituted with | D554 | to form | No. | 967908 | ~ | 968626 |
| R3 of | No. | 1~494, 496~720 | are substituted with | D555 | to form | No. | 968627 | ~ | 969345 |
| R3 of | No. | 1~495, 497~720 | are substituted with | D556 | to form | No. | 969346 | ~ | 970064 |

(continued)

| Group to be Substituted | | | Substituting Group | | | New Compound No. | | | |
|---|---|---|---|---|---|---|---|---|---|
| R3 of | No. | 1~496, 498~720 | are substituted with | D557 | to form | No. | 970065 | ~ | 970783 |
| R3 of | No. | 1~497, 499~720 | are substituted with | D558 | to form | No. | 970784 | ~ | 971502 |
| R3 of | No. | 1~498, 500~720 | are substituted with | D559 | to form | No. | 971503 | ~ | 972221 |
| R3 of | No. | 1~499, 501~720 | are substituted with | D560 | to form | No. | 972222 | ~ | 972940 |
| R3 of | No. | 1~500, 502~720 | are substituted with | D561 | to form | No. | 972941 | ~ | 973659 |
| R3 of | No. | 1~501, 503~720 | are substituted with | D562 | to form | No. | 973660 | ~ | 974378 |
| R3 of | No. | 1~502, 504~720 | are substituted with | D563 | to form | No. | 974379 | ~ | 975097 |
| R3 of | No. | 1~503, 505~720 | are substituted with | D564 | to form | No. | 975098 | ~ | 975816 |
| R3 of | No. | 1~504, 506~720 | are substituted with | D565 | to form | No. | 975817 | ~ | 976535 |
| R3 of | No. | 1~505, 507~720 | are substituted with | D566 | to form | No. | 976536 | ~ | 977254 |
| R3 of | No. | 1~506, 508~720 | are substituted with | D567 | to form | No. | 977255 | ~ | 977973 |
| R3 of | No. | 1~507, 509~720 | are substituted with | D568 | to form | No. | 977974 | ~ | 978692 |
| R3 of | No. | 1~508, 510~720 | are substituted with | D569 | to form | No. | 978693 | ~ | 979411 |
| R3 of | No. | 1~509, 511~720 | are substituted with | D570 | to form | No. | 979412 | ~ | 980130 |
| R3 of | No. | 1~510, 512~720 | are substituted with | D571 | to form | No. | 980131 | ~ | 980849 |
| R3 of | No. | 1~511, 513~720 | are substituted with | D572 | to form | No. | 980850 | ~ | 981568 |
| R3 of | No. | 1~512, 514~720 | are substituted with | D573 | to form | No. | 981569 | ~ | 982287 |
| R3 of | No. | 1~513, 515~720 | are substituted with | D574 | to form | No. | 982288 | ~ | 983006 |
| R3 of | No. | 1~514, 516~720 | are substituted with | D575 | to form | No. | 983007 | ~ | 983725 |
| R3 of | No. | 1~515, 517~720 | are substituted with | D576 | to form | No. | 983726 | ~ | 984444 |
| R3 of | No. | 1~516, 518~720 | are substituted with | D577 | to form | No. | 984445 | ~ | 985163 |
| R3 of | No. | 1~517, 519~720 | are substituted with | D578 | to form | No. | 985164 | ~ | 985882 |
| R3 of | No. | 1~518, 520~720 | are substituted with | D579 | to form | No. | 985883 | ~ | 986601 |
| R3 of | No. | 1~519, 521~720 | are substituted with | D580 | to form | No. | 986602 | ~ | 987320 |
| R3 of | No. | 1~520, 522~720 | are substituted with | D581 | to form | No. | 987321 | ~ | 988039 |
| R3 of | No. | 1~521, 523~720 | are substituted with | D582 | to form | No. | 988040 | ~ | 988758 |
| R3 of | No. | 1~522, 524~720 | are substituted with | D583 | to form | No. | 988759 | ~ | 989477 |
| R3 of | No. | 1~523, 525~720 | are substituted with | D584 | to form | No. | 989478 | ~ | 990196 |
| R3 of | No. | 1~524, 526~720 | are substituted with | D585 | to form | No. | 990197 | ~ | 990915 |
| R3 of | No. | 1~525, 527~720 | are substituted with | D586 | to form | No. | 990916 | ~ | 991634 |
| R3 of | No. | 1~526, 528~720 | are substituted with | D587 | to form | No. | 991635 | ~ | 992353 |
| R3 of | No. | 1~527, 529~720 | are substituted with | D588 | to form | No. | 992354 | ~ | 993072 |
| R3 of | No. | 1~528, 530~720 | are substituted with | D589 | to form | No. | 993073 | ~ | 993791 |
| R3 of | No. | 1~529, 531~720 | are substituted with | D590 | to form | No. | 993792 | ~ | 994510 |
| R3 of | No. | 1~530, 532~720 | are substituted with | D591 | to form | No. | 994511 | ~ | 995229 |
| R3 of | No. | 1~531, 533~720 | are substituted with | D592 | to form | No. | 995230 | ~ | 995948 |
| R3 of | No. | 1~532, 534~720 | are substituted with | D593 | to form | No. | 995949 | ~ | 996667 |
| R3 of | No. | 1~533, 535~720 | are substituted with | D594 | to form | No. | 996668 | ~ | 997386 |

(continued)

| Group to be Substituted | | | Substituting Group | | | New Compound No. | | | |
|---|---|---|---|---|---|---|---|---|---|
| R3 of | No. | 1~534, 536~720 | are substituted with | D595 | to form | No. | 997387 | ~ | 998105 |
| R3 of | No. | 1~535, 537~720 | are substituted with | D596 | to form | No. | 998106 | ~ | 998824 |
| R3 of | No. | 1~536, 538~720 | are substituted with | D597 | to form | No. | 998825 | ~ | 999543 |
| R3 of | No. | 1~537, 539~720 | are substituted with | D598 | to form | No. | 999544 | ~ | 1000262 |
| R3 of | No. | 1~538, 540~720 | are substituted with | D599 | to form | No. | 1000263 | ~ | 1000981 |
| R3 of | No. | 1~539, 541~720 | are substituted with | D600 | to form | No. | 1000982 | ~ | 1001700 |
| R3 of | No. | 1~540, 542~720 | are substituted with | D601 | to form | No. | 1001701 | ~ | 1002419 |
| R3 of | No. | 1~541, 543~720 | are substituted with | D602 | to form | No. | 1002420 | ~ | 1003138 |
| R3 of | No. | 1~542, 544~720 | are substituted with | D603 | to form | No. | 1003139 | ~ | 1003857 |
| R3 of | No. | 1~543, 545~720 | are substituted with | D604 | to form | No. | 1003858 | ~ | 1004576 |
| R3 of | No. | 1~544, 546~720 | are substituted with | D605 | to form | No. | 1004577 | ~ | 1005295 |
| R3 of | No. | 1~545, 547~720 | are substituted with | D606 | to form | No. | 1005296 | ~ | 1006014 |
| R3 of | No. | 1~546, 548~720 | are substituted with | D607 | to form | No. | 006015 | ~ | 1006733 |
| R3 of | No. | 1~547, 549~720 | are substituted with | D608 | to form | No. | 1006734 | ~ | 1007452 |
| R3 of | No. | 1~548, 550~720 | are substituted with | D609 | to form | No. | 1007453 | ~ | 1008171 |
| R3 of | No. | 1~549, 551~720 | are substituted with | D610 | to form | No. | 008172 | ~ | 1008890 |
| R3 of | No. | 1~550, 552~720 | are substituted with | D611 | to form | No. | 1008891 | ~ | 1009609 |
| R3 of | No. | 1~551, 553~720 | are substituted with | D612 | to form | No. | 009610 | ~ | 1010328 |
| R3 of | No. | 1~552, 554~720 | are substituted with | D613 | to form | No. | 1010329 | ~ | 1011047 |
| R3 of | No. | 1~553, 555~720 | are substituted with | D614 | to form | No. | 1011048 | ~ | 1011766 |
| R3 of | No. | 1~554, 556~720 | are substituted with | D615 | to form | No. | 011767 | ~ | 1012485 |
| R3 of | No. | 1~555, 557~720 | are substituted with | D616 | to form | No. | 1012486 | ~ | 1013204 |
| R3 of | No. | 1~556, 558~720 | are substituted with | D617 | to form | No. | 1013205 | ~ | 1013923 |
| R3 of | No. | 1~557, 559~720 | are substituted with | D618 | to form | No. | 1013924 | ~ | 1014642 |
| R3 of | No. | 1~558, 560~720 | are substituted with | D619 | to form | No. | 1014643 | ~ | 1015361 |
| R3 of | No. | 1~559, 561~720 | are substituted with | D620 | to form | No. | 1015362 | ~ | 1016080 |
| R3 of | No. | 1~560, 562~720 | are substituted with | D621 | to form | No. | 1016081 | ~ | 1016799 |
| R3 of | No. | 1~561, 563~720 | are substituted with | D622 | to form | No. | 1016800 | ~ | 1017518 |
| R3 of | No. | 1~562, 564~720 | are substituted with | D623 | to form | No. | 017519 | ~ | 1018237 |
| R3 of | No. | 1~563, 565~720 | are substituted with | D624 | to form | No. | 1018238 | ~ | 1018956 |
| R3 of | No. | 1~564, 566~720 | are substituted with | D625 | to form | No. | 1018957 | ~ | 1019675 |
| R3 of | No. | 1~565, 567~720 | are substituted with | D626 | to form | No. | 1019676 | ~ | 1020394 |
| R3 of | No. | 1~566, 568~720 | are substituted with | D627 | to form | No. | 1020395 | ~ | 1021113 |
| R3 of | No. | 1~567, 569~720 | are substituted with | D628 | to form | No. | 1021114 | ~ | 1021832 |
| R3 of | No. | 1~568, 570~720 | are substituted with | D629 | to form | No. | 021833 | ~ | 1022551 |
| R3 of | No. | 1~569, 571~720 | are substituted with | D630 | to form | No. | 1022552 | ~ | 1023270 |
| R3 of | No. | 1~570, 572~720 | are substituted with | D631 | to form | No. | 1023271 | ~ | 1023989 |
| R3 of | No. | 1~571, 573~720 | are substituted with | D632 | to form | No. | 023990 | ~ | 1024708 |

(continued)

| Group to be Substituted | | | Substituting Group | | | New Compound No. | | | |
|---|---|---|---|---|---|---|---|---|---|
| R3 of | No. | 1~572, 574~720 | are substituted with | D633 | to form | No. | 024 709 | ~ | 1025427 |
| R3 of | No. | 1~573, 575~720 | are substituted with | D634 | to form | No. | 1025428 | ~ | 1026146 |
| R3 of | No. | 1~574, 576~720 | are substituted with | D635 | to form | No. | 1026147 | ~ | 1026865 |
| R3 of | No. | 1~575, 577~720 | are substituted with | D636 | to form | No. | 1026866 | ~ | 1027584 |
| R3 of | No. | 1~576, 578~720 | are substituted with | D637 | to form | No. | 1027585 | ~ | 1028303 |
| R3 of | No. | 1~577, 579~720 | are substituted with | D638 | to form | No. | 1028304 | ~ | 1029022 |
| R3 of | No. | 1~578, 580~720 | are substituted with | D639 | to form | No. | 1029023 | ~ | 1029741 |
| R3 of | No. | 1~579, 581~720 | are substituted with | D640 | to form | No. | 1029742 | ~ | 1030460 |
| R3 of | No. | 1~580, 582~720 | are substituted with | D641 | to form | No. | 1030461 | ~ | 1031179 |
| R3 of | No. | 1~581, 583~720 | are substituted with | D642 | to form | No. | 031180 | ~ | 1031898 |
| R3 of | No. | 1~582, 584~720 | are substituted with | D643 | to form | No. | 031899 | ~ | 1032617 |
| R3 of | No. | 1~583, 585~720 | are substituted with | D644 | to form | No. | 032618 | ~ | 1033336 |
| R3 of | No. | 1~584, 586~720 | are substituted with | D645 | to form | No. | 1033337 | ~ | 1034055 |
| R3 of | No. | 1~585, 587~720 | are substituted with | D646 | to form | No. | 1034056 | ~ | 1034774 |
| R3 of | No. | 1~586, 588~720 | are substituted with | D647 | to form | No. | 034775 | ~ | 1035493 |
| R3 of | No. | 1~587, 589~720 | are substituted with | D648 | to form | No. | 1035494 | ~ | 1036212 |
| R3 of | No. | 1~588, 590~720 | are substituted with | D649 | to form | No. | 036213 | ~ | 1036931 |
| R3 of | No. | 1~589, 591~720 | are substituted with | D650 | to form | No. | 1036932 | ~ | 1037650 |
| R3 of | No. | 1~590, 592~720 | are substituted with | D651 | to form | No. | 1037651 | ~ | 1038369 |
| R3 of | No. | 1~591, 593~720 | are substituted with | D652 | to form | No. | 1038370 | ~ | 1039088 |
| R3 of | No. | 1~592, 594~720 | are substituted with | D653 | to form | No. | 1039089 | ~ | 1039807 |
| R3 of | No. | 1~593, 595~720 | are substituted with | D654 | to form | No. | 1039808 | ~ | 1040526 |
| R3 of | No. | 1~594, 596~720 | are substituted with | D655 | to form | No. | 1040527 | ~ | 1041245 |
| R3 of | No. | 1~595, 597~720 | are substituted with | D656 | to form | No. | 1041246 | ~ | 1041964 |
| R3 of | No. | 1~596, 598~720 | are substituted with | D657 | to form | No. | 041965 | ~ | 1042683 |
| R3 of | No. | 1~597, 599~720 | are substituted with | D658 | to form | No. | 1042684 | ~ | 1043402 |
| R3 of | No. | 1~598, 600~720 | are substituted with | D659 | to form | No. | 1043403 | ~ | 1044121 |
| R3 of | No. | 1~599, 601~720 | are substituted with | D660 | to form | No. | 1044122 | ~ | 1044840 |
| R3 of | No. | 1~600, 602~720 | are substituted with | D661 | to form | No. | 1044841 | ~ | 1045559 |
| R3 of | No. | 1~601, 603~720 | are substituted with | D662 | to form | No. | 1045560 | ~ | 1046278 |
| R3 of | No. | 1~602, 604~720 | are substituted with | D663 | to form | No. | 1046279 | ~ | 1046997 |
| R3 of | No. | 1~603, 605~720 | are substituted with | D664 | to form | No. | 1046998 | ~ | 1047716 |
| R3 of | No. | 1~604, 606~720 | are substituted with | D665 | to form | No. | 047717 | ~ | 1048435 |
| R3 of | No. | 1~605, 607~720 | are substituted with | D666 | to form | No. | 1048436 | ~ | 1049154 |
| R3 of | No. | 1~606, 608~720 | are substituted with | D667 | to form | No. | 1049155 | ~ | 1049873 |
| R3 of | No. | 1~607, 609~720 | are substituted with | D668 | to form | No. | 1049874 | ~ | 1050592 |
| R3 of | No. | 1~608, 610~720 | are substituted with | D669 | to form | No. | 1050593 | ~ | 1051311 |
| R3 of | No. | 1~609, 611~720 | are substituted with | D670 | to form | No. | 051312 | ~ | 1052030 |

(continued)

| Group to be Substituted | | | Substituting Group | | | New Compound No. | | | |
|---|---|---|---|---|---|---|---|---|---|
| R3 of | No. | 1~610, 612~720 | are substituted with | D671 | to form | No. | 1052031 | ~ | 1052749 |
| R3 of | No. | 1~611, 613~720 | are substituted with | D672 | to form | No. | 1052750 | ~ | 1053468 |
| R3 of | No. | 1~612, 614~720 | are substituted with | D673 | to form | No. | 1053469 | ~ | 1054187 |
| R3 of | No. | 1~613, 615~720 | are substituted with | D674 | to form | No. | 1054188 | ~ | 1054906 |
| R3 of | No. | 1~614, 616~720 | are substituted with | D675 | to form | No. | 1054907 | ~ | 1055625 |
| R3 of | No. | 1~615, 617~720 | are substituted with | D676 | to form | No. | 1055626 | ~ | 1056344 |
| R3 of | No. | 1~616, 618~720 | are substituted with | D677 | to form | No. | 1056345 | ~ | 1057063 |
| R3 of | No. | 1~617, 619~720 | are substituted with | D678 | to form | No. | 1057064 | ~ | 1057782 |
| R3 of | No. | 1~618, 620~720 | are substituted with | D679 | to form | No. | 1057783 | ~ | 1058501 |
| R3 of | No. | 1~619, 621~720 | are substituted with | D680 | to form | No. | 1058502 | ~ | 1059220 |
| R3 of | No. | 1~620, 622~720 | are substituted with | D681 | to form | No. | 1059221 | ~ | 1059939 |
| R3 of | No. | 1~621, 623~720 | are substituted with | D682 | to form | No. | 1059940 | ~ | 1060658 |
| R3 of | No. | 1~622, 624~720 | are substituted with | D683 | to form | No. | 1060659 | ~ | 1061377 |
| R3 of | No. | 1~623, 625~720 | are substituted with | D684 | to form | No. | 061378 | ~ | 1062096 |
| R3 of | No. | 1~624, 626~720 | are substituted with | D685 | to form | No. | 1062097 | ~ | 1062815 |
| R3 of | No. | 1~625, 627~720 | are substituted with | D686 | to form | No. | 062816 | ~ | 1063534 |
| R3 of | No. | 1~626, 628~720 | are substituted with | D687 | to form | No. | 1063535 | ~ | 1064253 |
| R3 of | No. | 1~627, 629~720 | are substituted with | D688 | to form | No. | 1064254 | ~ | 1064972 |
| R3 of | No. | 1~628, 630~720 | are substituted with | D689 | to form | No. | 1064973 | ~ | 1065691 |
| R3 of | No. | 1~629, 631~720 | are substituted with | D690 | to form | No. | 1065692 | ~ | 1066410 |
| R3 of | No. | 1~630, 632~720 | are substituted with | D691 | to form | No. | 1066411 | ~ | 1067129 |
| R3 of | No. | 1~631, 633~720 | are substituted with | D692 | to form | No. | 1067130 | ~ | 1067848 |
| R3 of | No. | 1~632, 634~720 | are substituted with | D693 | to form | No. | 1067849 | ~ | 1068567 |
| R3 of | No. | 1~633, 635~720 | are substituted with | D694 | to form | No. | 1068568 | ~ | 1069286 |
| R3 of | No. | 1~634, 636~720 | are substituted with | D695 | to form | No. | 1069287 | ~ | 1070005 |
| R3 of | No. | 1~635, 637~720 | are substituted with | D696 | to form | No. | 1070006 | ~ | 1070724 |
| R3 of | No. | 1~636, 638~720 | are substituted with | D697 | to form | No. | 1070725 | ~ | 1071443 |
| R3 of | No. | 1~637, 639~720 | are substituted with | D698 | to form | No. | 1071444 | ~ | 1072162 |
| R3 of | No. | 1~638, 640~720 | are substituted with | D699 | to form | No. | 1072163 | ~ | 1072881 |
| R3 of | No. | 1~639, 641~720 | are substituted with | D700 | to form | No. | 1072882 | ~ | 1073600 |
| R3 of | No. | 1~640, 642~720 | are substituted with | D701 | to form | No. | 1073601 | ~ | 1074319 |
| R3 of | No. | 1~641, 643~720 | are substituted with | D702 | to form | No. | 1074320 | ~ | 1075038 |
| R3 of | No. | 1~642, 644~720 | are substituted with | D703 | to form | No. | 1075039 | ~ | 1075757 |
| R3 of | No. | 1~643, 645~720 | are substituted with | D704 | to form | No. | 1075758 | ~ | 1076476 |
| R3 of | No. | 1~644, 646~720 | are substituted with | D705 | to form | No. | 1076477 | ~ | 1077195 |
| R3 of | No. | 1~645, 647~720 | are substituted with | D706 | to form | No. | 1077196 | ~ | 1077914 |
| R3 of | No. | 1~646, 648~720 | are substituted with | D707 | to form | No. | 077915 | ~ | 1078633 |
| R3 of | No. | 1~647, 649~720 | are substituted with | D708 | to form | No. | 1078634 | ~ | 1079352 |

(continued)

| Group to be Substituted | | | Substituting Group | | | New Compound No. | | | |
|---|---|---|---|---|---|---|---|---|---|
| R3 of | No. | 1~648, 650~720 | are substituted with | D709 | to form | No. | 1079353 | ~ | 1080071 |
| R3 of | No. | 1~649, 651~720 | are substituted with | D710 | to form | No. | 1080072 | ~ | 1080790 |
| R3 of | No. | 1~650, 652~720 | are substituted with | D711 | to form | No. | 1080791 | ~ | 1081509 |
| R3 of | No. | 1~651, 653~720 | are substituted with | D712 | to form | No. | 1081510 | ~ | 1082228 |
| R3 of | No. | 1~652, 654~720 | are substituted with | D713 | to form | No. | 1082229 | ~ | 1082947 |
| R3 of | No. | 1~653, 655~720 | are substituted with | D714 | to form | No. | 1082948 | ~ | 1083666 |
| R3 of | No. | 1~654, 656~720 | are substituted with | D715 | to form | No. | 1083667 | ~ | 1084385 |
| R3 of | No. | 1~655, 657~720 | are substituted with | D716 | to form | No. | 1084386 | ~ | 1085104 |
| R3 of | No. | 1~656, 658~720 | are substituted with | D717 | to form | No. | 1085105 | ~ | 1085823 |
| R3 of | No. | 1~657, 659~720 | are substituted with | D718 | to form | No. | 1085824 | ~ | 1086542 |
| R3 of | No. | 1~658, 660~720 | are substituted with | D719 | to form | No. | 1086543 | ~ | 1087261 |
| R3 of | No. | 1~659, 661~720 | are substituted with | D720 | to form | No. | 1087262 | ~ | 1087980 |
| R3 of | No. | 1~660, 662~720 | are substituted with | D721 | to form | No. | 1087981 | ~ | 1088699 |
| R3 of | No. | 1~661, 663~720 | are substituted with | D722 | to form | No. | 1088700 | ~ | 1089418 |
| R3 of | No. | 1~662, 664~720 | are substituted with | D723 | to form | No. | 1089419 | ~ | 1090137 |
| R3 of | No. | 1~663, 665~720 | are substituted with | D724 | to form | No. | 1090138 | ~ | 1090856 |
| R3 of | No. | 1~664, 666~720 | are substituted with | D725 | to form | No. | 1090857 | ~ | 1091575 |
| R3 of | No. | 1~665, 667~720 | are substituted with | D726 | to form | No. | 091576 | ~ | 1092294 |
| R3 of | No. | 1~666, 668~720 | are substituted with | D727 | to form | No. | 1092295 | ~ | 1093013 |
| R3 of | No. | 1~667, 669~720 | are substituted with | D728 | to form | No. | 1093014 | ~ | 1093732 |
| R3 of | No. | 1~668, 670~720 | are substituted with | D729 | to form | No. | 1093733 | ~ | 1094451 |
| R3 of | No. | 1~669, 671~720 | are substituted with | D730 | to form | No. | 1094452 | ~ | 1095170 |
| R3 of | No. | 1~670, 672~720 | are substituted with | D731 | to form | No. | 095171 | ~ | 1095889 |
| R3 of | No. | 1~671, 673~720 | are substituted with | D732 | to form | No. | 1095890 | ~ | 1096608 |
| R3 of | No. | 1~672, 674~720 | are substituted with | D733 | to form | No. | 1096609 | ~ | 1097327 |
| R3 of | No. | 1~673, 675~720 | are substituted with | D734 | to form | No. | 097328 | ~ | 1098046 |
| R3 of | No. | 1~674, 676~720 | are substituted with | D735 | to form | No. | 098047 | ~ | 1098765 |
| R3 of | No. | 1~675, 677~720 | are substituted with | D736 | to form | No. | 1098766 | ~ | 1099484 |
| R3 of | No. | 1~676, 678~720 | are substituted with | D737 | to form | No. | 1099485 | ~ | 1100203 |
| R3 of | No. | 1~677, 679~720 | are substituted with | D738 | to form | No. | 1100204 | ~ | 1100922 |
| R3 of | No. | 1~678, 680~720 | are substituted with | D739 | to form | No. | 1100923 | ~ | 1101641 |
| R3 of | No. | 1~679, 681~720 | are substituted with | D740 | to form | No. | 1101642 | ~ | 1102360 |
| R3 of | No. | 1~680, 682~720 | are substituted with | D741 | to form | No. | 1102361 | ~ | 1103079 |
| R3 of | No. | 1~681, 683~720 | are substituted with | D742 | to form | No. | 1103080 | ~ | 1103798 |
| R3 of | No. | 1~682, 684~720 | are substituted with | D743 | to form | No. | 1103799 | ~ | 1104517 |
| R3 of | No. | 1~683, 685~720 | are substituted with | D744 | to form | No. | 1104518 | ~ | 1105236 |
| R3 of | No. | 1~684, 686~720 | are substituted with | D745 | to form | No. | 1105237 | ~ | 1105955 |
| R3 of | No. | 1~685, 687~720 | are substituted with | D746 | to form | No. | 1105956 | ~ | 1106674 |

(continued)

| Group to be Substituted | | | Substituting Group | | | New Compound No. | | | |
|---|---|---|---|---|---|---|---|---|---|
| R3 of | No. | 1~686, 688~720 | are substituted with | D747 | to form | No. | 1106675 | ~ | 1107393 |
| R3 of | No. | 1~687, 689~720 | are substituted with | D748 | to form | No. | 1107394 | ~ | 1108112 |
| R3 of | No. | 1~688, 690~720 | are substituted with | D749 | to form | No. | 1108113 | ~ | 1108831 |
| R3 of | No. | 1~689, 691~720 | are substituted with | D750 | to form | No. | 1108832 | ~ | 1109550 |
| R3 of | No. | 1~690, 692~720 | are substituted with | D751 | to form | No. | 1109551 | ~ | 1110269 |
| R3 of | No. | 1~691, 693~720 | are substituted with | D752 | to form | No. | 1110270 | ~ | 1110988 |
| R3 of | No. | 1~692, 694~720 | are substituted with | D753 | to form | No. | 1110989 | ~ | 1111707 |
| R3 of | No. | 1~693, 695~720 | are substituted with | D754 | to form | No. | 1111708 | ~ | 1112426 |
| R3 of | No. | 1~694, 696~720 | are substituted with | D755 | to form | No. | 1112427 | ~ | 1113145 |
| R3 of | No. | 1~695, 697~720 | are substituted with | D756 | to form | No. | 1113146 | ~ | 1113864 |
| R3 of | No. | 1~696, 698~720 | are substituted with | D757 | to form | No. | 1113865 | ~ | 1114583 |
| R3 of | No. | 1~697, 699~720 | are substituted with | D758 | to form | No. | 1114584 | ~ | 1115302 |
| R3 of | No. | 1~698, 700~720 | are substituted with | D759 | to form | No. | 1115303 | ~ | 1116021 |
| R3 of | No. | 1~699, 701~720 | are substituted with | D760 | to form | No. | 1116022 | ~ | 1116740 |
| R3 of | No. | 1~700, 702~720 | are substituted with | D761 | to form | No. | 1116741 | ~ | 1117459 |
| R3 of | No. | 1~701, 703~720 | are substituted with | D762 | to form | No. | 1117460 | ~ | 1118178 |
| R3 of | No. | 1~702, 704~720 | are substituted with | D763 | to form | No. | 1118179 | ~ | 1118897 |
| R3 of | No. | 1~703, 705~720 | are substituted with | D764 | to form | No. | 1118898 | ~ | 1119616 |
| R3 of | No. | 1~704, 706~720 | are substituted with | D765 | to form | No. | 1119617 | ~ | 1120335 |
| R3 of | No. | 1~705, 707~720 | are substituted with | D766 | to form | No. | 1120336 | ~ | 1121054 |
| R3 of | No. | 1~706, 708~720 | are substituted with | D767 | to form | No. | 1121055 | ~ | 1121773 |
| R3 of | No. | 1~707, 709~720 | are substituted with | D768 | to form | No. | 1121774 | ~ | 1122492 |
| R3 of | No. | 1~708, 710~720 | are substituted with | D769 | to form | No. | 1122493 | ~ | 1123211 |
| R3 of | No. | 1~709, 711~720 | are substituted with | D770 | to form | No. | 1123212 | ~ | 1123930 |
| R3 of | No. | 1~710, 712~720 | are substituted with | D771 | to form | No. | 1123931 | ~ | 1124649 |
| R3 of | No. | 1~711, 713~720 | are substituted with | D772 | to form | No. | 1124650 | ~ | 1125368 |
| R3 of | No. | 1~712, 714~720 | are substituted with | D773 | to form | No. | 1125369 | ~ | 1126087 |
| R3 of | No. | 1~713, 715~720 | are substituted with | D774 | to form | No. | 1126088 | ~ | 1126806 |
| R3 of | No. | 1~714, 716~720 | are substituted with | D775 | to form | No. | 1126807 | ~ | 1127525 |
| R3 of | No. | 1~715, 717~720 | are substituted with | D776 | to form | No. | 1127526 | ~ | 1128244 |
| R3 of | No. | 1~716, 718~720 | are substituted with | D777 | to form | No. | 1128245 | ~ | 1128963 |
| R3 of | No. | 1~717, 719~720 | are substituted with | D778 | to form | No. | 1128964 | ~ | 1129682 |
| R3 of | No. | 1~718, 720~720 | are substituted with | D779 | to form | No. | 1129683 | ~ | 1130401 |
| R3 of | No. | 1~719, 721~720 | are substituted with | D780 | to form | No. | 1130402 | ~ | 1131120 |
| R3 of | No. | 1~720 | are substituted with | D781 | to form | No. | 1131121 | ~ | 1131840 |
| R3 of | No. | 1~720 | are substituted with | D782 | to form | No. | 1131841 | ~ | 1132560 |
| R3 of | No. | 1~720 | are substituted with | D783 | to form | No. | 1132561 | ~ | 1133280 |
| R3 of | No. | 1~720 | are substituted with | D784 | to form | No. | 1133281 | ~ | 1134000 |

(continued)

| Group to be Substituted | | | Substituting Group | | | New Compound No. | | | |
|---|---|---|---|---|---|---|---|---|---|
| R3 of | No. | 1~720 | are substituted with | D785 | to form | No. | 1134001 | ~ | 1134720 |
| R3 of | No. | 1~720 | are substituted with | D786 | to form | No. | 1134721 | ~ | 1135440 |
| R3 of | No. | 1 ~720 | are substituted with | D787 | to form | No. | 1135441 | ~ | 1136160 |
| R3 of | No. | 1 ~720 | are substituted with | D788 | to form | No. | 1136161 | ~ | 1136880 |
| R3 of | No. | 1 ~720 | are substituted with | D789 | to form | No. | 1136881 | ~ | 1137600 |
| R3 of | No. | 1 ~720 | are substituted with | D790 | to form | No. | 1137601 | ~ | 1138320 |
| R3 of | No. | 1 ~720 | are substituted with | D791 | to form | No. | 1138321 | ~ | 1139040 |
| R3 of | No. | 1 ~720 | are substituted with | D792 | to form | No. | 1139041 | ~ | 1139760 |

The molecular weight of the compound represented by the general formula (1) is, for example, when an organic layer containing the compound represented by the general formula (1) is intended to be formed by an evaporation method and used, preferably 1500 or less, more preferably 1200 or less, even more preferably 1000 or less, further more preferably 900 or less. The lower limit of the molecular weight is a molecular weight of the smallest compound represented by the general formula (1).

The compound represented by the general formula (1) can be formed into a layer by a coating method, irrespective of the molecular weight thereof. According to a coating method, the compound having a relatively large molecular weight can be formed into a layer. The compound represented by the general formula (1) has an advantage that, among cyanobenzene compounds, the compound is readily soluble in an organic compound. Consequently, a coating method is readily applicable to the compound represented by the general formula (1) and, in addition, the compound can be purified to have an increased purity.

By applying the present invention, it is considered that a compound containing plural number of structures represented by the general formula (1) in the molecule can be used as a light emitting material.

For example, it is considered that a polymerizable group is previously introduced into the structure represented by the general formula (1), and the polymer formed by polymerizing the polymerizable group is used as a light emitting material. Specifically, it is considered that a monomer containing a polymerizable functional group in any of $R^1$ to $R^4$ in the general formula (1) is prepared, and this is homo-polymerized, or is copolymerized with any other monomer to give a polymer having a repeating unit, and the polymer is used as a light emitting material. Or it is also considered that compounds each having the structure represented by the general formula (1) are coupled to give a dimer or a trimer, and these are used as a light emitting material.

Examples of the polymer having a repeating unit that contains the structure represented by the general formula (1) include polymers having a structure represented by the following general formula (2) or (3).

General Formula (2)

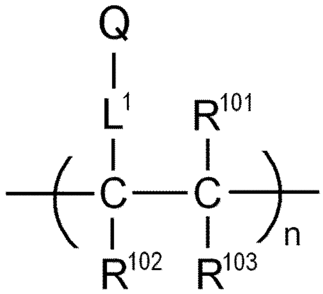

General Formula (3)

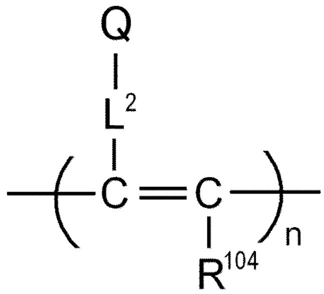

In the general formula (2) or (3), Q represents a group containing the structure represented by the general formula (1), $L^1$ and $L^2$ each represent a linking group. The carbon number of the linking group is preferably 0 to 20, more preferably 1 to 15, even more preferably 2 to 10. The linking group preferably has a structure represented by - $X^{11}$-$L^{11}$-. Here, $X^{11}$ represents an oxygen atom or a sulfur atom, and is preferably an oxygen atom. $L^{11}$ represents a linking group, and is preferably a substituted or unsubstituted alkylene group, or a substituted or unsubstituted arylene group, more preferably a substituted or unsubstituted alkylene group having 1 to 10 carbon atoms, or a substituted or unsubstituted phenylene group.

In the general formula (2) or (3), $R^{101}$, $R^{102}$, $R^{103}$ and $R^{104}$ each independently represent a substituent. Preferably, they each are a substituted or unsubstituted alkyl group having 1 to 6 carbon atoms, a substituted or unsubstituted alkoxy group having 1 to 6 carbon atoms, or a halogen atom, more preferably an unsubstituted alkyl group having 1 to 3 carbon atoms, an unsubstituted alkoxy group having 1 to 3 carbon atoms, a fluorine atom or a chlorine atom, even more preferably an unsubstituted alkyl group having 1 to 3 carbon atoms or an unsubstituted alkoxy group having 1 to 3 carbon atoms.

The linking group represented by $L^1$ and $L^2$ bonds to any of $R^1$ to $R^4$ in formula (1) that constitutes Q. Two or more linking groups can bond to one Q to form a crosslinked structure or a network structure.

Examples of specific structures of the repeating unit include structures represented by the following formulae (4) to (7).

Formula (4)

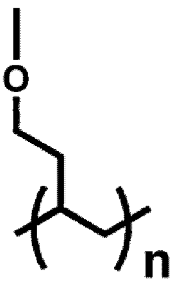

Formula (5)

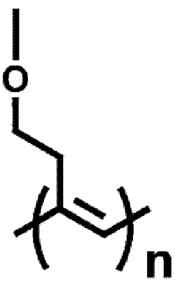

Formula (6)

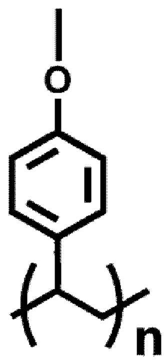

Formula (7)

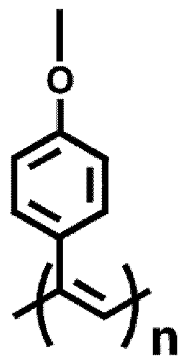

Polymers having a repeating unit that contains any of these formulae (4) to (7) can be synthesized by previously introducing a hydroxy group into any of $R^1$ to $R^4$ in the general formula (1), then reacting the group serving as a linker with the following compound to thereby introduce a polymerizable group, and polymerizing the polymerizable group.

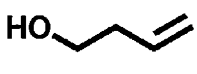

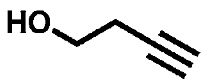

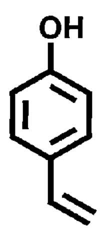

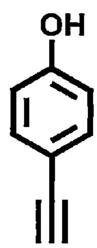

The polymer having a structure represented by the general formula (1) in the molecule can be a polymer having only a repeating unit that has the structure represented by the general formula (1), or can be a polymer containing a

repeating unit that has any other structure. The repeating unit having the structure represented by the general formula (1) to be contained in the polymer may be a single kind or two or more kinds. The repeating unit not having the structure of the general formula (1) includes those derived from monomers used in general copolymerization. For example, it includes repeating units derived from monomers having an ethylenically unsaturated bond, such as ethylene or styrene.

In some embodiments, the compound represented by the general formula (1) is a light-emitting material.

In some embodiments of the present disclosure, the compound represented by the general formula (1) is a compound capable of emitting delayed fluorescence.

In some embodiments, the compound represented by the general formula (1) is, when excited thermally or by an electronic means, able to emit light in a UV region, emit light of blue, green, yellow or orange in a visible region, or emit light in a red region (e.g., about 420 nm to about 500 nm, about 500 nm to about 600 nm, or about 600 nm to about 700 nm) or in a near IR region.

In some embodiments of the present disclosure, the compound represented by the general formula (1) is, when excited thermally or by an electronic means, able to emit light of red or orange in a visible region (e.g., about 620 nm to about 780 nm, about 650 nm).

In some embodiments of the present disclosure, the compound represented by the general formula (1) is, when excited thermally or by an electronic means, able to emit light of orange or yellow in a visible region (e.g., about 570 nm to about 620 nm, about 590 nm, about 570 nm).

In some embodiments of the present disclosure, the compound represented by the general formula (1) is, when excited thermally or by an electronic means, able to emit light of green in a visible region (e.g., about 490 nm to about 575 nm, about 510 nm).

In some embodiments of the present disclosure, the compound represented by the general formula (1) is, when excited thermally or by an electronic means, able to emit light of blue in a visible region (e.g., about 400 nm to about 490 nm, about 475 nm).

In some embodiments of the present disclosure, the compound represented by the general formula (1) is, when excited thermally or by an electronic means, able to emit light in a UV region (e.g., about 280 to 400 nm).

In some embodiments of the present disclosure, the compound represented by the general formula (1) is, when excited thermally or by an electronic means, able to emit light in an IR region (e.g., about 780 nm to 2 $\mu$m).

Electronic characteristics of small-molecule chemical substance libraries can be calculated by known *ab initio* quantum chemistry calculation. For example, according to time-dependent density functional theory calculation using 6-31G* as a basis, and a functional group known as Becke's three parameters, Lee-Yang-Parr hybrid functionals, the Hartree-Fock equation (TD-DFTB3LYP/6-31G*) is analyzed and molecular fractions (parts) having HOMO not lower than a specific threshold value and LUMO not higher than a specific threshold value can be screened, and the calculated triplet state of the parts is more than 2.75 eV

With that, for example, in the presence of a HOMO energy (for example, ionizing potential) of -6.5 eV or more, a donor part ("D") can be selected. On the other hand, for example, in the presence of a LUMO energy (for example, electron affinity) of -0.5 eV or less, an acceptor part ("A") can be selected. A bridge part ("B") is a strong conjugated system, for example, capable of strictly limiting the acceptor part and the donor part in a specific three-dimensional configuration, and therefore prevents the donor part and the acceptor part from overlapping in the pai-conjugated system.

In some embodiments, a compound library is screened using at least one of the following characteristics.

1. Light emission around a specific wavelength.
2. A triplet state over a calculated specific energy level.
3. $\Delta E_{ST}$ value lower than a specific value.
4. Quantum yield more than a specific value.
5. HOMO level.
6. LUMO level.

In some embodiments, the difference ($\Delta E_{ST}$) between the lowest singlet excited state and the lowest triplet excited state at 77 K is less than about 0.5 eV, less than about 0.4 eV, less than about 0.3 eV, less than about 0.2 eV, or less than about 0.1 eV In some embodiments, $\Delta E_{ST}$ value is less than about 0.09 eV, less than about 0.08 eV, less than about 0.07 eV, less than about 0.06 eV, less than about 0.05 eV, less than about 0.04 eV, less than about 0.03 eV, less than about 0.02 eV, or less than about 0.01 eV

In some embodiments, the compound represented by the general formula (1) shows a quantum yield of more than 25%, for example, about 30%, about 35%, about 40%, about 45%, about 50%, about 55%, about 60%, about 65%, about 70%, about 75%, about 80%, about 85%, about 90%, about 95% or more.

[Synthesis Method for Compound represented by general formula (1)]

The compound represented by the general formula (1) is a novel compound.

The compound represented by the general formula (1) can be synthesized by combining known reactions. For example, the compound can be synthesized by reacting a trifluoroisophthalonitrile, in which the positions where three donor groups are desired to be introduced are substituted with fluorine atoms, and a Donor-H (a donor group with a hydrogen atom bonding thereto) in tetrahydrofuran in the presence of sodium hydride. In the case where plural kinds of donor groups are desired to be introduced, the reaction with the donor group can be carried out in two stages. Regarding the specific condition of reaction and the reaction procedure, reference can be made to Synthesis Examples given hereinunder.

[Structure Using the Compound Represented by the General Formula (1)]

In some embodiments, the compound represented by the general formula (1) is used along with one or more materials (e.g., small molecules, polymers, metals, metal complexes), by combining them, or by dispersing the compound, or by covalent-bonding with the compound, or by coating with the compound, or by carrying the compound, or by associating with the compound, and solid films or layers are formed. For example, by combining the compound represented by the general formula (1) with an electroactive material, a film can be formed. In some cases, the compound represented by the general formula (1) can be combined with a hole transporting polymer. In some cases, the compound represented by the general formula (1) can be combined with an electron transporting polymer. In some cases, the compound represented by the general formula (1) can be combined with a hole transporting polymer and an electron transporting polymer. In some cases, the compound represented by the general formula (1) can be combined with a copolymer having both a hole transporting moiety and an electron transporting moiety. In the embodiments mentioned above, the electrons and/or the holes formed in a solid film or layer can be interacted with the compound represented by the general formula (1).

[Film Formation]

In some embodiments, a film containing the compound represented by the general formula (1) can be formed in a wet process. In a wet process, a solution prepared by dissolving a composition containing the compound of the present invention is applied onto a surface, and then the solvent is removed to form a film. The wet process includes a spin coating method, a slit coating method, an ink jet method (a spraying method), a gravure printing method, an offset printing method and flexographic printing method, which, however are not limitative. In the wet process, an appropriate organic solvent capable of dissolving a composition containing the compound of the present invention is selected and used. In some embodiments, a substituent (e.g., an alkyl group) capable of increasing the solubility in an organic solvent can be introduced into the compound contained in the composition.

In some embodiments, a film containing the compound of the present invention can be formed in a dry process. In some embodiments, a vacuum evaporation method is employable as a dry process, which, however, is not limitative. In the case where a vacuum evaporation method is employed, compounds to constitute a film can be co-evaporated from individual evaporation sources, or can be co-evaporated from a single evaporation source formed by mixing the compounds. In the case where a single evaporation source is used, a mixed powder prepared by mixing compound powders can be used, or a compression molded body prepared by compression-molding the mixed powder can be used, or a mixture prepared by heating and melting the constituent compounds and cooling the resulting melt can be used. In some embodiments, by coevaporation under the condition where the evaporation rate (weight reduction rate) of the plural compounds contained in a single evaporation source is the same or is nearly the same, a film having a compositional ratio corresponding to the compositional ratio of the plural compounds contained in the evaporation source can be formed. When plural compounds are mixed in the same compositional ratio as the compositional ratio of the film to be formed to prepare an evaporation source, a film having a desired compositional ratio can be formed in a simplified manner. In some embodiments, the temperature at which the compounds to be co-evaporated have the same weight reduction ratio is specifically defined, and the temperature can be employed as the temperature of coevaporation.

[Use Examples of Compound of the Present Disclosure]

Organic Light Emitting Diode:

One embodiment of the present invention relates to use of the compound represented by the general formula (1) of the present invention as a light emitting material for organic light emitting devices. In some embodiments, the compound represented by the general formula (1) of the present invention can be effectively used as a light emitting material in a light emitting layer in an organic light emitting device. In some embodiments, the compound represented by the general

formula (1) of the present invention includes delayed fluorescence (delayed fluorescent material) that emits delayed fluorescence. In some embodiments, the present invention provides a delayed fluorescent material having a structure represented by the general formula (1) of the present invention. In some embodiments, the present invention relates to use of the compound represented by the general formula (1) of the present invention as a delayed fluorescent material. In some embodiments, the compound represented by the general formula (1) of the present invention can be used as a host material, and can be used along with one or more light-emitting materials, and the light emitting material can be a fluorescent material, a phosphorescent material or a TADF. In some embodiments, the compound represented by the general formula (1) can be used as a hole transporting material. In some embodiments, the compound represented by the general formula (1) can be used as an electron transporting material. In some embodiments, the present invention relates to a method of generating delayed fluorescence from the compound represented by the general formula (1). In some embodiments, the organic light emitting device containing the compound as a light emitting material emits delayed fluorescence and shows a high light emission efficiency.

In some embodiments, the light emitting layer contains the compound represented by the general formula (1), and the compound represented by the general formula (1) is aligned in parallel to the substrate. In some embodiments, the substrate is a film-forming surface. In some embodiment, the alignment of the compound represented by the general formula (1) relative to the film-forming surface can have some influence on the propagation direction of light emitted by the aligned compounds, or can determine the direction. In some embodiments, by aligning the propagation direction of light emitted by the compound represented by the general formula (1), the light extraction efficiency from the light emitting layer can be improved.

One embodiment of the present invention relates to an organic light emitting device. In some embodiments, the organic light emitting device includes a light emitting layer. In some embodiments, the light emitting layer contains, as a light emitting material therein, the compound represented by the general formula (1). In some embodiments, the organic light emitting device is an organic photoluminescent device (organic PL device). In some embodiments, the organic light emitting device is an organic electroluminescent device (organic EL device). In some embodiments, the compound represented by the general formula (1) assists light irradiation from the other light emitting materials contained in the light emitting layer (as a so-called assist dopant). In some embodiments, the compound represented by the general formula (1) contained in the light emitting layer is in a lowest excited energy level, and is contained between the lowest excited single energy level of the host material contained in the light emitting layer and the lowest excited singlet energy level of the other light emitting materials contained in the light emitting layer.

In some embodiments, the organic photoluminescent device comprises at least one light-emitting layer. In some embodiments, the organic electroluminescent device comprises at least an anode, a cathode, and an organic layer between the anode and the cathode. In some embodiments, the organic layer comprises at least a light-emitting layer. In some embodiments, the organic layer comprises only a light-emitting layer. In some embodiments, the organic layer comprises one or more organic layers in addition to the light-emitting layer. Examples of the organic layer include a hole transporting layer, a hole injection layer, an electron barrier layer, a hole barrier layer, an electron injection layer, an electron transporting layer and an exciton barrier layer. In some embodiments, the hole transporting layer may be a hole injection and transporting layer having a hole injection function, and the electron transporting layer may be an electron injection and transporting layer having an electron injection function. An example of an organic electroluminescent device is shown in Fig. 1.

Light Emitting Layer:

In some embodiments, the light emitting layer is a layer where holes and electrons injected from the anode and the cathode, respectively, are recombined to form excitons. In some embodiments, the layer emits light.

In some embodiments, only a light emitting material is used as the light emitting layer. In some embodiments, the light emitting layer contains a light emitting material and a host material. In some embodiments, the light emitting material is one or more compounds of the general formula (1). In some embodiments, for improving luminous radiation efficiency of an organic electroluminescent device and an organic photoluminescence device, the singlet exciton and the triplet exciton generated in a light emitting material is confined inside the light emitting material. In some embodiments, a host material is used in the light emitting layer in addition to a light emitting material therein. In some embodiments, the host material is an organic compound. In some embodiments, the organic compound has an excited singlet energy and an excited triplet energy, and at least one of them is higher than those in the light emitting material of the present invention. In some embodiments, the singlet exciton and the triplet exciton generated in the light emitting material of the present invention are confined in the molecules of the light emitting material of the present invention. In some embodiments, the singlet and triplet excitons are fully confined for improving luminous radiation efficiency. In some embodiments, although high luminous radiation efficiency is still attained, singlet excitons and triplet excitons are not fully confined, that is, a host material capable of attaining high luminous radiation efficiency can be used in the present invention with no specific limitation. In some embodiments, in the light emitting material in the light emitting layer of the device of the

present invention, luminous radiation occurs. In some embodiments, radiated light includes both fluorescence and delayed fluorescence. In some embodiments, radiated light includes radiated light from a host material. In some embodiments, radiated light is composed of radiated light from a host material. In some embodiments, radiated light includes radiated light from the compound represented by the general formula (1) and radiated light from a host material. In some embodiment, a TADF molecule and a host material are used. In some embodiments, TADF is an assist dopant.

In the case where the compound of the general formula (1) is used as an assist dopant, various compounds can be employed as a light emitting material (preferably a fluorescent material). As such light emitting materials, employable are an anthracene derivative, a tetracene derivative, a naphthacene derivative, a pyrene derivative, a perylene derivative, a chrysene derivative, a rubrene derivative, a coumarin derivative, a pyran derivative, a stilbene derivative, a fluorenone derivative, an anthryl derivative, a pyrromethene derivative, a terphenyl derivative, a terphenylene derivative, a fluoranthene derivative, an amine derivative, a quinacridone derivative, an oxadiazole derivative, a malononitrile derivative, a pyran derivative, a carbazole derivative, a julolidine derivative, a thiazole derivative, and a metal (Al, Zn)-having derivative. These exemplified skeletons can have a substituent, or may not have a substituent. These exemplified skeletons can be combined.

Light emitting materials that can be used in combination with the assist dopant represented by the general formula (1) are shown below.

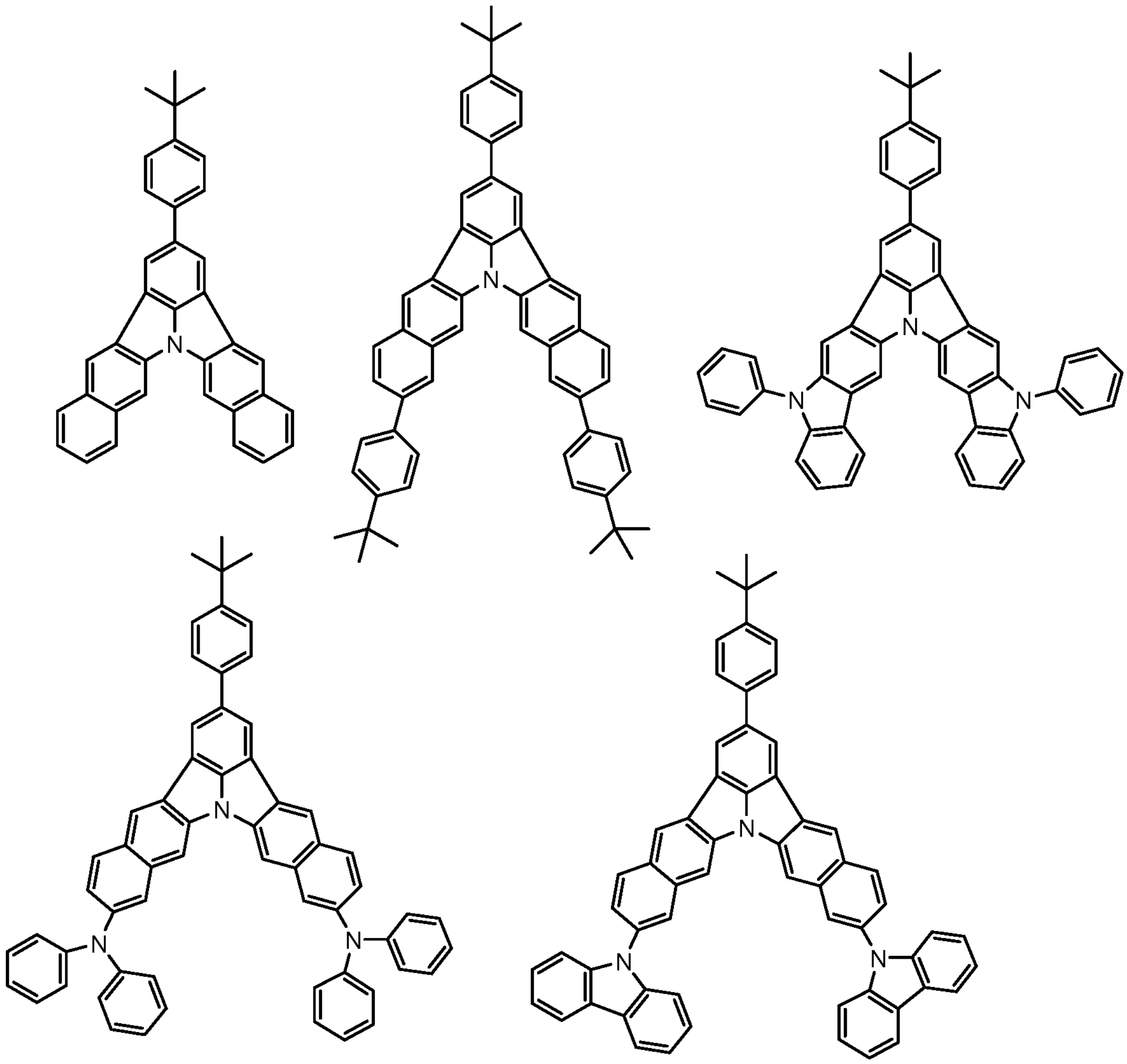

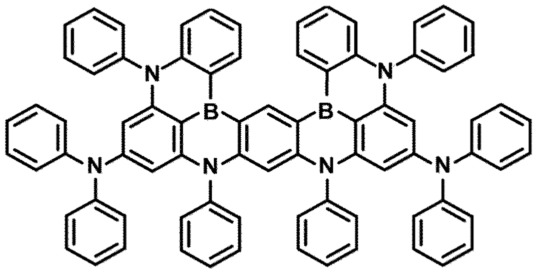
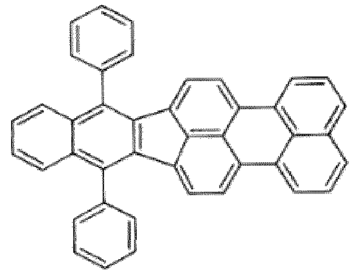

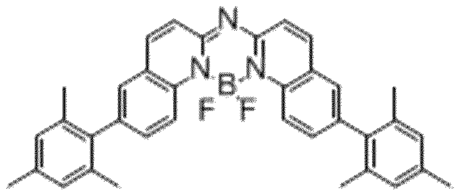

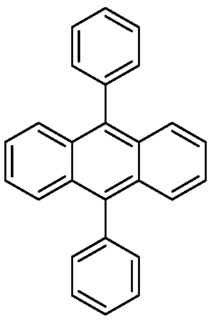
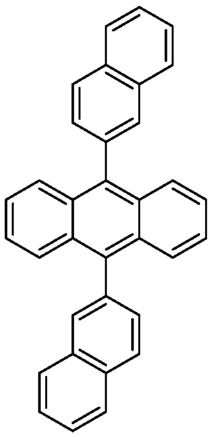
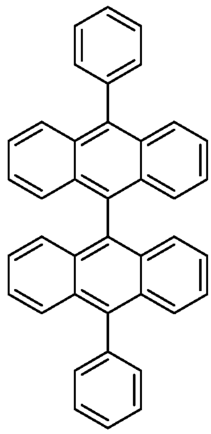
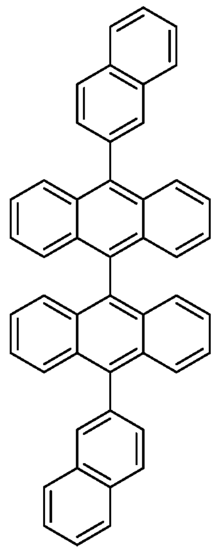

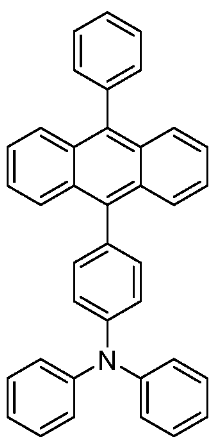
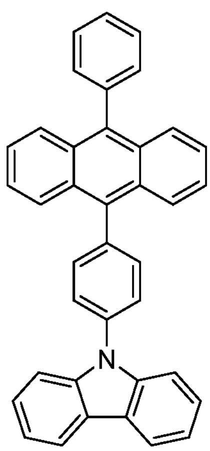
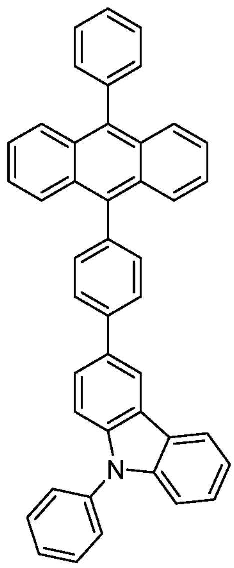
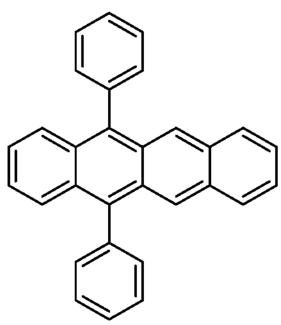

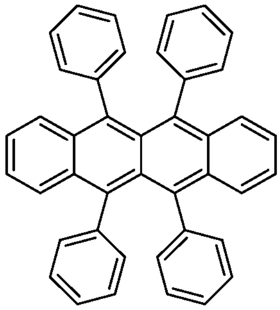
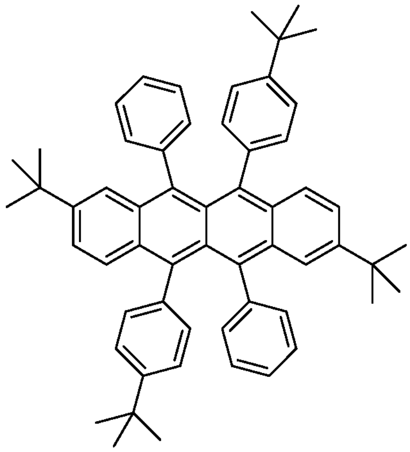
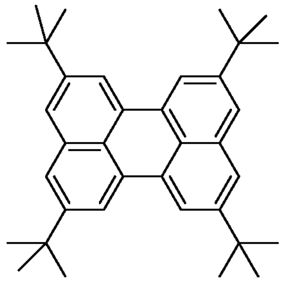

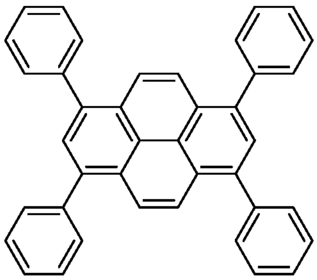
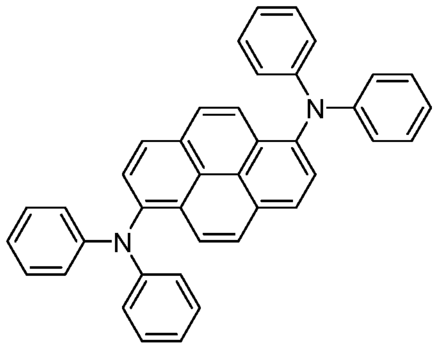
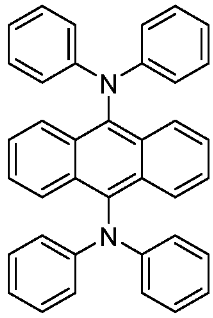

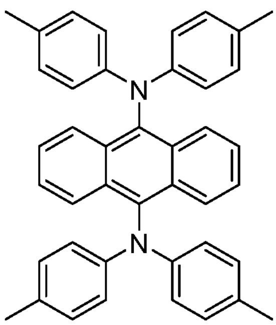
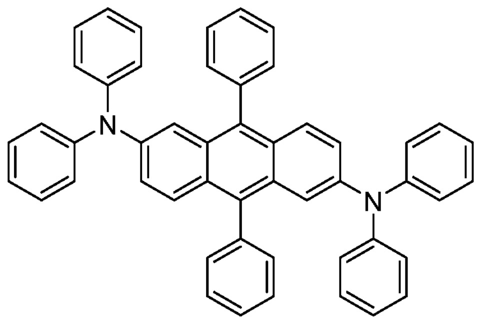
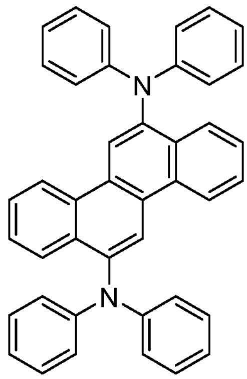

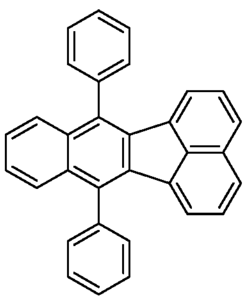
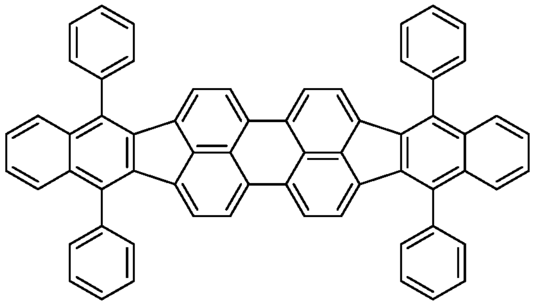
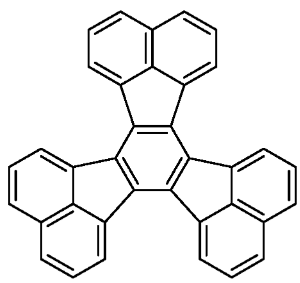

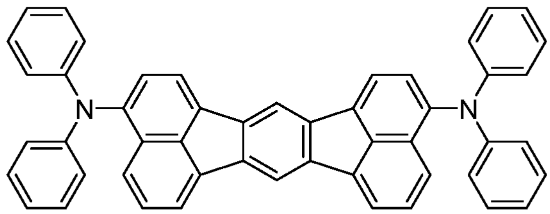

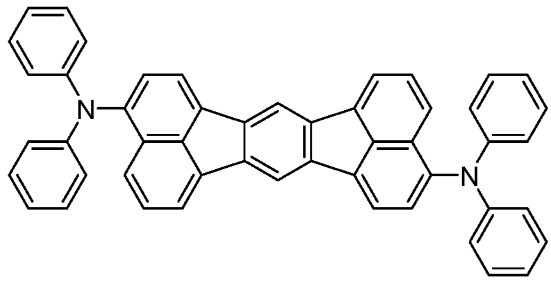

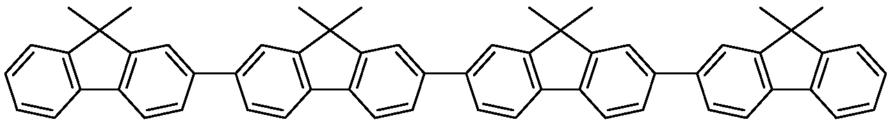

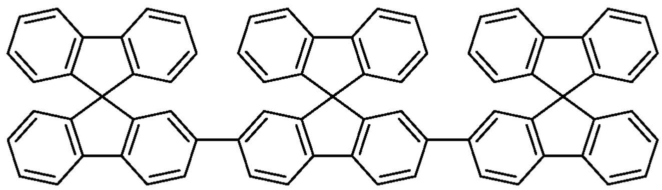

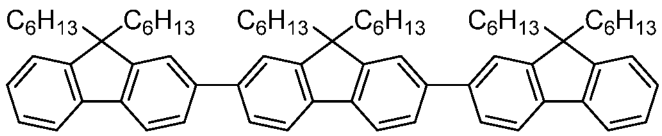

In addition, the compounds described in WO2015/022974, paragraphs 0220 to 0239 are also especially favorably employable as a light emitting material for use along with the assist dopant represented by the general formula (1).

In some embodiments where a host material is used, the amount of the compound of the present invention contained in a light emitting layer as a light emitting material is 0.1% by weight or more. In some embodiments where a host material is used, the amount of the compound of the present invention contained in a light emitting layer as a light emitting material is 1% by weight or more. In some embodiments where a host material is used, the amount of the compound of the present invention contained in a light emitting layer as a light emitting material is 50% by weight or less. In some embodiments where a host material is used, the amount of the compound of the present invention contained in a light emitting layer as a light emitting material is 20% by weight or less. In some embodiments where a host material is used, the amount of the compound of the present invention contained in a light emitting layer as a light emitting material is 10% by weight or less.

In some embodiments, the host material in a light emitting layer is an organic compound having a hole transporting capability and an electron transporting capability. In some embodiments, the host material in a light emitting layer is an organic compound that prevents increase in the wavelength of emitted light. In some embodiments, the host material in a light emitting layer is an organic compound having a high glass transition temperature.

In some embodiments, the host material is selected from the following group:

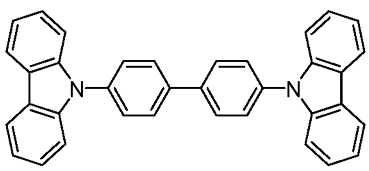

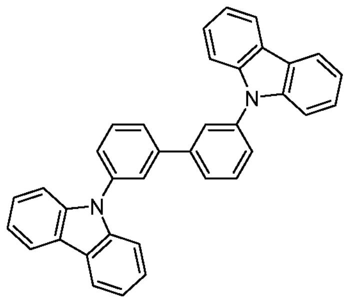

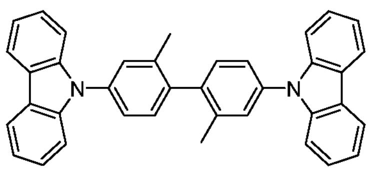

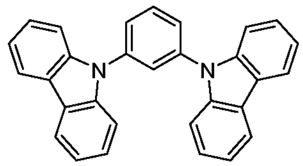
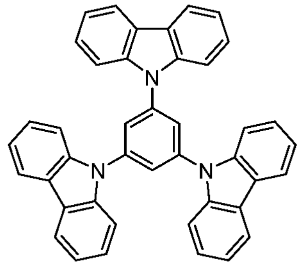
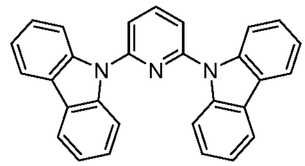

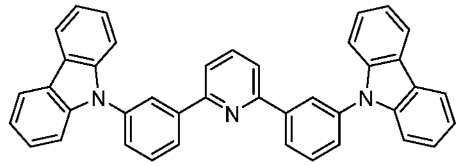
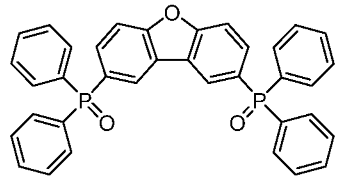

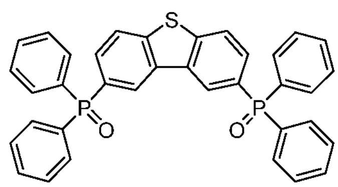
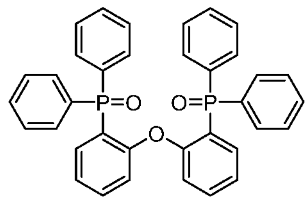
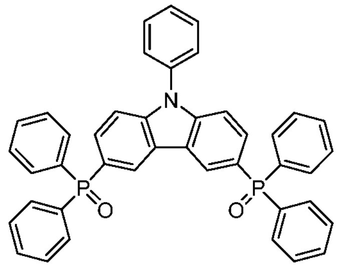

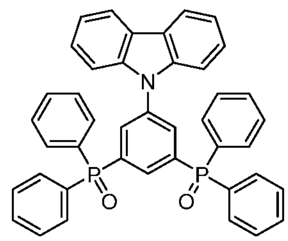
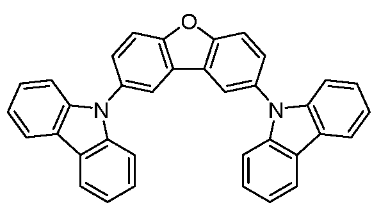
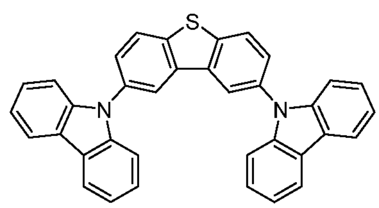

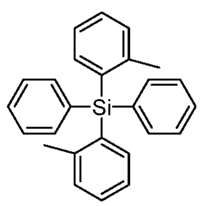
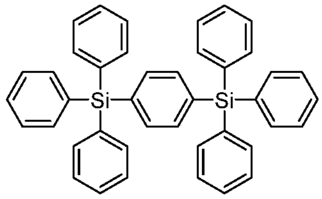
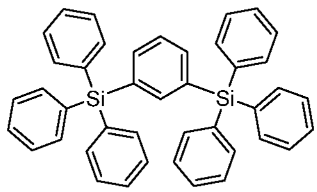
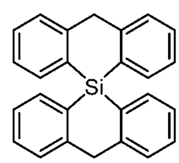

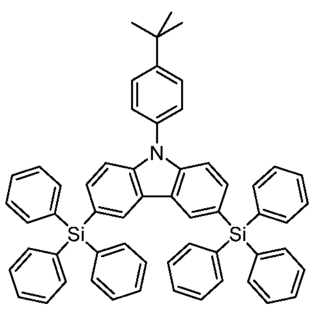
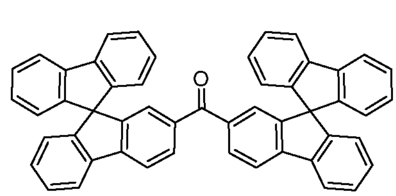
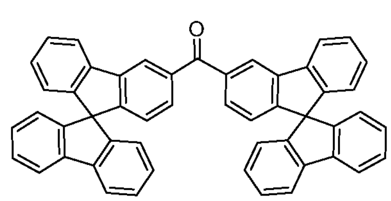

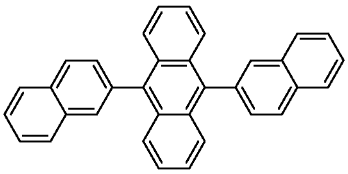

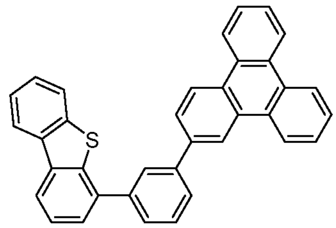

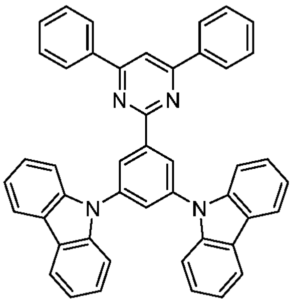

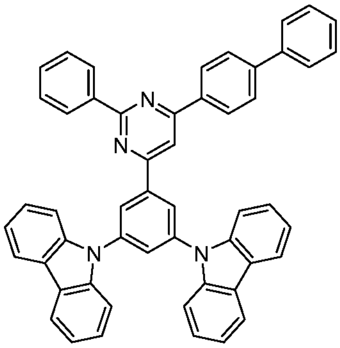

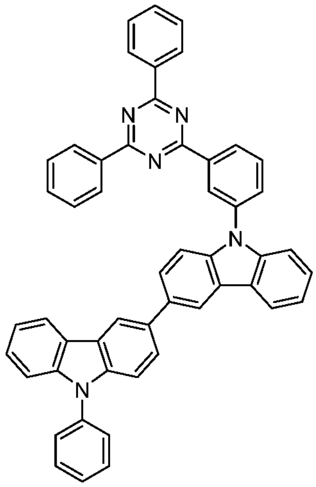

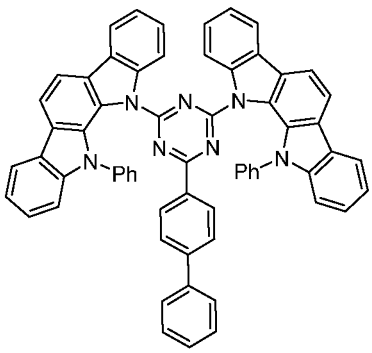

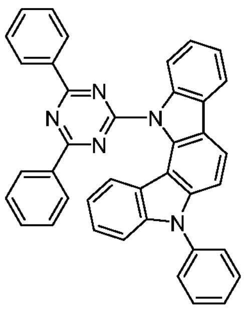

In some embodiments, the light emitting layer contains two or more kinds of TADF molecules differing in the structure. For example, the light emitting layer can contain three kinds of materials of a host material, a first TADF molecule and a second TADF molecule whose excited singlet energy level is higher in that order. In that case, both the first TADF molecule and the second TADF molecule are preferably such that the difference $\Delta E_{ST}$ between the lowest excited single energy level and the lowest excited triplet energy level at 77 K is 0.3 eV or less, more preferably 0.25 eV or less, even more preferably 0.2 eV or less, further more preferably 0.15 eV or less, further more preferably 0.1 eV or less, further more preferably 0.07 eV or less, further more preferably 0.05 eV or less, further more preferably 0.03 eV or less, further more preferably 0.01 eV or less. The content of the first TADF molecule in the light emitting layer is preferably larger than the content of the second TADF molecule therein. The content of the host material in the light emitting layer is preferably larger than the content of the second TADF molecule therein. The content of the first TADF molecule in the light emitting layer can be larger than or can be smaller than or can be the same as the content of the host material therein. In some embodiments, the composition in the light emitting layer can be 10 to 70% by weight of a host material, 10 to 80% by weight of a first TADF molecule, and 0.1 to 30% by weighty of a second TADF molecule. In some embodiments, the composition in the light emitting layer can be 20 to 45% by weight of a host material, 50 go 75% by weight of a first TADF molecule, and 5 to 20% by weighty of a second TADF molecule. In some embodiments, the emission quantum yield cpPLI(A) by photo-excitation of a co-deposited film of a first TADF molecule and a host material (the content of the first TADF molecule in the co-deposited film = A% by weight) and the emission quantum yield cpPL2(A) by photo-excitation of a co-deposited film of a second TADF molecule and a host material (the content of the second TADF molecule in the co-deposited film = A% by weight) satisfy a relational formula $\varphi$PL1(A)>$\varphi$PL2(A).

In some embodiments, the emission quantum yield φPL2(B) by photo-excitation of a co-deposited film of a second TADF molecule and a host material (the content of the second TADF molecule in the co-deposited film = B% by weight) and the emission quantum yield φPL2(100) by photo-excitation of a single film of a second TADF molecule satisfy a relational formula φPL2(B)>φPL2(100). In some embodiments, the light emitting layer can contain three kinds of TADF molecules differing in the structure. The compound of the present invention can be any of the plural TADF compounds contained in the light emitting layer.

In some embodiments, the light emitting layer can be composed of materials selected from the group consisting of a host material an assist dopant and a light emitting material. In some embodiments, the light emitting layer does not contain a metal element. In some embodiments, the light emitting layer can be formed of a material composed of atoms alone selected from the group consisting of a carbon atom, a hydrogen atom, a deuterium atom, a nitrogen atom, an oxygen atom and a sulfur atom. Or the light emitting layer can be formed of a material composed of atoms alone selected from the group consisting of a carbon atom, a hydrogen atom, a deuterium atom, a nitrogen atom and an oxygen atom. Or the light emitting layer can be formed of a material composed of atoms alone selected from the group consisting of a carbon atom, a hydrogen atom, a nitrogen atom and an oxygen atom.

In the case where the light emitting layer contain any other TADF material than the compound of the present invention, the TADF material can be a known delayed fluorescent material. As preferred delayed fluorescent materials, there can be mentioned compounds included in the general formulae described in WO2013/154064, paragraphs 0008 to 0048 and 0095 to 0133; WO2013/011954, paragraphs 0007 to 0047 and 0073~0085; WO2013/011955, paragraphs 0007 to 0033 and 0059 to 0066; WO2013/081088, paragraphs 0008 to 0071 and 0118 to 0133; JP 2013-256490 A, paragraphs 0009 to 0046 and 0093 to 0134; JP 2013-116975 A, paragraphs 0008 to 0020 and 0038 to 0040; WO2013/133359, paragraphs 0007 to 0032 and 0079 to 0084; WO2013/161437, paragraphs 0008 to 0054 and 0101~0121; JP 2014-9352 A, paragraphs 0007 to 0041 and 0060 to 0069; and JP 2014-9224 A, paragraphs 0008 to 0048 and 0067 to 0076; JP 2017-119663 A, paragraphs 0013 to 0025; JP 2017-119664 A, paragraphs 0013 to 0026; JP 2017-222623 A, paragraphs 0012 to 0025; JP 2017-226838 A, paragraphs 0010 to 0050; JP 2018-100411 A, paragraphs 0012 to 0043; WO2018/047853, paragraphs 0016 to 0044; and especially, exemplary compounds therein capable of emitting delayed fluorescence. In addition, also preferably employable here are light emitting materials capable of emitting delayed fluorescence, as described in JP 2013-253121 A, WO2013/133359, WO2014/034535, WO2014/115743, WO2014/122895, WO2014/126200, WO2014/136758, WO2014/133121, WO2014/136860, WO2014/196585, WO2014/189122, WO2014/168101, WO2015/008580, WO2014/203840, WO2015/002213, WO2015/016200, WO2015/019725, WO2015/072470, WO2015/108049, WO2015/080182, WO2015/072537, WO2015/080183, JP 2015-129240 A, WO2015/129714, WO2015/129715, WO2015/133501, WO2015/136880, WO2015/137244, WO2015/137202, WO2015/137136, WO2015/146541 and WO2015/159541. These patent publications described in this paragraph are hereby incorporated as a part of this description by reference.

In the following, the constituent members and the other layers than the light-emitting layer of the organic electroluminescent device are described.

Substrate:

In some embodiments, the organic electroluminescent device of the invention is supported by a substrate, wherein the substrate is not particularly limited and may be any of those that have been commonly used in an organic electroluminescent device, for example those formed of glass, transparent plastics, quartz and silicon.

Anode

In some embodiments, the anode of the organic electroluminescent device is made of a metal, an alloy, an electroconductive compound, or a combination thereof. In some embodiments, the metal, alloy, or electroconductive compound has a large work function (4 eV or more). In some embodiments, the metal is Au. In some embodiments, the electroconductive transparent material is selected from CuI, indium tin oxide (ITO), $SnO_2$, and ZnO. In some embodiments, an amorphous material capable of forming a transparent electroconductive film, such as IDIXO ($In_2O_3$-ZnO), is be used. In some embodiments, the anode is a thin film. In some embodiments the thin film is made by vapor deposition or sputtering. In some embodiments, the film is patterned by a photolithography method. In some embodiments, where the pattern may not require high accuracy (for example, approximately 100 μm or more), the pattern may be formed with a mask having a desired shape on vapor deposition or sputtering of the electrode material. In some embodiments, when a material can be applied as a coating, such as an organic electroconductive compound, a wet film forming method, such as a printing method and a coating method is used. In some embodiments, when the emitted light goes through the anode, the anode has a transmittance of more than 10%, and the anode has a sheet resistance of several hundred Ohm per square or less. In some embodiments, the thickness of the anode is from 10 to 1,000 nm. In some embodiments, the thickness of the anode is from 10 to 200 nm. In some embodiments, the thickness of the anode varies depending

on the material used.

Cathode

In some embodiments, the cathode is made of an electrode material a metal having a small work function (4 eV or less) (referred to as an electron injection metal), an alloy, an electroconductive compound, or a combination thereof. In some embodiments, the electrode material is selected from sodium, a sodium-potassium alloy, magnesium, lithium, a magnesium-cupper mixture, a magnesium-silver mixture, a magnesium-aluminum mixture, a magnesium-indium mixture, an aluminum-aluminum oxide ($Al_2O_3$) mixture, indium, a lithium-aluminum mixture, and a rare earth metal. In some embodiments, a mixture of an electron injection metal and a second metal that is a stable metal having a larger work function than the electron injection metal is used. In some embodiments, the mixture is selected from a magnesium-silver mixture, a magnesium-aluminum mixture, a magnesium-indium mixture, an aluminum-aluminum oxide ($Al_2O_3$) mixture, a lithium-aluminum mixture, and aluminum. In some embodiments, the mixture increases the electron injection property and the durability against oxidation. In some embodiments, the cathode is produced by forming the electrode material into a thin film by vapor deposition or sputtering. In some embodiments, the cathode has a sheet resistance of several hundred Ohm per square or less. In some embodiments, the thickness of the cathode ranges from 10 nm to 5 μm. In some embodiments, the thickness of the cathode ranges from 50 to 200 nm. In some embodiments, for transmitting the emitted light, any one of the anode and the cathode of the organic electroluminescent device is transparent or translucent. In some embodiments, the transparent or translucent electroluminescent devices enhances the light emission luminance.

In some embodiments, the cathode is formed with an electroconductive transparent material, as described for the anode, to form a transparent or translucent cathode. In some embodiments, a device comprises an anode and a cathode, both being transparent or translucent.

Injection Layer

An injection layer is a layer between the electrode and the organic layer. In some embodiments, the injection layer decreases the driving voltage and enhances the light emission luminance. In some embodiments the injection layer includes a hole injection layer and an electron injection layer. The injection layer can be positioned between the anode and the light-emitting layer or the hole transporting layer, and between the cathode and the light-emitting layer or the electron transporting layer. In some embodiments, an injection layer is present. In some embodiments, no injection layer is present.

Preferred compound examples for use as a hole injection material are shown below.

$MoO_3$,

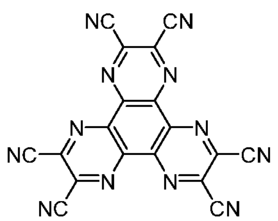
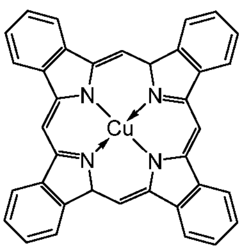
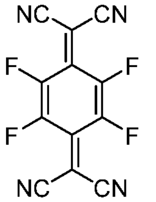
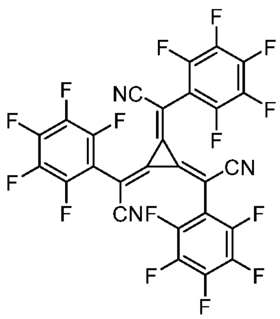

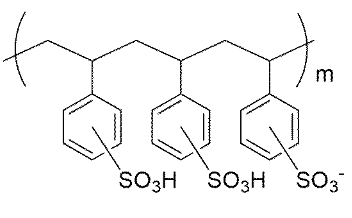
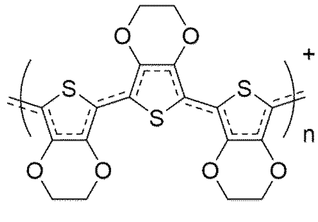

Next, preferred compound examples for use as an electron injection material are shown below.

LiF, CsF, 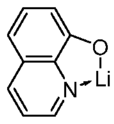

Barrier Layer

A barrier layer is a layer capable of inhibiting charges (electrons or holes) and/or excitons present in the light-emitting layer from being diffused outside the light-emitting layer. In some embodiments, the electron barrier layer is between the light-emitting layer and the hole transporting layer, and inhibits electrons from passing through the light-emitting layer toward the hole transporting layer. In some embodiments, the hole barrier layer is between the light-emitting layer and the electron transporting layer, and inhibits holes from passing through the light-emitting layer toward the electron transporting layer. In some embodiments, the barrier layer inhibits excitons from being diffused outside the light-emitting layer. In some embodiments, the electron barrier layer and the hole barrier layer are exciton barrier layers. As used herein, the term "electron barrier layer" or "exciton barrier layer" includes a layer that has the functions of both electron barrier layer and of an exciton barrier layer.

Hole Barrier Layer

A hole barrier layer acts as an electron transporting layer. In some embodiments, the hole barrier layer inhibits holes from reaching the electron transporting layer while transporting electrons. In some embodiments, the hole barrier layer enhances the recombination probability of electrons and holes in the light-emitting layer. The material for the hole barrier layer may be the same materials as the ones described for the electron transporting layer.

Preferred compound examples for use for the hole barrier layer are shown below.

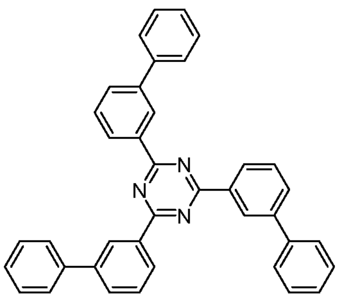
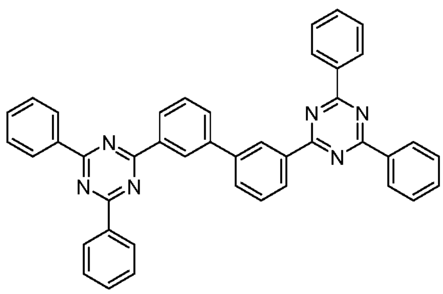
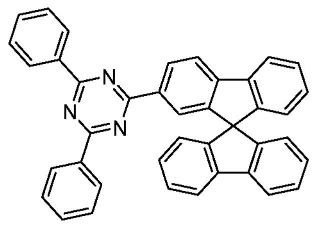
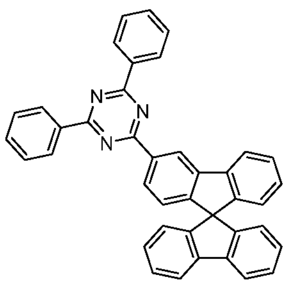
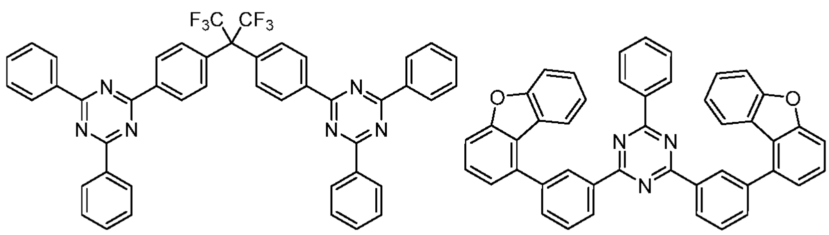
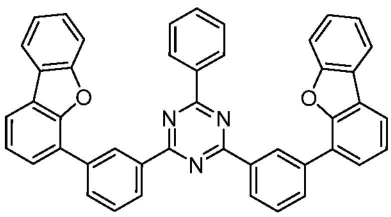
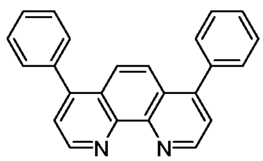
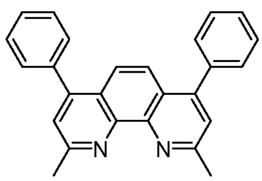

Electron Barrier Layer

As electron barrier layer transports holes. In some embodiments, the electron barrier layer inhibits electrons

from reaching the hole transporting layer while transporting holes. In some embodiments, the electron barrier layer enhances the recombination probability of electrons and holes in the light-emitting layer.

Preferred compound examples for use as the electron barrier material are shown below.

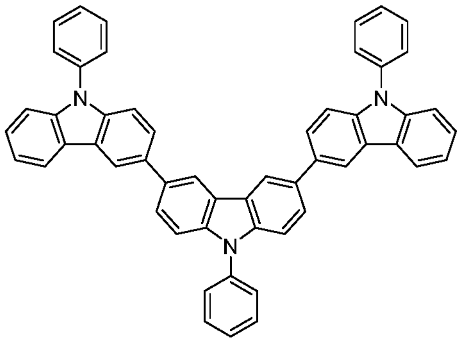

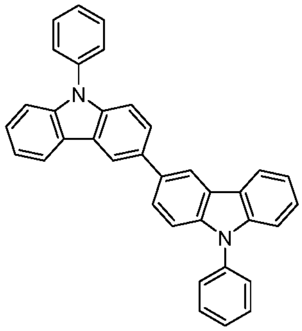

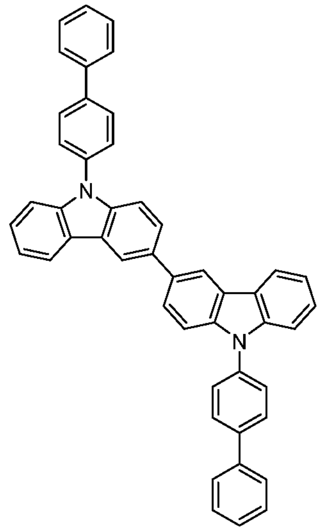

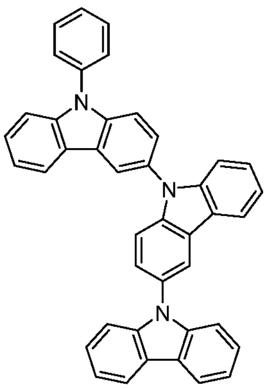

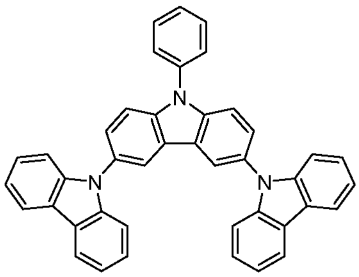

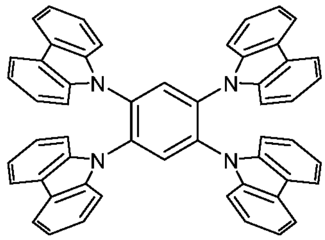

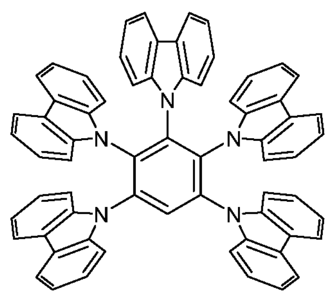

Exciton Barrier Layer

An exciton barrier layer inhibits excitons generated through recombination of holes and electrons in the light-emitting layer from being diffused to the charge transporting layer. In some embodiments, the exciton barrier layer enables effective confinement of excitons in the light-emitting layer. In some embodiments, the light emission efficiency of the device is enhanced. In some embodiments, the exciton barrier layer is adjacent to the light-emitting layer on any of the side of the anode and the side of the cathode, and on both the sides. In some embodiments, where the exciton barrier layer is on the side of the anode, the layer can be between the hole transporting layer and the light-emitting layer and adjacent to the light-emitting layer. In some embodiments, where the exciton barrier layer is on the side of the cathode, the layer can be between the light-emitting layer and the cathode and adjacent to the light-emitting layer. In some embodiments, a hole injection layer, an electron barrier layer, or a similar layer is between the anode and the

exciton barrier layer that is adjacent to the light-emitting layer on the side of the anode. In some embodiments, a hole injection layer, an electron barrier layer, a hole barrier layer, or a similar layer is between the cathode and the exciton barrier layer that is adjacent to the light-emitting layer on the side of the cathode. In some embodiments, the exciton barrier layer comprises excited singlet energy and excited triplet energy, at least one of which is higher than the excited singlet energy and the excited triplet energy of the light-emitting material, respectively.

Hole Transporting Layer

The hole transporting layer comprises a hole transporting material. In some embodiments, the hole transporting layer is a single layer. In some embodiments, the hole transporting layer comprises a plurality layers.

In some embodiments, the hole transporting material has one of injection or transporting property of holes and barrier property of electrons. In some embodiments, the hole transporting material is an organic material. In some embodiments, the hole transporting material is an inorganic material. Examples of known hole transporting materials that may be used herein include but are not limited to a triazole derivative, an oxadiazole derivative, an imidazole derivative, a carbazole derivative, an indolocarbazole derivative, a polyarylalkane derivative, a pyrazoline derivative, a pyrazolone derivative, a phenylenediamine derivative, an arylamine derivative, an amino-substituted chalcone derivative, an oxazole derivative, a styryl anthracene derivative, a fluorenone derivative, a hydrazone derivative, a stilbene derivative, a silazane derivative, an aniline copolymer and an electroconductive polymer oligomer, particularly a thiophene oligomer, or a combination thereof. In some embodiments, the hole transporting material is selected from a porphyrin compound, an aromatic tertiary amine compound, and a styrylamine compound. In some embodiments, the hole transporting material is an aromatic tertiary amine compound. Preferred compound examples for use as the hole transporting material are shown below.

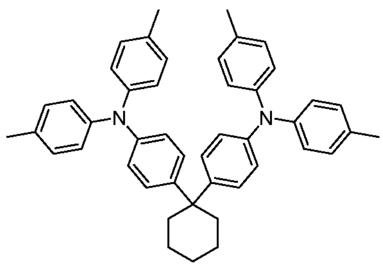
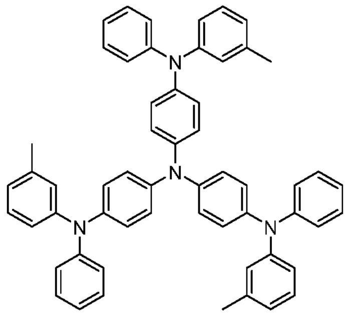
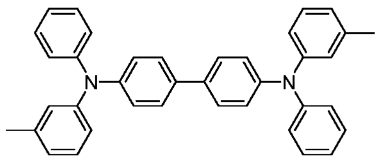

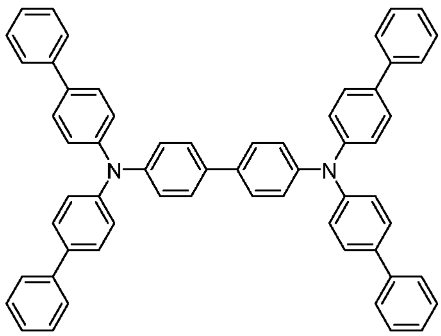
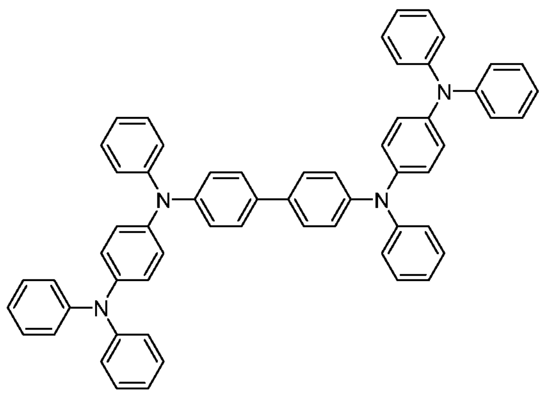

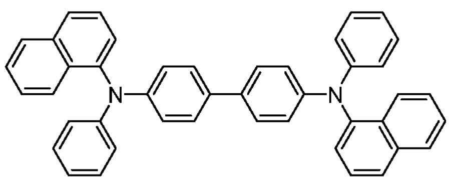
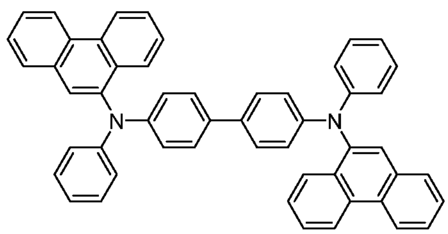

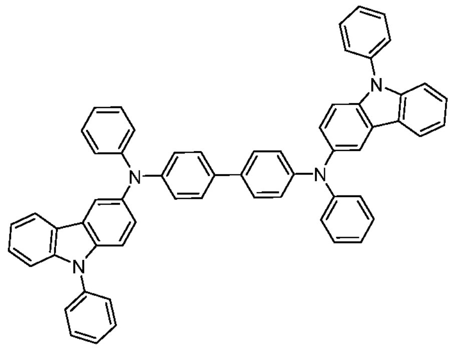
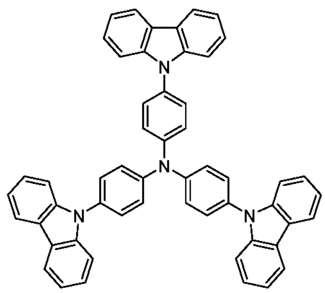
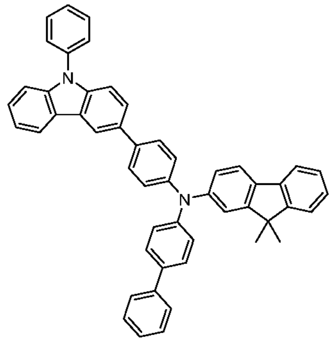

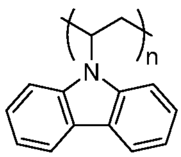
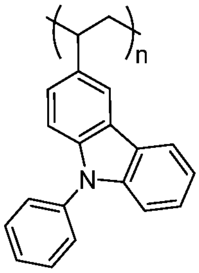
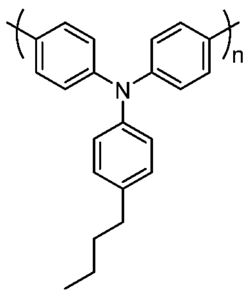

Electron Transporting Layer

The electron transporting layer comprises an electron transporting material. In some embodiments, the electron transporting layer is a single layer. In some embodiments, the electron transporting layer comprises a plurality of layer.

In some embodiments, the electron transporting material needs only to have a function of transporting electrons, which are injected from the cathode, to the light-emitting layer. In some embodiments, the electron transporting material also function as a hole barrier material. Examples of the electron transporting layer that may be used herein include but are not limited to a nitro-substituted fluorene derivative, a diphenylquinone derivative, a thiopyran dioxide derivative, carbodiimide, a fluorenylidene methane derivative, anthraquinodimethane, an anthrone derivatives, an azole derivative, an azine derivative, an oxadiazole derivative, or a combination thereof, or a polymer thereof. In some embodiments, the electron transporting material is a thiadiazole derivative, or a quinoxaline derivative. In some embodiments, the electron transporting material is a polymer material. Preferred compound examples for use as the electron transporting material are shown below.

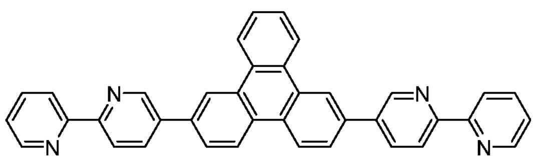
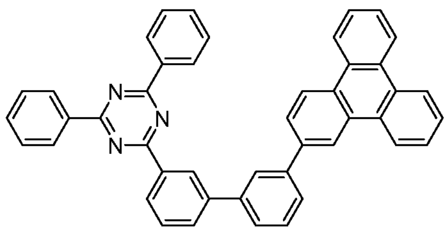

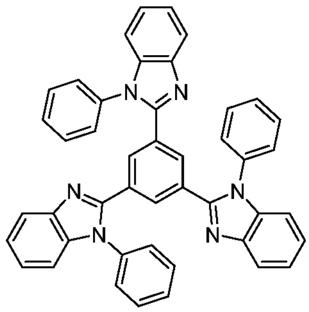
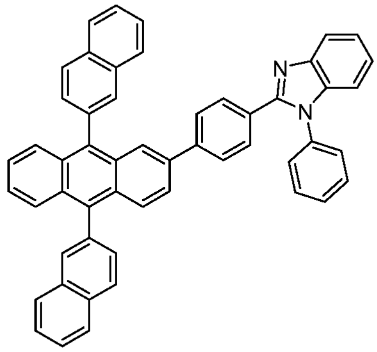

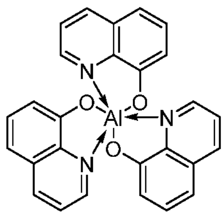
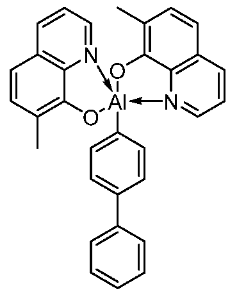

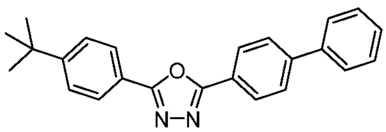
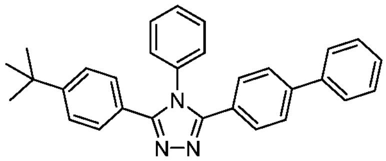

Hereinunder compound examples preferred as a material that can be added to the organic layers are shown. For example, these can be added as a stabilization material.

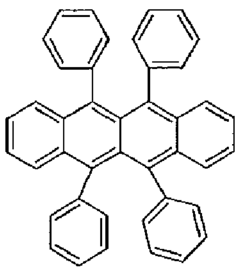
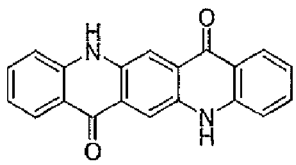
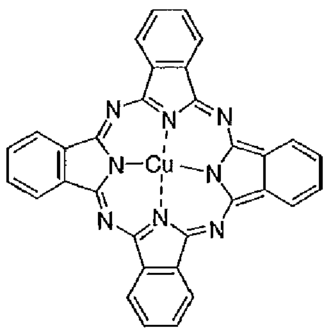

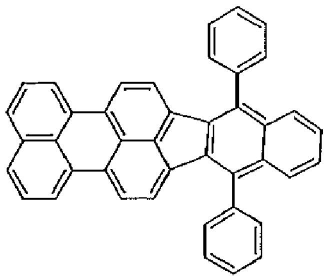
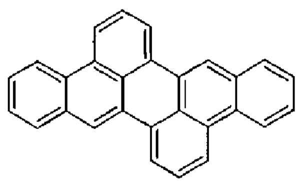

Preferred materials for use in the organic electroluminescent device are specifically shown. However, the materials usable in the invention should not be limitatively interpreted by the following exemplary compounds. Compounds

that are exemplified as materials having a specific function can also be used as materials having any other function.

Devices

In some embodiments, an light emitting layer is incorporated into a device. For example, the device includes, but is not limited to an OLED bulb, an OLED lamp, a television screen, a computer monitor, a mobile phone, and a tablet.
In some embodiments, an electronic device comprises an OLED comprising an anode, a cathode, and at least one organic layer comprising a light emitting layer between the anode and the cathode.
In some embodiments, compositions described herein may be incorporated into various light-sensitive or light-activated devices, such as a OLEDs or photovoltaic devices. In some embodiments, the composition may be useful in facilitating charge transfer or energy transfer within a device and/or as a hole-transport material. The device may be, for example, an organic light-emitting diode (OLED), an organic integrated circuit (O-IC), an organic field-effect transistor (O-FET), an organic thin-film transistor (O-TFT), an organic light-emitting transistor (O-LET), an organic solar cell (O-SC), an organic optical detector, an organic photoreceptor, an organic field-quench device (O-FQD), a light-emitting electrochemical cell (LEC) or an organic laser diode (O-laser).

Bulbs or Lamps

In some embodiments, an electronic device comprises an OLED comprising an anode, a cathode, and at least one organic layer comprising a light emitting layer between the anode and the cathode.
In some embodiments, a device comprises OLEDs that differ in color. In some embodiments, a device comprises an array comprising a combination of OLEDs. In some embodiments, the combination of OLEDs is a combination of three colors (e.g., RGB). In some embodiments, the combination of OLEDs is a combination of colors that are not red, green, or blue (for example, orange and yellow green). In some embodiments, the combination of OLEDs is a combination of two, four, or more colors.
In some embodiments, a device is an OLED light comprising:

a circuit board having a first side with a mounting surface and an opposing second side, and defining at least one aperture;
at least one OLED on the mounting surface, the at least one OLED configured to emanate light, comprising:
an anode, a cathode, and at least one organic layer comprising a light emitting layer between the anode and the cathode;
a housing for the circuit board; and
at least one connector arranged at an end of the housing, the housing and the connector defining a package adapted for installation in a light fixture.

In some embodiments, the OLED light comprises a plurality of OLEDs mounted on a circuit board such that light emanates in a plurality of directions. In some embodiments, a portion of the light emanated in a first direction is deflected to emanate in a second direction. In some embodiments, a reflector is used to deflect the light emanated in a first direction.

Displays or Screens

In some embodiments, the compounds of the invention can be used in a screen or a display. In some embodiments, the compounds of the invention are deposited onto a substrate using a process including, but not limited to, vacuum evaporation, deposition, vapor deposition, or chemical vapor deposition (CVD). In some embodiments, the substrate is a photoplate structure useful in a two-sided etch provides a unique aspect ratio pixel. The screen (which may also be referred to as a mask) is used in a process in the manufacturing of OLED displays. The corresponding artwork pattern design facilitates a very steep and narrow tie-bar between the pixels in the vertical direction and a large, sweeping bevel opening in the horizontal direction. This allows the close patterning of pixels needed for high definition displays while optimizing the chemical deposition onto a TFT backplane.
The internal patterning of the pixel allows the construction of a 3-dimensional pixel opening with varying aspect ratios in the horizontal and vertical directions. Additionally, the use of imaged "stripes" or halftone circles within the pixel area inhibits etching in specific areas until these specific patterns are undercut and fall off the substrate. At that point the entire pixel area is subjected to a similar etch rate but the depths are varying depending on the halftone pattern. Varying the size and spacing of the halftone pattern allows etching to be inhibited at different rates within the pixel allowing for a localized deeper etch needed to create steep vertical bevels.
A preferred material for the deposition mask is invar. Invar is a metal alloy that is cold rolled into long thin sheet

in a steel mill. Invar cannot be electrodeposited onto a rotating mandrel as the nickel mask. A preferred and more cost feasible method for forming the open areas in the mask used for deposition is through a wet chemical etching.

In some embodiments, a screen or display pattern is a pixel matrix on a substrate. In some embodiments, a screen or display pattern is fabricated using lithography (e.g., photolithography and e-beam lithography). In some embodiments, a screen or display pattern is fabricated using a wet chemical etch. In further embodiments, a screen or display pattern is fabricated using plasma etching.

Methods of Manufacturing Devices

An OLED display is generally manufactured by forming a large mother panel and then cutting the mother panel in units of cell panels. In general, each of the cell panels on the mother panel is formed by forming a thin film transistor (TFT) including an active layer and a source/drain electrode on a base substrate, applying a planarization film to the TFT, and sequentially forming a pixel electrode, a light-emitting layer, a counter electrode, and an encapsulation layer, and then is cut from the mother panel.

An OLED display is generally manufactured by forming a large mother panel and then cutting the mother panel in units of cell panels. In general, each of the cell panels on the mother panel is formed by forming a thin film transistor (TFT) including an active layer and a source/drain electrode on a base substrate, applying a planarization film to the TFT, and sequentially forming a pixel electrode, a light-emitting layer, a counter electrode, and an encapsulation layer, and then is cut from the mother panel.

In another aspect, provided herein is a method of manufacturing an organic light-emitting diode (OLED) display, the method comprising:

forming a barrier layer on a base substrate of a mother panel;
forming a plurality of display units in units of cell panels on the barrier layer;
forming an encapsulation layer on each of the display units of the cell panels;
applying an organic film to an interface portion between the cell panels.

In some embodiments, the barrier layer is an inorganic film formed of, for example, SiNx, and an edge portion of the barrier layer is covered with an organic film formed of polyimide or acryl. In some embodiments, the organic film helps the mother panel to be softly cut in units of the cell panel.

In some embodiments, the thin film transistor (TFT) layer includes a light-emitting layer, a gate electrode, and a source/drain electrode. Each of the plurality of display units may include a thin film transistor (TFT) layer, a planarization film formed on the TFT layer, and a light-emitting unit formed on the planarization film, wherein the organic film applied to the interface portion is formed of a same material as a material of the planarization film and is formed at a same time as the planarization film is formed. In some embodiments, a light-emitting unit is connected to the TFT layer with a passivation layer and a planarization film therebetween and an encapsulation layer that covers and protects the light-emitting unit. In some embodiments of the method of manufacturing, the organic film contacts neither the display units nor the encapsulation layer.

Each of the organic film and the planarization film may include any one of polyimide and acryl. In some embodiments, the barrier layer may be an inorganic film. In some embodiments, the base substrate may be formed of polyimide. The method may further include, before the forming of the barrier layer on one surface of the base substrate formed of polyimide, attaching a carrier substrate formed of a glass material to another surface of the base substrate, and before the cutting along the interface portion, separating the carrier substrate from the base substrate. In some embodiments, the OLED display is a flexible display.

In some embodiments, the passivation layer is an organic film disposed on the TFT layer to cover the TFT layer. In some embodiments, the planarization film is an organic film formed on the passivation layer. In some embodiments, the planarization film is formed of polyimide or acryl, like the organic film formed on the edge portion of the barrier layer. In some embodiments, the planarization film and the organic film are simultaneously formed when the OLED display is manufactured. In some embodiments, the organic film may be formed on the edge portion of the barrier layer such that a portion of the organic film directly contacts the base substrate and a remaining portion of the organic film contacts the barrier layer while surrounding the edge portion of the barrier layer.

In some embodiments, the light-emitting layer includes a pixel electrode, a counter electrode, and an organic light-emitting layer disposed between the pixel electrode and the counter electrode. In some embodiments, the pixel electrode is connected to the source/drain electrode of the TFT layer.

In some embodiments, when a voltage is applied to the pixel electrode through the TFT layer, an appropriate voltage is formed between the pixel electrode and the counter electrode, and thus the organic light-emitting layer emits light, thereby forming an image. Hereinafter, an image forming unit including the TFT layer and the light-emitting unit is referred to as a display unit.

In some embodiments, the encapsulation layer that covers the display unit and prevents penetration of external moisture may be formed to have a thin film encapsulation structure in which an organic film and an inorganic film are alternately stacked. In some embodiments, the encapsulation layer has a thin film encapsulation structure in which a plurality of thin films are stacked. In some embodiments, the organic film applied to the interface portion is spaced apart from each of the plurality of display units. In some embodiments, the organic film is formed such that a portion of the organic film directly contacts the base substrate and a remaining portion of the organic film contacts the barrier layer while surrounding an edge portion of the barrier layer.

In one embodiment, the OLED display is flexible and uses the soft base substrate formed of polyimide. In some embodiments, the base substrate is formed on a carrier substrate formed of a glass material, and then the carrier substrate is separated.

In some embodiments, the barrier layer is formed on a surface of the base substrate opposite to the carrier substrate. In one embodiment, the barrier layer is patterned according to a size of each of the cell panels. For example, while the base substrate is formed over the entire surface of a mother panel, the barrier layer is formed according to a size of each of the cell panels, and thus a groove is formed at an interface portion between the barrier layers of the cell panels. Each of the cell panels can be cut along the groove.

In some embodiments, the method of manufacture further comprises cutting along the interface portion, wherein a groove is formed in the barrier layer, wherein at least a portion of the organic film is formed in the groove, and wherein the groove does not penetrate into the base substrate. In some embodiments, the TFT layer of each of the cell panels is formed, and the passivation layer which is an inorganic film and the planarization film which is an organic film are disposed on the TFT layer to cover the TFT layer. At the same time as the planarization film formed of, for example, polyimide or acryl is formed, the groove at the interface portion is covered with the organic film formed of, for example, polyimide or acryl. This is to prevent cracks from occurring by allowing the organic film to absorb an impact generated when each of the cell panels is cut along the groove at the interface portion. That is, if the entire barrier layer is entirely exposed without the organic film, an impact generated when each of the cell panels is cut along the groove at the interface portion is transferred to the barrier layer, thereby increasing the risk of cracks. However, in one embodiment, since the groove at the interface portion between the barrier layers is covered with the organic film and the organic film absorbs an impact that would otherwise be transferred to the barrier layer, each of the cell panels may be softly cut and cracks may be prevented from occurring in the barrier layer. In one embodiment, the organic film covering the groove at the interface portion and the planarization film are spaced apart from each other. For example, if the organic film and the planarization film are connected to each other as one layer, since external moisture may penetrate into the display unit through the planarization film and a portion where the organic film remains, the organic film and the planarization film are spaced apart from each other such that the organic film is spaced apart from the display unit.

In some embodiments, the display unit is formed by forming the light-emitting unit, and the encapsulation layer is disposed on the display unit to cover the display unit. As such, once the mother panel is completely manufactured, the carrier substrate that supports the base substrate is separated from the base substrate. In some embodiments, when a laser beam is emitted toward the carrier substrate, the carrier substrate is separated from the base substrate due to a difference in a thermal expansion coefficient between the carrier substrate and the base substrate.

In some embodiments, the mother panel is cut in units of the cell panels. In some embodiments, the mother panel is cut along an interface portion between the cell panels by using a cutter. In some embodiments, since the groove at the interface portion along which the mother panel is cut is covered with the organic film, the organic film absorbs an impact during the cutting. In some embodiments, cracks may be prevented from occurring in the barrier layer during the cutting.

In some embodiments, the methods reduce a defect rate of a product and stabilize its quality.

Another aspect is an OLED display including: a barrier layer that is formed on a base substrate; a display unit that is formed on the barrier layer; an encapsulation layer that is formed on the display unit; and an organic film that is applied to an edge portion of the barrier layer.

Examples

The features of the present invention will be described more specifically with reference to Synthesis Examples and Examples given below. The materials, processes, procedures and the like shown below may be appropriately modified unless they deviate from the substance of the invention. Accordingly, the scope of the invention is not construed as being limited to the specific examples shown below. Hereinunder the light emission characteristics were evaluated using a source meter (available from Keithley Corporation, Keithley 2400), a semiconductor parameter analyzer (available from Agilent Technology Corporation, E5273A), a light power meter apparatus (available from Newport Corporation, 1930C), an optical spectroscope (available from Ocean Optics Corporation, USB 2000), a spectroradiometer (available from Topcon Corporation, SR-3) and a streak camera (available from Hamamatsu Photonics K.K., C4334).

(Synthesis Example 1) Synthesis of Compound 3

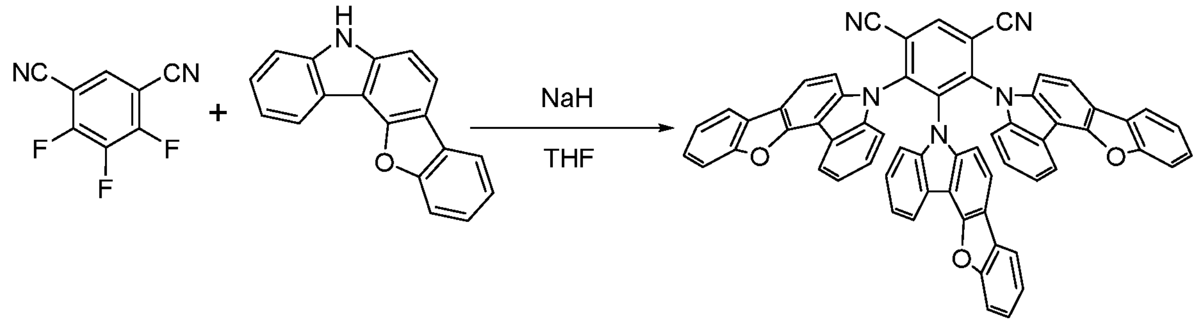


3

In a nitrogen stream atmosphere, sodium hydride (0.44 g, 11.0 mmol) and benzofuro[3,2-c]carbazole (2.83 g, 11.0 mmol) were stirred in tetrahydrofuran (20 mL) at 0°C for 30 minutes, and then 4,5,6-trifluoroisophthalonitrile was added, then heated up to room temperature and reacted at room temperature for 5 hours. Subsequently, the reaction was stopped with water and methanol. The precipitated yellow solid was filtered out, and the residue was purified by silica gel column chromatography (toluene/hexane/chloroform = 11/2/1) and reprecipitation (o-dichlorobenzene/methanol) to give a yellow solid, Compound 3 (0.91 g, 1.02 mmol, yield 37%).

$^1$H NMR (400 MHz, $CDCl_3$, $\delta$): 8.62 (s, 0.3H), 8.61 (s, 0.7H), 8.15-8.10 (m, 2H), 7.88-7.77 (s, 2H), 7.73-7.54 (m, 6H), 7.40-7.25 (m, 6H), 7.23-7.11 (m, 8H), 7.09-6.93 (m, 2H), 6.90-6.78 (m, 3H), 6.69-6.51 (m, 1H).
MS (ASAP): 894.43 ($M+H^+$). Calcd for C62H31N5O3: 893.24.

(Synthesis Example 2) Synthesis of Compound 5

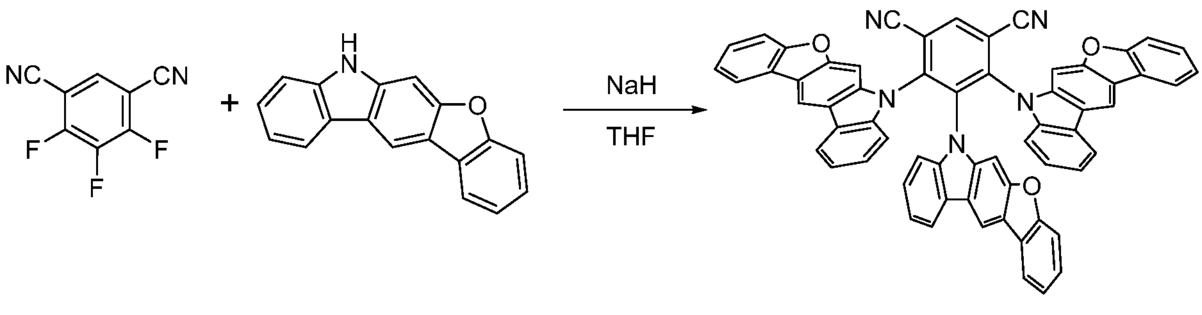


5

This was synthesized at a yield of 80%, according to the same method as in Synthesis Example 1.
$^1$H NMR (400 MHz, $CDCl_3$, $\delta$): 8.66 (s, 1H), 8.22 (s, 0.7H), 8.17 (s, 0.3H), 8.08 (s, 0.7H), 8.05 (s, 0.3H), 7.92-7.87 (m, 1H), 7.79 (d, *J* = 8.4 Hz, 1H), 7.74-7.69 (m, 2H), 7.66-7.63 (m, 2H), 7.53-7.37 (m, 5H), 7.35-7.27 (m, 4H), 7.23-7.17 (m, 2H), 7.15-7.08 (m, 2H), 7.06-6.98 (m, 3H), 6.95-6.86 (m, 3H), 6.80-6.68 (m, 2H), 6.57-6.53 (m, 1H). MS (ASAP): 894.44 ($M+H^+$). Calcd for C62H31N5O3: 893.24.

(Synthesis Example 3) Synthesis of Compound 6

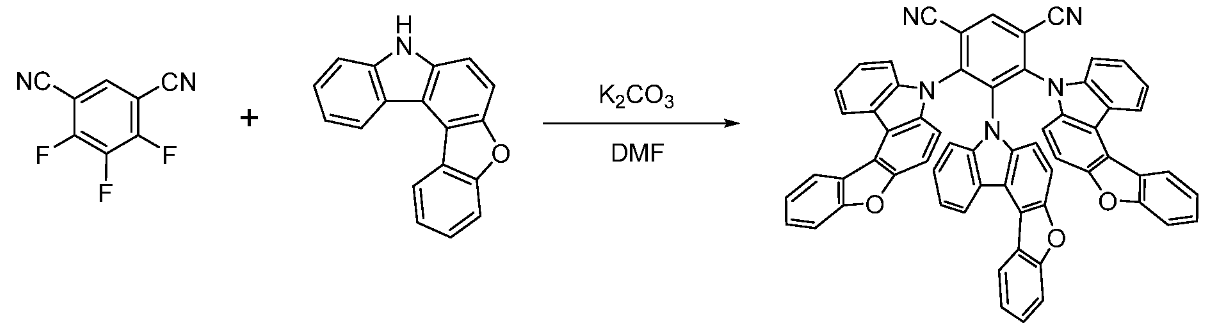


6

In a nitrogen stream atmosphere, potassium carbonate (1.20 g, 8.69 mmol) and benzofuro[2,3-c]carbazole (1.99 g, 7.72 mmol) were stirred in dimethylformamide (20 mL) at room temperature for 30 minutes, and then 4,5,6-trifluoroisophthalonitrile (0.35 mmol, 1.92 mmol) was added, then heated up to 60°C and reacted for 5 hours. Subsequently, this was restored to room temperature, and the reaction was stopped with water and methanol. The precipitated yellow solid was filtered out, and the residue was purified by silica gel column chromatography (toluene) and reprecipitation (o-toluene/methanol) to give a yellow solid, Compound 6 (1.35 g, 1.51 mmol, yield 79%). $^{1}$H NMR (400 MHz, $CDCl_3$, $\delta$): 8.65 (s, 1H), 8.35-8.28 (m, 5H), 7.98-7.7.95 (m, 1H), 7.81-7.78 (m, 1H), 7.57-7.50 (m, 2H), 7.47-7.28 (m, 11H), 7.23-7.10 (m, 5H), 7.02-6.79 (m, 4H), 6.72-6.60 (m, 1H).
MS (ASAP): 894.50 (M+H$^+$). Calcd for C62H31N5O3: 893.24.

(Synthesis Example 4) Synthesis of Compound 33

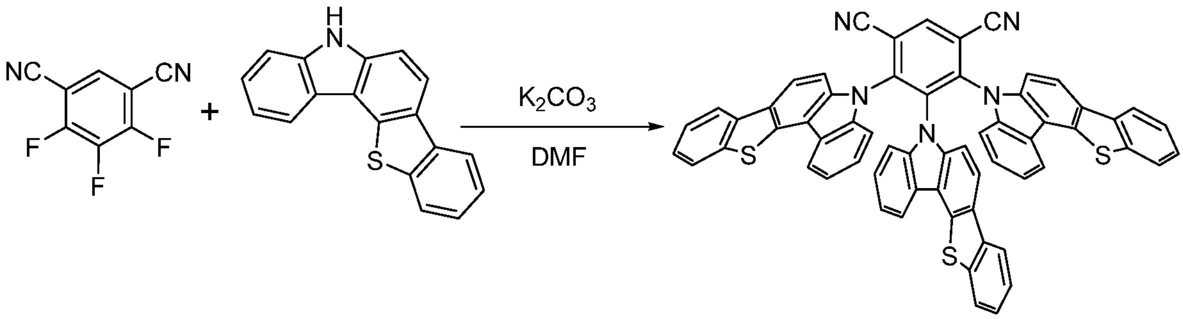


33

This was synthesized at a yield of 63%, according to the same method as in Synthesis Example 3.

$^{1}$H NMR (400 MHz, $CDCl_3$, $\delta$): 8.43 (d, *J = 8.0 Hz,* 1H), 8.38-8.35 (m, 3H), 8.17-8.13 (m, 2H), 8.10-8.03 (m, 3H), 7.88-7.82 (m, 1H), 7.13-7.77 (m, 4H), 7.70-7.64 (m, 4H), 7.58-7.43 (m, 5H), 7.38-7.32 (m, 3H), 7.24 (t, *J = 7.2 Hz,* 1H), 7.14-7.21 (m, 2H), 6.88 (t, *J = 7.6* $H_z$, 1H), 6.78 (t, *J = 8.4 Hz,* 1H).
MS (ASAP): 942.15 (M+H$^+$). Calcd for C62H31N5S3: 941.17.

(Synthesis Example 5) Synthesis of Compound 159

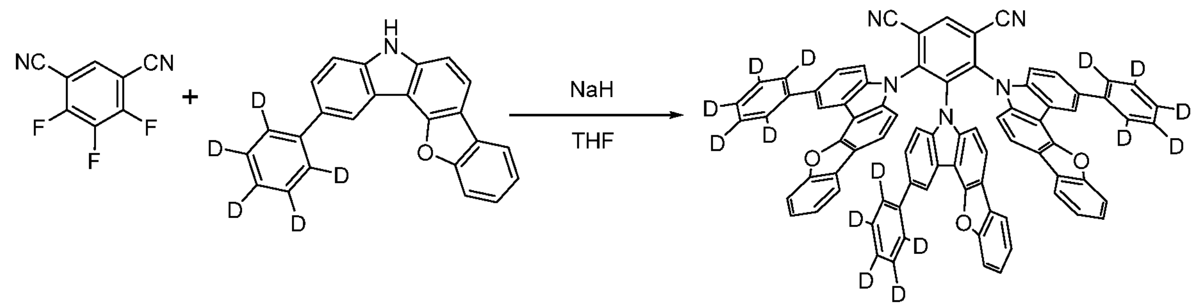


159

This was synthesized at a yield of 92%, according to the same method as in Synthesis Example 1.

$^{1}$H NMR (400 MHz, $CDCl_3$, $\delta$): 9.67-9.64 (m, 1H), 8.24-8.03 (m, 6-H), 7.94-7.87 (m, 3H), 7.81-7.74 (m, 3H), 7.71-7.22 (m, 13H), 7.16-7.03 (m, 0.5H), 7.16-7.03 (m, 0.5H), 6.81-6.79 (m, 0.5H), 6.64-6.61 (m, 0.5H), 6.47-6.44 (m, 0.5H). MS (ASAP): 1137.68 (M+H$^{+}$). Calcd for C80H28D15N5O3: 1136.43.

(Synthesis Example 6) Synthesis of Compound 4

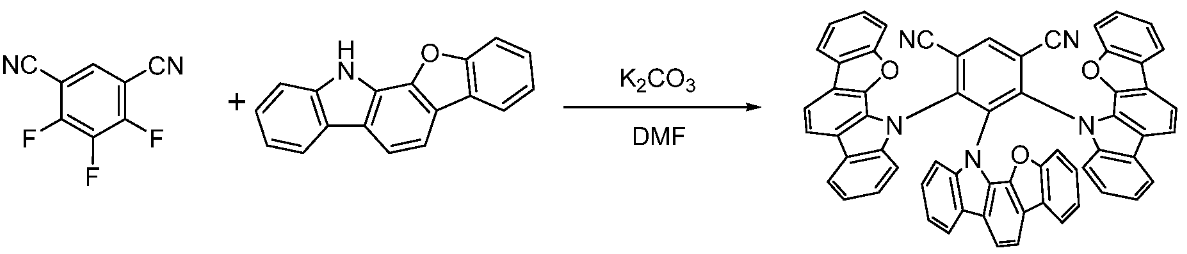


4

This was synthesized at a yield of 69%, according to the same method as in Synthesis Example 3. MS (ASAP): 894.43 (M+H$^{+}$). Calcd for $C_{62}H_{31}N_5O_3$: 893.24.

(Synthesis Example 7) Synthesis of Compound 2

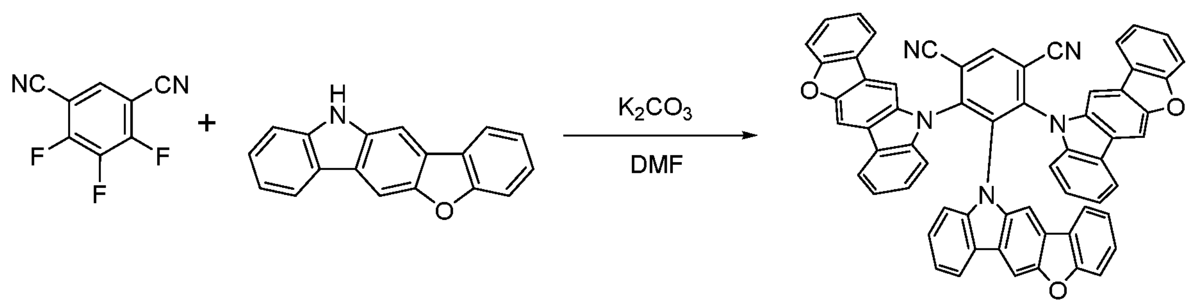


2

This was synthesized at a yield of 69%, according to the same method as in Synthesis Example 3. MS (ASAP): 894.46 (M+H$^{+}$). Calcd for $C_{62}H_{31}N_5O_3$: 893.24.

(Synthesis Example 8) Synthesis of Compound 570243

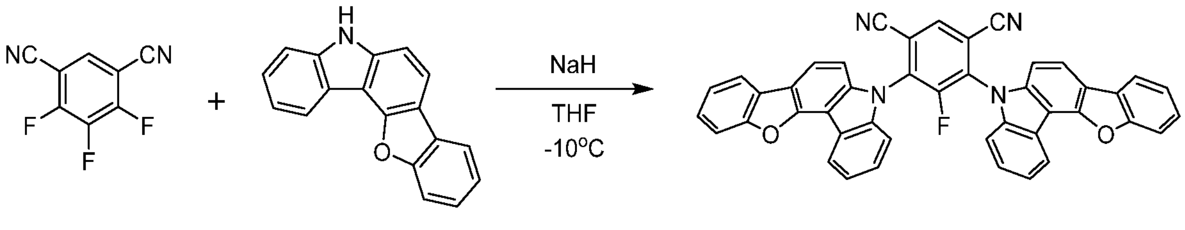


1a

In a nitrogen atmosphere, a tetrahydrofuran (150 mL) solution of 5H-benzofuro[3,2-c]carbazole (14.1 g, 54.8 mmol) and NaH (60%, 2.2 g, 55 mmol) was stirred at room temperature for 30 minutes. Subsequently, this was dropwise added to a tetrahydrofuran (275 mL) solution of 4,5,6-trifluoroisophthalonitrile (5 g, 27.5 mmol) cooled at -10°C, taking 20 minutes, and then stirred for 4 hours. The reaction mixture was neutralized with an aqueous saturated $NH_4Cl$ solution added thereto, then restored to room temperature to stop the reaction, extracted with ethyl acetate, and the organic layer was dried with $Mg_2SO_4$. An inorganic substance was separated by filtration, the solvent was evaporated away under reduced pressure, and the resultant mixture was purified by column chromatography (toluene/hexane/$CHCl_3$=6/3.5/0.5) and reprecipitation ($CHCl_3$/hexane) to give a yellow solid, Compound la (16.5 g, 25.2 mmol, 91.7%). $^1$H NMR (400 MHz, $CDCl_3$, $\delta$): 8.54 (d, *J=7.6Hz,* 2H), 8.23 (s, 1H), 8.00 (d, *J=8.8Hz,* 2H), 7.97 (d, *J=7.6Hz,* 2H),7.72 (d, *J=8.4Hz,* 2H) 7.567-7.443 (m, 6H), 7.38 (t, *J=8.0Hz,* 2H) 7.28-7.25 (m, 2H), 7.20-7.17 (m, 2H) .
MS (ASAP): 657.25 $[M+H]^+$. Calcd for. $C_{44}H_{21}FN_4O_2$: 656.16

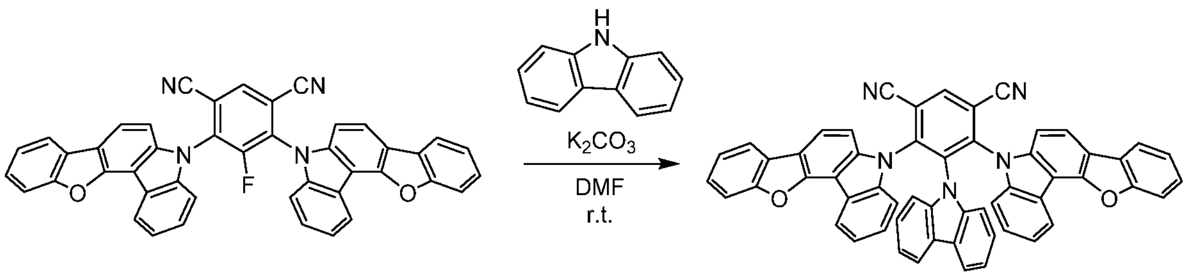


1a 570243

In a nitrogen atmosphere, a dimethylformamide solution (280 mL) of Compound la (16.5 g, 252.2 mmol), carbazole (7.9 g, 47.2 mmol) and $K_2CO_3$ (8.17 g, 59.1 mmol) was stirred at room temperature for 15 hours. Methanol (200 mL) and water (150 mL) were added to the reaction mixture to stop the reaction. Subsequently, the precipitated substance was filtered out to collect the solid. The resultant solid was purified by column chromatography (toluene/hexane/$CHCl_3$=6/3.5/0.5) and reprecipitation (toluene/hexane) to give a yellow solid, Compound 570243 (16.0 g, 19.9 mmol, 84.2%). $^1$H NMR (400 MHz, $CDCl_3$, $\delta$): 8.60 (s, 1H), 8.19-8.15 (m, 2H), 7.87 (t, *J=8.4Hz,* 2H), 7.90-7.86 (m, 4H), 7.44-7.39 (m, 2H), 7.36-7.31 (m, 2H), 7.22-7.02 (m, 10H), 6,83-6.66 (m, 4H), 6.62-6.46 (m, 2H).
MS (ASAP): 804.25 $[M+H]^+$. Calcd for. $C_{56}H_{29}N_5O_2$ : 803.23

(Synthesis Example 9) Synthesis of Compound 571683

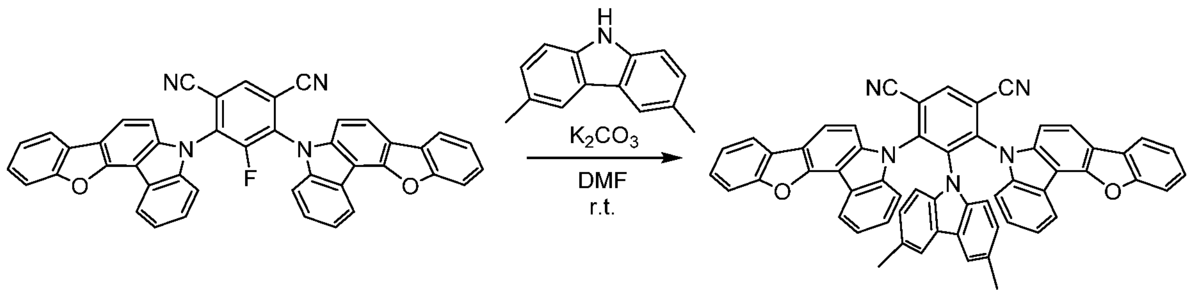


1a

571683

This was synthesized at a yield of 68%, according to the same method as in Synthesis Example 8.

$^1$H NMR (400 MHz, $CDCl_3$, $\delta$): 8.52 (s, 1H), 8.20-8.17 (m, 2H), 7.87 (t, *J=6.8Hz,* 2H), 7.65-7.59 (m, 4H),7.42-7.36 (m, 2H) 7.34-7.30 (m, 2H), 7.25-7.19 (m, 2H) 7.18-6.92 (m, 6H), 6.95 (s, 2H), 6.70-6.62 (m, 2H), 6.38-6.27 (m, 2H) 2.05 (s, 6H).
MS (ASAP):832.41 $[M+H]^+$. Calcd for. C58H33N5O2 : 831.26

(Synthesis Example 10) Synthesis of Compound 570244

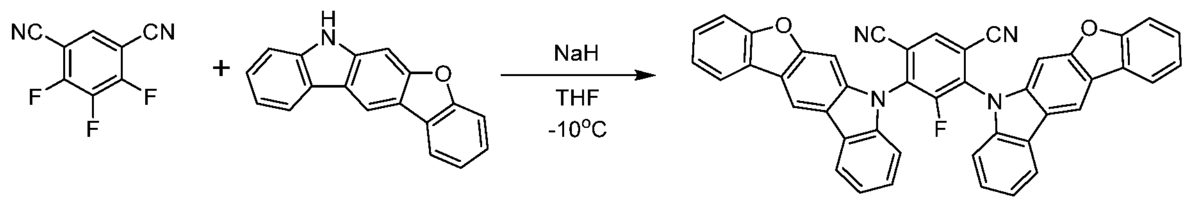


This was synthesized at a yield of 56%, according to the same method of the first reaction in Synthesis Example 8.

$^1$H NMR (400 MHz, $CDCl_3$, $\delta$): 8.64 (s, 2H), 8.45 (s, 1H), 8.23 (d, *J = 8.0 Hz,* 2H), 8.06 (d, *J = 6.8 Hz,* 2H),7.59-7.35 (m, 12H), 7.29-7.24 (m, 2H).
MS (ASAP): 657.35 $[M+H]^+$. Calcd for. $C_{44}H_{21}FN_4O_2$: 656.16

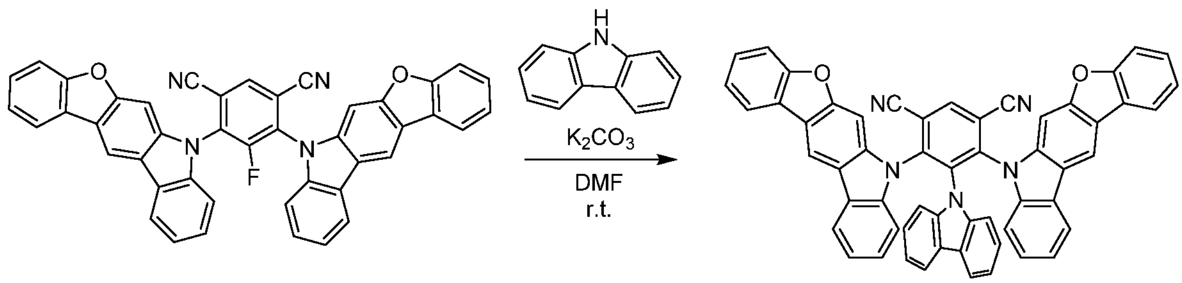


570244

This was synthesized at a yield of 67%, according to the same method of the second reaction in Synthesis Example 8.

$^1$H NMR (400 MHz, $CDCl_3$, $\delta$): 8.62 (s, 1H), 8.24 (d, *J = 10.8 Hz,* 2H), 7.95-7.90 (m, 2H), 7.81-7.76 (m, 2H),7.52-7.30 (m, 7H), 7.21-7.00 (m, 7H), 6.92-6.54 (m, 8H).
MS (ASAP): 804.37 $[M+H]^+$. Calcd for. $C_{56}H_{29}N_5O_2$ : 803.23

(Synthesis Example 11) Synthesis of Compound 570273

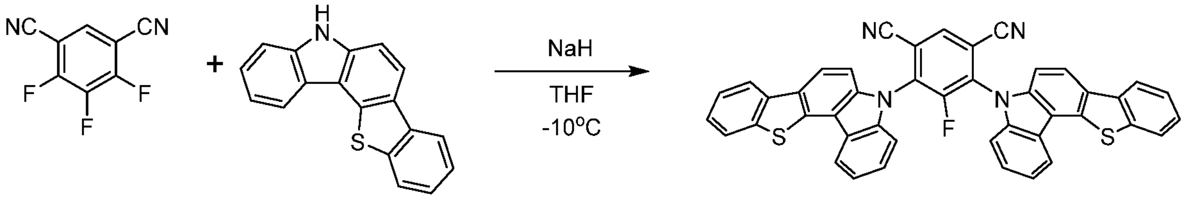


This was synthesized at a yield of 80%, according to the same method of the first reaction in Synthesis Example 8. MS (ASAP): 689.43 $[M+H]^+$. Calcd for. C44H21FN4S2: 688.12

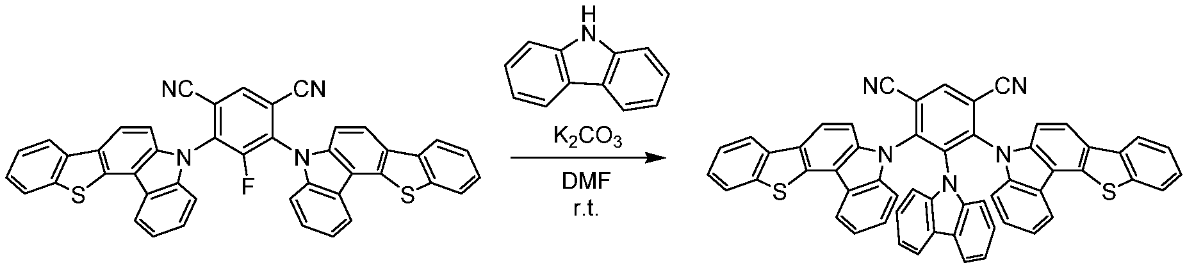


570273

This was synthesized at a yield of 89%, according to the same method of the second reaction in Synthesis Example 8.

$^1$H NMR (400 MHz, $CDCl_3$, δ): 8.61 (s, 1H), 8.08 (t, *J = 8.4 Hz,* 2H), 7.90-7.81 (m, 7H), 7.48-7.40 (m, 2H),7.30-7.02 (m, 10H), 6.80-6.43 (m, 7H).
MS (ASAP): 836.14 $[M+H]^+$. Calcd for. C56H29N5S2: 835.19

(Synthesis Example 12) Synthesis of Compound 570274

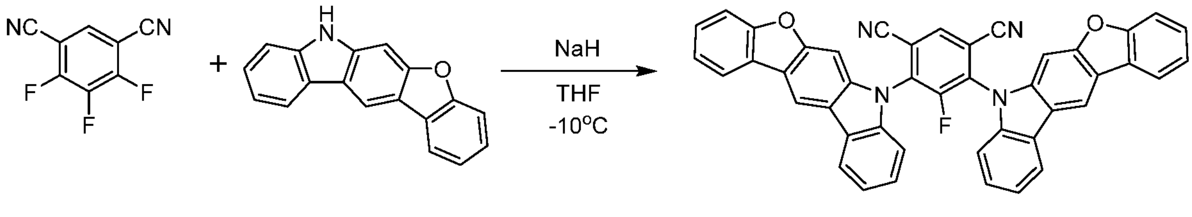


This was synthesized at a yield of 79%, according to the same method of the first reaction in Synthesis Example 8.

$^1$H NMR (400 MHz, $CDCl_3$, δ): 9.53 (s, 1H), 8.46 (t, *J = 7.6Hz,* 4H), 7.40 (d, *J = 8.0 Hz,* 4H), 8.02 (d, *J = 7.6Hz,* 2H), 7.40 (d, *J = 8.0 Hz,* 2H), 7.62-748 (m, 8H).
MS (ASAP): 688.97 $[M+H]^+$. Calcd for. C44H21FN4S2: 688.12

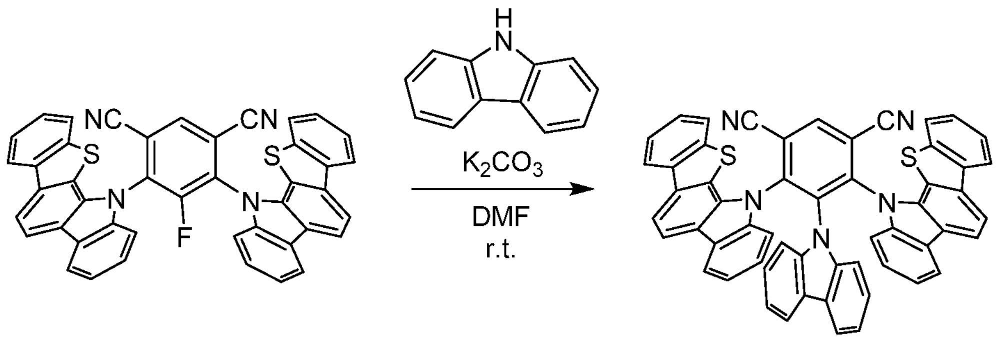


570274

This was synthesized at a yield of 48%, according to the same method of the second reaction in Synthesis Example 8.

$^1$H NMR (400 MHz, DMSO, δ): 9.75 (s, 1H), 8.24 (d, *J = 7.2 Hz,* 2H), 8.13 (d, *J = 7.2 Hz,* 2H), 7.99 (d, *J = 7.6Hz,* 2H), 7.89 (d, *J = 8.4 Hz,* 2H), 7.81 (d, *J = 7.2 Hz,* 2H), 7.61 (d, *J = 7.2 Hz,* 2H), 7.47 (d, *J = 8.8 Hz,* 4H), 7.26-7.22 (m, 2H), 7.11-7.06 (m, 5H), 6.90 (d, *J = 8. 0 Hz,* 1H), 6.67 (t, *J = 7.6 Hz,* 1H), 6.44 (t, *J = 7.2 Hz,* 1H), 6.08 (t, *J = 8. 0 Hz,* 1H), 5.16 (t, *J = 8.4Hz,* 1H).
MS (ASAP): 836.20 [M+H]$^+$. Calcd for. C56H29N5S2: 835.19

(Synthesis Example 13) Synthesis of Compound 570261

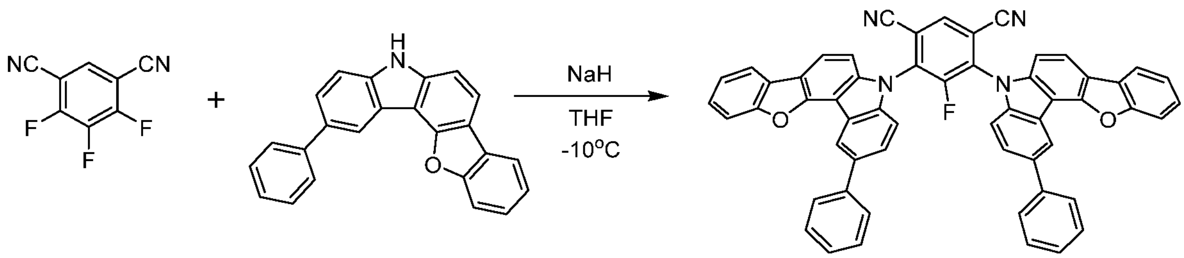


This was synthesized at a yield of 90%, according to the same method of the first reaction in Synthesis Example 8.

$^1$H NMR (400 MHz, $CDCl_3$, δ): 8.77 (s, 2H), 8.37 (s, 1H), 8.08 (d, *J = 8.8 Hz,* 2H), 8.01 (d, *J = 7.6Hz,* 2H), 7.82-7.75(m, 9H), 7.55-7.47(m, 6H), 7.43-7.37 (m, 6H), 7.28-7.25 (m, 1H).
MS (ASAP): 809.33[M+H]$^+$. Calcd for. C56H29FN4O2: 808.23

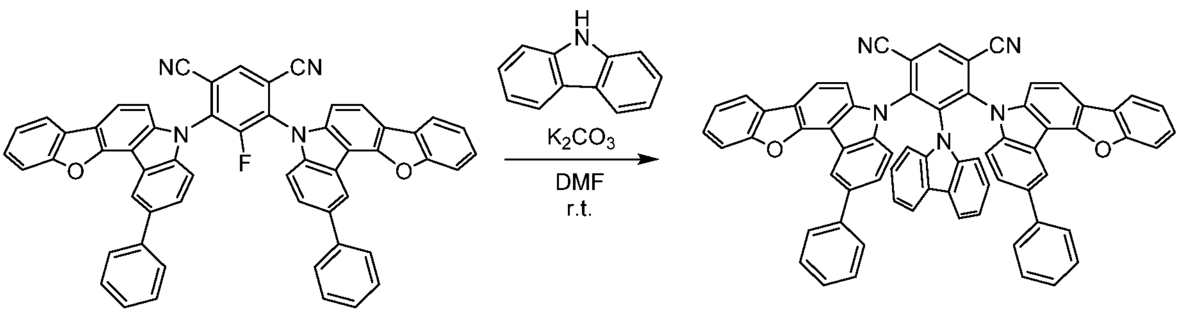


570261

This was synthesized at a yield of 94%, according to the same method of the second reaction in Synthesis Example 8.

$^1$H NMR (400 MHz, $CDCl_3$, $\delta$): 8.63 (s, 1H), 8.35 (d, *J = 10.4 Hz,* 2H), 7.92-7.86 (m, 2H), 7.74 (d, *J = 8.4 Hz,* 1H), 7.66-7.57(m, 7H), 7.48-7.31(m, 11H), 7.29-7.10 (m, 8H), 6.84-6.46 (m, 5H).
MS (ASAP): 956.43 $[M+H]^+$. Calcd for. C68H37N5O2: 955.29

(Synthesis Example 14) Synthesis of Compound 570399

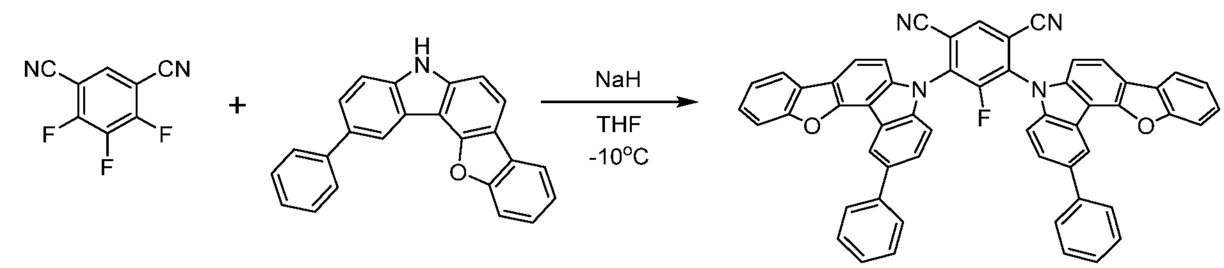


This was synthesized at a yield of 89%, according to the same method of the first reaction in Synthesis Example 8.

$^1$H NMR (400 MHz, $CDCl_3$, $\delta$): 8.77 (s, 2H), 8.38 (s, 1H), 8.09 (d, *J=8.4 Hz,* 2H), 8.01 (d, *J = 6.4 Hz,* 2H),7.83-7.76 (m, 4H) 7.49 (t, *J = 7.2 Hz,* 2H), 7.43-7.38 (m, 4H) 7.28-7.25 (m, 2H) .
MS (ASAP): 819.45 $[M+H]^+$. Calcd for. C56H19D10FN4O2: 818.29

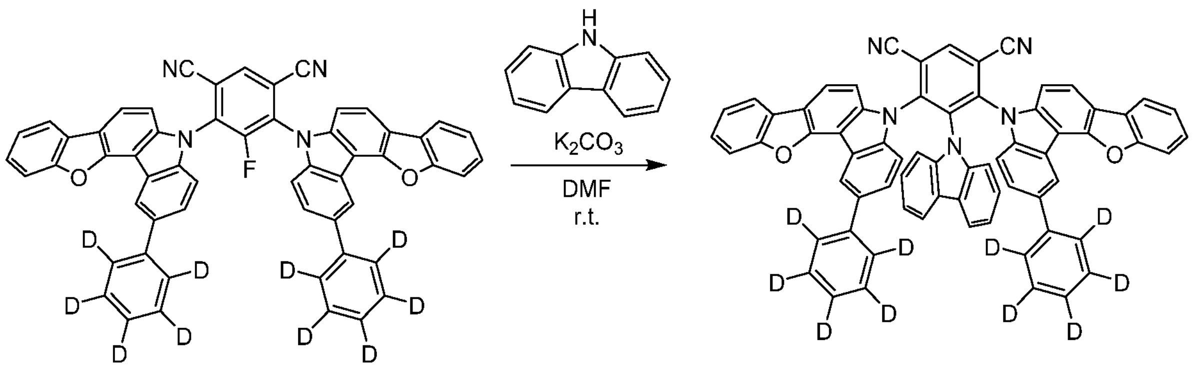


570399

This was synthesized at a yield of 89%, according to the same method of the second reaction in Synthesis Example 8.

$^1$H NMR (400 MHz, $CDCl_3$, $\delta$): 8.63 (s, 1H), 8.35 (d, *J=8.8 Hz,* 2H), 7.93-7.86 (m, 2H), 7.74 (d, *J = 8.8 Hz,* 1H),7.68-7.61 (m, 3H), 7.46-7.32 (m, 5H) 7.27-7.10 (m, 7H), 6.84-6.46 (m, 6H).
MS (APCI): 966 $[M+H]^+$. Calcd for. C68H27D10N5O2: 965.36

(Synthesis Example 15) Synthesis of Compound 570309

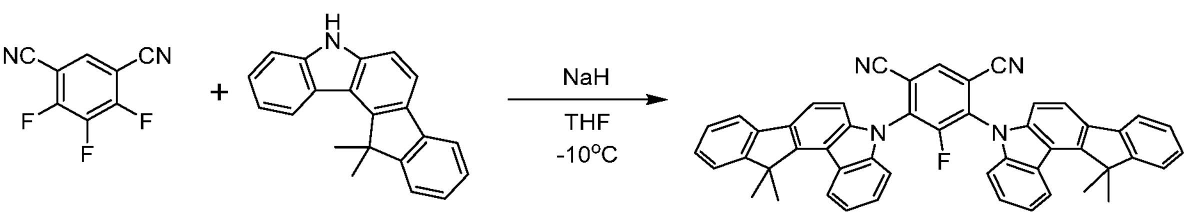

This was synthesized at a yield of 64%, according to the same method of the first reaction in Synthesis Example 8.

$^1$H NMR (400 MHz, $CDCl_3$, $\delta$): 8.52 (d, *J = 8.0 Hz,* 2H),8.34(s, 1H), 7.91-7.88 (m, 2H),7.76-7.74 (m, 2H), 7.54-7.44(m, 6H), 7.38-7.32 (m, 4H), 7.25-7.21 (m, 4H), 1.91 (s, 6H), 1.86 (s, 6H).
MS (ASAP): 709.26[M+H]$^+$. Calcd for. C50H33FN4: 708.27

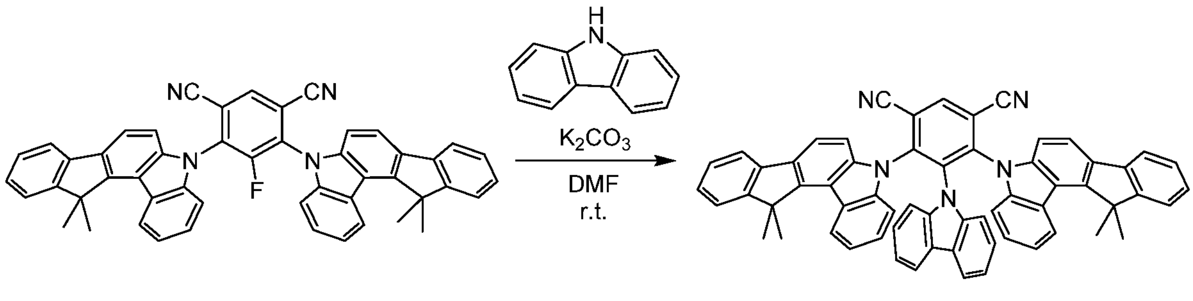


570309

This was synthesized at a yield of 64%, according to the same method of the second reaction in Synthesis Example 8.

$^1$H NMR (400 MHz, $CDCl_3$, $\delta$): 8.62 (s, 1H), 8.04(t, *J = 8.0 Hz,* 2H), 7.62-7.57 (m, 2H), 7.46 (d, *J = 8. 0 Hz,* 2H), 7.44-7.27 (m, 8H), 7.19-6.99 (m, 10H), 6.92 (t, *J = 8.0 Hz,* 1H), 6.70-6.43 (m, 6H), 1.54 (s, 6H), 1.49 (s, 6H).
MS (ASAP): 856.49 [M+H]$^+$. Calcd for. C62H41N5: 855.34

(Synthesis Example 16) Synthesis of Compound 570378

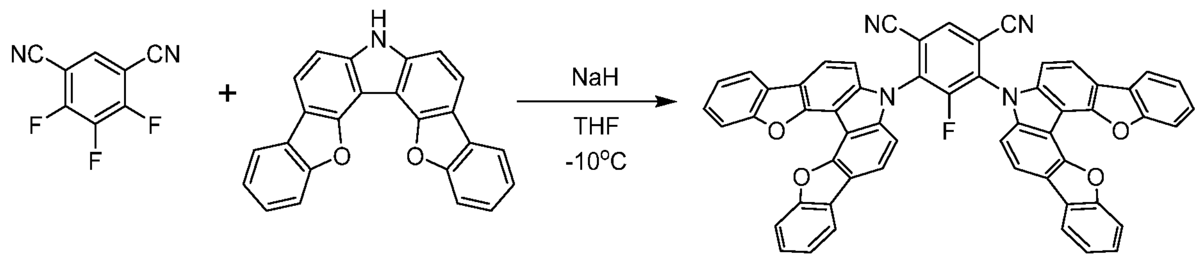


This was synthesized at a yield of 63%, according to the same method of the first reaction in Synthesis Example 8.

$^1$H NMR (400 MHz, DMSO, $\delta$): 9.35 (s, 1H), 8.42 (d, *J = 8.4 Hz,* 4H), 8.25 (d, *J = 8.4 Hz,* 4H), 7.97 (d, *J = 8.4 Hz,* 4H), 7.84 (d, *J = 6.8 Hz,* 4H), 7.59 (t, *J = 6.8 Hz,* 4H), 7.46 (t, *J = 7.2 Hz* ,4H), 7.23-7.10 (m ,4H).
MS (ASAP): 837.33 [M+H]$^+$. Calcd for. C56H25FN4O4: 836.19

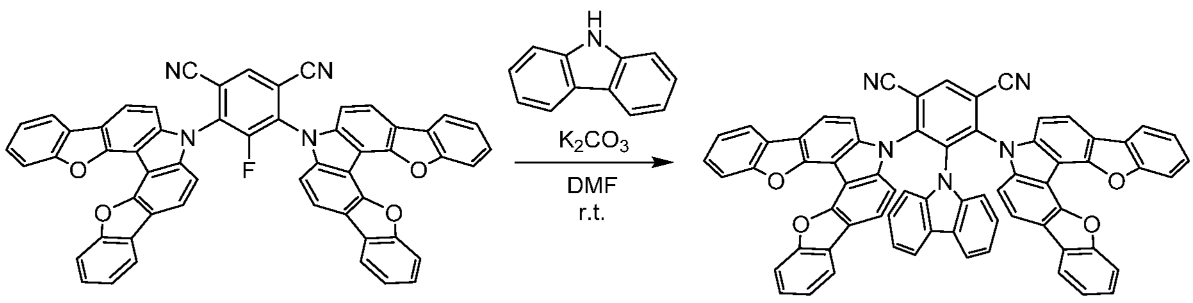


570378

This was synthesized at a yield of 61%, according to the same method of the second reaction in Synthesis

Example 8.

$^1$H NMR (400 MHz, DMSO, δ): 9.64 (s, 1H), 8.12 (d, *J = 8.4 Hz,* 4H), 7.99 (d, *J = 8.4 Hz,* 4H), 7.84 (d, *J = 8.4 Hz,* 8H), 7.53-7.47 (m, 6H), 7.39 (t, *J = 6.8 Hz,* 4H), 7.23 (m, 2H), 6.64-6.58 (m, 4H).
MS (ASAP): 984.45 [M+H]$^+$. Calcd for. C68H33N5O4: 983.25

(Synthesis Example 17) Synthesis of Compound 570262

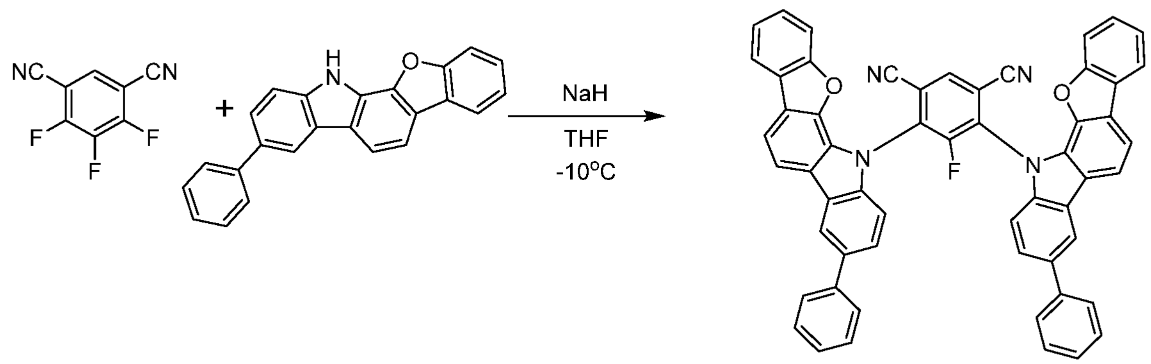


This was synthesized at a yield of 63%, according to the same method of the first reaction in Synthesis Example 8.

$^1$H NMR (400 MHz, $CDCl_3$, δ): 8.42 (s, 1H), 8.37 (s, 2H), 8.15 (d, *J = 8.0 Hz,* 2H), 8.06 (d, *J = 7.6 Hz,* 2H), 8.06 (d, *J = 8.4 Hz,* 2H), 7.69-7.36 (m, 20H).
MS (ASAP): 809.55[M+H]$^+$. Calcd for. C56H29FN4O2: 808.23

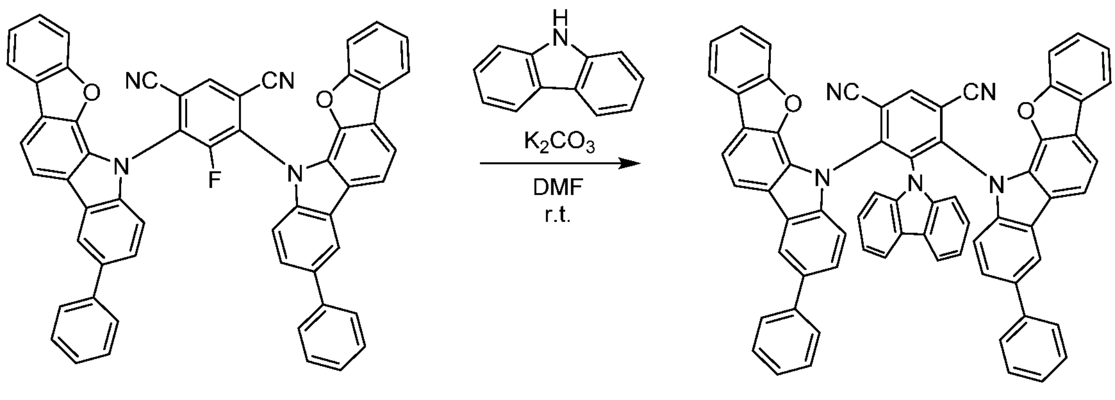


570262

This was synthesized at a yield of 60%, according to the same method of the second reaction in Synthesis Example 8.

$^1$H NMR (400 MHz, $CDCl_3$, δ): 8.70 (s, 1H), 7.92 (s, 2H), 7.89-7.85 (m, 2H), 7.70-7.64 (m, 4H), 7.54 (d, *J = 7.6Hz,* 4H), 7.46-7.38 (m, 6H), 7.33-7.22 (m, 11H), 6.95 (d, *J = 8.8 Hz,* 1H), 6.78 (t, *J = 8.8 Hz,* 2H), 6.64 (d, *J = 8.8 Hz,* 1H), 6.54 (t, *J = 8.8 Hz,* 1H), 6.12 (t, *J = 8.8 Hz,* 1H), 5.40 (t, *J = 8.8 Hz,* 1H),.
MS (ASAP): 956.57 [M+H]$^+$. Calcd for. C68H37N5O2: 955.29

(Synthesis Example 18) Synthesis of Compound 570264

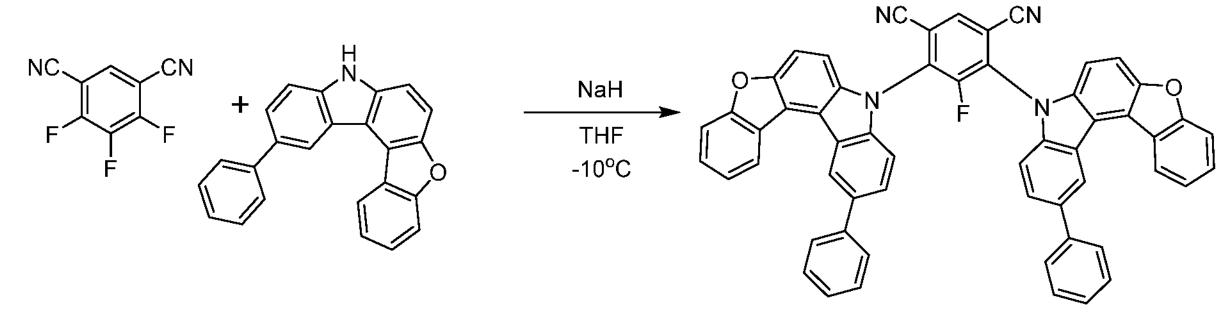


This was synthesized at a yield of 56%, according to the same method of the first reaction in Synthesis Example 8.

$^1$H NMR (400 MHz, $CDCl_3$, δ): 8.92 (s, 2H), 8.64 (d, *J = 6.8 Hz,* 2H), 8.39 (s, 1H), 7.84-7.79 (m, 8H), 7.72 (d, *J = 8.4 Hz,* 2H), 7.58-7.52 (m, 8H), 7.46-7.36 (m, 6H).
MS (ASAP): 809.44[M+H]$^+$. Calcd for. C56H29FN4O2: 808.23

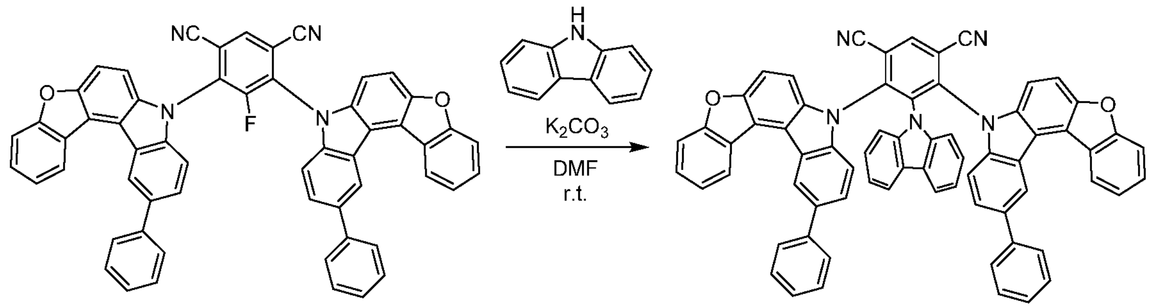


570264

This was synthesized at a yield of 71%, according to the same method of the second reaction in Synthesis Example 8.

$^1$H NMR (400 MHz, $CDCl_3$, δ): 8.66 (s, 1H), 8.52 (s, 2H), 8.40 (d, *J = 7.2 Hz,* 2H), 7.65-7.61 (m, 6H), 7.54-7.48 (m, 6H), 7.46-7.38 (m, 5H), 7.36-7.25 (m, 7H),7.19 (d, *J = 7.6 Hz,* 2H), 6.86-6.80 (m, 2H), 6.76-6.51 (m, 4H).
MS (ASAP): 956.57 [M+H]$^+$. Calcd for. C68H37N5O2: 955.29

(Synthesis Example 19) Synthesis of Compound 570507

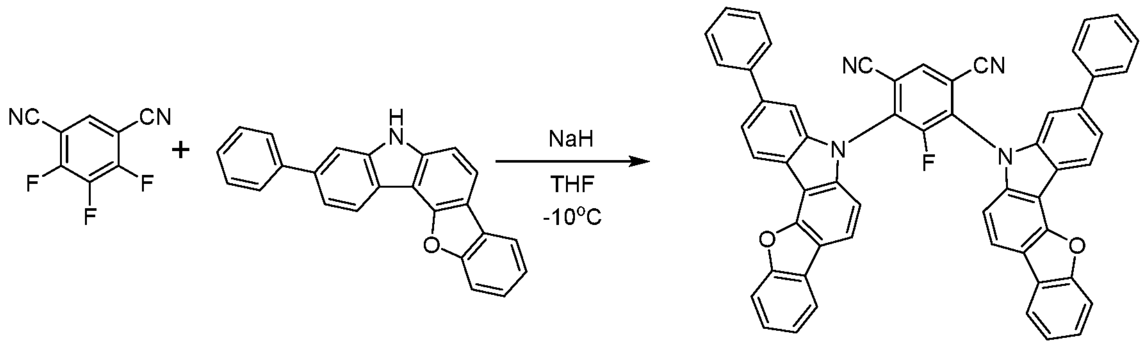


This was synthesized at a yield of 32%, according to the same method for Compound 1a.

$^1$H NMR (400 MHz, $CDCl_3$, δ): 8.61 (d, *J = 8.0 Hz,* 2H), 8.38 (s, 1H), 7.99 (d, *J = 7.6 Hz,* 2H), 7.94 (d, *J = 8.0 Hz,* 2H), 7.77-7.72 (m, 9H), 7.59-7.40 (m, 13H).
MS (ASAP): 809.44[M+H]$^+$. Calcd for. C56H29FN4O2: 808.23

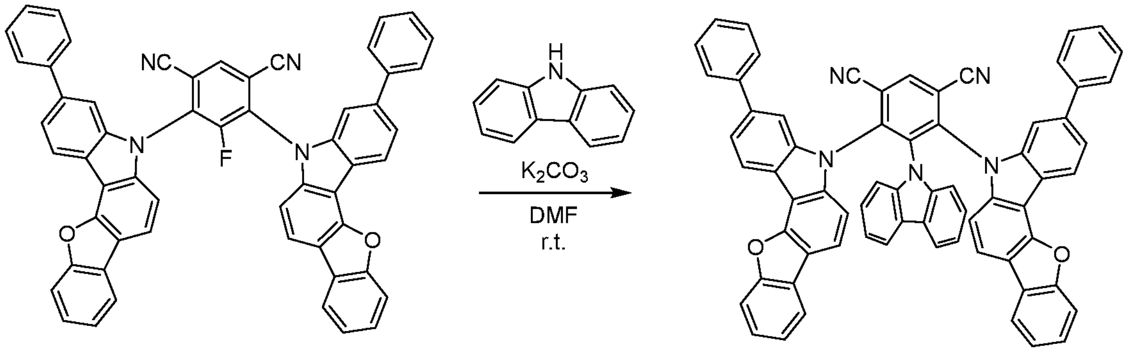


570507

This was synthesized at a yield of 64%, according to the same method of the second reaction in Synthesis Example 8.

$^1$H NMR (400 MHz, $CDCl_3$, $\delta$): 8.62 (s, 0.5H), 8.58 (s, 0.5H), 8.24 (d, *J = 8.0 Hz,* 1H), 8.18 (d, *J = 8.0 Hz,* 1H), 8.02-7.99 (m, 1.5H), 7.90 (d, *J = 6.8 Hz,* 1H), 7.70-7.60 (m, 2H), 7.51-7.26 (m, 13H), 7.21-7.14 (m, 7H), 6.95-6.62 (m, 5.5H), 6.58 (t, *J = 8.0 Hz,* 0.5H), 6.46-6.38 (m, 1.5H).
MS (ASAP): 956.57 $[M+H]^+$. Calcd for. C68H37N5O2: 955.29

(Synthesis Example 20) Synthesis of Compound 723

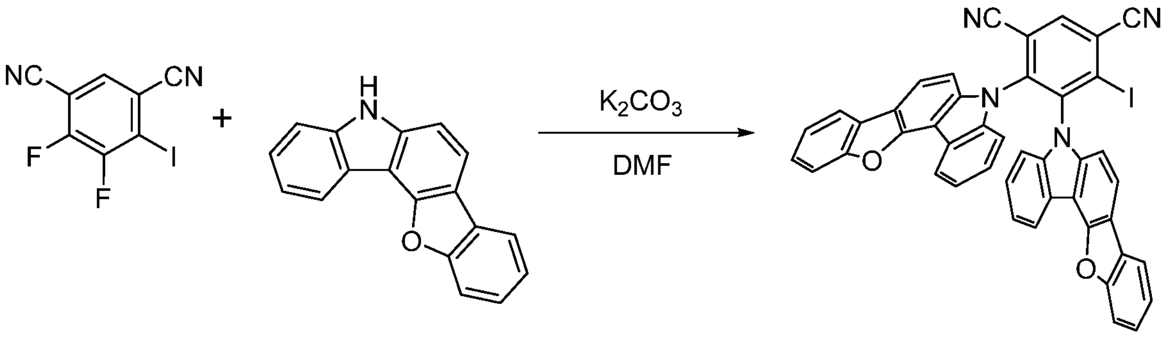


2a

In a nitrogen stream atmosphere, potassium carbonate (1.47 g, 10.35 mmol) and 5H-benzofuro[3,2-c]carbazole (1.95 g, 7.59 mmol) were stirred in dimethylformamide (60 mL) at room temperature for 30 minutes, then 4,5,6-trifluoroisophthalonitrile (0.35 mmol, 1.92 mmol) was added, and reacted at room temperature for 5 hours. Subsequently, the reaction was stopped with water and methanol, and the precipitated yellow solid was filtered out, and the residue was purified by silica gel column chromatography (toluene) and reprecipitation (toluene/methanol) to give a yellow solid, Compound 2a (0.90 g, 1.17 mmol, yield 34%).

$^1$H NMR (400 MHz, $CDCl_3$, $\delta$): 8.31 (s, 1H), 8.13-8.06 (m, 2H), 7.90-7.87 (m, 0.5H), 7.79-7.76 (m, 1.5H), 7.69-7.65 (m, 0.5H), 7.59-7.53 (m, 3.5H), 7.41-7.34 (m, 2H), 7.30-7.25 (m, 2H), 7.23-7.09 (m, 4H), 7.04-6.99 (m, 2), 6.93-6.86 (m, 2H).
MS (ASAP): 765.27 $(M+H^+)$. Calcd for C44H21IN4O2: 764.07.

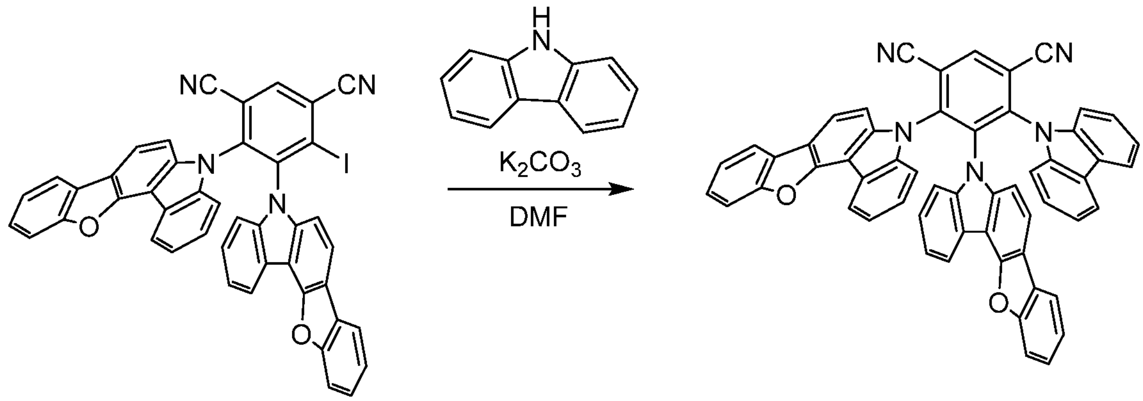


2a

723

In a nitrogen atmosphere, a dimethylformamide solution (20 mL) of Compound 2a (0.75 g, 0.98 mmol), carbazole (0.33 g, 1.96 mmol) and $K_2CO_3$ (0.35 g, 2.45 mmol) was stirred with heating at 120°C for 5 hours. Methanol and water were added to the reaction mixture to stop the reaction. Subsequently, the precipitated substance was filtered out to collect a solid. The resultant solid was purified by column chromatography (toluene/hexane/$CHCl_3$=7/2.5/0.5) and reprecipitation (toluene/hexane) to give a yellow solid, Compound 37027 (0.45 g, 0.56 mmol, 57%).

$^1$H NMR (400 MHz, $CDCl_3$, $\delta$): 8.58 (s, 1H), 8.13 (d, *J = 8.0* Hz, 1H), 7.86-7.78 (m, 2H), 7.74-7.55 (m, 6H), 7.45-7.42 (m, 1H), 7.39-6.97 (m, 14H), 6.93-6.74 (m, 3H), 6,66-6.55 (m, 1H).
MS (ASAP): 804.43 $[M+H]^+$. Calcd for. $C_{56}H_{29}N_5O_2$ : 803.23

(Examples 1 to 3, Comparative Example 1)

Production and Evaluation of Thin Film

On a quartz substrate according to a vacuum evaporation method, Compound 3 and mCBP were evaporated from different evaporation sources under the condition of a vacuum degree of lower than $1 \times 10^{-3}$ Pa to form a thin film having a thickness of 100 nm in which the concentration of Compound 3 was 20% by weight, and this is a doped thin film of Example 1.

Compound 5, Compound 6 and Comparative Compound A were individually used in place of Compound 3 to produce thin films of Example 2, Example 3 and Comparative Example 1.

The resultant thin films were individually irradiated with 300-nm excitation light, and all the thin films gave photoluminescence. The lifetime ($\tau_d$) of delayed fluorescence was derived from the transient decay curve of emission. The results are as shown in the following Table. It is confirmed that the delayed fluorescence lifetime ($\tau_d$) in Examples 1 to 3 is short.

[Table 4]

| | $\tau_d$ ($\mu$s) |
|---|---|
| Example 1 | 1.68 |
| Example 2 | 1.99 |
| Example 3 | 1.89 |
| Comparative Example 1 | 2.99 |

(Example 4)

Production of Organic Electroluminescent Device

On a glass substrate having, as formed thereon, an indium-tin oxide (ITO) anode film having a thickness of 100 nm, thin films were layered according to a vacuum evaporation method under a vacuum degree of $1 \times 10^{-6}$ Pa. First, on ITO, HATCN was formed at a thickness of 10 nm, and then NPD was formed thereon at a thickness of 30 nm.

Next, TrisPCz was formed on it at a thickness of 10 nm, and further thereon, formed Host1 at a thickness of 5 nm. Next, Compound 3 and Host1 were co-evaporated from different evaporation sources to form a light emitting layer at a thickness of 30 nm. At that time, the concentration of Compound 3 was 35% by weight. Further on this, SF3TRZ was formed at a thickness of 10 nm, and further thereon SF3TRZ and Liq were co-evaporated from different evaporation sources to form a layer at a thickness of 30 nm. At that time, SF3TRZ/Liq (by weight) was 7/3. Further, Liq was formed at a thickness of 2 nm, and then aluminum (Al) was evaporated at a thickness of 100 nm to form a cathode. According to the process, an organic electroluminescent device of Example 1 was produced.

(Comparative Example 2)

According to the same process as in Example 1 but using Comparative Compound A in place of Compound 3, an organic electroluminescent device of Comparative Example 2 was produced.

(Evaluation)

Emission from the organic electroluminescent devices of Example 1 and Comparative Example 2 was analyzed to measure x and y in the CIE chromaticity coordinate. Of the organic electroluminescent devices, the time in which the emission intensity at 12.6 mA/cm$^2$ lowered to 95% (LT95) was measured. LT95 of Comparative Example 2 is referred to as 100%, and based on this, a relative value of Example 1 was calculated. The results are as shown in the following Table. The chromaticity of the organic electroluminescent device of Example 1 was good, and the device lifetime (device durability) thereof significantly improved.

[Table 5]

| | CIE | | LT95 (%) |
|---|---|---|---|
| | x | Y | |
| Example 1 | 0.41 | 0.57 | 194 |
| Comparative Example 2 | 0.34 | 0.60 | 100 |

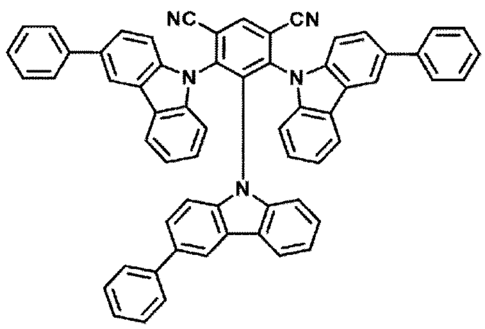

**Comparative Compound A**

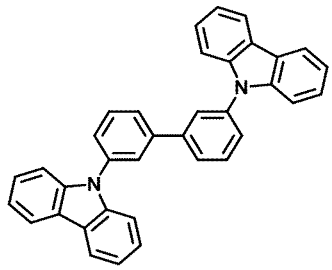

**mCBP**

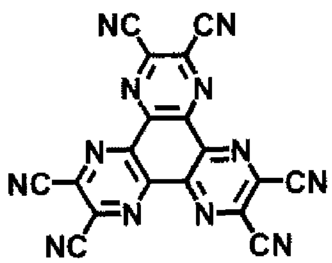

**HATCN**

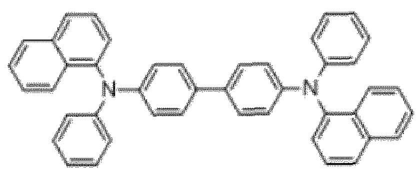

**NPD**

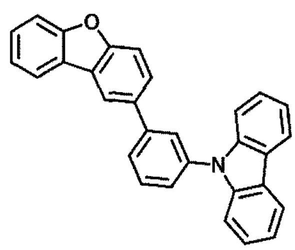

**Host1**

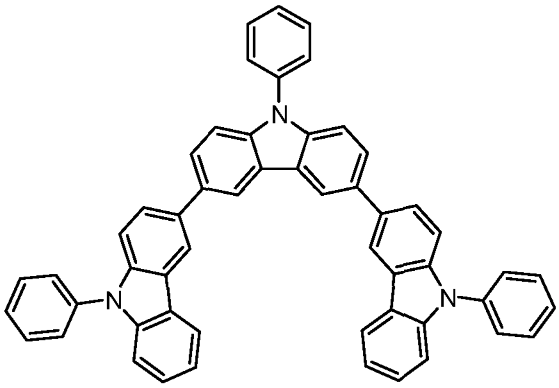

TrisPCz

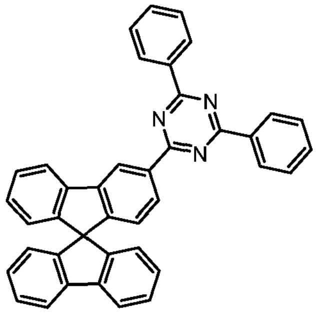

SF3TRZ

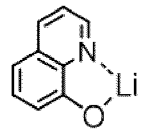

Liq

Reference Signs List

1 Substrate
2 Anode
3 Hole Injection Layer
4 Hole Transporting Layer
5 Light Emitting Layer
6 Electron Transporting Layer
7 Cathode

## Claims

**1.** A compound represented by the following general formula (1):

General Formula (1)

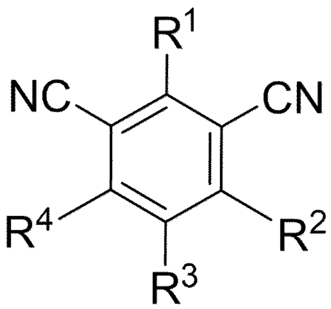

wherein:

any one of $R^1$, $R^2$ and $R^4$ is a hydrogen atom or a deuterium atom,
the remaining two and $R^3$ each independently represent a donor group, of which at least one is a benzofuran-condensed carbazol-9-yl group having a skeleton of a carbazole ring condensed with a benzofuran ring at the 2,3-positions, a benzothiophene-condensed carbazol-9-yl group having a skeleton of a carbazole ring condensed with a benzothiophene ring at the 2,3-positions, an indole-condensed carbazol-9-yl group having a skeleton of a carbazole ring condensed with an indole ring at the 2,3-positions, an indene-condensed carbazol-9-yl group having a skeleton of a carbazole ring condensed with an indene ring at the 2,3-positions, or a silaindene-condensed carbazol-9-yl group having a skeleton of a carbazole ring condensed with a silaindene ring at the 2,3-positions.

**2.** The compound according to claim 1, wherein the remaining two and $R^3$ all contain a carbazole ring.

**3.** The compound according to claim 1 or 2, wherein $R^3$ is the benzofuran-condensed carbazol-9-yl group, the benzothiophene-condensed carbazol-9-yl group, the indole-condensed carbazol-9-yl group, the indene-condensed carbazol-9-yl group, or the silaindene-condensed carbazol-9-yl group.

**4.** The compound according to any one of claims 1 to 3 wherein $R^2$ and $R^4$ each are independently the benzofuran-condensed carbazol-9-yl group, the benzothiophene-condensed carbazol-9-yl group, the indole-condensed carbazol-9-yl group, the indene-condensed carbazol-9-yl group, or the silaindene-condensed carbazol-9-yl group.

**5.** The compound according to any one of claims 1 to 4, wherein $R^2$ and $R^4$ are the same.

**6.** The compound according to any one of claims 1 to 5, wherein at least one of the remaining two and $R^3$ is the benzofuran-condensed carbazol-9-yl group or the benzothiophene-condensed carbazol-9-yl group.

**7.** The compound according to any one of claims 1 to 5, wherein the remaining two and $R^3$ each are independently the benzofuran-condensed carbazol-9-yl group or the benzothiophene-condensed carbazol-9-yl group.

**8.** The compound according to any one of claims 1 to 7, wherein $R^1$ is a hydrogen atom or a deuterium atom.

**9.** The compound according to any one of claims 1 to 8, wherein the remaining two and $R^3$ are the same.

**10.** The compound according to any one of claims 1 to 9, wherein at least one of the remaining two and $R^3$ is a benzofuran-condensed carbazol-9-yl group having a skeleton of a carbazole ring condensed with two benzofuran rings at the 2,3-positions, a benzothiophene-condensed carbazol-9-yl group having a skeleton of a carbazole ring condensed with two benzothiophene rings at the 2,3-positions, an indole-condensed carbazol-9-yl group having a skeleton of a carbazole ring condensed with two indole rings at the 2,3-positions, an indene-condensed carbazol-9-yl group having a skeleton of a carbazole ring condensed with two indene rings at the 2,3-positions, or a silaindene-condensed carbazol-9-yl group having a skeleton of a carbazole ring condensed with two silaindene rings at the 2,3-positions.

**11.** The compound according to any one of claims 1 to 10, having a symmetric structure.

**12.** The compound according to any one of claims 1 to 11, composed of atoms selected from the group consisting of a carbon atom, a hydrogen atom, a deuterium atom, a nitrogen atom, an oxgen atom and a sulfur atom.

**13.** The compound according to any one of claims 1 to 12, wherein the benzofuran-condensed carbazol-9-yl group has a structure of any of the following:

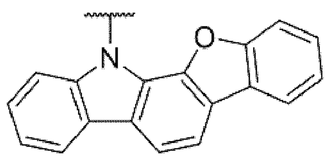
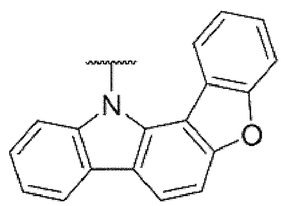
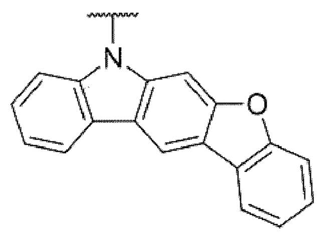

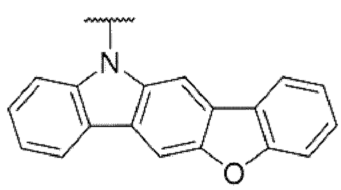
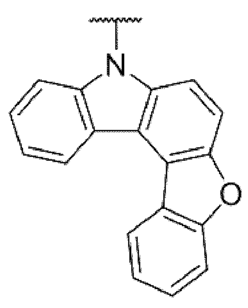
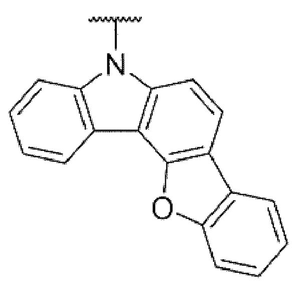

wherein the hydrogen atom can be substituted, but is not further condensed.

**14.** The compound according to any one of claims 1 to 13, wherein the benzothiophene-condensed carbazol-9-yl group has a structure of any of the following:

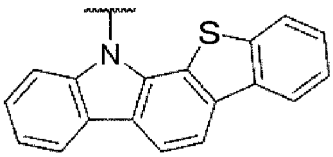
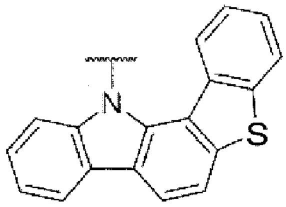
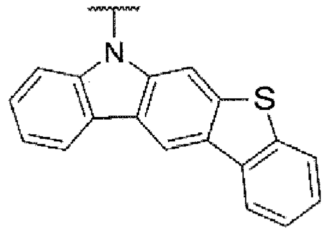
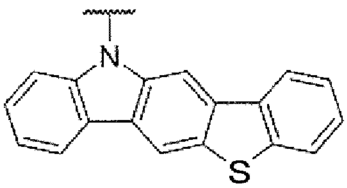
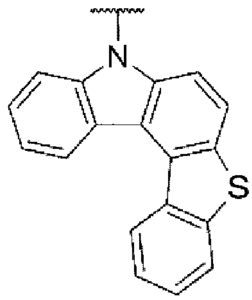
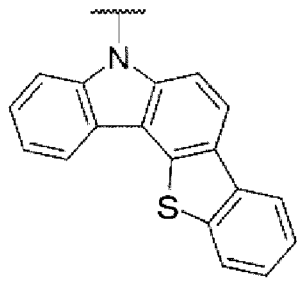

wherein the hydrogen atom can be substituted, but is not further condensed.

**15.** Alight emitting material of a compound of any one of claims 1 to 14.

**16.** Alight emitting device containing a compound of any one of claims 1 to 14.

**17.** The light emitting device according to claim 16, wherein the light emitting device has a light emitting layer and the light emitting layer contains the above compound and a host material.

**18.** The light emitting device according to claim 16, wherein the light emitting device has a light emitting layer, the light emitting layer contains the above compound and a light emitting material, and the light emitting material mainly emits light.

Fig. 1

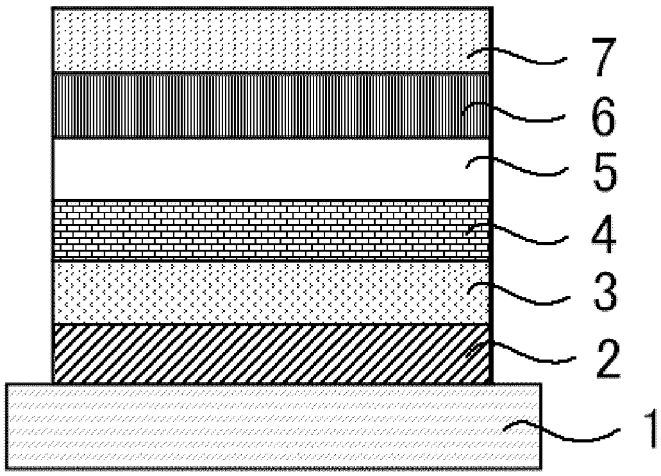

**INTERNATIONAL SEARCH REPORT**

International application No.
**PCT/JP2021/028342**

**A. CLASSIFICATION OF SUBJECT MATTER**

***C07D 519/00***(2006.01)i; ***C09K 11/06***(2006.01)i; ***H01L 51/50***(2006.01)i
FI: C07D519/00 301; C09K11/06 655; H05B33/14 B; C09K11/06 645; C07D519/00 CSP

According to International Patent Classification (IPC) or to both national classification and IPC

**B. FIELDS SEARCHED**

Minimum documentation searched (classification system followed by classification symbols)

C09K11/06; C07D519/00; H01L51/50

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X<br>A | WO 2019/190235 A1 (LG CHEM, LTD.) 03 October 2019 (2019-10-03)<br>pages 44, 49-54 | 1-8, 10-18<br>9 |
| X | CN 108264478 A (KUNSHAN GOVISIONOX OPTOELECTRONICS CO., LTD.) 10 July 2018 (2018-07-10)<br>pages 2, 4 | 1-18 |
| A | KR 10-2019-0062250 A (LG CHEM, LTD.) 05 June 2019 (2019-06-05)<br>claims | 1-18 |
| A | WO 2018/237389 A1 (KYULUX INC.) 27 December 2018 (2018-12-27)<br>claims | 1-18 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 September 2021** | **28 September 2021** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2021/028342**

| Patent document cited in search report | Publication date (day/month/year) | Patent family member(s) | Publication date (day/month/year) |
|---|---|---|---|
| WO 2019/190235 A1 | 03 October 2019 | CN 111051283 A<br>claims<br>KR 10-2019-0113672 A | |
| CN 108264478 A | 10 July 2018 | (Family: none) | |
| KR 10-2019-0062250 A | 05 June 2019 | WO 2019/107934 A1<br>claims<br>CN 110799496 A | |
| WO 2018/237389 A1 | 27 December 2018 | US 2020/0115364 A1<br>claims<br>US 2020/0115376 A1<br>WO 2018/237385 A1<br>WO 2018/237393 A1<br>KR 10-2020-0019694 A<br>CN 110914378 A<br>KR 10-2020-0023371 A<br>CN 110997866 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015022974 A
- WO 2013154064 A
- WO 2013011954 A
- WO 2013011955 A
- WO 2013081088 A
- JP 2013256490 A
- JP 2013116975 A
- WO 2013133359 A
- WO 2013161437 A
- JP 2014009352 A
- JP 2014009224 A
- JP 2017119663 A
- JP 2017119664 A
- JP 2017222623 A
- JP 2017226838 A
- JP 2018100411 A
- WO 2018047853 A
- JP 2013253121 A
- WO 2014034535 A
- WO 2014115743 A
- WO 2014122895 A
- WO 2014126200 A
- WO 2014136758 A
- WO 2014133121 A
- WO 2014136860 A
- WO 2014196585 A
- WO 2014189122 A
- WO 2014168101 A
- WO 2015008580 A
- WO 2014203840 A
- WO 2015002213 A
- WO 2015016200 A
- WO 2015019725 A
- WO 2015072470 A
- WO 2015108049 A
- WO 2015080182 A
- WO 2015072537 A
- WO 2015080183 A
- JP 2015129240 A
- WO 2015129714 A
- WO 2015129715 A
- WO 2015133501 A
- WO 2015136880 A
- WO 2015137244 A
- WO 2015137202 A
- WO 2015137136 A
- WO 2015146541 A
- WO 2015159541 A


### Non-patent literature cited in the description


- *Organic Electronics,* 2013, vol. 14, 2721-2726
- **HANSCH, C.** *Chem. Rev.,* 1991, vol. 91, 165-195